# DEMANDE DE BREVET EUROPEEN

(11) **EP 2 081 028 A1**
(43) Date de publication de la demande: **22.07.2009**
(21) Numéro de dépôt: 08172758.8
(22) Date de dépôt: 17.07.2000
(51) Int. Cl.: G01N 33/68, G01N 33/564, C07K 14/47, A61K 38/17

(54) **Utilisation d'un polypeptide pour détecter, prévenir ou traiter un état pathologique associé à une maladie dégénérative, neurologique autoimmune**

(30) Priorité: 15.07.1999 FR 9909372
(62) Demande divisionnaire de: 00953247.4
(71) Demandeur: bioMérieux S.A., 69280 Marcy l'Etoile (FR)
(72) Inventeur: Roecklin, Dominique, 67500 Niederschaeffolsheim (FR); Kolbe, Bruno, 67204 Achenheim (FR); Charles, Marie-Hélène, 69420 Condrieu (FR); Malcus, Carine, 69530 Brignais (FR); Santoro, Lyse, 69269 Charbonnieres les Bains (FR); Perron, Hervé, 69290 Saint Genis Les Ollieres (FR)
(74) Mandataire: Agasse, Stéphane

(57) **Abrégé**

Utilisation d'au moins un polypeptide comprenant au moins un fragment d'une protéine pour obtenir une composition diagnostique, pronostique, prophylactique ou thérapeutique destinée à détecter, prévenir ou traiter un état pathologique associé à une maladie dégénérative et/ou neurologique et/ou auto-immune, ladite protéine étant choisie parmi les protéines dont la séquence peptidique à l'état natif correspond à SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19, SEQ ID N° 20, SEQ ID N° 21, SEQ ID N° 22, SEQ ID N° 23, SEQ ID N° 24, SEQ ID N° 25, SEQ ID N° 26, SEQ ID N° 27, SEQ ID N° 28 et SEQ ID N° 29, et les séquences peptidiques qui présentent au moins 70 % d'identité, de préférence au moins 80 % d'identité et avantageusement au moins 98 % d'identité avec l'une quelconque des séquences peptidiques SEQ ID N° 1 à SEQ ID N° 8 et SEQ ID N° 10 à SEQ ID N° 29, et les séquences peptidiques ou les fragments desdites séquences appartenant à une même famille de protéines choisies parmi le pcrlccan, le précurseur de la protéine plasmatique de liaison au rétinol, de la protéine activatrice du ganglioside GM2, de la calgranuline B et de la saposine B.

## Description

La présente invention concerne notamment l'utilisation d'au moins un polypeptide, pour obtenir une composition diagnostique, pronostique, prophylactique ou thérapeutique destinée à détecter, prévenir ou traiter un état pathologique associé à une maladie dégénérative et/ou auto-immune et/ou neurologique.

Selon l'invention, on entend par maladie dégénérative, une maladie dans laquelle un processus de mort cellulaire ou de destruction cellulaire est associé à des troubles physiologiques et/ou cliniques. La maladie d'Alzheimer, la sclérose latérale amyotrophique, la maladie de Parkinson sont classées parmi les maladies neurodégénératives. On entend par maladie auto-immune, une hyperréactivité du système immunitaire vis à vis d'un ou de plusieurs auto-antigène(s). La sclérose en plaques (SEP), la polyarthrite rhumatoïde (PR) et le lupus érythémateux sont classés dans les maladies auto-immunes.

La sclérose en plaques est une maladie chronique du système nerveux central de l'homme, évoluant par succession de phases de rémission et de poussée ou selon une progression régulière, dont la caractéristique anatomopathologique consiste en la formation de zones de démyélinisation bien délimitées dans la substance blanche du cerveau et de la moelle épinière.

Au niveau histologique, ces zones présentent au stade précoce du processus lésionnel, une dégradation de la myéline péri-axonale associée à une atteinte des cellules gliales responsable de cette démyélinisation. Une activation macrophagique inflammatoire impliquant les cellules microgliales (macrophages tissulaires résidants du système nerveux central), ainsi que, probablement, des macrophages provenant de monocytes sanguins infiltrés, est associée à ce processus de démyélinisation et contribue à la destruction des feuillets myélinisés. Au centre de la zone démyélinisée, une déplétion relative en cellules gliales est retrouvée alors qu'une prolifération d'astrocytes se développe à la périphérie et peut envahir la plaque démyélinisée pour générer une plaque fibreuse ou gliotique. Ces structures sclérotiques sont à l'origine du nom donné à la maladie.

Une autre caractéristique de ces plaques est leur association quasi systématique avec un élément vasculaire autour duquel elles se développent.

Au niveau histologique, on observe une altération fréquente de la barrière hémato-encéphalique (BHE) constituée par l'endothélium capillaire. Un des éléments déterminants dans le maintien de la BHE est constitué par la présence sous-jacente d'extensions cytoplasmiques des astrocytes, appelées pieds astrocytaires. Vraisemblablement, les pieds astrocytaires induisent la formation ou permettent le maintien de structures de jonction étanches qui assurent la cohésion de la barrière endothéliale capillaire concrétisant la BHE. Or, différents modèles pathologiques font état de l'altération de la BHE et d'une déplétion des pieds astrocytaires.

Par ailleurs, dans le processus lésionnel de la SEP, l'altération de la BHE contribue à amplifier la réponse inflammatoire associée, par l'afflux de cellules lymphoïdes provenant de la circulation sanguine. La contribution de l'inflammation associée aux cellules immunitaires est importante dans la SEP et participe au processus lésionnel.

L'étiologie de la SEP est source d'un débat d'actualité car la maladie pourrait avoir des origines diverses. Des hypothèses ont été émises sur une origine bactérienne et/ou virale. Par ailleurs, comme décrit dans la demande de brevet WO 95/21859, H. Perron et al. ont été conduits à rechercher un ou des agents effecteurs du processus pathogénique aboutissant à la formation typique de plaques de démyélinisation et à une gliose astrocytaire. Dans le cadre de cette étude, ils ont mis en évidence la présence dans le liquide céphalo-rachidien (LCR) et le sérum de patients SEP d'au moins un facteur qui présente une activité toxique vis à vis des cellules astrocytaires et oligodendrocytaires humaines ou animales. Cette activité toxique se caractérise par une désorganisation cytomorphologique du réseau de filaments intermédiaires et/ou une dégradation des protéines desdits filaments et/ou une mort cellulaires par apoptose des cellules gliales. Ils ont établi une corrélation significative entre la détection *in vitro* de cette activité toxique dans des échantillons de LCR et de sérum de patients SEP et la sclérose en plaques par un dosage colorimétrique quantitatif au bromure de méthyltétrazolium (MTT) des cellules vivantes, comme décrit dans la demande de brevet WO 95/21859. Par ailleurs, C. Malcus-Vocanson *et al*. ont montré que l'urine est un fluide biologique très favorable pour la détection de l'activité de ce facteur toxique et développé un procédé utilisant la cytométrie de flux pour détecter et/ou quantifier les cellules gliales adhérentes mortes par apoptose. Toutes les informations concernant ce procédé sont décrites dans la demande de brevet WO 98/11439.

Des essais ont été réalisés à partir d'une fraction protéique de LCR et d'urine de patients SEP pour tenter d'identifier ce facteur toxique. Le contenu protéique de chaque fraction a été séparé sur gel SDS-PAGE 12 % et observé après coloration du gel à l'argent. Parmi les protéines observées, une fraction protéique centrée sur un poids moléculaire apparent d'environ 21 kD a été trouvée minoritairement associée à l'activité toxique détectée *in vitro* et une fraction centrée sur un poids moléculaire apparent d'environ 17 kD a été trouvée majoritairement associée à cette activité toxique.

Une injection de la fraction provenant de LCR de patients SEP dans le cerveau de rat Lewis et une observation histologique post-mortem de coupes de cerveau des rats a permis d'observer, trois mois après l'injection, une apoptose de la population astrocytaire et la formation de plaques de démyélinisation. Toutes les informations sont contenues dans la demande de brevet WO 97/33466. Ces observations sont conformes à celles qui ont pu être faites sur des coupes de cerveau de patients atteints de SEP, après biopsie (N. Benjelloun et al. Cell. Mol. Biol., 1998, 44 (4), 579-583).

Les présents inventeurs ont maintenant identifié et analysé les protéines associées à cette activité toxique vis à vis des cellules gliales dans des échantillons biologiques de patients SEP, en particulier dans l'urine, le liquide céphalo-rachidien et le sérum.

Après purification des protéines et séparation sur gel SDS-TRICINE, les inventeurs ont mis en évidence la présence de quatre bandes d'intérêt de différents poids moléculaires apparents, respectivement de 8, 14, 18 et 20 kD correspondant à au moins cinq familles de protéines différentes. Les protéines de ces familles ont ensuite été analysées par spectrométrie de masse et/ou séquençage et recherche d'homologie dans les banques de données (NCBI http :/ww.ncbi.nlm.nih.gov, Basic Blast Search, Protein Blastp, les séquences protéiques sont entrées en format FASTA dans la base de données nr, l'algorithme utilisé est Matrix BLOSUM62, l'identité dénommée "Identities" correspond au nombre d'acides aminés identiques donné en pourcentage et la positivité " Positives "correspond aux acides aminés présentant une équivalence biologique selon les paramètres susmentionnés du logiciel donnés en pourcentage). Ces protéines appartiennent aux familles des protéines du Perlecan, du précurseur de la protéine plasmatique de liaison au rétinol, de la protéine activatrice du ganglioside GM2, de la calgranuline et de la saposine B. Plus précisément, les protéines sont (i) pour la bande de 20 kD le fragment C-terminal du Perlecan qui commence à l'acide aminé 3464 et se termine à l'acide aminé 3707 (Murdoch AD et al. J Biol Chem, 1992, April 25 ;267 (12) :8544-47), et référencé dans l'identificateur de séquences SEQ ID N° 2 (la protéine entière Perlecan étant référencée en SEQ ID N°1), (ii) pour la bande de 20 kD le précurseur de la protéine plasmatique de liaison au rétinol (Monaco HL et al., Science, 1995, 268 (5213) :1039-1041) dont la séquence est donnée en SEQ ID N° 4, (iii) pour la bande de 18 kD la protéine activatrice du ganglioside GM2 (Furst W et al., Euro J Biochem, 1990, Sep 24 ; 193(3) :709-14) identifié en SEQ ID N° 8, (iv) pour la bande de 14 kD la calgranuline B (Lagasse E et al., Mol Cell Biol, 1988, Jun ;8(6) :2402-10) identifiée en SEQ ID N° 17 et (v) pour la bande de 8 kD la saposine B (Kleinschmidt T et al., Biol Chem Hoppe Seyler, 1988, Dec ;369(12) :1361-5) représentée en SEQ ID N° 24. Ils ont par ailleurs mis également en évidence la présence de séquences variantes auxdites séquences de référence, en particulier pour la bande de 18 kD une séquence variante de la protéine activatrice du ganglioside GM2 référencée SEQ ID N° 9. Ces séquences protéiques variantes sont le produit de mutations au niveau des gènes codant pour lesdites protéines ou sont le résultat de phénomènes d'épissage. Il est à noter par exemple que la calprotectine est un variant de la calgranuline B.

Le fragment C-terminal de la protéine Perlecan (SEQ ID N° 2) est codé par exemple par la séquence nucléotidique ADN SEQ ID N° 69, en tenant compte du code génétique. La protéine précurseur de la protéine plasmatique de liaison au rétinol (SEQ ID N° 4) est codée par exemple par la séquence nucléotidique ADN SEQ ID N° 70, en tenant compte du code génétique. La protéine activatrice du GM2 (SEQ ID N° 8) est codée par exemple par la séquence nucléotidique ADN SEQ ID N° 31, en tenant compte du code génétique. Les peptides FSWDNCFEGK DPAVIR et YSLPKSEFAV PDLELP issus du polypeptide muté activateur du GM2 (SEQ ID N°9) sont codés par les séquences nucléotidiques ADN SEQ ID N° 66 et SEQ ID N° 67 respectivement, en tenant compte du code génétique. La protéine calgranuline B (SEQ ID N° 17) est codée par exemple par la séquence nucléotidique ADN SEQ ID N° 42, en tenant compte du code génétique. La protéine saposine B (SEQ ID N° 24) est codée par exemple par la séquence nucléotidique ADN SEQ ID N° 53, en tenant compte du code génétique.

Par famille de protéines on entend l'ensemble des protéines codées à partir d'un même gène d'ADN et qui résultent d'un multi-épissage différentiel du gène et/ou d'un cadre de lecture différent. Le gène ADN est transcrit avec des phénomènes d'épissage alternatif ce qui conduit à la traduction de différentes séquences primaires de protéines. Toutes ces protéines appartiennent à une même famille protéique. On inclut également dans le terme " famille protéique ", les protéines qui présentent au moins 70% d'identité, de préférence au moins 80 % d'identité et avantageusement au moins 98 % d'identité avec une séquence protéique de référence de la famille.

On entend par multi-épissage, un épissage intervenant au moins une fois dans la région nucléotidique d'intérêt.

Par exemple, par famille de protéine précurseur de la protéine plasmatique de liaison au rétinol, on désigne la famille de protéines comprenant *au moins* les protéines ou fragment de protéines de séquence SEQ ID N° 4, SEQ IDN° 5, SEQ ID N° 6, SEQ ID N° 7, et les protéines codées par le gène correspondant selon différents cadres de lecture.

Par exemple, par famille de protéine activatrice du GM2, on désigne la famille de protéines comprenant *au moins* les protéines ou fragments de protéines de séquence SEQ ID N° 8, SEQ ID N° 9, SEQ ID N° 10, SEQ ID N° 11, SEQ ID N° 12, SEQ ID N° 13, SEQ ID N° 14, SEQ ID N° 15, SEQ ID N° 16, et les protéines codées par le gène correspondant selon différents cadres de lecture, qui résultent d'un multi-épissage différentiel du gène et/ou d'un cadre de lecture différent.

Par exemple, par famille de protéine calgranuline B, on désigne la famille de protéines comprenant *au moins* les protéines ou fragments de protéines de séquence SEQ ID N° 17, SEQ ID N° 18, SEQ ID N° 19, SEQ ID N° 20, SEQ ID N° 21, SEQ ID N° 22, SEQ ID N° 23, et les protéines codées par le gène correspondant selon différents cadres de lecture, qui résultent d'un multi-épissage différentiel du gène et/ou d'un cadre de lecture différent. Les protéines MRP14 (SEQ ID N° 17) et MRP8 (SEQ ID N° 18) ont une séquence protéique différente tout en étant codées par un même gène ; elles appartiennent à la même famille protéique.

Par exemple, par famille de protéine saposine B, on désigne la famille de protéines comprenant *au moins* les protéines ou fragments de protéines de séquence SEQ ID N° 24, SEQ ID N° 25, SEQ ID N° 26, SEQ ID N° 27, SEQ ID N° 28, SEQ IDN° 29, et les protéines codées par le gène correspondant selon différents cadres de lecture, qui résultent d'un multi-épissage différentiel du gène et/ou d'un cadre de lecture différent.

Par famille d'acides nucléiques codant pour une protéine on entend l'ensemble des séquences nucléiques ADNc et/ou ARN transcrits à partir d'un même gène ADN et, qui résultent d'un multi-épissage différentiel. Le gène ADN est transcrit avec des phénomènes d'épissage différentiels et conduit à la synthèse de différents acides nucléiques (ADNc, ARN) de séquences différentes. Toutes ces séquences ADNc et ARNm sont considérées comme appartenant à une même famille d'acides nucléiques.

Par exemple, par famille d'acides nucléiques codant pour la famille de protéine précurseur de la protéine plasmatique de liaison au rétinol, on désigne la famille d'acides nucléiques comprenant *au moins* les acides nucléiques ou fragments de séquence SEQ ID N°30.

Par exemple, par famille d'acides nucléiques codant pour la famille de protéine activatrice du GM2, on désigne la famille d'acides nucléiques comprenant *au moins* les acides nucléiques ou fragments de séquences SEQ ID N° 31, SEQ ID N° 32, SEQ ID N° 33, SEQ ID N° 34, SEQ ID N° 35, SEQ ID N° 36, SEQ ID N° 37, SEQ ID N° 38, SEQ ID N° 39, SEQ ID N° 40, SEQ ID N° 41 qui résultent d'un multi-épissage différentiel du gène et/ou d'un cadre de lecture différent.

Par exemple, par famille d'acides nucléiques codant pour la famille de protéine calgranuline B, on désigne la famille d'acides nucléiques comprenant *au moins* les acides nucléiques ou fragments de séquences SEQ ID N° 42, SEQ ID N° 43, SEQ ID N° 44, SEQ ID N° 45, SEQ ID N° 46, SEQ ID N° 47, SEQ ID N° 48, SEQ ID N° 49, SEQ ID N° 50, SEQ ID N° 51, SEQ ID N° 52 qui résultent d'un multi-épissage différentiel du gène et/ou d'un cadre de lecture différent.

Par exemple, par famille d'acides nucléiques codant pour la famille de protéine saposine B, on désigne la famille d'acides nucléiques comprenant *au moins* les acides nucléiques ou fragment de séquences SEQ ID N° 53, SEQ ID N° 54, SEQ ID N° 55 qui résultent d'un multi-épissage différentiel du gène et/ou d'un cadre de lecture différent.

Par « épissage » on entend un mécanisme d'excision des introns et de raboutage des exons au cours de la maturation des transcrits et par " épissage différentiel " on entend l'existence de plusieurs schémas d'épissage d'un transcrit primaire aboutissant à la formation de différents ARN messagers et, pouvant donner lieu à la synthèse de plusieurs protéines différentes (Kaplan et Delpech, Biologie Moléculaire et Médecine, 1993, 2ème édition, Médecine et Sciences, Flammarion, pages 73-77). Ce phénomène est largement décrit dans la littérature scientifique. A titre d'exemple, on peut citer le modèle des gènes qui codent pour les chaînes lourdes et légères des immunoglobulines, le modèle du gène de la dystrophine, le modèle du gène de l'alpha amylase, le gène de la myéline, etc...

II est connu que les gènes eucaryotes, notamment, comprennent des régions (exons) qui codent pour des fragments de la protéine codée par ledit gène et d'autres régions (introns) qui n'ont pas d'équivalent protéique. Ceci est dû au fait que les gènes sont d'abord transcrits en un ARN " primaire " qui est ensuite coupé par des enzymes d'épissage au niveau de sites nucléotidiques spécifiques (sites d'épissage). Ces enzymes raboutent ensuite les régions codant pour la protéine, reconstituant ainsi un ARN " secondaire " dont les régions introniques ont été éliminées. Par ailleurs, selon les phénotypes cellulaires (et donc les tissus ou la différenciation) ces enzymes ne sont pas toutes exprimées et, ainsi, un même ARN peut être épissé différemment dans les cellules d'un même individu, générant ainsi des protéines avec des différences de séquence. Cependant, ces phénomènes peuvent aussi s'appliquer à des régions nucléotidiques qui sont entièrement codantes (exons), mais qui, selon différents épissages possibles vont générer plusieurs protéines différentes à partir de la même région nucléotidique, par phénomène d'épissage différentiel entre les différents produits protéiques.

De plus, il est connu que des régions nucléotidiques peuvent avoir plusieurs cadres de lecture selon les trois trames potentielles du code génétique. Ainsi, la présence de plusieurs codons initiateurs de traduction dans plusieurs phases de lecture et/ou un épissage d'ARN primaire raboutant des séquences nucléotiques présentes dans des phases de lectures différentes sur l'ADN, permet à une même région ADN de générer des produits protéiques sans rapports entre eux, du point de vue de la séquence peptidique.

Enfin, le polymorphisme génétique existant entre les individus d'une même espèce et/ou des mutations individuelles peuvent créer ou supprimer des sites d'épissage dans une région ADN donnée et, ainsi, modifier la séquence et la structure du ou des produits protéiques normalement produits par cette région.

Ainsi, la combinaison de ces différents phénomènes peut permettre qu'une même séquence nucléotidique correspondant à un segment d'ADN, identifiée comme déterminant une région génétique d'intérêt dans une étude donnée, comprenne l'information nécessaire et suffisante pour définir toute une famille d'ARN épissés selon des schémas différentiels et alternatifs, dans des cadres de lecture divers et, par là évidemment, de protéines et de polypeptides ayant des séquences " mosaïques " selon un cadre de lecture voire selon les trois cadres potentiels et des mutations éventuellement liées au polymorphisme génétique.

Un exemple de ce phénomène peut être représenté par la région nucléotidique du gène *env* du rétrovirus HIV-1. En effet, plusieurs protéines différentes sont codées par des segments de la même séquence : par exemple, la glycoprotéine d'enveloppe, et les protéines régulatrices TAT, REV, NEF, VIF.

II est encore connu que des protéines peuvent résulter de I'assemblage de sous-unités identiques (homodimères, homomultimères) ou différentes (hétérodimères, hétéromultimères). Ainsi, les différents produits protéiques codés par une même région ADN peuvent aussi s'assembler entre eux pour constituer des entités protéiques complexes multimériques. Ce phénomène s'ajoute aux précédents et, lorsqu'une protéine est identifiée par un fragment peptidique, on peut logiquement identifier tous les autres éléments constitutifs de cette protéine complexe et les segments ADN et ARN épissé qui les codent, ainsi que tous les membres de la famille de produits protéiques et leurs assemblages.

Un autre exemple est fourni par la région d'ADN humain codant pour la famille de protéines MRP14 ou calgranuline B, MRP8, calprotectine, psoriasine etc...

Aussi, la présente invention a pour objet l'utilisation d'au moins un polypeptide comprenant au moins un fragment d'une protéine pour obtenir une composition diagnostique, pronostique, prophylactique ou thérapeutique destinée à détecter, pronostiquer, prévenir ou traiter un état pathologique associé à une maladie dégénérative et/ou auto-immune, ladite protéine étant choisie parmi les protéines dont la séquence peptidique à l'état natif correspond à SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19, SEQ ID N° 20, SEQ ID N° 21, SEQ ID N° 22, SEQ ID N° 23, SEQ ID N° 24, SEQ ID N° 25, SEQ ID N° 26, SEQ ID N° 27, SEQ ID N° 28 et SEQ ID N° 29 et les séquences peptidiques qui présentent au moins 70 % d'identité, de préférence au moins 80 % d'identité et avantageusement au moins 98 % d'identité avec l'une quelconque des séquences peptidiques précitées, et les séquences peptidiques ou les fragments desdites séquences appartenant à une même famille de protéines choisies parmi le perlecan, le précurseur de la protéine plasmatique de liaison au rétinol, que de la protéine activatrice du ganglioside GM2, de la calgranuline B et de la saposine B. Dans des modes de réalisation particuliers au moins deux polypeptides précités sont utilisés en combinaison pour obtenir une composition diagnostique, pronostique, prophylactique ou thérapeutique destinée à détecter, pronostiquer, prévenir ou traiter un état pathologique associé à une maladie dégénérative et/ou auto-immune.

L'invention concerne également l'utilisation d'au moins un polypeptide comprenant au moins un fragment d'une protéine pour obtenir une composition diagnostique, pronostique, prophylactique ou thérapeutique destinée à détecter, pronostiquer, prévenir ou traiter un état pathologique associé à une maladie dégénérative et/ou auto-immune, ladite protéine étant choisie parmi les protéines dont la séquence peptidique à l'état natif correspond à SEQ ID N° 2 , SEQ ID N° 4, SEQ ID N° 8, SEQ ID N° 17 et SEQ ID N° 24 et les séquences peptidiques qui présentent au moins 70 % d'identité, de préférence au moins 80 % d'identité et avantageusement au moins 98 % d'identité avec l'une quelconque des séquences peptidiques précitées. Avantageusement les cinq polypeptides qui répondent à la définition précédente sont utilisés en combinaison.

De préférence, la séquence peptidique dudit polypeptide comprend, ou consiste en une séquence choisie parmi l'une quelconque des SEQ ID N° 2, SEQ ID N° 4, SEQ ID N° 8, SEQ ID N° 17 et SEQ ID N° 24.

L'invention concerne encore l'utilisation d'au moins un fragment d'un des polypeptiques précités pour la préparation d'un peptide immunogène, ledit peptide comprenant tout ou partie d'au moins une des séquences référencées SEQ ID N°s 58 à 65 et étant utilisé pour la production d'anticorps monoclonaux.

L'invention a également pour objet, l'utilisation d'au moins un fragment nucléotidique, pour obtenir une composition diagnostique, pronostique, prophylactique ou thérapeutique destinée à détecter, pronostiquer, prévenir ou traiter un état pathologique associé à une maladie dégénérative et/ou auto-immune, selon laquelle ledit fragment nucléotidique est choisi parmi des fragments qui codent pour au moins un fragment d'une protéine, ladite protéine étant choisie parmi les protéines dont la séquence peptidique à l'état natif correspond à SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19, SEQ ID N° 20, SEQ ID N° 21, SEQ ID N° 22, SEQ ID N° 23, SEQ ID N° 24, SEQ ID N° 25, SEQ ID N° 26, SEQ ID N° 27, SEQ ID N° 28 ET SEQ ID N° 29 et les séquences peptidiques qui présentent au moins 70 % d'identité, de préférence au moins 80 % et avantageusement au moins 98 % d'identité avec l'une quelconque des séquences peptidiques ci dessus, et les fragments complémentaires desdits fragments, et les séquences peptidiques ou les fragments desdites séquences appartenant à une même famille de protéines choisies parmi le perlecan, le précurseur de la protéine plasmatique de liaison au rétinol, la protéine activatrice du ganglioside GM2, de la calgranuline B et de la saposine B. Il est à la portée de l'homme du métier de déterminer les séquences nucléiques des fragments nucléotidiques à partir des séquences peptidiques et du code génétique, ceci faisant partie de ses connaissances générales.

De préférence, ledit fragment nucléotidique code pour une protéine qui à l'état natif consiste en une séquence choisie parmi l'une quelconque des SEQ ID N°s 1 à 8 et SEQ ID N°s 10 à 29 précitées, et parmi les séquences peptidiques ou les fragments desdites séquences appartenant à une même famille de protéines choisies parmi le perlecan, le précurseur de la protéine plasmatique de liaison au rétinol, la protéine activatrice du ganglioside GM2, de la calgranuline B et de la saposine B.

Un autre objet de l'invention est l'utilisation d'au moins un fragment nucléotidique pour obtenir une composition diagnostique, pronostique, prophylactique ou thérapeutique destinée à détecter, pronostiquer, prévenir ou traiter un état pathologique associé à une maladie dégénérative et/ou neurologique et/ou auto-immune selon laquelle ledit fragment est un fragment d'une séquence nucléique choisie parmi l'une quelconque des SEQ ID N° 30, SEQ ID N° 31, SEQ ID N° 32, SEQ ID N° 33, SEQ ID N° 34, SEQ ID N° 35, SEQ ID N° 36, SEQ ID N° 37, SEQ ID N° 38, SEQ ID N° 39, SEQ ID N° 40, SEQ ID N° 41, SEQ ID N° 42, SEQ ID N° 43, SEQ ID N° 44, SEQ ID N° 45, SEQ ID N° 46 et SEQ ID N° 47, SEQ ID N° 48, SEQ ID N° 49 et SEQ ID N° 50, SEQ ID N° 51, SEQ ID N° 52, SEQ ID N° 53, SEQ ID N° 54, SEQ ID N° 55, SEQ ID N° 56, SEQ ID N° 57, SEQ ID N° 67, SEQ ID N° 66, SEQ ID N° 69, SEQ ID N° 70 et SEQ ID N° 71 et leurs séquences complémentaires.

L'invention concerne également l'utilisation d'un ligand spécifique d'un polypeptide ou d'un fragment nucléotidique tel que défini ci dessus pour obtenir une composition diagnostique, pronostique, prophylactique ou thérapeutique destinée à détecter, pronostiquer, prévenir ou traiter un état pathologique associé à une maladie dégénérative et/ou auto-immune.

Par ligand, on entend toute molécule susceptible de s'associer au polypeptide, tel que un anticorps monoclonal, un anticorps polyclonal, un récepteur, un substrat d'activité enzymatique, une enzyme dont ledit polypeptide est un cofacteur. La production d'anticorps polyclonaux et monoclonaux fait partie des connaissances générales de l'homme du métier. On peut citer à titre de référence Köhler G. et Milstein C. (1975) : Continuous culture of fused cells secreting antibody of predefined specificity, Nature 256 :495-497 et Galfre G. et al. (1977) Nature, 266 : 522-550 pour la production d'anticorps monoclonaux et Roda A., Bolelli G.F. : Production of high-titer antibody to bile acids, Journal of Steroid Biochemistry, Vol. 13, pp. 449-454 (1980) pour la production d'anticorps polyclonaux.

Par ligand, on entend également toute molécule susceptible de s'associer à un fragment nucléotidique, tel qu'un fragment nucléotidique partiellement ou totalement complémentaire, un polynucléotide complémentaire, un anticorps anti-acides nucléiques. La production de fragments nucléotidiques ou de polynucléotides fait partie des connaissances générales de l'homme du métier. On peut notamment citer l'utilisation d'enzymes de restriction, et la synthèse chimique sur synthétiseur automatique, par exemple sur des synthétiseurs commercialisés par la société Applied Biosystem. Par ailleurs, on connaît des techniques pour la production d'anticorps anti-acides nucléiques. On peut citer à titre d'exemples Philippe Cros et al., Nucleic Acides Researc, 1994, Vol. 22, N°. 15, 2951-2957 ; Anderson, W.F. et al. (1988) Bioessays, 8 (2), 69-74 ; Lee, J.S. et al. (1984) FEBS Lett., 168, 303-306 ; Malfoy, B. et al. (1982) Biochemistry, 21(22), 5463-5467 ; Stollar, B.D. et al., J.J. (eds) Methods in Enzymology, Academic Press, pp. 70-85 ; Traincard, F. et al. (1989) J. Immunol. Meth., 123, 83-91 et Traincard, F. et al. (1989) Mol. Cell. Probes, 3, 27-38).

L'invention a encore pour objet un procédé pour détecter au moins une protéine associée à une maladie dégénérative et/ou auto-immune, dans un échantillon biologique dans lequel on met en contact l'échantillon biologique avec au moins un ligand spécifique d'au moins un polypeptide, ledit polypeptide comprenant au moins un fragment d'une protéine et ladite protéine étant choisie parmi les protéines dont la séquence peptidique à l'état natif correspond à SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19, SEQ ID N° 20, SEQ ID N° 21, SEQ ID N° 22, SEQ ID N° 23, SEQ ID N° 24, SEQ ID N° 25, SEQ ID N° 26, SEQ ID N° 27, SEQ ID N° 28 et SEQ ID N° 29 et les séquences peptidiques qui présentent au moins 70 % d'identité de préférence au moins 80 % d'identité et avantageusement au moins 98 % d'identité avec l'une quelconque des séquences peptidiques SEQ ID N° 1 à SEQ ID N° 8 et SEQ ID N° 10 à 29, et les séquences peptidiques ou les fragments desdites séquences appartenant à une même famille de protéines choisies parmi le perlecan, le précurseur de la protéine plasmatique de liaison au rétinol, la protéine activatrice du ganglioside GM2, de la calgranuline B et de la saposine B, puis on détecte la formation d'un complexe entre ledit polypeptide et ledit ligand. Ledit ligand est avantageusement un anticorps monoclonal, un anticorps polyclonal, un récepteur, un substrat d'activité enzymatique ou une enzyme dont ledit polypeptide est un cofacteur.

De même, l'invention concerne un procédé pour détecter au moins un ligand associé à une maladie dégénérative et/ou auto-immune, dans un échantillon biologique, caractérisé en ce que l'on met en contact l'échantillon biologique avec au moins un polypeptide comprenant au moins un fragment d'une protéine, ladite protéine étant choisie parmi les protéines dont la séquence peptidique à l'état natif correspond à SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19, SEQ ID N° 20, SEQ ID N° 21, SEQ ID N° 22, SEQ ID N° 23, SEQ ID N° 24, SEQ ID N° 25, SEQ ID N° 26, SEQ ID N° 27, SEQ ID N° 28 et SEQ ID N° 29 et les séquences peptidiques qui présentent au moins 70 % d'identité, de préférence au moins 80 % d'identité et avantageusement au moins 98 % d'identité avec l'une quelconque des séquences peptidiques SEQ ID N° 1 à SEQ ID N° 8 et SEQ ID N°s 10 à SEQ ID N° 29, et les séquences peptidiques ou les fragments desdites séquences appartenant à une même famille de protéines choisies parmi le perlecan, le précurseur de la protéine plasmatique de liaison au rétinol, la protéine activatrice du ganglioside GM2, de la calgranuline B et de la saposine B, puis on détecte la formation d'un complexe entre ledit polypeptide et ledit ligand. Le ligand est toute molécule qui répond aux conditions précédemment décrites.

De préférence, dans les procédés décrits ci dessus la séquence du polypeptide comprend ou consiste en une séquence peptidique choisie parmi l'une quelconque des SEQ ID N° 1 à 8 et SEQ ID N° 10 à 29 précédentes et les séquences peptidiques ou les fragments desdites séquences appartenant à une même famille de protéines choisies parmi le perlecan, le précurseur de la protéine plasmatique de liaison au rétinol, la protéine activatrice du ganglioside GM2, de la calgranuline B et de la saposine B.

L'invention concerne également un nouveau polypeptide qui comprend au moins un fragment d'une protéine dont la séquence peptidique correspond à SEQ ID N° 9, ledit fragment présentant au moins une mutation, en particulier au moins deux mutations par rapport à la séquence de référence SEQ ID N° 8. Le polypeptide est avantageusement choisi parmi les polypeptides qui comprennent la séquence en acides aminés FSWDNCFEGKDPAVIR, référencée SEQ ID N° 68 et la séquence en acides aminés YSLPKSEFAVPDLELP, référencée SEQ ID N° 72.

En particulier, ledit polypeptide comprend ou consiste en SEQ ID N° 9. Ce polypeptide est utilisé pour obtenir une composition diagnostique, pronostique, prophylactique ou thérapeutique destinée à détecter, pronostiquer, prévenir ou traiter un état pathologique associé à une maladie dégénérative et/ou auto-immune, seul ou en mélange avec au moins un polypeptide tel que défini précédemment.

L'un des objets de l'invention est également un fragment nucléotidique qui code pour le fragment de la protéine dont la séquence peptidique correspond à SEQ ID N° 9, ledit fragment de ladite protéine présentant au moins une mutation, en particulier deux mutations par rapport à la séquence de référence SEQ ID N° 8. Ledit fragment nucléotidique, en particulier, comprend ou consiste en un fragment qui code pour SEQ ID N° 9. Ce fragment est utilisé pour obtenir une composition diagnostique, pronostique, prophylactique ou thérapeutique destinée à détecter, prévenir ou traiter un état pathologique associé à une maladie dégénérative et/ou auto-immune, seul ou en mélange avec au moins un fragment nucléotidique tel que défini précédemment.

L'invention a aussi pour objet un procédé pour détecter au moins un ligand associé à une maladie dégénérative et/ou auto-immune, dans un échantillon biologique, selon lequel on met en contact l'échantillon biologique avec au moins le polypeptide qui comprend ou consiste en SEQ ID N° 9 ou un mélange de polypeptides comprenant ce polypeptide et au moins un polypeptide tel que décrit ci dessus, puis on détecte la formation d'un complexe ou de complexes entre le ou les polypeptides et le ou les ligands correspondants ; étant entendu que par ligand on entend une molécule qui répond aux conditions précitées.

L'invention concerne également un procédé pour détecter au moins le polypeptide référence SEQ ID N° 9 ou un fragment dudit polypeptide, ce fragment comprenant au moins une et de préférence deux mutations par rapport à la séquence de référence SEQ ID N°8, dans un échantillon biologique selon lequel on met en contact l'échantillon biologique avec au moins un ligand spécifique dudit polypeptide, puis on détecte la formation d'un complexe entre ledit polypeptide et ledit ligand. La définition de ligand correspond à celle définie précédemment. Il peut s'agir entre autres d'un anticorps monolonal , d'un anticorps polyclonal, d'un substrat d'activité enzymatique, ou d'une enzyme dont ledit polypeptide est un cofacteur, d'un récepteur.

On peut également mettre en contact l'échantillon biologique avec un ligand spécifique du polypeptide référence SEQ ID N°9 et au moins un ligand spécifique d'au moins un autre polypeptide tel que défini précédemment, puis on détecte la formation de complexes entre lesdits polypeptides et lesdits ligands spécifiques desdits polypeptides ; étant entendu que par ligand on entend une molécule qui répond aux conditions décrites précédemment.

Un autre objet de l'invention est un fragment nucléotidique codant pour tout ou partie du polypeptide SEQ ID N° 9, et son utilisation pour obtenir une composition diagnostique, pronostique, prophylactique ou thérapeutique destinée à détecter, pronostiquer, prévenir ou traiter un état pathologique associé à une maladie dégénérative et/ou auto-immune, éventuellement en association avec au moins un fragment nucléotidique tel que défini précédemment, et les fragments complémentaires desdits fragments.

Par fragment polypeptidique, on entend au moins tout ou partie de la séquence peptidique d'une protéine, en particulier un fragment polypeptique qui comprend environ entre 5 et 15 acides aminés et plus précisément environ entre 5 et 10 acides aminés et 6 et 15 acides aminés. Et par fragment nucléotidique, on entend au moins tout ou partie d'une séquence nucléotidique, étant entendu que par séquence nucléotidique, sont couvertes les séquences ADN et ARN.

En particulier, par fragment polypeptidique ou nucléotidique, on entend soit des fragments associés à une même unité moléculaire, soit des fragments dans un complexe moléculaire comprenant plusieurs sous-unités homologues ou hétérologues obtenues de manière naturelle ou artificielle, notamment par multi-épissage différentiel ou par synthèse sélective.

L'invention concerne aussi un procédé pour détecter au moins un polypeptide tel que défini précédemment, selon lequel on prélève un échantillon d'un fluide biologique d'un patient présentant un état pathologique associé à une maladie dégénérative et/ou neurologique et/ou auto-immune et éventuellement après purification dudit échantillon de fluide biologique, on analyse par spectrométrie de masse le profil de masse obtenu à partir du fluide biologique et on compare à un profil de masse de référence.

La présente invention concerne également l'utilisation *d'au moins* un polypeptide de l'invention pour définir des agents efficaces thérapeutiquement, et l'utilisation de ces agents pour prévenir et/ou traiter une maladie auto-immune et/ou neurologique et/ou dégénérative, en particulier la sclérose en plaques.

Ainsi, d'autres objets de l'invention sont les suivants :
- Utilisation d'au moins un polypeptide comprenant au moins un fragment d'une protéine pour tester l'efficacité d'un agent thérapeutique, ladite protéine étant choisie parmi les protéines dont la séquence peptidique à l'état natif correspond à SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ ID N° 9, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19, SEQ ID N° 20, SEQ ID N° 21, SEQ ID N° 22, SEQ ID N° 23, SEQ ID N° 24, SEQ ID N° 25, SEQ ID N° 26, SEQ ID N° 27, SEQ ID N° 28 et SEQ ID N° 29, les séquences peptidiques qui présentent au moins 70 % d'identité de préférence au moins 80 % d'identité et avantageusement au moins 98 d'identité avec l'une quelconque des séquences peptidiques SEQ ID N° 1 à 29, et les séquences peptidiques ou les fragments desdites séquences appartenant à une même famille de protéines choisie parmi le perlecan, le précurseur de la protéine plasmatique de liaison au rétinol, la protéine activatrice du ganglioside GM2, de la calgranuline B et de la saposine B;
- Utilisation d'au moins un polypeptide comprenant au moins un fragment d'une protéine pour définir un matériel biologique pour la préparation d'une composition pharmaceutique destinée au traitement d'une maladie dégénérative et/ou neurologique et/ou auto-immune, telle que la sclérose en plaques, ladite protéine étant choisie parmi les protéines dont la séquence peptidique à l'état natif correspond à SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ ID N° 9, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19, SEQ ID N° 20, SEQ ID N° 21, SEQ ID N° 22, SEQ ID N° 23, SEQ ID N° 24, SEQ ID N° 25, SEQ ID N° 26, SEQ ID N° 27, SEQ ID N° 28 et SEQ ID N° 29, les séquences peptidiques qui présentent au moins 70 % d'identité de préférence au moins 80 % d'identité et avantageusement au moins 98 d'identité avec l'une quelconque des séquences peptidiques SEQ ID N° 1 à 29, et les séquences peptidiques ou les fragments desdites séquences appartenant à une même famille de protéines choisie parmi le perlacan, le précurseur de la protéine plasmatique de liaison au rétinol, de la protéine activatrice de l'activateur du ganglioside GM2, de la calgranuline et de la saposine ;

Selon une variante avantageuse de l'une des utilisations précédentes, le polypeptide est choisi parmi SEQ ID N° 2, 4,8, 9, 17, 24 ;
- Utilisation d'au moins un fragment nucléotidique, pour tester l'efficacité d'un agent thérapeutique pour un état pathologique associé à une maladie dégénérative et/ou neurologique et/ou auto-immune, selon laquelle ledit fragment nucléotidique est choisi parmi les fragments qui codent pour au moins un fragment d'une protéine, ladite protéine étant choisie parmi les protéines dont la séquence peptidique à l'état natif correspond à SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ IDN° 9, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19, SEQ ID N° 20, SEQ ID N° 21, SEQ ID N° 22, SEQ ID N° 23, SEQ ID N° 24, SEQ ID N° 25, SEQ ID N° 26, SEQ ID N° 27, SEQ ID N° 28 et SEQ ID N° 29, les séquences peptidiques qui présentent au moins 70 % d'identité, de préférence au moins 80 % et avantageusement au moins 98 % d'identité avec l'une quelconque des séquences peptidiques SEQ ID N° 1 à 29, et les fragments complémentaires desdits fragments et les fragments qui codent pour les séquences peptidiques ou les fragments desdites séquences appartenant à une même famille de protéines choisie parmi le perlecan, le précurseur de la protéine plasmatique de liaison au rétinol, de la protéine activatrice du ganglioside GM2, de la calgranuline B et de la saposine B.
- Utilisation pour tester l'efficacité d'un agent thérapeutique pour un état pathologique associé à une maladie dégénérative et/ou neurologique et/ou auto-immune, de protéines recombinantes et/ou codées par tout ou partie des fragments nucléotidiques définis au paragraphe précédent ;
- Utilisation d'au moins un fragment nucléotidique pour la préparation d'une composition pharmaceutique destinée au traitement d'une maladie dégénérative et/ou neurologique et/ou auto-immune, telle que la sclérose en plaques, selon laquelle ledit fragment nucléotidique est choisi parmi des fragments qui codent pour au moins un fragment d'une protéine, ladite protéine étant choisie parmi les protéines dont la séquence peptidique à l'état natif correspond à SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ IDN° 9, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19, SEQ ID N° 20, SEQ ID N° 21, SEQ ID N° 22, SEQ ID N° 23, SEQ ID N° 24, SEQ ID N° 25, SEQ ID N° 26, SEQ ID N° 27, SEQ ID N° 28 et SEQ ID N° 29, les séquences peptidiques qui présentent au moins 70 % d'identité, de préférence au moins 80 % et avantageusement au moins 98 % d'identité avec l'une quelconque des séquences peptidiques SEQ ID N° 1 à 29, et les fragments complémentaires desdits fragments et les fragments qui codent pour les séquences peptidiques ou les fragments desdites séquences appartenant à une même famille de protéines choisie parmi le perlecan, le précurseur de la protéine plasmatique de liaison au rétinol, de la protéine activatrice du ganglioside GM2, de la calgranuline B et de la saposine B ;
- Utilisation pour la préparation d'une composition pharmaceutique destinée au traitement d'une maladie dégénérative et/ou neurologique et/ou auto-immune, telle que la sclérose en plaques, de protéines recombinantes et/ou codées par tout ou partie des fragments nucléotidiques définis au paragraphe précédent.

Avantageusement, ledit fragment nucléotidique utilisé code pour ladite protéine.

De préférence, la séquence peptidique de ladite protéine à l'état natif consiste en une séquence choisie parmi l'une quelconque des SEQ ID N° 1 à 29, les séquences peptidiques qui présentent au moins 70 % d'identité de préférence au moins 80 % d'identité et avantageusement au moins 98 d'identité avec l'une quelconque des séquences peptidiques SEQ ID N° 1 à 29, et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B. Les polypeptides sont préférentiellement choisis parmi SEQ ID N° 2, 4, 8, 9, 17, 24.
- Utilisation d'au moins un fragment nucléotidique, pour tester l'efficacité d'un agent thérapeutique pour un état pathologique associé à une maladie dégénérative et/ou neurologique et/ou auto-immune selon laquelle ledit fragment est un fragment d'une séquence nucléique choisie parmi l'une quelconque des SEQ ID N° 30, SEQ ID N° 31, SEQ ID N° 32, SEQ ID N° 33, SEQ ID N° 34, SEQ ID N° 35, SEQ ID N° 36, SEQ ID N° 37, SEQ ID N° 38, SEQ ID N° 39, SEQ ID N° 40, SEQ ID N° 41, SEQ ID N° 42, SEQ ID N° 43, SEQ ID N° 44, SEQ ID N° 45, SEQ ID N° 46 et SEQ ID N° 47, SEQ ID N° 48, SEQ ID N° 49 et SEQ ID N° 50, SEQ ID N° 51, SEQ ID N° 52, SEQ ID N° 53, SEQ ID N° 54, SEQ ID N° 55, SEQ ID N° 56, SEQ ID N° 57, SEQ ID N° 66, SEQ ID N° 67, SEQ ID N° 69, SEQ ID N°70 , SEQ ID N° 71, et leurs séquences complémentaires.
- Utilisation d'au moins un fragment nucléotidique pour la préparation d'une composition pharmaceutique destinée au traitement d'une maladie dégénérative et/ou neurologique et/ou auto-immune, telle que la sclérose en plaques selon laquelle ledit fragment est un fragment d'une séquence nucléique choisie parmi l'une quelconque des SEQ ID N° 30, SEQ ID N° 31, SEQ ID N° 32, SEQ ID N° 33, SEQ ID N° 34, SEQ ID N° 35, SEQ ID N° 36, SEQ ID N° 37, SEQ ID N° 38, SEQ ID N° 39, SEQ ID N° 40, SEQ ID N° 41, SEQ ID N° 42, SEQ ID N° 43, SEQ ID N° 44, SEQ ID N° 45, SEQ ID N° 46 et SEQ ID N° 47, SEQ ID N° 48, SEQ ID N° 49 et SEQ ID N° 50, SEQ ID N° 51, SEQ ID N° 52, SEQ ID N° 53, SEQ ID N° 54, SEQ ID N° 55, SEQ ID N° 56, SEQ ID N° 57, SEQ ID N° 66, SEQ ID N°67, SEQ ID N° 68, SEQ ID N° 69, SEQ ID N°70 , SEQ ID N° 71, et leurs séquences complémentaires.

La séquence nucléique est de préférence choisie parmi SEQ ID N° 30, 31, 42,53.
- Utilisation de la lycorine pour la préparation d'une composition pour la prévention et/ou le traitement de maladie dégénérative et/ou neurologique et/ou auto-immune.

Par efficacité thérapeutique, on entend le bénéfice clinique et biologique acquis après administration d'un agent thérapeutique en vue d'une amélioration, voire d'une guérison de la maladie. Ce bénéfice se traduit entre autre par une diminution des signes cliniques, biologiques, et des effets pathologiques de la maladie après une analyse clinique par le médecin et/ou des analyses biologiques, telles que imagerie par résonance magnétique, analyse des bandes oligoclonales dans le liquide céphalo-rachidien, analyse de potentiels évoqués et le test de détection de gliotoxicité appelé bio-essai, dont le principe est décrit dans la demande de brevet WO 98/11439 précédemment citée. Cette diminution des signes cliniques et effets pathologiques doit entraîner un bénéfice pour le patient (Schwartz et Lazar, 1995, Elements de statistique médicale et biologique, eds Flammarion ; Lazar et Schwartz, 1995, Eléments de statistique médicale et biologique, eds Flammarion ). La maladie étudiée de préférence est la sclérose en plaques.

On entend par composition à usage prophylactique et/ou thérapeutique, toute composition qui comprend un agent thérapeutiquement efficace. Ces agents thérapeutiques sont capables (i) d'influencer de manière qualitative et/ou quantitative l'activité biologique et/ou la fonction des protéines d'intérêt identifiées dans la présente invention, de préférence l'activité gliotoxique et/ou (ii) de moduler et/ou d'inhiber l'expression de ces protéines et/ou (iii) de diminuer la concentration de ces protéines dans un compartiment extracellulaire et/ou intracellulaire, et/ou de substituer une forme non pathogène à une forme pathogène, par exemple mutée, d'une de ces protéines et/ou de moduler leur fixation à au moins un de leur ligand ; ledit ligand étant une molécule qui répond aux critères précédemment décrits. Différents agents thérapeutiques sont produits en suivant les approches classiques largement décrites dans la littérature. Les différents groupes d'agents thérapeutiques définis à partir des protéines d'intérêt identifiées dans cette présente invention sont décrits ci-dessous. Leur activité ou efficacité prophylactique et/ou thérapeutique est évaluée *in vitro* et/ou *in vivo.*

Evaluation de l'efficacité d'un agent thérapeutique *in vitro* : des échantillons d'urine d'individus sains et de patients atteints de la sclérose en plaque, de préférence en phase active, sont testés pour leur activité gliotoxique *in vitro* en suivant le protocole du bio-essai décrit dans la demande de brevet WO 98/11439, précédemment citée. L'expérience est réalisée en parallèle en ajoutant ou non dans les échantillons d'urine testés l'agent thérapeutique dont l'efficacité est à tester. Des essais sont réalisés à différentes concentrations de cet agent, et après différents temps d'incubation avec l'échantillon, à une température d'environ 37°C ou à température ambiante, pour chaque concentration d'agent testé, avant la réalisation du test bio-essai. L'activité gliotoxique est déterminée pour chaque échantillon brut ou purifié d'urine témoin et de patient en présence ou en absence de l'agent thérapeutique testé. Un agent prophylactique et/ou thérapeutique pour la sclérose en plaques est un agent qui permet une diminution ou une inhibition de l'activité gliotoxique dans un fluide biologique des patients, en particulier dans l'urine. Cette diminution ou inhibition est évaluée par rapport à l'activité gliotoxique détectée dans le fluide biologique des patients SEP en absence de l'agent testé qui fixe la borne supérieure et par rapport à l'activité gliotoxique détectée dans l'urine d'individu sain qui détermine la borne inférieure (Schwartz et Lazar, 1995, Elements de statistique médicale et biologique, eds Flammarion ; Lazar et Schwartz, 1995, Elements de statistique médicale et biologique, eds Flammarion). L'efficacité thérapeutique de plusieurs agents peuvent être évaluée en combinaison dans un même essai.

Evaluation de l'efficacité d'un agent thérapeutique utilisant un modèle animal : à un animal sont injectées des fractions d'urine purifiée et/ou au moins un polypeptide de l'invention et/ou au moins une protéine obtenue par recombinaison génétique qui correspond à au moins un polypeptide de l'invention et/ou au moins un polypeptide de synthèse dont la séquence en acides aminés correspond à la séquence d'au moins un polypeptide de l'invention. Les injections sont effectuées, à différentes concentrations établies, à des animaux mammifères, tels que souris ou rat, de préférence un rat Lewis selon le protocole décrit dans la demande de brevet WO97/33466 citée précédemment. A des séries d'animaux sont injectées, par voie intradermique, intraveineuse, intrathécale, intracérébrale, intramusculaire, ou autres, différentes concentrations d'une fraction d'urine brute ou purifiée ou d'au moins un polypeptide et/ou une protéine, tels que définis ci-dessus. Un contrôle négatif est effectué en parallèle. L'agent prophylactique et/ou thérapeutique à évaluer et ensuite injecté à différentes concentrations et par différentes voies d'administration à un animal mammifère, de préférence à une souris ou à un rat. Les injections sont réalisées en une seule dose ou en doses répétées, avec différents temps d'intervalle entre chaque administration. Quelques heures à quelques semaines après l'administration, des échantillons biologiques, de préférence du sang, du sérum, du liquide céphalo-rachidien, de l'urine sont prélevés. Sur ces échantillons sont réalisés :
(i) une mesure de l'activité gliotoxique par le bio-essai, et/ou
(ii) une mesure d'activité des polypeptides et/ou protéines d'intérêt de l'invention, seuls ou en combinaison comme décrit au moins dans : Li et al., 1983, Am J Hum Genet 35 :629-634 ; Li et al., 1988 J Biol Chem 263 : 6588-6591 ; Li et al., 1981 J Biol Chem 256 : 6234-6240 ;Li et al., 1976 J Biol Chem 251 :1159 ; Kase et al., 1996, FebsLetters 393 : 74-76 ; Kishimoto et al., 1992, J Lipid Res 33 : 1255-1267 ; O'Brien et al., 1991 Faseb J 5 : 301-308 ; Murthy et al.,1993 J Immunol 151 : 6291-6301 ; Murao et al., 1990 Cell growth Differ 1 : 447-454, et/ou
(iii) un dosage des polypeptides et/ou protéines d'intérêt, seuls ou en combinaison, par ELISA (Enzyme Linked-Immunosorbant Assay) et/ou Western Blot, en utilisant des anticorps ou des fragments d'anticorps capables de se fixer à au moins un des polypeptides et/ou protéines de l'invention, ou leur fragment, et/ou
iv) un dosage d'anticorps spécifiques des polypeptides et/ou protéines d'intérêt ou leurs fragments, seuls ou en combinaison ou le dosage d'au moins un ligand capable de se fixer aux polypeptides et/ou protéines d'intérêt ou leurs fragments, et/ou
(v) un dosage de la réponse immune cellulaire « helper » et/ou cytotoxique induite contre les polypeptides et protéines d'intérêt ou leurs fragments et tout peptide immunogène dérivant de ces polypeptides, protéines et fragments, en réalisant, par exemple, un test d'activation *in vitro* de cellules lymphocytes T " helper " spécifiques de l'antigène administré ; en quantifiant les lymphocytes T cytotoxiques selon la technique dite ELISPOT décrite par Scheibenbogen et al.,1997 Clinical Cancer Research 3 : 221-226. Une telle détermination est particulièrement avantageuse lorsque l'on veut évaluer l'efficacité d'une approche vaccinale pour la mise en oeuvre chez un patient donné ou pour diagnostiquer et/ou pronostiquer un état pathologique potentiel en cherchant à mettre en évidence une réponse immune naturellement développée par le patient contre l'antigène, les polypeptides, les protéines d'intérêt ou les fragments immunogènes dérivés de ces protéines.

Par « ligand capable de se fixer à une protéine », on entend toute molécule capable de reconnaître la protéine ou une partie de la protéine. Cela peut être vérifié par exemple *in vitro* par tests Elisa et/ou Western blot .

On désigne par « polypeptides et/ou protéines d'intérêt de l'invention » le fragment C-terminal du perlecan (SEQ ID N°2), le précurseur de la protéine plasmatique de liaison au rétinol (SEQ ID N°4), la protéine activateur du GM2 (SEQ ID N° 8), la protéine mutée de l'activateur du GM2 (SEQ ID N° 9), la calgranuline B (SEQ ID N° 17), la saposine B (SEQ ID N° 24), les protéines ou fragments appartenant à la famille du précurseur de la protéine plasmatique de liaison au rétinol (par exemple SEQ ID N° 5 à 7), les protéines ou fragments appartenant à la famille de la protéine activateur du GM2 (par exemple SEQ ID N° 10 à 16), les protéines ou fragments appartenant à la famille de la protéine calgranuline B (par exemple SEQ ID N° 18 à 23), les protéines ou fragments appartenant à la famille de la protéine saposine B (par exemple SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29.

L'animal est ensuite sacrifié et des coupes histologiques de différents tissus sont réalisées, de préférence des coupes de cerveaux. Différentes études et observations sont réalisées pour détecter et/ou quantifier les effets caractéristiques des polypeptides et/ou protéines actives associées à la fraction gliotoxique, c'est à dire une apoptose des cellules gliales, et/ou l'ouverture de la barrière hémato-encéphalique, et/ou une démyélinisation. La présence ou l'expression des polypeptides et/ou protéines d'intérêt identifiées est également observée et/ou quantifiée dans ces tissus :
(i) par des analyses d'immunohistologie classiques en utilisant des ligands des polypeptides et/ou protéines d'intérêt et/ou leurs fragments et/ou des anticorps monoclonaux ou polyclonaux ou des fragments desdits qui se lient aux polypeptides et/ou protéines d'intérêt, ou à leurs fragments, et/ou
(ii) par des techniques d'hybridation *in situ* classiques en utilisant des fragments d'acides nucléiques ou des oligonucléotides définis à partir des séquences polypeptidiques et/ou protéiques d'intérêt ; et/ou
(iii) par des techniques d'amplification par PCR et/ou RT-PCR in situ en utilisant des fragments d'acides nucléiques ou des amorces définis à partir des séquences polypeptidiques et/ou protéiques d'intérêt.

Par anticorps capable de se fixer à un polypeptide, à une protéine ou à leurs fragments, on entend tout anticorps monoclonal ou polyclonal et tout fragment desdits anticorps capable de reconnaître le polypeptide, la protéine ou leurs fragments. La capacité des anticorps à reconnaître lesdits polypeptides, protéines ou leurs fragments est vérifiée *in vitro,* par exemple en ELISA et/ou Western Blot. Un anticorps capable de se fixer à la protéine saposine B (SEQ ID N° 24) ou à tout fragment de cette protéine est décrit par Misasi et al. 1998, J. NeuroChem. 71 : 2313 et Klein et al. 1994, BBRC 200 : 1440-1448 ou peut être produit en utilisant les méthodes conventionnelles, par exemple celles référencées précédemment pour la production d'anticorps monoclonaux et polyclonaux, par immunisation à partir de la protéine naturelle, d'une protéine recombinante, d'un polypeptide de synthèse ou de leurs fragments. Les peptides immunogènes pour la production d'anticorps monoclonaux anti-saposine B sont les peptides correspondant aux séquences SEQ ID N° 61 et SEQ ID N° 62.

Par exemple, un anticorps capable de se fixer à la protéine activatrice du GM2 (SEQ ID N° 8) ou à tout fragment de cette protéine est illustré par Yuziuk et al., 1998 J Biol Chem 273 : 66-72 ou peut être produit en utilisant les méthodes conventionnelles connues de l'homme de l'art. Cet anticorps peut être par exemple produit après injection à des souris ou lapin de la protéine naturelle ou tout fragment, et/ou de la protéine recombinante ou tout fragment, et/ou de peptides définis et synthétisés à partir de la séquence protéique de la protéine. Les peptides immunogènes utilisés pour la production d'anticorps monoclonaux anti-GM2 sont les peptides références SEQ ID N° 58, SEQ ID N° 59 et SEQ ID N° 60. Un anticorps capable de se fixer à la protéine Galgranuline B (SEQ ID N° 17) ou à tout fragment de cette protéine est décrit par Saintigny et al., 1992 J Invest Dermatol 99 : 639-644 et Goebeler et al 1994 J Leukoc Biol 55 : 259-261, ou peut être produit en utilisant les méthodes conventionnelles. Les peptides immunogènes pour la production d'anticorps monoclonaux anti-calgranuline B sont les peptides correspondant aux séquences SEQ ID N° 63, SEQ ID N° 64 et SEQ ID N° 65. Un anticorps capable de se fixer à la protéine mutée activatrice du GM2 (SEQ ID N°9) ou à tout fragment de cette protéine peut être produit en utilisant les méthodes conventionnelles définies ci dessus.

Par protéine naturelle et fragment, on entend toute protéine isolée, purifiée totalement ou partiellement obtenue à partir d'échantillon humain ou animal et tout fragment obtenu à partir de cette protéine. Par exemple, on obtient la protéine naturelle correspondant à la saposine B (SEQ ID N° 24) en suivant la technique décrite par Waring et al. 1998 Mol Genet Metab 63 : 14-25 ; la protéine naturelle correspondant à la protéine activatrice du GM2 (SEQ ID N° 8) en suivant la technique décrite par DeGasperi et al.,1989 Biochem J 260 : 777-783, Vogel et al., 1987 Arch Biochem Biophys 259 : 627-638, Mitsuyama, 1983 Hokkaido Igaku Zasshi 58: 502-512 ; Hirabayashi et al 1983 J Neurochem 40 : 168-175, Conzelmann et al, 1979 Hoppe Seylers Z Physiol Chem 360 : 1837-1849, Li et al., 1976 J Biol Chem 251 : 1159-1163. La protéine naturelle correspondant à la calgranuline B (SEQ ID N° 17) est obtenue en suivant la technique décrite par Hitomi et al. 1996 J Cell Sci 109 : 805-815, Van den Bos et al. 1998 Protein Expr Purif 13 : 313-318 et Raftery et al. 1996 Biochem J 316 : 285-293.

Par protéine recombinante ou fragment d'une protéine recombinante, on fait référence à toute protéine ou fragment de protéine produit dans une cellule procaryote ou eucaryote à partir d'une séquence nucléotidique codant pour la protéine ou son fragment et transfectée dans la cellule, cette protéine ou son fragment étant ensuite purifiée. D'une manière générale, toute cellule issue d'un organisme procaryote ou eucaryote peut être utilisée dans le cadre de la présente invention, mais les cellules issues d'organismes eucaryotes sont préférées. On peut citer à titre d'exemple les cellules CHO, les cellules COS, les cellules Semliki. Aux fins de la présente invention, ladite cellule peut être sauvage ou mutante. Par exemple, la protéine recombinante correspondant à la saposine B (SEQ ID N° 24) peut être obtenue en suivant les techniques décrites par Zaltash et al. 1998 Bebbs letter 423 : 1-4 et Qi et al. 1994 J Biol Chem 269 : 16746-16753. Une telle protéine recombinante est au moins disponible auprès de Kase et al. 1996 Febs Lett 393 : 74-76. La protéine recombinante correspondant à la protéine activatrice du GM2 (SEQ ID N° 8) peut être produite par les techniques décrites par Yuziuk et al. 1998 J Biol Chem 273 : 66-72 et Bierfreund et al., 1999 Neurochem Res 24 : 295-300. La protéine recombinante correspondant à la calgranuline B (SEQ ID N° 17) peut être obtenue selon le protocole de Longbottom et al.1992 Biochim Biophys Acta 1120 :215-222, Raftery et al. 1999 Protein Expr Purif 15 :228-235. Une telle protéine recombinante est disponible au moins auprès de Klempt et al. 1997 Febs Letter 408 : 81-84.

Par séquence nucléotidique d'ADN ou fragment nucléotidique d'ADN codant pour tout ou partie de la protéine saposine B (SEQ ID N°24), on entend la séquence d'acides nucléiques SEQ ID N° 53 ou un fragment de cette séquence. Par séquence ou fragment nucléotidique ARN codant pour tout ou partie de la protéine saposine B (SEQ ID N° 24), on entend toute séquence déduite de la séquence d'ADN SEQ ID N° 53, en tenant compte du code génétique et des phénomènes d'épissage.

Par séquence nucléotidique d'ADN ou fragment nucléotidique d'ADN codant pour tout ou partie de la protéine activatrice du GM2 (SEQ ID N° 8), on entend la séquence d'acides nucléiques SEQ ID N° 31 ou un fragment de cette séquence. Par séquence ou fragment nucléotidique d'ARN codant pour tout ou partie de la protéine activatrice du GM2 (SEQ ID N° 8), on entend toute séquence déduite de la séquence ADN SEQ ID N° 31, en tenant compte du code génétique et des phénomènes d'épissage.

Par séquence nucléotidique d'ADN ou fragment nucléotidique d'ADN codant pour tout ou partie de la protéine calgranuline B (SEQ ID N° 17), on entend la séquence d'acides nucléiques SEQ ID N° 42 ou un fragment de cette séquence. Par séquence ou fragment nucléotidique d'ARN codant pour tout ou partie de la protéine calgranuline B (SEQ ID N° 17), on entend toute séquence déduite de la séquence ADN SEQ ID N° 42, en tenant compte du code génétique et des phénomènes d'épissage.

Par séquence ou fragment nucléotidique codant pour tout ou partie de la protéine mutée (SEQ ID N° 9), on entend la séquence d'acides nucléiques déduite de la séquence SEQ ID N° 9, en tenant compte du code génétique. Par séquence ou fragment nucléotidique ARN codant pour tout ou partie de cette protéine mutée B (SEQ ID N° 9), on entend toute séquence déduite de la séquence ADN, en tenant compte du code génétique et des phénomènes d'épissage.

Par activité protéique, on entend une fonction caractéristique biologique de la protéine. Cette activité protéique peut être mise en évidence par des techniques connues de l'homme de l'art. Par exemple, l'activité de la saposine B (SEQ ID N° 24) et des protéines de la famille de la saposine B (par exemple SEQ ID N° 25 à 29), peut être détectée par la mise en oeuvre des protocoles décrits par Li et al., 1983, Am J Hum Genet 35 :629-634,; Li et al., 1988 J Biol Chem 263 : 6588-6591, Li et al., 1981 J Biol Chem 256 : 6234-6240 et Li et al., 1976 J Biol Chem 251 :1159. Par activité de la protéine activatrice du GM2 (SEQ ID N° 8) et des protéines de la même famille (par exemple SEQ ID N° 10 à 16), on entend au moins l'activité détectée par la mise en oeuvre des protocoles décrits par exemple par Kase et al., 1996, Febs Letters 393 : 74-76, Kishimoto et al., 1992, J Lipid Res 33 : 1255-1267 et O'Brien et al., 1991 Faseb J 5 : 301-308. Par activité de la calgranuline B (SEQ ID N° 17) et les protéines de la même famille de la calgranuline b (par exemple SEQ ID N° 18à 23) et toute, on entend au moins l'activité détectée par la mise en oeuvre des protocoles décrits par exemple par Murthy et al.,1993 J Immunol 151 : 6291-6301 et Murao et al., 1990 Cell growth Differ 1 : 447-454.

L'obtention d'un modèle animal transgénique, de préférence murin, pour une pathologie humaine est techniquement réalisable. Brièvement, l'animal transgénique est produit en utilisant les techniques conventionnelles décrites et possède intégré dans son génome les acides nucléiques codant pour les protéines ou leurs fragments.

Evaluation de l'efficacité d'un agent thérapeutique et suivi thérapeutique ex *vivo*, chez l'homme :
les agents thérapeutiques à tester pour une activité thérapeutique et/ou pour un suivi thérapeutique sont administrés par différentes voies à l'homme, telles que les voies intradermique, intraveineuse, intramusculaire, intracérébrale, orale, ou autres. Différentes doses sont administrées à l'être humain. Le dossier clinique du patient au moment de la première administration est parfaitement connu. Une ou plusieurs administrations peuvent être réalisées avec des temps d'intervalle différents entre chaque administration pouvant aller de quelques jours à quelques années. Des échantillons biologiques sont prélevés à des intervalles de temps déterminés après administration de l'agent thérapeutique, de préférence du sang, du sérum, du liquide céphalo-rachidien et de l'urine. Différentes analyses sont réalisées à partir de ces échantillons. Juste avant la première administration de l'agent thérapeutique, ces prélèvements et ces mêmes analyses sont également réalisés. Un examen clinique et biologique classique (IRM, bandes oligoclonales dans le liquide céphalo-rachidien, potentiels évoqués) est réalisé également en parallèle des analyses supplémentaires qui sont être décrites ci dessous, à différentes temps de l'analyse. Les analyses réalisées sont :
   (i) une mesure de l'activité gliotoxique par le bio-essai à partir d'échantillons de sérum, de LCR et d'urine, et/ou
   (ii) une mesure d'activité des protéines d'intérêt identifiées dans la présente invention seules ou en combinaison comme décrit par exemple par : Li et al., 1983, Am J Hum Genet 35 :629-634 ; Li et al., 1988 J Biol Chem 263 : 6588-6591 ; Li et al., 1981 J Biol Chem 256 : 6234-6240 ;Li et al., 1976 J Biol Chem 251 :1159 ; Kase et al., 1996, FebsLetters 393 : 74-76 ; Kishimoto et al., 1992, J Lipid Res 33 : 1255-1267 ; O'Brien et al., 1991 Faseb J 5 : 301-308 ; Murthy et al.,1993 J Immunol 151 : 6291-6301 ; Murao et al., 1990 Cell growth Differ 1 : 447-454, et/ou
   (iii) un dosage des protéines d'intérêt ou de leurs fragments, seuls ou en combinaison, dans les échantillons de sang/sérum, LCR, urine par ELISA et/ou Western Blot, en utilisant des anticorps ou des fragments d'anticorps capables de se fixer à au moins une des protéines ou à un de leur fragment, et/ou
   (iv) un dosage d'anticorps spécifiques des protéines d'intérêt ou de leurs fragments dans des échantillons de sang/sérum, LCR, urine, par ELISA et/ou Western blot en utilisant une protéine naturelle ou un fragment de la protéine naturelle et/ou une protéine recombinante ou un fragment de cette protéine recombinante, seuls ou en combinaison. De même un dosage de ligands capables de se fixer aux protéines d'intérêt identifiées, seules ou en combinaison, peut être réalisé, et/ou
   (v) un dosage de la réponse immune cellulaire « helper » et/ou cytotoxique induite contre les protéines d'intérêt et tout peptide immunogène dérivant de ces protéines, par exemple en réalisant un test d'activation *in vitro* de cellules lymphocytes T spécifiques de l'antigène administré (exemple). Par exemple en réalisant un test d'activation *in vitro* de cellules lymphocytes T helper spécifiques de l'antigène administré (exemple) ; Par exemple en quantifiant les lymphocytes T cytotoxiques selon la technique dite ELISPOT décrite par Scheibenbogen et al.,1997 Clinical Cancer Research 3 : 221-226. Une telle détermination est particulièrement avantageuse lorsque l'on souhaite évaluer l'efficacité d'une approche vaccinale mise en oeuvre chez un patient donné ou pour diagnostiquer un état pathologique potentiel chez un patient en cherchant à mettre en évidence une réponse immune naturellement développée par ledit patient contre l'antigène les protéines d'intérêt ou tout fragment immunogène dérivés de ces protéines, seuls ou en combinaison, et/ou
   (vi) une détection de fragments d'ADN et/ou d'ARN codant pour les protéines ou un fragment des protéines d'intérêt par hybridation nucléotidique selon les techniques bien connues de l'homme de l'art (Southern blot, Northern blot, ELOSA " Enzyme-Linked Oligosorbent Assay " (Katz JB et al., Am. J. Vet. Res., 1993 Dec ; 54 (12) :2021-6 et François Mallet et al., Journal of Clinical Microbiology, June 1993, p1444-1449)) et/ou par méthode d'amplification de l'ADN et/ou l'ARN , par exemple par PCR, RT-PCR, en utilisant des fragments d'acides nucléiques codant pour la séquence des protéines d'intérêt, et/ou
   (vii) par biopsie de tissus, de préférence du cerveau, et l'observation des effets caractéristiques des protéines actives associées à la fraction gliotoxique, c'est à dire une apoptose des cellules gliales et/ou l'ouverture de la barrière hémato-encéphalique et/ou l'observation de phénomènes de démyélinisation, et/ou
   (viii) par biopsie de tissus ou sur cellules circulantes (sang, LCR), l'observation de la présence des protéines d'intérêt et l'estimation de leur expression par observation immunohistologique sur des coupes histologiques réalisées à partir des tissus, en utilisant des ligands et/ou des anticorps ou leurs fragments capables de se fixer aux protéines d'intérêt, et/ou
   (ix) par biopsie de tissus ou sur cellules circulantes (sang, LCR), l'observation de l'expression des protéines d'intérêt par hybridation in situ des molécules d'ARN codant pour les protéines d'intérêt en utilisant des acides nucléiques définis à partir des séquences des protéines d'intérêt, et/ou
   (x) par biopsie de tissus ou sur cellules circulantes (sang, LCR), la détermination de l'expression des protéines d'intérêt par amplification de ces ARN par des techniques classiques, comme par exemple, la RT-PCR, en utilisant des acides nucléiques définis à partir des séquences des protéines d'intérêt.

On désigne par « polypeptides et/ou protéines d'intérêt de l'invention » le fragment C-terminal du perlecan (SEQ ID N°2), le précurseur de la protéine plasmatique de liaison au rétinol (SEQ ID N°4), la protéine activateur du GM2 (SEQ ID N° 8), la protéine mutée de l'activateur du GM2 (SEQ ID N° 9), la calgranuline B (SEQ ID N° 17), la saposine B (SEQ ID N° 24), les protéines ou fragments appartenant à la famille du précurseur de la protéine plasmatique de liaison au rétinol (par exemple SEQ ID N° 5 à 7), les protéines ou fragments appartenant à la famille de la protéine activateur du GM2 (par exemple SEQ ID N° 10 à 16), les protéines ou fragments appartenant à la famille de la protéine calgranuline B (par exemple SEQ ID N° 18 à 23), les protéines ou fragments appartenant à la famille de la protéine saposine B (par exemple SEQ ID N° 25 à 29), et les séquences peptidiques qui présentent au moins 70 % d'identité de préférence au moins 80 % d'identité et avantageusement au moins 98 d'identité avec l'une quelconque des séquences peptidiques SEQ ID N° 1 à 29.

On désigne par séquence d'acides nucléiques ADN ou fragments codant pour les 'polypeptides et/ou protéines d'intérêt de l'invention' la séquence d'acides nucléiques codant pour le fragment C-terminal du perlecan (SEQ ID N°2), la séquence d'acides nucléiques codant pour le précurseur de la protéine plasmatique de liaison au rétinol (SEQ ID N°4), la séquence d'acides nucléiques (SEQ ID N° 31) codant pour la protéine activateur du GM2 (SEQ ID N° 8), la séquence d'acides nucléiques codant pour la protéine mutée de l'activateur du GM2 (SEQ ID N° 9), la séquence d'acides nucléique (SEQ ID N° 42) codant pour la calgranuline B (SEQ ID N° 17), la séquence d'acides nucléiques (SEQ ID N°53) codant pour la saposine B (SEQ ID N° 24), les séquences d'acides nucléiques ADN et/ou ARN (SEQ ID N° 30 à 57) codant pour les protéines ou fragments appartenant à la famille du précurseur de la protéine plasmatique de liaison au rétinol (par exemple SEQ ID N° 5 à 7), les protéines ou fragments appartenant à la famille de la protéine activateur du GM2 (par exemple SEQ ID N° 10 à 16), les protéines ou fragments appartenant à la famille de la protéine calgranuline B (par exemple SEQ ID N° 18 à 23), les protéines ou fragments appartenant à la famille de la protéine saposine B (par exemple SEQ ID N° 25 à 29).

Une protéine ou un variant d'une protéine choisie plus particulièrement parmi les séquences définies dans les identificateurs SEQ ID N°s 2, 4, 8 , 9, 17 et 24 ou leurs fragments, ou parmi les séquences correspondant aux protéines des familles de ces dites séquences (par exemple SEQ ID N° 1, SEQ ID N° 3, SEQ ID N° 5 à 7, SEQ ID N° 10 à 16, SEQ ID N° 18 à 24, SEQ ID N° 25 à 29), et les séquences peptidiques qui présentent au moins 70 % d'identité de préférence au moins 80 % d'identité et avantageusement au moins 98 d'identité avec l'une quelconque des séquences peptidiques SEQ ID N° 1 à 29, indépendamment ou en combinaison, présente un effet toxique directement ou indirectement, vis à vis de cellules, en particulier vis à vis des cellules gliales, qui est mis en évidence par le bio-essai précité. Les auto-anticorps produits en réponse à la présence de cette protéine ou de ces protéines sont associés au processus auto-immun. Ainsi, la cible du ou des agent(s) thérapeutique(s) est par exemple (i) la protéine naturelle ou les protéines naturelles ou leurs variants dans le but de réguler leur expression et/ou leur concentration intracellulaire et/ou leur concentration dans la circulation, (ii) un anticorps spécifique d'au moins une telle protéine. L'agent thérapeutique ou les agents thérapeutiques définis éliminent la cible directement, par induction d'une réponse immune spécifique et/ou la neutralisent.

La présente invention concerne donc un matériel biologique pour la préparation d'une composition pharmaceutique destinée au traitement de mammifères atteints de pathologies dégénérative et/ou auto-immune et/ou neurologique, de préférence la sclérose en plaques, ladite composition comprenant :
(i) soit au moins une protéine naturelle et/ou une protéine recombinante ou leurs fragments dont la séquence correspond à tout ou partie des séquences référencées SEQ ID N° 2, 4, 8, 9, 17 et 24 et les séquences peptidiques ou les fragments desdites séquences appartenant à une même famille de protéines choisie parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29), et les séquences peptidiques qui présentent au moins 70 % d'identité de préférence au moins 80 % d'identité et avantageusement au moins 98 d'identité avec l'une quelconque des séquences peptidiques SEQ ID N° là 29, indépendamment ou en combinaison,
(ii) soit au moins un ligand spécifique d'au moins une desdites protéines ou leurs fragments dont la séquence correspond à tout ou partie des séquences référencées SEQ ID N° 2, 4, 8, 9, 17 et 24, et les séquences peptidiques ou les fragments desdites séquences appartenant à une même famille de protéines choisie parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29), et les séquences peptidiques qui présentent au moins 70 % d'identité de préférence au moins 80 % d'identité et avantageusement au moins 98 d'identité avec l'une quelconque des séquences peptidiques SEQ ID N° 1 à 29, indépendamment ou en combinaison,
(iii) soit au moins un anticorps polyclonal ou monoclonal spécifique d'au moins une desdites protéines ou leurs fragments dont la séquence correspond à tout ou partie des séquences référencées SEQ ID N° 2, 4, 8, 9, 17 et 24, et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29), et les séquences peptidiques qui présentent au moins 70 % d'identité de préférence au moins 80 % d'identité et avantageusement au moins 98 d'identité avec l'une quelconque des séquences peptidiques SEQ ID N° 1 à 29, indépendamment ou en combinaison,
(iv) soit au moins une séquence d'acide nucléique comprenant au moins un gène d'intérêt thérapeutique dont la séquence nucléique est déduite des séquences d'ADN et d'ARN codant pour tout ou partie des protéines dont les séquences sont référencées SEQ ID N° 2, 4, 8, 9, 17 et 24, et les séquences d'ADN et/ou ARN (par exemple SEQ ID N° 30 à 57) codant pour tout ou partie des protéines appartenant à une même famille de protéines choisies parmi le perlecan, le précurseur de la protéine plasmatique de liaison au rétinol, de la protéine activatrice du GM2, de la calgranuline B et de la saposine B, en association avec des éléments assurant l'expression dudit gène d'intérêt thérapeutique *in vivo* dans des cellules cibles destinées à être génétiquement modifiées par la séquence nucléique du gène d'intérêt thérapeutique,
(v) soit au moins une cellule de mammifère ne produisant pas naturellement la protéine d'intérêt ou les protéines d'intérêt ou tout fragment de cette ou de ces protéine(s) ou des anticorps spécifiques d'au moins une desdites protéines ou de ses fragments ladite cellule mammifère étant génétiquement modifiée *in vitro* par au moins une séquence d'acide nucléique ou un fragment d'une séquence d'acide nucléique ou une association de séquences d'acides nucléiques correspondant à des fragments d'acides nucléiques issus d'un même gène ou de gènes différents, la ou lesdites séquences nucléiques étant déduite(s) des séquences d'ADN et ARN codant pour les protéines référencées SEQ ID N° 2, 4, 8, 9, 17 et 24, et les séquences d'ADN et/ou ARN (par exemple SEQ ID N° 30 à 57) codant pour tout ou partie des protéines appartenant à une même famille de protéines choisies parmi le perlecan, le précurseur de la protéine plasmatique de liaison au rétinol, de la protéine activatrice du GM2, de la calgranuline B et de la saposine B, ledit gène d'intérêt thérapeutique codant pour tout ou partie de la protéine d'intérêt, d'un fragment de la protéine d'intérêt ou d'un anticorps spécifique de la protéine d'intérêt qui sera exprimé à la surface de ladite cellule de mammifère (Toes et al., 1997,PNAS 94 : 14660-14665). La composition pharmaceutique peut contenir un agent thérapeutique seul dirigé contre une cible seule ou des agents pris en combinaison dirigés contre plusieurs cibles.

On désigne par « polypeptides et/ou protéines d'intérêt de l'invention » le fragment C-terminal du perlecan (SEQ ID N° 2), le précurseur de la protéine plasmatique de liaison au rétinol (SEQ ID N°4), la protéine activateur du GM2 (SEQ ID N° 8), la protéine mutée de l'activateur du GM2 (SEQ ID N° 9), la calgranuline B (SEQ ID N° 17), la saposine B (SEQ ID N° 24), les protéines ou fragments appartenant à la famille du précurseur de la protéine plasmatique de liaison au rétinol (par exemple SEQ ID N° 5 à 7), les protéines ou fragments appartenant à la famille de la protéine activateur du GM2 (par exemple SEQ ID N° 10 à 16), les protéines ou fragments appartenant à la famille de la protéine calgranuline B (par exemple SEQ ID N° 18 à 23), les protéines ou fragments appartenant à la famille de la protéine saposine B (par exemple SEQ ID N° 25 à 29 et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29.

A partir des connaissances des séquences en acides aminés des protéines d'intérêt identifiées dans la présente invention, il est à la portée de l'homme de l'art de définir et utiliser les molécules décrites ci dessus et/ou toute molécule capable de se fixer au dites molécules, et/ou toute molécule capable d'inhiber lesdites molécules. Ainsi la présente invention concerne l'utilisation de protéines naturelles et/ou recombinantes et/ou de polypeptides de synthèse et leurs fragments, de ligand capables de se fixer au dites protéines ou à leur(s) fragment(s), par exemple des anticorps ; de protéines inhibitrices de la fonction et/ou de l'expression et/ou de la fixation desdites protéines.

Utilisation de protéine(s) et/ou peptide(s) naturel(s) et/ou de protéine(s) recombinante(s) et/ou de polypeptide(s) de synthèse correspondant aux protéines d'intérêt identifiées dans la présente invention.

La présente invention concerne un matériel biologique pour la préparation de compositions pharmaceutiques destinées au traitement de mammifères atteint de maladie auto-immune, de préférence la sclérose en plaques, comprenant :
(i) soit au moins une protéine naturelle et/ou une protéine recombinante et/ou un polypeptide de synthèse choisi parmi les protéines dont les séquences en acides aminés sont référencées SEQ ID N° 2, 4, 8, 9, 17 et 24, et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29), et les séquences peptidiques qui présentent au moins 70 % d'identité de préférence au moins 80 % d'identité et avantageusement au moins 98 d'identité avec l'une quelconque des séquences peptidiques SEQ ID N° 1 à 29, seules ou en combinaison,
(ii) soit au moins un fragment naturel et/ou synthétique de ces protéines d'intérêt, par exemple un fragment immunogène capable d'induire une réponse immune contre un polypeptide cible,
(iii) soit au moins un peptide mimotope défini à partir des séquences de référence SEQ ID N° 2, 4, 8, 9, 17 et 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29), et les séquences peptidiques qui présentent au moins 70 % d'identité de préférence au moins 80 % d'identité et avantageusement au moins 98 d'identité avec l'une quelconque des séquences peptidiques SEQ ID N° 1 à 29, ou une combinaison de mimotopes, capable d'induire une réponse immune contre le polypeptide cible,
(iv) soit au moins toute protéine ou peptide pouvant réguler *in vivo* la transcription et/ou la traduction des protéines d'intérêt (SEQ ID N° 2, 4, 8, 9, 17 et 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 d'identité avec l'une quelconque des séquences peptidiques SEQ ID N° 1 à 29. L'administration de ces protéines et/ou peptides seuls ou en combinaison peut rétablir la concentration d'une protéine d'intérêt dans l'organisme.

La réponse immune dirigée contre un antigène spécifique peut être divisée en deux catégories distinctes, l'une mettant en jeu les anticorps (réponse immune de type humorale), l'autre les cellules effectrices cytotoxiques telles que par exemple les macrophages, les lymphocytes cytotoxiques (CTL) ou les cellules tueuses (NK) ainsi que les lymphocytes T « helper », notamment les lymphocytes T CD4+ (réponse immune de type cellulaire). Plus particulièrement, les deux types de réponse se distinguent en ce que les anticorps reconnaissent les antigènes sous leur forme tridimensionnelle alors que les lymphocytes T, par exemple, reconnaissent des portions peptidiques desdits antigènes, associés à des glycoprotéines codées par les gènes du complexe majeur d'histocompatibilité (CMH), notamment les gènes du complexe majeur d'histocompatibilité de type I qui sont exprimés de façon ubiquitaire à la surface des cellules ou les gènes du complexe majeur d'histocompatibilité de type II qui sont exprimés de façon spécifique à la surface des cellules impliquées dans la présentation des antigènes (APC). 1) Selon un premier aspect, la réponse immune de type cellulaire est caractérisée en ce que les cellules T de type CD4+ (cellules T helper), suite à un phénomène d'activation bien connu (pour une revue voir Alberola-lia 1997, Annu Rev Immunol 15, 125-154) produisent des cytokines qui à leur tour induisent la prolifération de cellules APC capables de produire lesdites cytokines, la différenciation cellulaire des lymphocytes B capables de produire des anticorps spécifiques de l'antigène, et la stimulation des lymphocytes T cytotoxiques (CTL). 2) Selon un second aspect de la réponse immune cellulaire, les cellules effectrices cytotoxiques telles que par exemple les lymphocytes de type CD8+ (CTL) sont activés a) après interaction avec des peptides antigéniques fixés sur et présentés par les glycoprotéines portées par les cellules ubiquitaires et codées par les gènes appartenant au système CMHI, et b) éventuellement par les cytokines produites par les CD4+.

La présente invention concerne l'administration d'une protéine ou d'un peptide dérivés des protéines d'intérêt (SEQ ID N° 2, 4, 8, 9, 17 et 24) ou de leur(s) fragment(s), et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29), et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, seuls ou en combinaison, pour la prophylaxie et/ou la thérapie d'une maladie auto-immune, telle que la sclérose en plaques. Ces protéines et peptides administrés sont caractérisés en ce que ils doivent avoir perdu leur activité toxique, par exemple leur activité gliotoxique, ou avoir perdu leur capacité à se fixer à un ligand, et peuvent induire significativement une réponse immune médiée par les lymphocytes T ou/et les anticorps dirigée contre cette protéine sont utilisés. De telles protéines sont dites 'modifiées', cependant leur immunogénicité est conservée. De telles molécules immunogéniques modifiées sont obtenues par un nombre de traitements conventionnels, par exemple la dénaturation chimique ou à la chaleur, la troncation ou la mutation avec délétion, insertion ou emplacement d'acides aminés. Un exemple de troncation consiste en la troncation d'acides aminés à l'extrémité carboxy-terminale pouvant aller jusqu'à 5-30 acides aminés. Les molécules modifiées peuvent être obtenues par des techniques synthétiques ou/et recombinantes ou par des traitements chimiques ou physiques des molécules naturelles.

Les protéines d'intérêt naturelles et/ou recombinantes identifiées dans la présente invention (SEQ ID N° 2, 4, 8, 9, 17 et 25), et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29), et les séquences peptidiques qui présentent au moins 70 % d'identité de préférence au moins 80 % d'identité et avantageusement au moins 98 d'identité avec l'une quelconque des séquences peptidiques SEQ ID N° 1 à 29, ou leur(s) fragment(s), sont utilisées en vaccination prophylactique et thérapeutique contre les maladies auto-immunes, de préférence la SEP. Un vaccin comprend une quantité immunogénique effective de la protéine immunogène en association avec un véhicule pharmaceutiquement acceptable et éventuellement un adjuvant et/ou un diluant. Les véhicules, adjuvants et diluants pharmaceutiquement acceptables sont bien connus de l'homme du métier. On peut citer à titre de référence le Remington's Pharmaceutical Sciences. L'utilisation de compositions vaccinales est particulièrement avantageuse en association avec un diagnostic précoce de la maladie. La protéine immunogène est utilisée dans la préparation de médicament pour la vaccination prophylactique ou thérapeutique. Les protéines d'intérêt peuvent être éliminées de l'organisme sans induire d'effets secondaires indésirables. L'identification de telles protéines ou peptides vaccins est réalisée comme suit : les molécules candidates modifiées comme décrit précédemment (protéines naturelles, recombinantes, peptides) sont analysées dans un test fonctionnel pour vérifier qu'elles ont perdues leur toxicité, par exemple leur activité gliotoxique en utilisant le test appelé bio-essai, et pour vérifier leur immunogénicité (i) en réalisant un test *in vitro* de prolifération de lymphocytes T CD4+ spécifiques de l'antigène administré (T cell assay) ou un test *in vitro* de cytotoxicité des lymphocytes CD8+ spécifiques de l'antigène administré et (ii) en mesurant entre autre le taux d'anticorps circulants dirigés contre la protéine naturelle. Ces formes modifiées sont utilisées pour immuniser des hommes par des procédures standard avec des adjuvants appropriés.

Les vaccins préparés sont injectables, c'est-à-dire en solution liquide ou en suspension. En option, la préparation peut aussi être émulsifiée. La molécule antigénique peut être mélangée avec des excipients qui sont pharmaceutiquement acceptables et compatibles avec l'ingrédient actif. Des exemples d'excipients favorables sont l'eau, une solution saline, le dextrose, le glycérol, l'éthanol ou des équivalents et leurs combinaisons. Si désiré, le vaccin peut contenir des quantités mineures de substances auxiliaires comme des agents " wetting " ou émulsifiants, des agents qui tamponnent le pH ou des adjuvants comme l'hydroxide d'aluminium, le dipeptide muramyl ou leurs variations. Dans le cas des peptides, leur couplage à une plus grosse molécule (KLH, toxine tétanique) augmente quelquefois l'immunogénicité. Les vaccins sont administrés conventionnellement par injection par exemple sous cutanée ou intramusculaire. Des formulations additionnelles favorables avec d'autres modes d'administration incluent des suppositoires et quelquefois des formulations orales.

En général la concentration du polynucléotide dans la composition utilisée pour une administration *in vivo* est de 0.1 µg /ml jusqu'à 20 mg /ml. Le polynucléotide peut être homologue ou hétérologue de la cellule cible dans laquelle il va être introduit.

La présente invention concerne également l'utilisation de vaccins incluant des molécules d'acides nucléiques qui codent pour les protéines d'intérêt ou des peptides immunogènes ou leur fragment(s), non actifs, correspondant aux protéines d'intérêt (SEQ ID N° 2, 4, 8, 9, 17 et 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29. Les vaccins d'acides nucléiques, en particulier les vaccins ADN, sont administrés généralement en association avec un véhicule pharmaceutiquement acceptable en injection intramusculaire.

A partir de la séquence en acides aminés des protéines d'intérêt décrites (SEQ ID N° 2, 4, 8, 9, 17 et 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, des peptides ou des fragments correspondant à tout ou partie de la séquence primaire de ces protéines peuvent être synthétisés par des méthodes classiques de synthèse peptidique ou obtenus par recombinaison génétique.

Des protéines recombinantes correspondante aux protéines d'intérêt, produites dans un système cellulaire procaryote ou eucaryote, sont disponibles auprès de différentes équipes et sont décrites dans la littérature. Elles peuvent être également produite par l'homme du métier à partir de la connaissance des séquences des gènes correspondants décrits dans la littérature et en tenant compte de la dégénérescence du code génétique. Toutes les séquences protéiques identifiées dans la présente invention sont ainsi susceptibles d'être obtenues par recombinaison génétique. Les gènes sont clonés dans des vecteurs adaptés. Des vecteurs différents sont utilisés pour transformer des cellules procaryotes (par exemple *E*. *coli*) et des cellules eucaryotes (par exemple cellules COS, CHO et cellules Simliki). Les protéines recombinantes correspondant aux protéines d'intérêt ou à des fragments des protéines d'intérêt peuvent être ainsi produits dans des systèmes cellulaires procaryotes et/ou encaryotes. Dans les cellules *E*. *coli*, les protéines recombinantes sont produites avec une queue poly-histidine. La fraction protéique insoluble est solubilisée dans de l'urée 8M. L'enrichissement du produit a été effectué sur résine chélatée au nickel (Qiagen). La colonne a été lavée avec des concentrations décroissantes d'urée. L'élution a été faite avec de l'imidazole en l'absence d'urée. La séquence complète des protéines d'intérêt peut être également clonée dans un plasmide adapté puis transférée dans le virus de la vaccine pour obtenir un virus recombinant.

Utilisation de ligands capables de se fixer aux protéines d'intérêt identifiées dans la présente invention.

La présente invention concerne un matériel biologique pour la préparation de compositions pharmaceutiques destinées au traitement de mammifères atteint de maladie auto-immune, de préférence la sclérose en plaques, comprenant :
(i) soit au moins un ligand capable de se fixer aux protéines et/ou fragments des protéines choisies parmi les protéines cibles SEQ ID N° 2, 4, 8, 9, 14 et 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, le ligand étant capable ou non d'inhiber l'activité protéique,
(ii) soit au moins un anticorps polyclonal ou monoclonal capable de se fixer à au moins une protéine ou un de ses fragments choisie parmi les protéines cibles SEQ ID N° 2, 4, 8, 9, 14 et 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29. Cet anticorps peut être ou non neutralisant, c'est-à-dire capable ou non d'inhiber l'activité de la protéine d'intérêt. Le ligand peut être choisi parmi toute molécule ou fragment molécule capable de se fixer aux protéines cibles, par exemple les récepteurs de ce protéines, les cofacteurs de ces protéines, les anticorps polyclonaux ou monoclonaux capables de se fixer aux protéines ou tout fragment de ces protéines.

Ces anticorps sont très utiles notamment pour permettent la mise en oeuvre de compositions thérapeutiques car ils conduisent par exemple, à des réactions immunes, dirigées spécifiquement à l'encontre d'épitopes immunodominants ou contre des antigènes présentant une grande variabilité. On administre chez le patient soit des anticorps solubles neutralisants pour inhiber leur fonction, soit des anticorps solubles spécifiques pour éliminer le peptide par formation de complexes immuns. L'invention décrit l'utilisation d'anticorps capables de reconnaître spécifiquement au moins une protéine décrite dans la présente invention pour le traitement et /ou pour le suivi thérapeutique de maladie dégénérative et/ou neurologique et/ou auto-immune, de préférence la sclérose en plaques. Ces anticorps sont polyclonaux et de préférence monoclonaux. De préférence ces anticorps reconnaissent le site actif de la protéine et en se fixant, inhibe la fonction de la protéine. La capacité de l'anticorps à se fixer spécifiquement à la protéine est analysé par des techniques conventionnelle décrites, comme par exemple par des tests ELISA ou de Western blot en utilisant la protéine ou le peptide immunogène naturel ou synthétique. Le titre de l'anticorps est déterminé. La capacité de l'anticorps à neutraliser la fonction de la protéine peut être analysée par différents moyen, par exemple en déterminant la diminution de l'activité de la protéine ou du peptide immunogène en présence de l'anticorps, de préférence en déterminant la diminution de l'activité gliotoxique en utilisant le test bio-essai *in vitro.*

Par exemple, les anticorps monoclonaux dirigés contre la protéine cible ou une partie de cette protéine sont produits par des techniques conventionnelles utilisées pour produire des anticorps contre des antigènes de surface Des souris ou des lapins sont immunisées (i) soit avec la protéine naturelle ou recombinante d'intérêt, (ii) soit avec tout peptide immunogène de cette protéine d'intérêt, (iii) soit avec des cellules murines qui expriment la protéine ou le peptide d'intérêt et les molécules du CMHII. La lignée murine Balb/c est la plus fréquemment utilisée. L'immunogène est également un peptide choisi parmi les peptides définis à partir des séquences primaires des protéines d'intérêt. Par exemple, l'immunogène suivant a été préparé : les peptides SEQ ID N°s 58, 59, 60 issus de la séquence du précurseur du ganglioside GM2, les peptides SEQ ID N°s 61, 62 issus de la séquence de la saposine B et les peptides SEQ ID N°s 63, 64, 65 issus de la calgranuline B ont été couplé à de l'hémocyanine de Lymphet Keyhole, en abrégé peptide-KLH, comme support pour son utilisation en immunisation, ou couplé à de l'albumine de sérique humaine, en abrégé peptide-HSA. Les animaux ont été soumis à une injection de peptide-KLH ou de peptide-HSA en utilisant de l'adjuvant complet de Freund (IFA). Les sérums et les surnageants de culture d'hybridome issus des animaux immunisés avec chaque peptide ont été analysés pour la présence d'anticorps anti-protéines par un test ELISA utilisant les protéines initiales. Les cellules spléniques de ces souris ont par conséquent été récupérées et fusionnées avec des cellules de myélome. Le polyéthylèneglycol (PEG) est l'agent de fusion le plus fréquemment utilisé. Les hybridomes produisant les anticorps les plus spécifiques et les plus sensibles sont sélectionnés. Les anticorps monoclonaux peuvent être produits *in vitro* par culture cellulaire des hybridomes produits ou par récupération de liquide d'ascite murin après injection intrapéritonéale des hybridomes chez la souris. Quel que soit le mode de production en surnageant ou en ascite, il importe ensuite de purifier l'anticorps monoclonal. Les méthodes de purification utilisées sont essentiellement la filtration sur gel échangeur d'ions ou par chromatographie d'exclusion, voire l'immunoprécipitation. Pour chaque anticorps il faut choisir la méthode qui permettra d'obtenir le meilleur rendement. Un nombre suffisant d'anticorps anti-protéines sont criblés dans des tests fonctionnels pour identifier les anticorps les plus performants pour fixer la protéine d'intérêt et/ou pour bloquer l'activité de la protéine d'intérêt. Les anticorps monoclonaux sélectionnés sont humanisés par des méthodes standard de « CDR grafting » (protocole réalisé par de nombreuses compagnies, sous forme de service). Ces anticorps humanisés peuvent être testés cliniquement chez le patient. L'efficacité de ces anticorps peut être suivie par des paramètres cliniques.

La production *in vitro* d'anticorps, de fragments d'anticorps ou de dérivés d'anticorps, tels que les anticorps chimères, produits par génie génétique, dans des cellules eucaryotes a été décrite (EP 120 694 ou EP 125 023) et est aussi applicable à la présente invention.

Utilisation de molécules inhibitrices des protéines d'intérêt identifiées dans la présente invention.

La présente invention concerne un matériel biologique pour la préparation de compositions pharmaceutiques destinées au traitement de mammifères atteint de maladie dégénérative et/ou neurologique et/ou auto-immune, de préférence la sclérose en plaques, ladite composition comprenant (i) soit au moins une molécule inhibitrice de la fonction d'au moins une protéine choisie parmi les protéines identifiées dans la présente invention (SEQ ID N° 2, 4, 8, 9, 17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, par exemple inhibitrice de l'activité gliotoxique, (ii) soit au moins une molécule régulatrice de l'expression d'au moins une protéine choisie parmi les protéines SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, par exemple pour bloquer la transcription ou la traduction, (iii) soit au moins une molécule régulatrice du métabolisme d'au moins une protéine choisie parmi les protéines SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, (iv) soit au moins une molécule régulatrice de l'expression et/ou du métabolisme d'un ligand d'au moins une protéine choisie parmi les protéines SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à SEQ ID N° 8 et SEQ ID N° 10 à 29 et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, par exemple un récepteur ou un cofacteur. On peut penser que ces protéines de l'organisme humain peuvent être inhibées sans effet secondaire.

Un autre aspect important de l'invention concerne l'identification et l'évaluation de l'efficacité thérapeutique de substances naturelles et/ou synthétiques (i) capables de bloquer et/ou d'inhiber l'activité des protéines d'intérêt de l'invention et/ou de leur fragment : SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29 et/ou (ii) capables d'inhiber leur métabolisme tels les inhibiteurs du métabolisme correspondant, les inhibiteurs d'enzymes activées par les coenzymes, (iii) capables de réguler l'expression des protéines d'intérêt (SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, (iv) capables d'inhiber la fonction et/ou l'expression des ligands des protéines d'intérêt SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, comme par exemple des récepteurs. Ces substances peuvent être utilisées dans des traitements prophylactiques et thérapeutiques de la maladie. L'invention concerne également des méthodes pour traiter et prévenir une maladie auto-immune, par exemple la SEP, en administrant des quantités effectives de ces substances. Les substances peuvent être des protéines, des anticorps, de petites molécules synthétiques ou naturelles, des dérivés des protéines identifiées dans cette invention, des lipides, des glycolipides etc... Les petites molécules peuvent être criblées et identifiées en grande quantité en utilisant des librairies combinatoires chimiques. L'invention concerne également des compositions pharmaceutiques comprenant ces substances en association avec des carriers physiologiques acceptables, et des méthodes pour la préparation de médicaments à utiliser en thérapie ou en prévention de maladies auto-immunes dont la SEP en utilisant ces substances.

Pour identifier des molécules inhibitrices de faible poids moléculaire comme des drogues candidates pour les maladies dégénératives et/ou neurologiques et/ou auto-immunes, telles que la sclérose en plaques, on utilise les tests et protocoles décrits dans précédemment et dans les demandes de brevet incorporés à titre de référence, en utilisant des échantillons prélevés du patient non traité ou traité, du modèle animal non traité ou traité, ou de tissus du modèle animal non traité ou traité. Cet aspect de l'invention inclue également un procédé pour identifier des substances capables de bloquer ou d'inhiber l'activité des protéines d'intérêt, comprenant l'introduction de ces substances dans un test *in vitro* ou dans un modèle animal *in vivo.* Les molécules sélectionnées sont testées à différentes concentrations. Ces inhibiteurs sont aussi testés dans des essais de toxicité et pharmacocinétique pour savoir si ils peuvent représenter des drogues candidates valables. Les substances testées pour l'inhibition ou le blocage des activités protéiques ou de l'expression des protéines, dans ces procédures de criblage peuvent être des protéines, des anticorps, des fragments d'anticorps, de petites molécules synthétiques ou naturelles, des dérivés des protéines d'intérêt, etc ..... Les petites molécules peuvent être criblées et identifiées en grande quantité en utilisant des librairies combinatoires chimiques.

A titre d'exemple, on peut citer comme substances inhibitrices :

Les inhibiteurs des protéines identifiées dans la présente invention (SEQ ID N° 2, 4, 8, 9, 17, 24), les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, et les inhibiteurs des fragments desdites protéines. Ces inhibiteurs peuvent être compris dans une composition prophylactique et thérapeutique, en particulier pour le traitement de la sclérose en plaques. Par exemple, la lycorine, alcaloïde extrait de Amaryllidaceae (ex : Crinum Asiaticum) est utilisée *in vitro* à une concentration comprise entre 0.1 et 0.5 µg /ml et *in vivo* à une concentration comprise entre 0.1 et 1 mg / kg /jour. Par exemple, le Rolipram (nom commercial) et l'Ibudilast (nom commercial), qui sont deux molécules de la même famille des inhibiteurs des phosphodiestérases 4(PDE4) sont utilisées *in vitro* à des concentrations comprises entre 1 et 10 µM/1 et *in vivo* à des concentrations comprises entre environ 10 mg/kg/jour.

**→** A partir des séquences d'acides aminés des protéines SEQ ID N° 2,_4, 8, 9, 17, 24_et des séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29), il est évident que l'on peut déduire les séquences nucléotidiques ADN et ARN (SEQ ID N° 30, 31, 42, 53) correspondant aux protéines d'intérêt et les séquences codant pour les protéines de la famille de ces protéines d'intérêt ( par exemple SEQ ID N° 32 à 41, SEQ ID N° 43 à 52, SEQ ID N° 54 à 57, SEQ ID N° 66 à 67), en tenant compte du code génétique et de sa dégénérescence. Ainsi la présente invention concerne l'utilisation de ces séquences nucléotidiques sous forme :
- de séquences anti-sens,
- de séquences codant pour un gène thérapeutique,
- de séquences pouvant être contenue dans un vecteur pour la réalisation de transformation cellulaire ex vitro et/ou in vivo (thérapie génique).

Utilisation d'acides nucléiques déduits des séquences en acides aminés des protéines d'intérêt identifiées dans la présente invention ; acides nucléiques anti-sens et/ou codant pour un gène thérapeutique.

La présente invention concerne un matériel biologique pour la préparation de compositions pharmaceutiques destinées au traitement de mammifères atteint de maladie dégénérative et/ou neurologique et/ou auto-immune, en particulier la sclérose en plaques, la composition comprenant (i) soit au moins une séquence d'acide nucléique capable de s'hybrider à une séquence d'acides nucléiques codant pour les protéines d'intérêt (SEQ ID N° 2, 4, 8, 9, 17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, ou leur(s) fragment(s), (ii) soit au moins une séquence d'acide nucléique comprenant au moins un gène d'intérêt thérapeutique codant pour les protéines ou un fragment de protéines (SEQ ID N° 2, 4, 8, 9, 17, 24), les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, et des éléments assurant l'expression dudit gène *in vivo* dans des cellules cibles destinées à être génétiquement modifiées par ladite séquence nucléique.

Par séquence d'acide nucléique, on entend un fragment d'ADN et/ou d'ARN, double brin ou simple brin, linéaire ou circulaire, naturel et isolé ou de synthèse, désignant un enchaînement précis de nucléotides, modifiés ou non, permettant de définir un fragment ou une région d'un acide nucléique choisi dans le groupe consistant en un ADNc ; un ADN génomique ; un ADN plasmidique ; un ARN messager. Ces séquences d'acides nucléiques sont déduites de la séquence d'acides aminés des protéines SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, en utilisant le code génétique. En raison de la dégénérescence du code génétique l'invention englobe également des séquences équivalentes ou homologues. Ces séquences définies permettent à l'homme de l'art de définir lui-même les acides nucléiques adaptés.

Aussi, la présente invention concerne un matériel biologique pour la préparation de compositions pharmaceutiques comprenant au moins une séquence d'acide nucléique capable de s'hybrider à une séquence d'acides nucléiques codant pour les protéines d'intérêt ou leur(s) fragment(s) (SEQ ID N° 2, 4, 8, 9, 17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29.

L'invention consiste à définir et utiliser des molécules d'acides nucléiques complémentaires des séquences ADN et/ou ARN codant pour les protéines d'intérêt ou leur(s) fragment(s). Ces fragments correspondent à des molécules anti-sens ou ribozyme et peuvent être synthétisés à l'aide de synthétiseurs automatiques, tels que ceux commercialisés par la société Applied Biosystem. L'invention décrit l'utilisation de ces acides nucléiques capables de s'hybrider dans des conditions stringentes à l'ADN ou/et ARN codant pour les protéines de l'invention ou pour leu(s) fragment(s). Des conditions de stringence caractéristiques sont celles qui correspondent à une combinaison de la température et de la concentration saline choisie approximativement entre 12 à 20°C sous le Tm (« melting temperature ») de l'hybride à l'étude. De telles molécules sont synthétisées et peuvent être marquées en utilisant des méthodes de marquage conventionnelles utilisées pour les sondes moléculaires, ou peuvent être utilisées comme amorces dans les réactions d'amplification. Les séquences qui présentent au moins 90% d'homologie par rapport à une séquence de référence font également partie de l'invention, de même que les fragments de ces séquences qui présentent au moins 20 nucléotides et de préférence 30 nucléotides contigus homologues par rapport à une séquence de référence. Afin de réduire la proportion de peptides naturels ou variants, il est possible d'envisager une approche anti-sens et/ou ribozyme. Une telle approche est largement décrite dans la littérature. Bien entendu, de telles molécules anti-sens peuvent constituer en tant que telles des vecteurs. On peut également utiliser des vecteurs qui comprennent une séquence d'acides nucléique qui code pour un anti-sens.

La présente invention concerne un matériel biologique pour la préparation de compositions pharmaceutiques destinées au traitement de mammifères atteint de maladie dégénérative et/ou neurologique et/ou auto-immune, telle que la sclérose en plaques, ladite composition comprenant au moins une séquence d'acide nucléique contenant au moins un gène d'intérêt thérapeutique et des éléments assurant l'expression dudit gène *in vivo* dans des cellules cibles destinées à être génétiquement modifiées par ladite séquence nucléique.

Ces séquences d'acides nucléiques et/ou vecteurs (anti-sens ou codant pour une protéine ou un fragment d'une protéine) permettent de cibler les cellules dans lesquelles le peptide est exprimé, telles que les cellules macrophages : (i) soit par l'utilisation d'une molécule de ciblage introduite sur le vecteur, (ii) soit par l'utilisation d'une propriété particulière de ces cellules.

Utilisation de vecteurs comprenant un gène d'intérêt thérapeutique correspondant aux gènes des protéines d'intérêt identifiées dans la présente invention.

La présente invention concerne un matériel biologique pour la préparation de compositions pharmaceutiques destinées à la prévention et au traitement de maladies dégénérative et/ou neurologique et/ou auto-immune, telles que la sclérose en plaques, la composition comprenant une séquence d'acide nucléique comprenant un gène d'intérêt thérapeutique et des éléments d'expression dudit gène d'intérêt. Les gènes peuvent être non mutés ou mutés. Ils peuvent également consister en des acides nucléiques modifiés de sorte qu'il ne leur est pas possible de s'intégrer dans le génome de la cellule cible ou des acides nucléiques stabilisés à l'aide d'agents, tels que la spermine.

Un tel gène d'intérêt thérapeutique code notamment :
(i) soit au moins pour une protéine choisie parmi les protéines identifiées dans la présente invention (SEQ ID N° 2, 4, 8, 9, 17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, ou leur(s) fragment(s),
(ii) soit au moins pour tout ou partie d'un anticorps polyclonal ou monoclonal capable de se fixer à au moins une protéine ou un fragment de protéine choisi parmi les protéines identifiées dans la présente invention (SEQ ID N° 2, 4, 8, 9, 17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29. Il peut notamment s'agir d'anticorps transmembranaire natif, ou de fragment ou dérivé d'un tel anticorps, pour autant que ledit anticorps, fragment ou dérivé d'anticorps soit exprimé à la surface de la cellule cible du mammifère génétiquement modifiée et soit capable de se fixer à un polypeptide présent à la surface d'une cellule effectrice cytotoxique ou d'un lymphocyte T helper impliqué dans le procédé d'activation d'une telle cellule,
(iii) soit au moins pour une molécule inhibitrice d'au moins une protéine ou de ses fragments, ladite protéine étant choisie parmi les protéines identifiées (SEQ ID N° 2, 4, 8, 9, 17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29; les protéines inhibitrices de la fonction et/ou du métabolisme et/ou de la fixation des protéines SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29,
(iv) soit au moins pour un ligand ou toute partie d'un ligand capable de se fixer à au moins une protéine ou un fragment de protéine choisi parmi les protéines identifiées (SEQ ID N° 2, 4, 8, 9,17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, et/ou d'inhiber sa fonction.

Plus particulièrement, par fragment d'anticorps, on entend les fragments F(ab)2, Fab', Fab, sFv (Blazar et al., 1997, Journal of Immunology 159 : 5821-5833 ; Bird et al., 1988 Science 242 : 423-426) d'un anticorps natif et par dérivé on entend, par exemple, un dérivé chimérique d'un tel anticorps (voir par exemple les chimères des anticorps antiCD3 Souris/Homme dans Arakawa et al., 1996 J Biochem 120 : 657-662 ou les immunotoxines telles que sFv-toxine de Chaudary et al 1989, Nature 339 : 394-397). Par anticorps transmembranaire on entend un anticorps dont au moins la région fonctionnelle capable de reconnaître et de se fixer à son antigène spécifique est exprimée à la surface des cellules cibles pour permettre lesdites reconnaissance et fixation. Plus particulièrement, les anticorps selon la présente invention consistent en des polypeptides de fusion comprenant les amino acides définissant ladite région fonctionnelle et une séquence d'amino acides (polypeptide transmembranaire) permettant l'ancrage au sein de la double couche lipidique membranaire de la cellule cible ou à la surface externe de cette bi-couche. Les séquences nucléiques codant pour de nombreux polypeptides transmembranaires sont décrites dans la littérature. Selon un cas tout à fait avantageux, la séquence d'acide nucléique codant pour la chaîne lourde de l'anticorps est fusionnée avec la séquence d'acide nucléique codant pour undit polypeptide transmembranaire.

Par éléments assurant l'expression dudit gène *in vivo* on fait notamment référence aux éléments nécessaires pour assurer l'expression dudit gène après son transfert dans une cellule cible. Il s'agit notamment des séquences promotrices et/ou des séquences de régulation efficaces dans ladite cellule, et éventuellement les séquences requises pour permettre l'expression à la surface des cellules cibles dudit polypeptide. Le promoteur utilisé peut être un promoteur viral, ubiquitaire ou spécifique de tissu ou encore un promoteur synthétique. A titre d'exemple, on mentionnera les promoteurs tels que les promoteurs des virus RSV (Rous Sarcoma Virus), MPSV, SV40 (Simian Virus), CMV (Cytomegalovirus) ou du virus de la vaccine, les promoteurs du gène codant pour la créatine kinase musculaire, pour l'actine. Il est en outre possible de choisir une séquence promotrice spécifique d'un type cellulaire donné, ou activable dans des conditions définies. La littérature procure un grand nombre d'informations relatives à de telles séquences promotrices.

Par ailleurs, ledit acide nucléique peut comprendre au moins deux séquences, identiques ou différentes, présentant une activité de promoteur transcriptionnel et/ou au moins deux gènes, identiques ou différents, situés l'un par rapport à l'autre de manière contiguë, éloignée, dans le même sens ou dans le sens inverse, pour autant que la fonction de promoteur transcriptionnel ou la transcription desdits gènes ne soit pas affectée.

De même dans ce type de construction d'acide nucléique, il est possible d'introduire des séquences nucléiques « neutres » ou introns qui ne nuisent pas à la transcription et sont épissées avant l'étape de traduction. De telles séquences et leurs utilisations sont décrites dans la littérature (référence : demande de brevet PCT WO 94/29471).

Ledit acide nucléique peut également comprendre des séquences requises pour le transport intracellulaire, pour la réplication et/ou pour l'intégration, pour la transcription et/ou la traduction. De telles séquences sont bien connues de l'homme de l'art.

Par ailleurs, les acides nucléiques utilisables selon la présente invention peuvent également être des acides nucléiques modifiés de sorte qu'il ne leur est pas possible de s'intégrer dans le génome de la cellule cible ou des acides nucléiques stabilisés à l'aide d'agents, tels que par exemple la spermine, qui en tant que tels n'ont pas d'effet sur l'efficacité de la transfection.

Selon un mode de réalisation de l'invention, la séquence d'acide nucléique est une séquence d'ADN ou ARN nue, c'est à dire libre de tout composé facilitant son introduction dans les cellules (transfert de séquence d'acide nucléique). Toutefois, selon un second mode de réalisation de l'invention, afin de favoriser son introduction dans les cellules cibles et afin d'obtenir les cellules génétiquement modifiées de l'invention, cette séquence d'acide nucléique peut être sous la forme d'un « vecteur », et plus particulièrement sous la forme d'un vecteur viral, tel que par exemple un vecteur adénoviral, rétroviral, un vecteur dérivé d'un poxvirus, notamment dérivé du virus de la vaccine ou du Modified Virus Ankara (MVA) ou d'un vecteur non viral tel que, par exemple, un vecteur consistant en au moins une dite séquence d'acide nucléique complexée ou conjuguée à au moins une molécule ou substance porteuse sélectionnée parmi le groupe consistant en un amphiphile cationique, notamment un lipide cationique, un polymère cationique ou neutre, un composé polaire pratique notamment choisi parmi le propylène glycol, le polyéthylène glycol, le glycérol, l'éthanol, la 1-méthyl L-2-pyrrolidone ou leurs dérivés, et un composé polaire aprotique notamment choisi parmi le diméthylsulfoxide (DMSO), le diéthylsulfoxide, le di-n-propylsulfoxide, le diméthylsulfone, le sulfolane, la diméthylformamide, le diméthylacetamide, la tetraméthylurée, l'acétonitrile ou leurs dérivés. La littérature procure un nombre important d'exemples de tels vecteurs viraux et non viraux.

De tels vecteurs peuvent en outre et de préférence comprendre des éléments de ciblage pouvant permettre de diriger le transfert de séquence d'acide nucléique vers certains types cellulaires ou certains tissus particuliers tels que les cellules cytotoxiques et les cellules présentatrices de l'antigène). Ils peuvent également permettre de diriger le transfert d'une substance active vers certains compartiments intracellulaires préférés tel que le noyau, les mitochondries ou les peroxysomes, par exemple. Il peut en outre s'agir d'éléments facilitant la pénétration à l'intérieur de la cellule ou la lyse de compartiments intracellulaires. De tels éléments de ciblage sont largement décrits dans la littérature. Il peut par exemple s'agir de tout ou partie de lectines, de peptides, notamment le peptide JTS-1 (voir demande de brevet PCT WO 94/40958), d'oligonucléotides, de lipides, d'hormones, de vitamines, d'antigènes, d'anticorps, de ligands spécifiques de récepteurs membranaires, de ligands susceptibles de réagir avec un anti-ligand, de peptides fusogènes, de peptides de localisation nucléaire, ou d'une composition de tels composés.

Utilisation de cellules transformées *in vivo* après injection de vecteurs contenant au moins un gène d'intérêt thérapeutique défini à partir des protéines d'intérêt identifiées dans la présente invention (SEQ ID N° 2, 4, 8, 9, 17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29.

La présente invention concerne un matériel biologique pour la préparation de compositions pharmaceutiques destinée à la prévention et au traitement de mammifères atteint de maladies dégénérative et/ou neurologique et/ou auto-immune, de préférence la sclérose en plaques, la composition comprenant au moins un vecteur contenant un gène thérapeutique comme décrit ci-dessous, capable d'être introduit dans une cellule cible *in vivo* et d'exprimer le gène d'intérêt thérapeutique *in vivo*. L'avantage de cette invention repose sur la possibilité de maintenir sur le long terme un niveau basal de molécules exprimées dans le patient traité. Des vecteurs ou acides nucléiques codant pour des gènes d'intérêt thérapeutique sont injectés. Ces vecteurs et acides nucléiques doivent être transportés jusqu'aux cellules cibles et transfecter ces cellules dans lesquelles ils doivent être exprimés *in vivo*.

L'invention concerne l'expression *in vivo* de séquences nucléotidiques et/ou de vecteurs tels que désignés dans le paragraphe précédent, c'est-à-dire des séquences correspondant à des gènes d'intérêt thérapeutique codant notamment :
(i) soit au moins pour une protéine choisie parmi les protéines identifiées dans la présente invention (SEQ ID N° 2, 4, 8, 9, 17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, ou leur(s) fragment(s),

(i) soit au moins pour tout ou partie d'un anticorps polyclonal ou monoclonal capable de se fixer à au moins une protéine choisie parmi les protéines identifiées dans la présente invention (SEQ ID N° 2, 4, 8, 9, 17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29. Il peut s'agir d'anticorps transmembranaire natif, ou de fragment ou dérivé d'un tel anticorps, pour autant que ledit anticorps, fragment ou dérivé d'anticorps soit exprimé à la surface de la cellule cible de mammifère génétiquement modifiée et en ce que ledit anticorps est capable de se fixer à un polypeptide présent à la surface d'une cellule effectrice cytotoxique ou d'un lymphocyte T helper et impliqué dans le procédé d'activation d'une telle cellule. Il peut s'agir de fragments d'anticorps exprimés par des cellules capables de sécréter lesdits anticorps dans la circulation sanguine d'un mammifère ou patient porteur des cellules génétiquement modifiées par le gène codant pour l'anticorps,
(ii) soit au moins pour une molécule inhibitrice d'au moins une protéine choisie parmi les protéines identifiées (SEQ ID N° 2, 4, 8, 9, 17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29; protéine inhibitrice de la fonction et/ou du métabolisme et/ou de la fixation des protéines SEQ ID N° 2, 4, 8,9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29,
(iii) soit au moins pour un ligand ou toute partie du ligand capable de se fixer sur au moins une protéine choisie parmi les protéines identifiées (SEQ ID N° 2, 4, 8, 9, 17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, et/ou d'inhiber sa fonction.

Selon un mode de réalisation particulier, il s'agit d'utiliser la thérapie génique de manière à diriger la réponse immune contre la protéine, le peptide ou la molécule d'intérêt cible, c'est-à-dire contre toute protéine choisie parmi les protéines SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, leur(s) fragment(s) et/ou contre toute molécule inhibitrice de la fonction et/ou de l'expression et/ou du métabolisme desdites protéines d'intérêt, et/ou des ligands desdites protéines comme par exemple les récepteurs. Pour cela il est évident que les cellules à cibler pour la transformation avec un vecteur sont des cellules appartenant au système immun, soit des cellules de type lymphocytes (CD4/CD8), soit des cellules présentatrices de l'antigène (cellules dendritiques, macrophages, ...).

Selon un mode de réalisation particulier, on modifie génétiquement, notamment *in vivo*, les cellules présentatrices de l'antigène (CPA). Les CPA comme les macrophages, les cellules dendritiques, les microgliocytes, les astrocytes jouent un rôle dans l'initiation de la réponse immune. Elles sont les premiers composants cellulaires qui capturent l'antigène, l'apprête dans la cellule et expriment des molécules du CMHI et CMHII transmembranaires impliquées dans la présentation de l'immunogène aux cellules T CD4+ et CD8+, elles produisent des protéines accessoires spécifiques qui participent à l'activation des cellules T (Debrick et al ;, 1991, J. Immunol 147 : 2846 ; Reis et al., 1993, J Ep Med 178 : 509 ; Kovacsovics-bankowski et al., 1993, PNAS 90 : 4942; Kovacsovics-bankowski et al., 1995 Science 267 : 243 ; Svensson et al., 1997, J Immunol 158 : 4229 ; Norbury et al ;, 1997, Eur J Immunol 27 : 280). Pour une vaccination, il peut être avantageux de disposer d'un système de thérapie génique qui peut cibler le transfert de gène dans de telles cellules APC, c'est-à-dire un gène qui code pour un polypeptide qui peut, après sa production intracellulaire et son « processing », être présenté aux cellules CD8+ et/ou CD4+ par les molécules des complexes CMHI et CMHII respectivement à la surface de ces cellules.

On choisit d'exprimer à la surface des cellules CPA *in vivo* tout ou partie d'un anticorps et/ou d'un ligand comme par exemple un récepteur, capable de réagir avec la protéine ou le peptide cible choisis parmi les protéines SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29. De telles cellules vont alors spécifiquement phagocyter ladite protéine ou ledit peptide, le « processer » de façon à ce que des fragments de ce peptide soient présentés à la surface des cellules présentatrices de l'antigène.

La littérature propose un grand nombre d'exemples de gènes codant pour des anticorps capables de réagir avec des polypeptides ou récepteurs. Il est à la protée de l'homme de l'art d'obtenir les séquences d'acides nucléiques codant pour de tels anticorps. Citons par exemple les gènes codant pour les chaînes légère et lourde de l'anticorps YTH 12.5 (anti-CD3) (Routledge et al. 1991, Eur J Immunol 21 : 2717-2725), de l'anti-CD3 selon Arakawa et al ; 1996, J. Biochem. 120 : 657-662. Les séquences d'acide nucléique de tels anticorps sont aisément identifiables à partir des bases de données communément utilisées par l'homme du métier. Il est également possible à partir d'hybridomes disponibles auprès de l'ATCC de cloner les séquences d'acides nucléiques codant pour les chaînes lourdes et/ou légères de ces différents anticorps par les méthodes d'amplification telles que la RT-PCR à l'aide d'oligonucléotides spécifiques ou les techniques mettant en oeuvre des banques d'ADNc (Maniatis et al ., 1982, Molecular cloning. A laboratory manual CSH Laboratory, Cold Spring Harbor, New York). Les séquences ainsi clonées sont alors disponibles pour leur clonage dans des vecteurs. Selon un cas préféré de l'invention, la séquence d'acide nucléique codant pour la chaîne lourde de l'anticorps est fusionnée par recombinaison homologue avec la séquence d'acide nucléique codant pour un polypeptide transmembranaire tel que la glycoprotéine rabique ou la gp160 (Polydefkis et al., 1990, J Exp Med 171 : 875-887). Ces techniques de biologie moléculaire ont été parfaitement bien décrites.

On choisit d'exprimer à la surface des cellules CPA *in vivo* des fragments immunogènes correspondant à au moins une protéines choisie parmi les protéines SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29. Pour cela, on peut choisir de faire exprimer par le vecteur soit le polypeptide complet soit de manière préféré des polypeptides sélectionnés pour réagir avec des ligands et/ou récepteurs spécifiques. Le peptide immunogène codé par le polynucléotide introduit dans la cellule du vertébré *in vivo* peut être produit et/ou sécrété, apprêté puis présenté à une cellule présentatrice de l'antigène (APC) dans le contexte des molécules du CMH. Les APC ainsi transférées *in vivo* induisent une réponse immune dirigée contre l'immunogène exprimé *in vivo*. Les APC possèdent différents mécanismes pour capturer les antigènes : (a) la capture des antigènes par des récepteurs membranaires comme les récepteurs aux immunoglobulines (Fc) ou pour le complément disponibles à la surface des granulocytes, des monocytes ou macrophages permettant une délivrance efficace de l'antigène dans les compartiments intracellulaires après phagocytose médiée par les récepteurs. (b) l'entrée dans les APC par pinocytose en phase fluide, impliquant différents mécanismes : la micropinocytose c'est-à-dire la capture de petites vésicules (0.1 µm) par les puits recouverts de clathrine et la macropinocytose c'est-à-dire la capture de plus grosses vésicules (avec une taille variant ente 0.5 µm et environ 6 µm) (Sallusto et al. 1995, J Exp Med 182 : 389-400). Tandis que la micropinocytose existe de façon constitutive dans toutes les cellules, la macropinocytose est limitée à des types cellulaires, comme par exemple les macrophages, les cellules dendritiques, les astrocytes, les cellules épithéliales stimulées par des facteurs de croissance (Racoosin et al., J Cell Sci 1992, 102 : 867-880). Dans cette invention, on entend par cellules capables de macropinocytose, les cellules qui peuvent réaliser les événements décrits ci-dessus et les cellules qui peuvent capturer des macromolécules de préférence entre 0.5 µm et environ 6 µm dans le cytoplasme.

Selon un mode de réalisation particulier, on modifie génétiquement notamment *in vivo*, les cellules effectrices cytotoxiques ou les lymphocytes T helper de façon à ce qu'elles expriment à leur surface un polypeptide correspondant aux protéines SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, à des ligands desdites protéines, naturellement non exprimés par ces cellules, et capables d'induire le procédé d'activation de telles cellules, par l'introduction dans ces cellules de séquences d'acide nucléique renfermant le gène codant pour un tel polypeptide. Conformément à la présente invention, il est également possible de sélectionner une séquence d'acide nucléique contenant un gène d'intérêt thérapeutique codant pour tout ou partie d'un anticorps dirigé contre une protéine choisie parmi les protéines SEQ ID N° 2, 4, 8, 9, 17, 24 et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, susceptible d'être exprimé à la surface des cellules cibles du patient à traiter, ledit anticorps étant capable de se fixer à un polypeptide naturellement non exprimé par ces cellules effectrices cytotoxiques ou lymphocytes T helper.

Par cellules effectrices cytotoxiques, on entend désigner les macrophages, les astrocytes, les lymphocytes T cytotoxiques (TCL) et les cellules tueuses (NK) ainsi que leurs dérivés telles que par exemple les LAK (Versteeg 1992 Immunology today 13 : 244-247 ;Brittende et al 1996, Cancer 77 :1226-1243). Par 'lymphocytes T helper' on entend désigner notamment les CD4 qui permettent après activation la sécrétion de facteurs d'activation des cellules effectrices de la réponse immune. Les polypeptides et notamment les récepteurs exprimés à la surface de ces cellules et qui sont impliqués dans l'activation de telles cellules consistent notamment en tout ou partie du complexe TCR ou le CD3, tout ou partie des complexes CD8, CD4, CD28, LFA-1, 4-1BB (Melero et al., 1998, Eur J Immunol 28 : 1116-1121), CD47, CD2, CD1, CD9, CD45, CD30, CD40, tout ou partie des récepteurs de cytokines (Finke et al., 1998, Gene therapy 5 : 31-39), telles que IL-7, IL-4, IL-2, IL-15 ou GM-CSF, tout ou partie du complexe récepteur des cellules NK tel que par exemple NKAR, Nkp46, .. ; (Kawano et al., 1998 Immunology 95 :5690-5693 ; Pessino et al., 1998 J Exp Med188 :953-960), Nkp44, tout ou partie des récepteurs de macrophages tels que par exemple le récepteur Fc (Deo et al., 1997, Immunology Today 18 : 127-135).

De nombreux outils ont été développés pour introduire différents gènes hétérologues et/ou vecteurs dans des cellules, en particulier des cellules de mammifères. Ces techniques peuvent être divisées en deux catégories : la première catégorie implique des technique physiques comme la micro-injection, l'électroporation ou le bombardement de particules. La seconde catégorie est basée sur l'utilisation de techniques en biologie moléculaire et cellulaire avec lesquelles le gène est transféré avec un vecteur biologique ou synthétique qui facilite l'introduction du matériel dans la cellule in vivo. Aujourd'hui, les vecteurs les plus efficaces sont les vecteurs viraux en particulier les adénoviraux et rétroviraux. Ces virus possèdent des propriétés naturelles pour traverser les membranes plasmiques, éviter la dégradation de leur matériel génétique et introduire leur génome dans le noyau de la cellule. Ces virus ont été largement étudiés et certains sont déjà utilisés expérimentalement dans des applications humaines en vaccination, en immunothérapie, ou pour compenser des déficiences génétiques. Cependant cette approche virale a des limitations notamment due à la capacité de clonage restreinte dans ces génomes viraux, le risque de disséminer les particules virales produites dans l'organisme et l'environnement, le risque de mutagenèse artéfactuelle par insertion dans la cellule hôte dans le cas des rétrovirus, et la possibilité d'induire une forte réponse immune inflammatoire in vivo pendant le traitement, ce qui limite le nombre d'injections possibles (Mc Coy et al. 11995, Human Gene Therapy 6 : 1553-1560 ; Yang et al., 1996 Immunity 1 : 433-422). D'autres systèmes alternatifs à ces vecteurs viraux existent. L'utilisation de méthodes non virales comme par exemple la co-précipitation avec le phosphate de calcium, l'utilisation de récepteurs qui miment les systèmes viraux (pour un résumé voir Cotten et Wagner 1993, Current Opinion in Biotechnology, 4 : 705-710), ou l'utilisation de polymères comme les polyamidoamines (Haensler et Szoka 1993, Bioconjugate Chem 4 : 372-379). D'autres techniques non virales sont basées sur l'utilisation de liposomes dont l'efficacité pour l'introduction de macromolécules biologiques comme l'ADN, l'ARN des protéines ou des substances pharmaceutiques actives a été largement décrite dans la littérature scientifique. Dans ce domaine des équipes ont proposé l'utilisation de lipides cationiques ayant une forte affinité pour les membranes cellulaires et/ou les acides nucléiques. En fait, il a été montré qu'une molécule d'acide nucléique elle-même pouvait traverser la membrane plasmique de certaines cellules *in vivo* (WO 90/11092), l'efficacité étant dépendante en particulier de la nature polyanionique de l'acide nucléique. Dès 1989 (Felgner et al., Nature 337 : 387-388) les lipides cationiques ont été proposés pour faciliter l'introduction de larges molécules anioniques, ce qui neutralise les charges négatives de ces molécules et favorise leur introduction dans les cellules. Différentes équipes ont développés de tels lipides cationiques : le DOTMA ( Felgner et al., 1987, PNAS 84 : 7413-7417), le DOGS ou Transfectam^{™} (Behr et al., 1989, PNAS 86 : 6982-6986), le DMRIE et le DORIE (Felgner et al., 1993 methods 5 : 67-75), le DC-CHOL (Gao et Huang 1991, BBRC 179 : 280-285), le DOTAP^{™} (McLachlan et al., 1995, Gene therapy 2 : 674-622) ou la Lipofectamine^{™}, et les autres molécules décrites dans les brevets WO9116024, WO9514651, WO9405624. D'autres groupes ont développés des polymères cationiques qui facilitent le transfert de macromolécules en particulier des macromolécules anioniques dans les cellules. Le brevet WO95/24221 décrit l'utilisation de polymères dendritiques, le document WO96/02655 décrit l'utilisation du polyéthylèneimine ou polypropylèneimine et les document US-A-5595897 et FR 2719316, l'utilisation des conjugués polylysine.

Etant donné que l'on souhaite obtenir *in vivo* une transformation ciblée vers un type cellulaire donné, il est évident que le vecteur utilisé doit pouvoir être lui-même « ciblé », comme décrit ci dessus.

Utilisation de cellules transformées *in vitro* ou *ex vivo* avec des vecteurs contenant un gène d'intérêt thérapeutique défini par rapport aux protéines d'intérêt identifiées dans la présente invention (SEQ ID N° 2, 4, 8, 9, 17, 24) et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29.

La présente invention concerne un matériel biologique pour la préparation de compositions pharmaceutiques destinée à la prévention et au traitement de maladies dégénérative et/ou neurologique et/ou auto-immune, de préférence la sclérose en plaques, la composition comprenant au moins une cellule, notamment une cellule ne produisant pas naturellement des anticorps, sous une forme permettant leur administration dans l'organisme d'un mammifère, humain ou animal, ainsi qu'éventuellement leur culture préalable, ladite cellule étant génétiquement modifiée *in vitro* par au moins une séquence d'acide nucléique contenant au moins un gène codant *in vivo* pour :
(i) au moins une protéine choisie parmi les protéines SEQ ID N° 2, 4, 8, 9, 17, 24, et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29 et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, et tout fragment
(ii) au moins un peptide défini à partir de la séquence primaire d'au moins une protéine choisie parmi les protéines SEQ ID N° 2, 4, 8, 9, 17, 24, et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29,
(iii) au moins toute molécule inhibitrice de la fonction et/ou de la fixation et/ou de l'expression de ces protéines,
(iv) au moins un peptide issu de la séquence primaire d'une protéine choisie parmi les protéines SEQ ID N° 2, 4, 8, 9, 17, 24, et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29, et capable de se fixer sur au moins une glycoprotéine du CMHI,
(v) au moins tout anticorps et toute partie d'anticorps capables de se fixer à au moins une protéine choisie parmi les protéines SEQ ID N° 2, 4, 8, 9, 17, 24, et les séquences peptidiques ou les fragments desdites séquences appartenant à une même famille de protéines choisies parmi le perlecan, le précurseur de la protéine plasmatique de liaison au rétinol, de la protéine activatrice du ganglioside GM2, de la calgranuline B et de la saposine B (par exemple SEQ ID N°1, SEQ ID N° 3, SEQ ID N° 5 à 7, SEQ ID N° 10 à 16, SEQ ID N° 18 à 23, SEQ ID N° 25 à 29) et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29.

Plus particulièrement, ladite cellule cible provient soit du mammifère à traiter, soit d'un autre mammifère que celui à traiter. Dans ce dernier cas, il convient de noter que ladite cellule cible aura subi un traitement la rendant compatible avec le mammifère à traiter. Par « mammifère » on entend, de préférence, un mammifère humain. Ces cellules sont établies en lignées cellulaires et sont préférentiellement CMHII+ ou CMHII+-inductibles comme les lymphocytes, les monocytes, les astrocytes, les oligodendrocytes.

L'invention concerne également les cellules modifiées et un procédé de préparation d'une cellule telle que décrite ci dessus caractérisé en ce que l'on introduit dans une cellule de mammifère ne produisant pas naturellement d'anticorps, par tout moyen approprié, au moins une séquence d'acide nucléique contenant au moins un gène d'intérêt thérapeutique et des éléments assurant l'expression dudit gène dans ladite cellule, ledit gène d'intérêt thérapeutique contenant une séquence d'acide nucléique codant pour une molécule ou un fragment de molécule *in vivo*, comme décrit juste ci-dessus. Plus particulièrement, elle concerne des cellules procaryotes , des cellules de levure et des cellules animales, en particulier des cellules de mammifères transformées par au moins une séquence nucléotidique et/ou un vecteur tel que décrit précédemment.

Selon un mode de réalisation particulier, les cellules (cellules dendritiques, macrophages, astrocytes, lymphocytes T CD4+, lymphocytes T CD8+, .......) du patient ou allogéniques sont placées en contact d'une préparation purifiée du polypeptide cible, celui-ci étant internalisé, apprêté et présenté à la surface cellulaire associé aux molécules du CMHI et/ou CMHII et ainsi induire une réponse immune spécifique contre le peptide. Les cellules « activées » sont ensuite administrées au patient chez lequel elles vont induire une réponse immune spécifique des antigènes (on utilise une voie naturelle de la réponse immune, mais on contrôle ce que la cellule présentatrice de l'antigène va présenter)

Selon un mode de réalisation particulier, les cellules présentatrices d'antigène (cellule dendritique, macrophage, astrocytes,..) sont modifiées *in vitro* pour exprimer les antigènes dans la cellule transformée qui vont s'associer aux molécules du CMHI et/ou CMHII et être présentées à la surface des cellules pour induire chez le patient chez lequel on administre la cellule modifiée une réaction immune parfaitement ciblée.

Toutes les approches vaccinales ne sont pas toujours satisfaisantes et conduisent par exemple à des réactions immunes limitées dirigées uniquement à l'encontre d'épitopes immunodominants ou contre des antigènes présentant une grande variabilité. De même la présentation incorrecte des antigènes par les glycoprotéines du système CMH à la surface des cellules, ne permet pas de développer chez le patient traité une immunité anti-protéine d'intérêt convenable. Afin de pallier ces problèmes, certains auteurs ont proposé dans le cadre de tels procédés vaccinaux, de sélectionner les fragments minimaux antigéniques correspondant aux portions de peptide susceptibles d'être reconnus spécifiquement par les lymphocytes T cytotoxiques, de les exprimer dans les cellules afin qu'ils s'associent aux molécules du CMHI et soient présentés à la surface des cellules pour induire chez le patient traité une réaction immunitaire parfaitement ciblée (Toes et al. 1997, PNAS 94 : 14660-14665). Plus particulièrement, il a été montré que des épitopes de très petites tailles (variant de 7 à environ 13 acides aminés) qui sont exprimés à partir de minigènes introduits dans un virus de la vaccine, pouvaient induire une immunisation de type cellulaire. Il a par ailleurs été montré que plusieurs minigènes pouvaient être exprimés conjointement à partir d'un même vecteur (cette construction particulière est appelée « string of beads »). Une telle construction présente l'avantage d'induire une réaction immune de type CTL synergique (Whitton et al ;, 1993 J. of Virology 67 : 348-352).

Protocole de mise en contact des cellules et du fragment antigénique :

La présentation des fragments antigéniques par les molécules CMHI repose sur un procédé intracellulaire identifié (voir Groettrup et al., 1996 Immunology Today 17 : 429-435 pour une revue) au cours duquel des peptides antigéniques de très courtes tailles (environ 7-13 acides aminés) sont produits par dégradation d'un polypeptide plus complexe contre lequel la réaction immune finale sera dirigée. Ces courts peptides sont ensuite associés aux molécules du CMHI ou du CMHII pour former un complexe protéique qui est transporté à la surface cellulaire afin de présenter lesdits peptides aux lymphocytes T cytotoxiques circulants ou aux lymphocytes T helper circulants, respectivement. Il convient en outre de noter que la spécificité des molécules CMH I ou CMH II vis-à-vis des peptides antigéniques varie en fonction des molécules CMH I ou CMH II (exemple pour le CMHI : HLA-A, HLA-B, ...) et de l'allèle (exemple pour le CMH I : HLA-A2, HLA-A3, HLA-A11) considérés. Au sein d'une même espèce animale, d'un individu à l'autre, il existe une grande variabilité des gènes codant pour les molécules du système CMH (à ce sujet, voir notamment George et al., 1995, Immunology Today 16 : 209-212).

Selon un mode de réalisation particulier, les cellules, telles que les cellules dendritiques, les macrophages, les astrocytes, les lymphocytes T CD4+, les lymphocytes T CD8+, sont modifiées de manière à exprimer à leur surface des anticorps spécifiques du peptide ciblé. Le peptide est neutralisé par les anticorps exprimés à la surface des cellules. Ces cellules sont de préférence immunes, de préférence du patient, de préférence cytotoxiques, modifiées pour exprimer tout ou partie d'un anticorps spécifique du polypeptide cible.

Isolement de cellules mononucléées à partir de sang périphérique :

En 1968, Boyum décrivit une technique rapide qui permet par centrifugation du sang sur gradient de densité, de séparer les cellules mononucléées (lymphocytes et monocytes) avec un bon rendement (rendement théorique 50 %, c'est-à-dire 10⁶ cellules /ml de sang). 50 ml de sang périphérique prélevés stérilement dans des tubes héparinés sont centrifugés 20 minutes à 150g à 20°C. Les cellules récupérées sont diluées dans deux volumes de sang périphérique initial de PBS stérile. 10 ml de cette suspension sont déposés sur 3ml d'une solution de Ficoll-Hypaque (milieu de séparation des lymphocytes, Flow). Après centrifugation pendant 20 minutes à 400g et 20°C sans freinage de décélération , les cellules mononucléées sédimentent à l'interface PBS-Ficoll, en une couche dense, opalescente, alors que la quasi-totalité des globules rouges et des polynucléaires sédimentent au fond du tube. Les cellules mononucléées sont récupérées et lavées en PBS stérile.

Internalisation des antigènes par les cellules présentatrices de l'antigène :

Traitement préalable des cellules présentatrices de l'antigène : les cellules présentatrices de l'antigène sont préalablement lavées avec un tampon PBS-BSA à 0.5% (p/v) puis énumérées puis elles sont préincubées en présence de différents inhibiteurs de réduction trois fois en PBS-BSA 0.5% contenant de 10 µM à 10 mM final de DTNB (acide 5,5'-dithio-bis-2-nitrobenzoïque) ou de NEM (N-éthylmaléimide). Les étapes ultérieures de fixation d'antigènes à la surface cellulaire ou d 'internalisation d'antigènes se réalisent aussi en présence des différentes concentrations d'inhibiteurs.

Protocole d'internalisation des antigènes par les cellules présentatrices de l'antigène :

8.10⁶ cellules sont internalisées en présence de quantité saturante de protéines radiomarquées à l'iode 125 (1 µg) dans des micropuits dans 70 µl. Après une heure d'incubation à 4°C sous agitation, les antigènes sont fixés à la surface des cellules. La suspension cellulaire est lavée deux fois en PBS-BSA et les culots cellulaires sont repris dans 70 µl de tampon et incubées à 37°C pendant différentes périodes allant jusqu'à 2 heures. Cellules et surnageants sont séparés par centrifugation à 800g pendant 5 minutes 4°C. Pour des plus longues périodes d'incubation, l'étape préliminaire de préfixation des antigènes à la surface des cellules est supprimée. Les cellules sont diluées dans un milieu RPMI-10% SVF en présence de 20 mM Hépès, à 10⁶cellules /ml. Les cellules sont incubées en présence d'un excès d'antigène pendant différentes périodes à 37°C (1 µg de molécules /5.10⁷ cellules monocytes/macrophages ou /10⁸ cellules B-EBV).

Tous les agents thérapeutiques définis dans le cadre de la présente invention sont utilisés pour prévenir et/ou traiter une maladie dégénérative et/ou neurologique et/ou auto-immune, telle que la sclérose en plaques, seuls ou en combinaison. Ils peuvent être utilisés également pour évaluer leur efficacité *in vitro* ou *in vivo*.

Administration chez l'homme des agents thérapeutiques:

Le matériel biologique selon l'invention peut être administré *in vivo* notamment sous forme injectable. On peut également envisager une injection par voie épidermique, intraveineuse, intra-artérielle, intramusculaire, intracérébrale par seringue ou tout autre moyen équivalent. Selon un autre mode de réalisation par administration orale ou tout autre moyen parfaitement connu de l'homme de l'art et applicable à la présente invention. L'administration peut avoir lieu en dose unique ou répétée, une ou plusieurs fois après un certain délai d'intervalle. La voie d'administration et le dosage les mieux appropriés varient en fonction de différents paramètres tels que par exemple l'individu ou la maladie à traiter, du stade et/ou de l'évolution de la maladie, ou encore de l'acide nucléique et/ou de la protéine et/ou peptide et/ou molécule et/ou cellule à transférer ou de l'organe/tissus cible.

Pour la mise en oeuvre du traitement du mammifère mentionné dans la présente invention, il est possible de disposer de compositions pharmaceutiques comprenant un matériel biologique tel que précédemment décrit, avantageusement associé avec un véhicule pharmaceutiquement acceptable pour l'administration à l'homme ou à l'animal. L'utilisation de tels supports est décrite dans la littérature (voir par exemple Remington's Pharmaceutical Sciences 16th ed. 1980, Mack Publishing Co). Ce véhicule pharmaceutiquement acceptable est préférentiellement isotonique, hypotonique ou présente une faible hypertonicité et a une force ionique relativement basse, tel que par exemple une solution de sucrose. Par ailleurs, ladite composition peut contenir des solvants, des véhicules aqueux ou partiellement aqueux tels que de l'eau stérile, libre d'agents pyrogène et des milieux de dispersion par exemple. Le pH de ces compositions pharmaceutiques est convenablement ajusté et tamponné selon les techniques conventionnelles.

**Figures :**

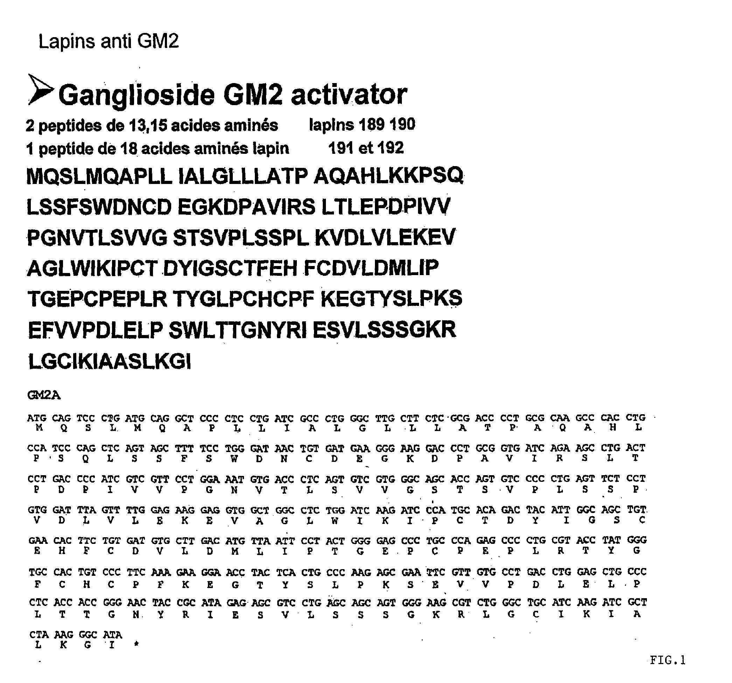

La figure 1 représente la séquence en amino acides de la protéine GM2AP, et la localisation des peptides, qui est soulignée, et qui sont utilisés pour la production des anticorps anti-peptides GM2AP.

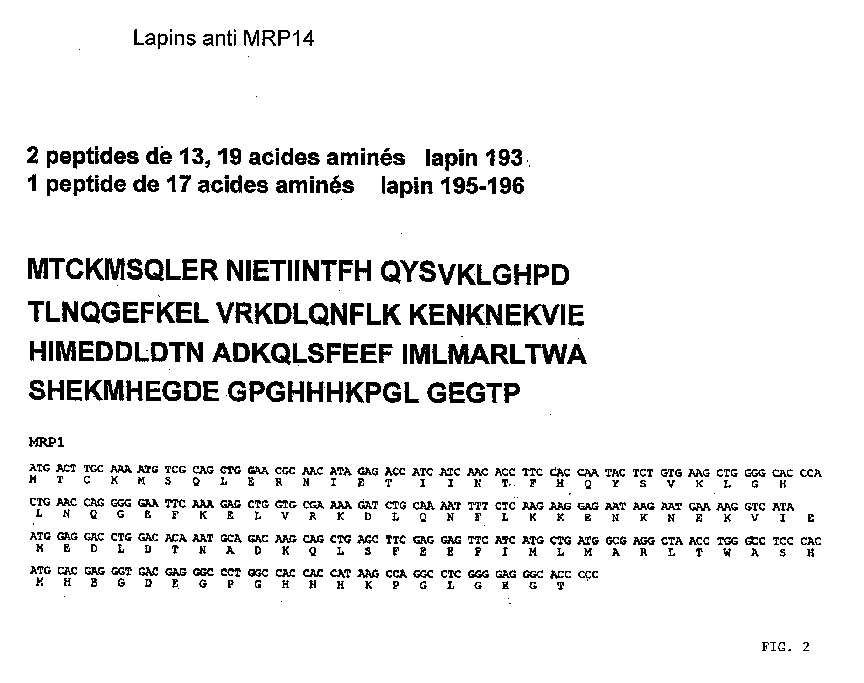

La figure 2 représente la séquence en amino acides de la protéine MRP14, et la localisation des peptides, qui est soulignée, et qui sont utilisés pour la production des anticorps anti-peptides MRP14.

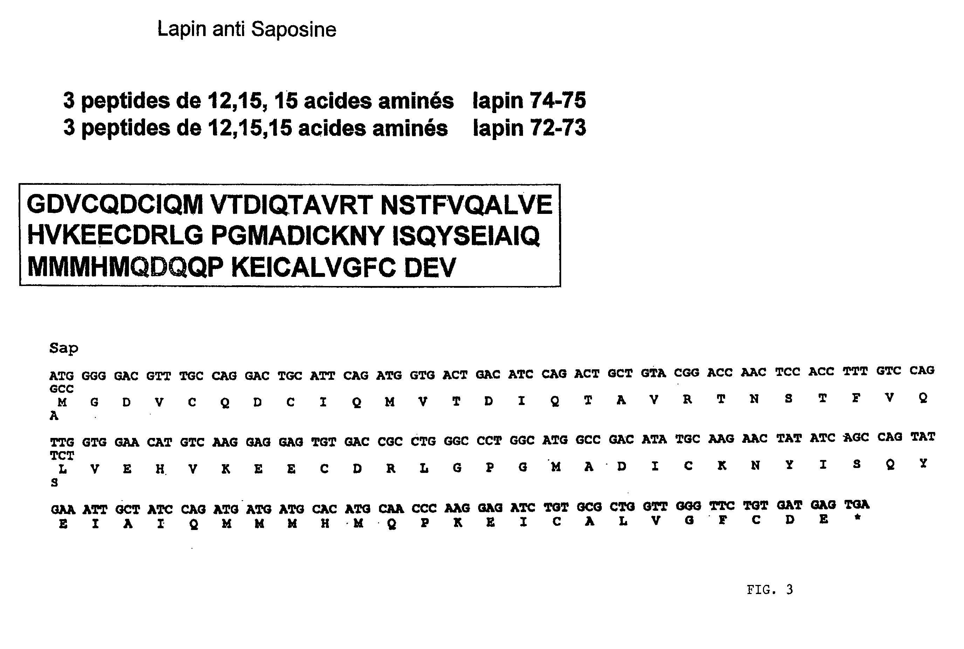

La figure 3 représente la séquence en amino acides de la protéine Saposine B, et la localisation des peptides, qui est soulignée, et qui sont utilisés pour la production des anticorps anti-peptides Saposine B.

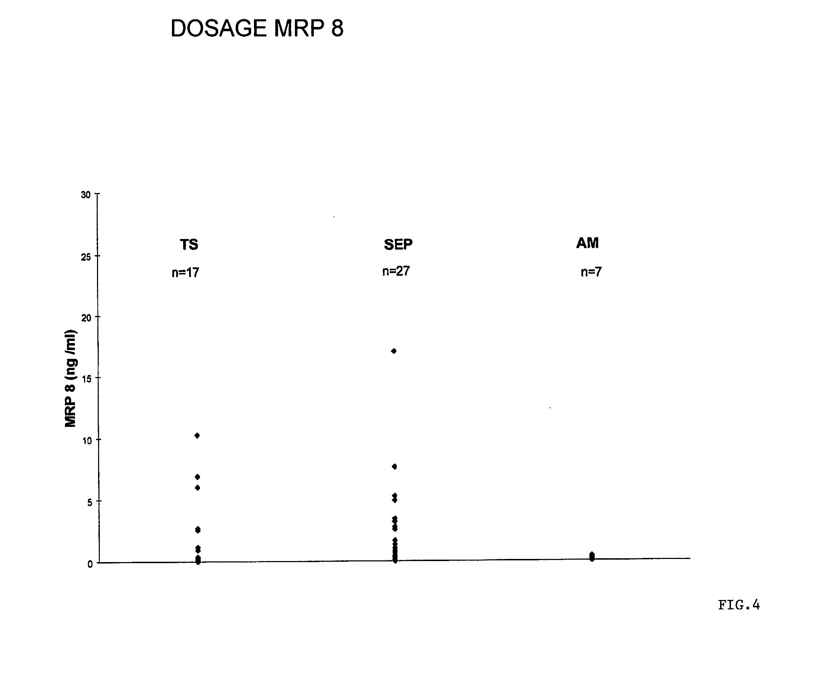

La figure 4 représente le dosage de la protéine MRP8 (ng/ml - en ordonnée) dans les urines de patients atteints de sclérose en plaques (SEP), dans les urines de patients atteints d'autres maladies neurologiques (AMN) et dans les urines de témoins considérés sains (TS). n signifie le nombre d'urines testées par catégorie.

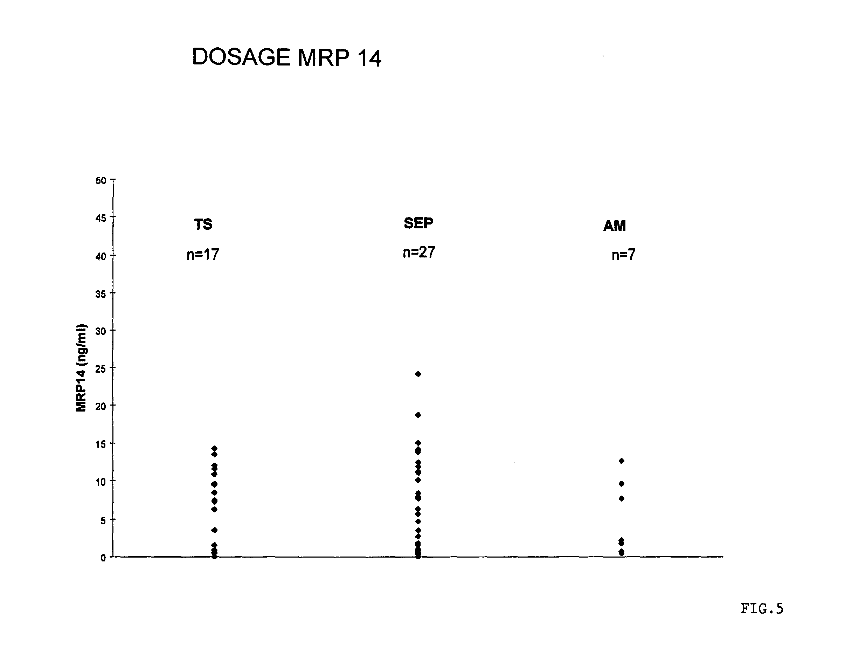

La figure 5 représente le dosage de la protéine MRP14 (ng/ml - en ordonnée) dans les urines de patients atteints de sclérose en plaques (SEP), dans les urines de patients atteints d'autres maladies neurologiques (AMN) et dans les urines de témoins considérés sains (TS). n signifie le nombre d'urines testées par catégorie.

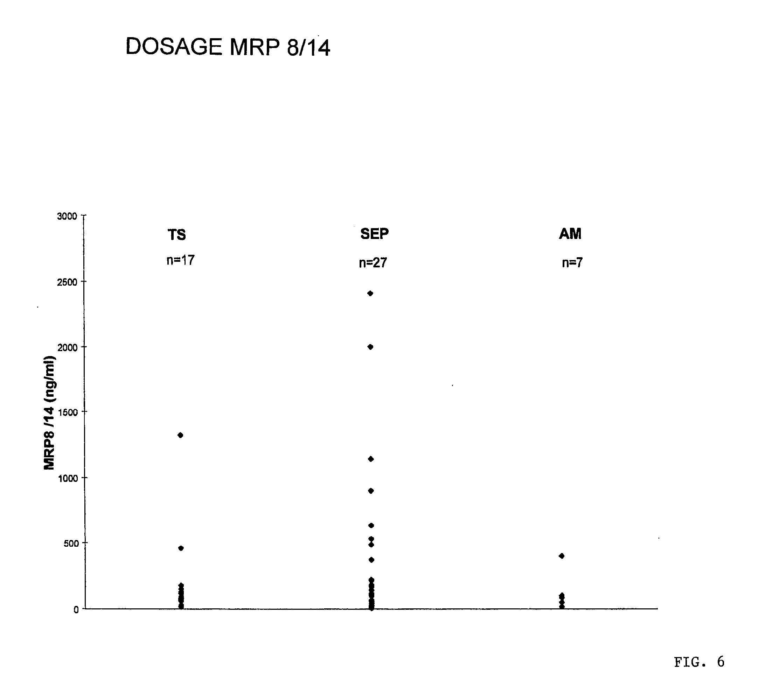

La figure 6 représente le dosage de la protéine MRP8/14 (ng/ml - en ordonnée) dans les urines de patients atteints de sclérose en plaques (SEP), dans les urines de patients atteints d'autres maladies neurologiques (AMN) et dans les urines de témoins considérés sains (TS). n signifie le nombre d'urines testées par catégorie.

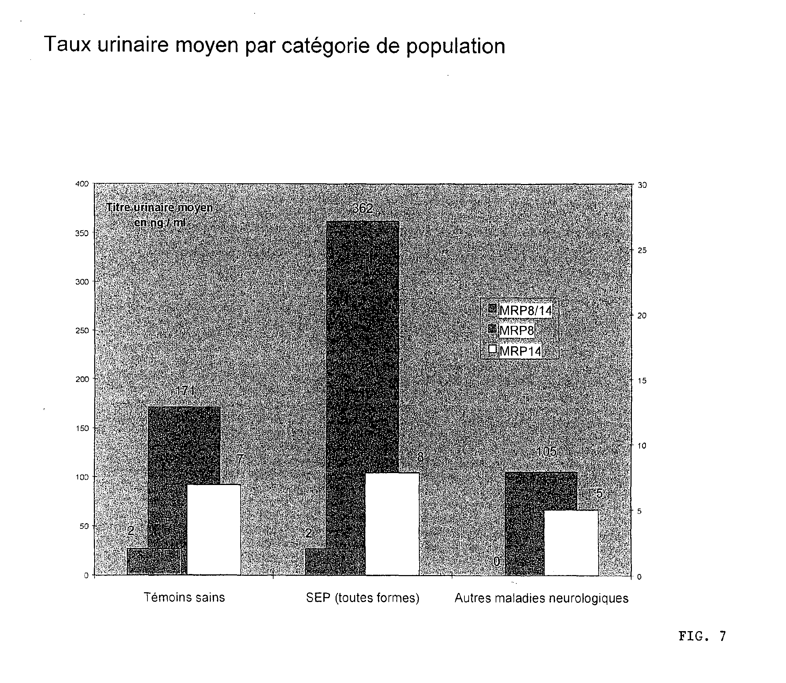

La figure 7 représente les concentrations moyennes des protéines MRP8, MRP14, MRP8/14 (ng/ml - en ordonnée) dans les urines de patients atteints de sclérose en plaques (SEP), dans les urines de patients atteints d'autres maladies neurologiques (AMN) et dans les urines de témoins considérés sains (TS). n signifie le nombre d'urines testées par catégorie.

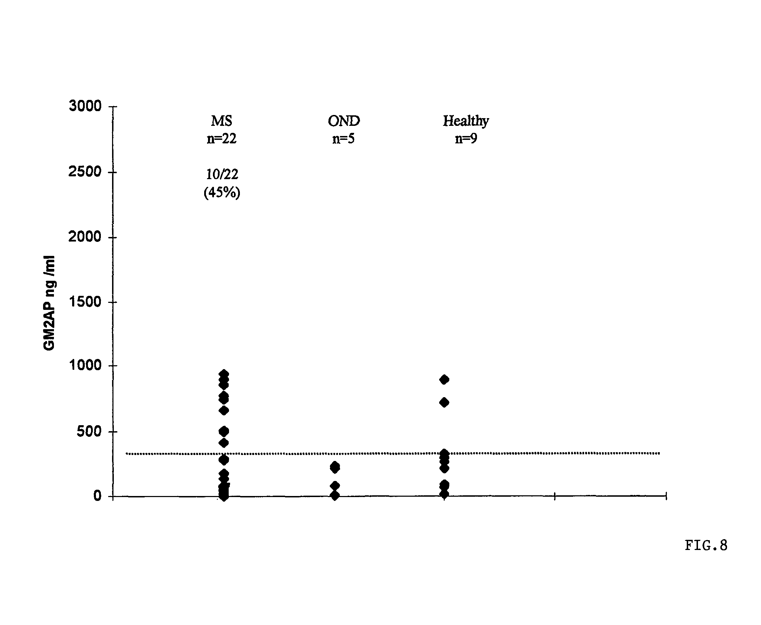

La figure 8 représente le dosage de la protéine GM2AP (ng/ml - en ordonnée) dans les urines de patients atteints de sclérose en plaques (SEP), dans les urines de patients atteints d'autres maladies neurologiques (AMN) et dans les urines de témoins considérés sains (TS). n signifie le nombre d'urines testées par catégorie. MS signifie SEP, OND signifie AMN et Healthy signifie prélèvements de témoins supposés sains (TS).

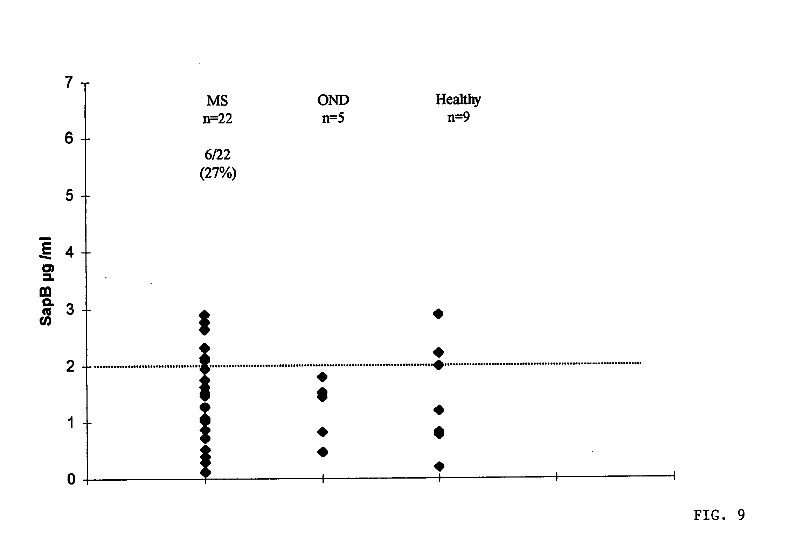

La figure 9 représente le dosage de la protéine Saposine B (µg/ml - en ordonnée) dans les urines de patients atteints de sclérose en plaques (SEP), dans les urines de patients atteints d'autres maladies neurologiques (AMN) et dans les urines de témoins considérés sains (TS). n signifie le nombre d'urines testées par catégorie. MS signifie SEP, OND signifie AMN et Healthy signifie prélèvements de témoins supposés sains (TS).

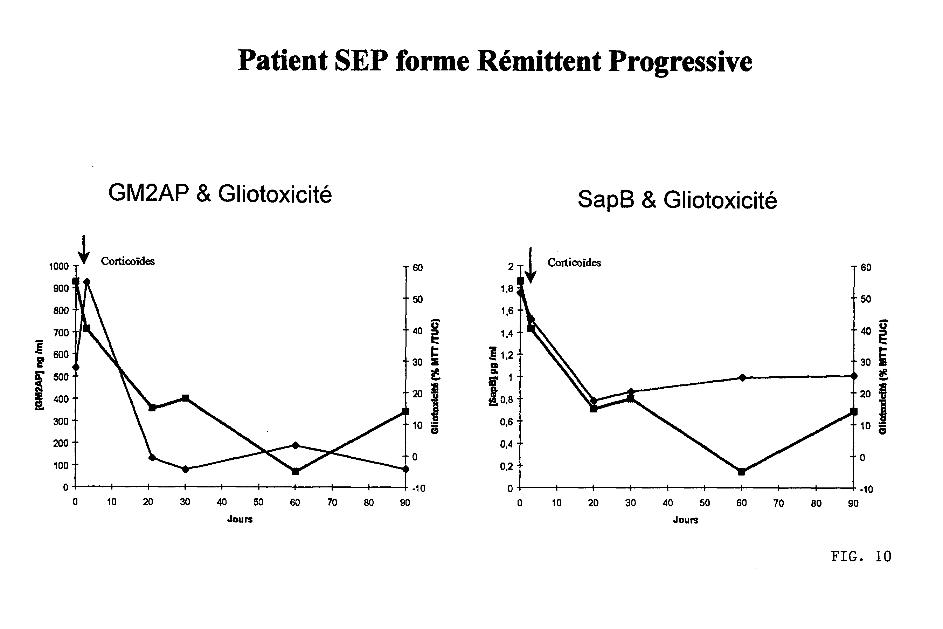

La figure 10 représente la co-détection des protéines Saposine B (µg/ml - en ordonnée) et GM2AP (ng/ml - en abscisse) dans des échantillons d'urine de patients SEP, de témoins supposés sains et de patients atteints d'autres maladies neurologiques et la corrélation observée entre les taux des deux protéines.

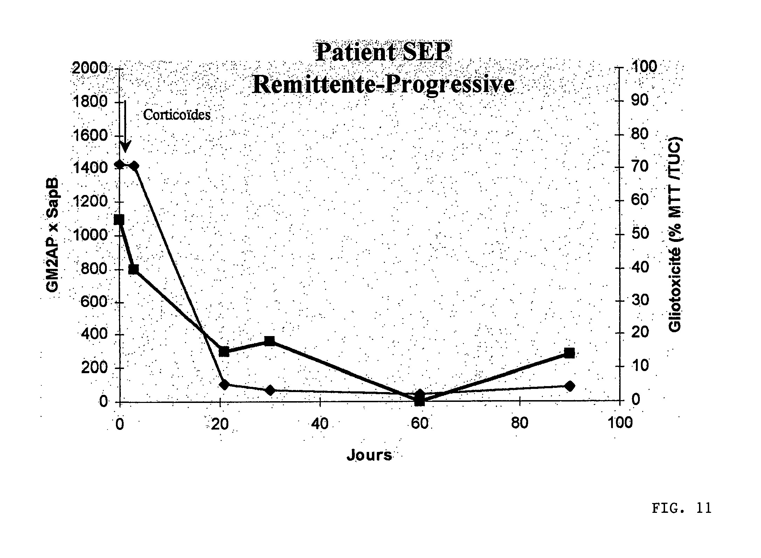

La figure 11 représente : figure 11A, le dosage de la protéine GM2AP en ng/ml dans les urines d'un patient SEP en forme rémittente progressive (courbe claire) et la gliotoxicité en pourcentage de cellules mortes estimées par le test MTT (courbe foncée) ; figure 11B le dosage de la protéine Saposine B en µg/ml dans les urines d'un patient SEP en forme rémittente progressive (courbe claire) et la gliotoxicité en pourcentage de cellules mortes estimées par le test MTT (courbe foncée).

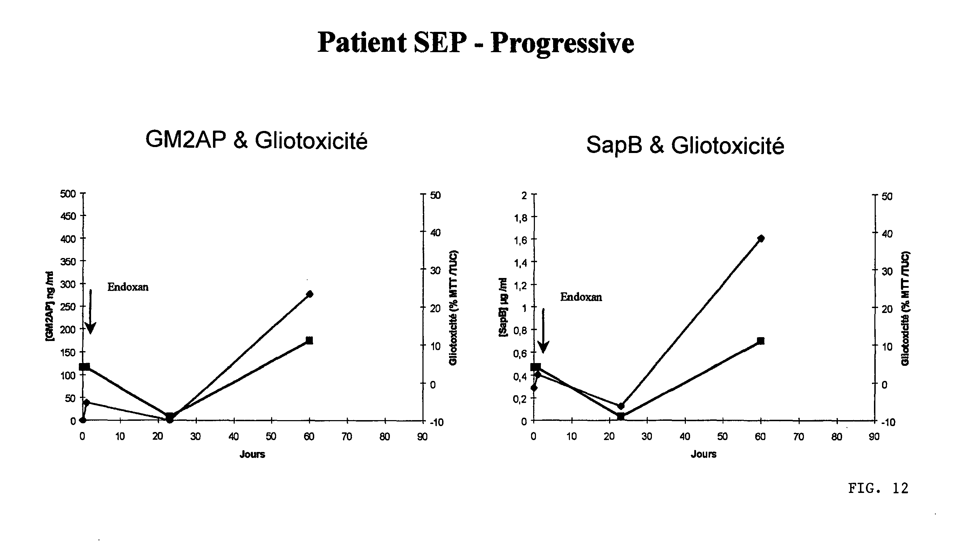

La figure 12 représente le produit des concentrations des protéines GM2AP et saposine B en ngxµg/ml² dans les urines d'un patient SEP en forme rémittente progressive (courbe claire) et la gliotoxicité en pourcentage de cellules mortes estimées par le test MTT (courbe foncée).

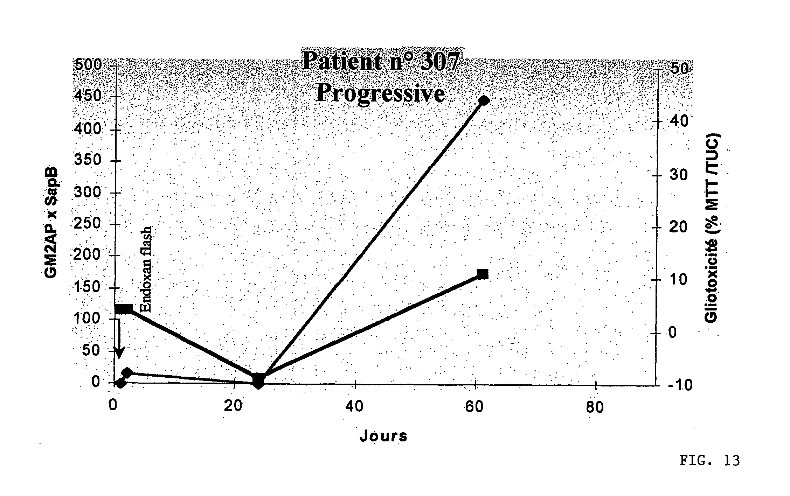

La figure 13 : figure 13A, le dosage de la protéine GM2AP en ng/ml dans les urines d'un patient SEP en forme progressive (courbe claire) et la gliotoxicité en pourcentage de cellules mortes estimées par le test MTT (courbe foncée) ; figure 13B le dosage de la protéine Saposine B en µg/ml dans les urines d'un patient SEP en forme progressive (courbe claire) et la gliotoxicité en pourcentage de cellules mortes estimées par le test MTT (courbe foncée).

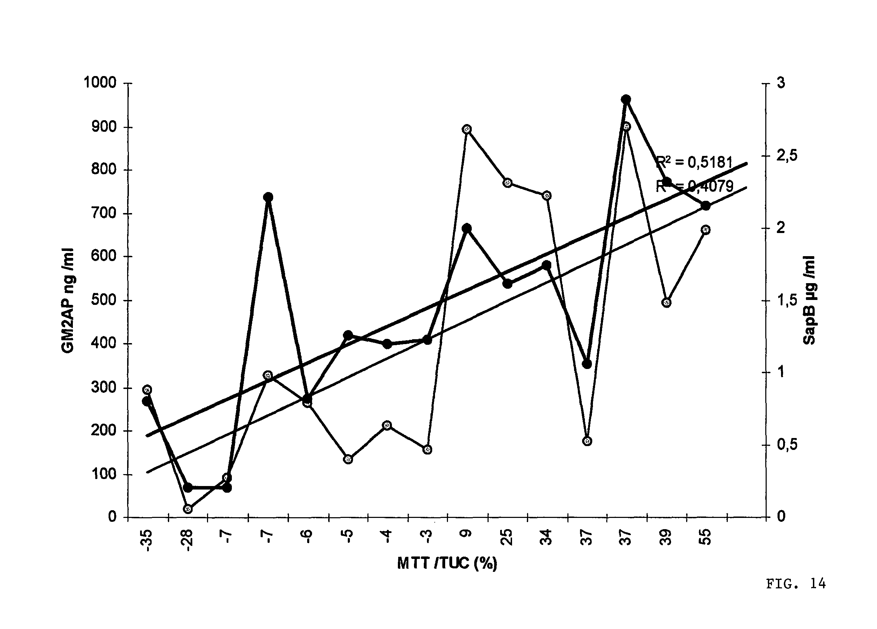

La figure 14 représente le produit des concentrations des protéines GM2AP et saposine B en ngxµg/ml² dans les urines d'un patient SEP en forme progressive (courbe claire) et la gliotoxicité en pourcentage de cellules mortes estimées par le test MTT (courbe foncée).
La figure 15 représente la corrélation entre les concentrations de GM2AP en ng/ml (abscisse) et de gliotoxicité en pourcentage de cellules mortes estimées par le test MTT (ordonnée) déterminées dans des urines de patients SEP et de témoins.
La figure 16 représente la corrélation entre les concentrations de Saposine B en µg/ml (abscisse) et de gliotoxicité en pourcentage de cellules mortes estimées par le test MTT (ordonnée) déterminées dans des urines de patients SEP et de témoins.
La figure 17 représente la corrélation entre le produit des concentrations de GM2AP et Saposine B en ngxµg/ml² (abscisse) et de gliotoxicité en pourcentage de cellules mortes estimées par le test MTT (ordonnée) déterminées dans des urines de patients SEP et de témoins.
La figure 18 représente la corrélation entre les concentrations en GM2AP (ng/ml - en ordonnée gauche), les concentrations en Saposine B (µg/ml - ordonnée droite) et la gliotoxicité en pourcentage de cellules mortes estimées par le test MTT (abscisse). Deux droites de corrélation estimées sont représentées sur le graphe. Les lignes en gras sont relatives aux concentrations en saposine B ; les lignes en noir clair sont relatives aux concentrations en GM2AP.

### Exemples :

### Exemple 1 : Recueil et pool d'urines.

Des échantillons d'urine de volumes différents ont été prélevés à partir d'individus sains (SEP négatifs) n'ayant a priori aucune maladie neurologique ou auto-immune. L'activité toxique de chaque prélèvement vis à vis de cellules astrocytaires murines a été testée *in vitro* en utilisant le test MTT. Au total un pool de 20 litres d'urine a été constitué (pool SEP négatif). Parallèlement, des échantillons d'urine de volumes différents ont été prélevés à partir d'individus atteints de sclérose en plaques (SEP positifs) à différents stade de la maladie. L'activité toxique de chaque prélèvement vis à vis de cellules astrocytaires murines a été testée *in vitro* en utilisant le test MTT. Au total un pool de 80 litres d'urine a été constitué (pool SEP positif).

### Exemple 2 : Purification des protéines urinaires.

Les pools d'urine SEP positif et SEP négatif, recueillis et testés selon l'exemple 1, ont été purifiés pour obtenir une concentration en protéines élevée et éliminer au maximum les protéines de haut poids moléculaire.

Précipitation : des précipitations au sulfate d'ammonium (Prolabo - réf. 21 333 365) ont été effectuées sur les pools d'urine SEP positif et SEP négatif. Le pourcentage de 60 % de sulfate d'ammonium saturé pour 40 % d'urine, soit 390 grammes de sulfate d'ammonium par litre d'urine a été utilisé. Chaque pool est réparti en fractions de 1,8 litres dans des flacons de 2 litres pour améliorer la précipitation. La précipitation a été effectuée durant 2 x 8 heures, à température ambiante, sous agitation douce. Après centrifugation des pools d'urine à 3 000 tpm pendant 10 min., à une température de 10°C, le culot obtenu est repris dans un tampon Tris 20 mM contenant du CaCl₂ 1 mM et de l'urée à 0,25 M. Le mélange a ensuite été centrifugé à 3 000 tpm pendant 10 min. Le surnageant contient les protéines concentrées. Il est soit utilisé immédiatement pour l'étape suivante, soit congelé si l'étape suivante ne peut être effectuée en continu.

Chromatographie par échange d'ions : la solution contenant les protéines a ensuite été passée sur un gel DEAE fast Flow (commercialisé par PHARMACIA). Cette étape est effectuée à basse pression sur une colonne PHARMACIA remplie de gel. Les tampons sont amenés sur la colonne par une pompe péristaltique qui permet un débit régulier. Le tampon d'équilibration de la colonne est le tampon Tris 20 mM, pH 7. La fraction correspondant au surnageant de précipitation et contenant une quantité de sels trop élevée est dialysée contre ce tampon avant dépôt sur la colonne. Une élution par un gradient salin permet de récupérer les protéines. Le gradient d'élution est effectué par palier de NaCl 100, 200, 300, 500 mM dans le tampon d'équilibration de la colonne. Les fractions d'élution sont testées par le test MTT et ne seront conservées que les fractions positives, soit la fraction éluée à 200 Mm NaCl. Ces fractions pourront être traitées immédiatement ou conservées à l'état lyophilisé.

Purification : Une chromatographie d'exclusion stérique basée sur la différence de taille et de forme des protéines à éluer a été utilisée. La fraction correspondant à l'élution 200 mM NaCl est déposée sur la colonne. Au cours de l'élution, les protéines de faible masse moléculaire sont retenues et donc éluées plus tardivement que les grosses molécules. Les purifications ont été effectuées sur HPLC avec une colonne TosoHaas TSK Prep G 3000 SW, d'un diamètre de 21,5 mm et d'une longueur de 300 mm, la limite d'exclusion en masse moléculaire est de 500 000 daltons . Le tampon d'élution utilisé contient du phosphate 100 mM, du sulfate de sodium 100 mM, à pH 6,8. La séparation du mélange de protéines a été effectué en 60 min. Seule la fraction correspondant à une masse de 15-20 000 daltons a été conservée. Cette fraction est dialysée dans un tampon Tris 20 mM contenant du CaCl₂ 0,2 mM, pH 7,2, puis lyophilisée.

A chaque étape, seules les fractions présentant une activité toxique significative ont été retenues pour l'étape suivante. Un contrôle de l'activité toxique des protéines a été effectué à chaque étape, à l'aide du test MTT. Seules les fractions présentant une activité toxique significative ont été retenues pour l'étape de purification supplémentaire décrite dans l'exemple 3.

### Exemple 3 : Purification supplémentaire des protéines urinaires par chromatographie phase inverse.

Des pools d'urine provenant de patients SEP (pool SEP positif) et de patients non SEP (pool SEP négatif), obtenus après purification selon l'exemple 2, ont été repris dans de l'eau distillée, puis dilués avec une solution 0,2% TFA/10% acétonitrile pour obtenir une concentration finale d'environ 130 à 140 µg/ml.

La séparation par HPLC phase inverse C8 a été effectuée sur une colonne Brownlee Aquapore (nom commercial) commercialisée par la société Perkin Elmer (caractéristiques de la colonne : 300 angstroms/7 µm/(100x4,6) mm). Deux colonnes distinctes ont été utilisées respectivement pour les pools positif et négatif. Les injections ont été réalisées par multi-injections de 250 µl. Les protéines ont été éluées avec un gradient linéaire de 5% à 15% de tampon B en 5 min., puis de 15% à 100% de tampon B en 95 min., à un débit de 0,5 ml/min. Les tampons de séparation A et B utilisés sont respectivement le tampon 0,1% TFA (Pierce n° 28904)/ eau MilliQ et le tampon 0,09% TFA/80% acétonitrile (Baker). La détection a été effectuée par mesure de l'absorbance UV à 205 et 280 nm. La collecte des fractions a été effectuée en fractions de 1,5 ml et de 0,5-1 ml dans la zone d'intérêt. Les fractions ont été congelées après la collecte dans de la carboglace.

Les fractions collectées ont ensuite été séchées en speed vac et reprises dans 100 µl de 0,1% TFA/30% acétonitrile. 20µl des fractions ont été transférés dans des eppendorfs de 500 µl, séchés et lavés à deux reprises avec 100 µl d'eau MilliQ, puis séchés de nouveau.

L'activité toxique des protéines contenues dans chaque fraction recueillie après élution a été déterminée à l'aide du test MTT. Seule la fraction 21 présentant une activité toxique significative a été retenue. Le numéro de cette fraction correspond à l'ordre de l'élution en fonction des conditions d'élution énoncée dans cet exemple.

### Exemple 4: Analyse des protéines obtenues par séparation sur HPLC sur gel SDS-TRICINE.

Le pool de collecte de la fraction 21 obtenue par HPLC, comme décrit dans l'exemple 3, et provenant de 20 injections du pool SEP positif, a été déposé sur un gel SDS-TRICINE 16% précoulé de 10 puits et de 1 mm d'épaisseur (commercialisé par la société Novex). Les conditions d'utilisation du gel correspondent à celles préconisées par le fournisseur. L'échantillon est repris dans 75 µl du tampon d'échantillon 1 fois concentré (SDS-TRICINE N° LC 1676, 1 ml deux fois concentré + 50µl de β-mercaptoéthanol (Pierce) dilué au 1/2 dans de l'eau) et 25µl de l'échantillon sont déposés sur le gel en trois fois. Le pool de collecte de la fraction 21 provenant de 6 injections du pool SEP négatif a été déposé sur le gel dans les mêmes conditions que celles décrites pour le pool SEP positif. La migration sur les deux gels a été effectuée en parallèle dans la même cuve de migration (XCELL II NOVEX (nom commercial)) à un voltage constant de 125 mV pendant 2 heures. La cuve est placée dans un bac contenant de la glace. Les gels ont été colorés directement après la migration par coloration au zinc/imidazole (kit de coloration 161-0440 commercialisé par la société BIORAD) pour obtenir une coloration négative réversible. Les bandes de protéines sont translucides sur fond opaque.

### Exemple 5 : Digestion à la trypsine des bandes de gel.

Toutes les bandes de protéines visualisées dans les dépôts de la fraction 21 ont été découpées et soumises à une protéolyse par la trypsine.

Les bandes de gels sont découpées au scalpel en tranches de 1 mm et transférées dans des tubes eppendorfs. Les eppendorfs sont soumis à un pic de centrifugation pour faire tomber les morceaux de gel et après centrifugation 100 µl de tampon de lavage (100 Mm NH₄CO₃/50% CH₃CN) sont ajoutés aux morceaux de gel. Après 30 min. d'agitation à température ambiante, le surnageant est enlevé par fractions de 20 µl et l'étape de lavage est renouvelée deux fois. Les eppendorfs sont séchés pendant 5 min. en speed vac. 20 µg de trypsine (Modified sequenal grade PROMEGA V5111) sont repris dans 200 µl de tampon de digestion (5 mM TRIS, pH 8) et sont dissous pendant 30 min. à température ambiante, sous agitation intermittente et 20 à 30 µl de trypsine resuspendue sont ajoutés aux morceaux de gel. Les eppendorfs sont centrifugés et conservés en chambre chaude à 28°C pendant une nuit. Après digestion les bandes de gel peuvent être utilisées immédiatement pour les mesures de masse ou congelées pour usage ultérieur.

### Exemple 6 : Digestion chimique au CNBR des bandes de gel.

Dans l'éventualité d'une protéine résistante aux clivages enzymatiques, en particulier à l'action de la trypsine comme décrit dans l'exemple 5, les bandes entre 16kD et 20kD ont été traitées avec du CNBR. Les bandes de gel, déjà utilisées pour les digestions avec la trypsine, sont séchées 5 à 10 min. en speed vac.
Une solution de CNBR (FLUKA) à 200 mg/ml a été préparée dans 70 % acide formique (BAKER). 20 µl de cette solution ont été utilisées pour réhydrater les morceaux de gel. La réaction s'est faite pendant 20 h à température ambiante et à l'obscurité. Les peptides sont extraits 3 fois 30 min. avec 100 µl de 0.1 % TFA / 60% Acétonitrile. Les solutions d'extraction sont réunies et concentrées à 20 µl. Ces échantillons sont dilués 5 fois dans 0,1 % TFA/eau. Les conditions de séparation sont celles décrites pour les peptides de la digestion avec la trypsine.

### Exemple 7 : Analyse par spectrométrie MALDI-TOF.

30 µl de tampon d'extraction (2 % TFA/50 % acétronitrile) sont ajoutés aux échantillons. Les eppendorfs à analyser sont soumis à une centrifugation de 5 min., puis à une sonication de 5 min. et finalement à une centrifugation de 1 min. Sur un disque en acier inoxydable, 14 dépôts de 0,5 µl de matrice (acide α-cyano-4-hydroxy-trans-cinnamique à saturation dans de l'acétone) sont réalisés. Une fine couche microcristalline uniforme est obtenue. 0,5 µl d'une solution de 2 % TFA/eau sont déposés sur cette sous-couche sur les 14 dépôts, puis 0,5 µl d'échantillon à analyser sont ajoutés. Dans cette goutte ainsi formée, 0,5 µl d'une solution à saturation d'acide d'acide α-cyano-4-hydroxy-trans-cinnamique dans 50 % acétonitrile/eau sont ajoutés. Après un séchage à température ambiante pendant 30 min., les dépôts cristallins sont lavés avec 2 µl d'eau qui sont immédiatement évacués par un souffle d'air. Tous les spectres sont obtenus sur un spectromètre de masse BRUKER BIFLEX (marque de commerce) équipé d'un réflectron. Les mesures (90 à 120 tirs laser sur l'ensemble du dépôt) sont accumulées pour obtenir un spectre de masse qui soit le plus représentatif de l'ensemble des peptides présents dans le sandwich matrice-échantillon. Pour chaque dépôt, une calibration avec les peptides de l'autolyse de la trypsine a été faite afin de pouvoir utiliser une précision de mesure inférieure à 100 ppm.
Les recherches dans les banques de données ont été exécutées dans MS-FIT PROTEINPROSPECTOR (http ://prospector.ucsf.edu). Les paramètres communs, utilisés dans ces recherches, sont (1) base de données : NCBInr, (2) une tolérance de 100-50 ppm, (3) les cystéines ne sont pas modifiées, (4) les méthionines peuvent être oxydées, (5) gamme de poids moléculaire : 1000-100000 Da, (6) jusqu'à 3 sites de coupure peuvent être ignorés.

### Exemple 8 : Séquençage N-terminal des peptides de digestion.

### (i) Extraction et séparation par HPLC des peptides de digestion.

Après les mesures de masse sur la totalité de la digestion, le reste des peptides est extrait en 3 fois 30 min. dans un bain de sonication avec 0,1 % TFA/60 % acétonitrile. Les solutions d'extraction sont réunies et séchées jusqu'à 20 µl en speed vac. Après dilution dans 80 µl de tampon A (0,1 % TFA/eau), les extractions des bandes de gel, digérées avec de la trypsine, sont injectées sur une colonne C18/MZ-Vydac/(125x1,6)mm/5 µm. L'élution des peptides se fait à un débit de 150 µl/min. et dans un gradient allant de 5 % de tampon B (0,09 % TFA/80 % acétronitrile) à 40 % de tampon B en 40 min., puis de 40 % de tampon B à 100 % de tampon B en 10 min. La détection est faite par mesure de l'absorbance UV à 205 nm. La collecte des pics est effectuée dans des tubes eppendorf de 500 µl. Les fractions sont conservées sur la glace et pour la bande de 18-20 kD du pool 21 SEP positif analysées par spectrométrie de masse MALDI-TOF.

### (ii) Séquençage N-terminal.

Les fractions ne correspondant qu'à un seul pic de masse ont été analysées par dégradation d'Edman sur un séquenceur (Modèle 477A PERKIN ELMER/Applied Biosystems). Les conditions de séquençage sont celles décrites par le constructeur. Une micro cartouche a été utilisée pour le dépôt des échantillons et les PTH-AminoAcid sont identifiés avec un système HPLC online (Modèle 120A PERKIN ELMER/Applied Biosystems).

Le dépôt de la fraction à séquencer s'est fait en plusieurs dépôts de 15 µl avec des séchages intermédiaires. Le tube ayant contenu le peptide est lavé avec 15 µl d'acide formique 85 % (BAKER). Les séquences d'acides aminés correspondent toujours aux masses mesurées. Les peptides, dont les masses ne correspondent pas à la protéine principale identifiée, ont été séquencés en priorité. De cette manière, il a été possible d'identifier jusqu'à trois protéines dans une bande de gel.

### Exemple 9 : Résultats et discussion.

Après HPLC inverse du pool témoin SEP négatif et du pool SEP positif, l'activité toxique de chaque fraction d'élution a été déterminée en utilisant le test MTT. Seule la fraction 21 du pool SEP positif présente une activité toxique *in vitro.* La fraction 21 du pool témoin SEP négatif ne présente aucune activité toxique. L'activité toxique de la fraction 21 du pool SEP positif a été confirmée *in vitro* par FACS, comme décrit dans la demande de brevet WO 98/11439 sur des cellules astrocytaires murines.

Le contenu protéique de la fraction 21 du pool témoin SEP négatif et du pool SEP positif a été observé après séparation sur gel SDS-TRICINE 16% et coloration du gel au zinc/imidazole. Des protéines de poids moléculaires apparents élevés ont été trouvées dans les deux fractions. Par contre cinq bandes différentes et de poids moléculaires apparents faibles ne sont visibles que dans la fraction 21 du pool SEP positif (bandes 8, 14, 18 et 20 kD). A chaque bande correspond au moins une protéine et des variants desdites protéines qui ont un poids moléculaire apparent proche de celui de la protéine native. Ces séquences variantes présentent un pourcentage d'homologie ou d'identité avec les séquences natives d'au moins 70%, de préférence d'au moins 80% et avantageusement d'au moins 98 %.

Les protéines d'intérêt de la fraction 21 du pool SEP positif ont ensuite été analysées par spectrométrie de masse et/ou séquençage et recherche d'homologie dans les banques de données. Les résultats montrent la présence de cinq bandes de protéines migrant entre 22 et 5 kD dans la fraction 21 du pool SEP positif et des variants desdites protéines.

Ces protéines sont le fragment C-terminal du Perlecan, qui commence à l'acide aminé 3464 et se termine à l'acide aminé 3707 de la séquence protéique complète, identifiée dans l'identificateur de séquences SEQ ID N° 2, le précurseur de la protéine plasmatique de liaison au rétinol dont la séquence est donnée en SEQ ID N° 4, la protéine activatrice du ganglioside GM2 identifié en SEQ ID N° 8, la calgranuline B identifiée en SEQ ID N° 17 et la saposine B représentée en SEQ ID N° 24. Comme décrit ci dessus des homologues ou variants desdites protéines ont également été identifiés par séquençage. Ces séquences protéiques homologues ou variantes sont le produit de mutations au niveau des gènes codant pour lesdites protéines. A titre d'exemple, la SEQ ID N° 9 présente 99 % d'homologie ou d'identité avec la SEQ ID N° 8 de la protéine activatrice du ganglioside GM2 et le fragment de SEQ ID N° 9 qui commence à l'acide aminé 34 et se termine à l'acide aminé 202 présente 98,88 % d'homologie ou d'identité avec le fragment correspondant de la protéine native identifiée en SEQ ID N° 8.

### Exemple 10 : Mise en évidence des protéines dans un échantillon urinaire.

Des échantillons d'urine provenant d'un individu SEP négatif et d'un patient SEP positif ont été prélevés. Ces échantillons d'urine ont été purifiés selon le protocole décrit précédemment. Les fractions d'élution finales 21 ont été analysées séparément par spectrométrie de masse. Le profil de masse de chaque fraction correspondant à chaque échantillon d'urine a été comparé au profil de masse obtenu pour les protéines identifiées dans les exemples précédents. Les résultats montrent que pour l'échantillon d'urine provenant du patient SEP positif les masses correspondent aux molécules (i) fragment C-terminal du Perlecan, (ii) précurseur de la protéine activatrice du ganglioside GM2, (iii) calgranuline B et (iv) saposine B identifiées précédemment. Par contre aucune de ces masses n'a été identifiée dans le profil de masse obtenu après analyse de l'échantillon d'urine provenant de l'individu SEP négatif. Le procédé décrit est utilisable comme essai de diagnostic.

### Exemple 11 : Essai en Western Blot.

Des Western Blot ont été réalisés sur différentes fractions d'urine brute ou purifiée comme décrit dans l'exemple 2. Des échantillons d'urine provenant d'individus sains et de patients atteints de sclérose en plaques sont testés en parallèle. Les échantillons sont déposés sur un gel d'électrophorèse permettant de séparer les différentes protéines en fonction de leur masse moléculaire sous l'action d'un champ électrique. Les Western Blot sont réalisés après transfert des protéines du gel sur une membrane. Pour révéler les protéines transférées, la membrane est saturée en tampon de saturation, puis incubée avec un anticorps directement marqué à la phosphatase alcaline. L'anticorps utilisé est un anticorps anti-calgranuline (anticorps monoclonal de souris, clone CF 145 sous-type IgG 2b commercialisé par la société Valbiotech : référence MAS 696p lot PC96G696). Le substrat de l'enzyme est le dichlorure de 3,3'-(1,1'-biphényl)4,4'diazonium et 2-naphtalenyl phosphate de sodium (commercialisé sous la dénomination β Naphtyl acid phosphate Sigma réf. N7375 et ô dianisine Tetrazotized D3502) est ajouté pour la révélation des bandes et la visualisation des protéines liées à l'anticorps. Une molécule de masse moléculaire apparente d'environ 14 000 est révélée dans les urines purifiées de patients atteints de SEP, avec un signal relativement intense. Cette protéine correspond à la calgranuline B (masse moléculaire apparente : 14 kD). Par contre, aucun signal n'est observé à partir d'urine d'individus sains. Cette observation confirme la présence de cette protéine spécifiquement dans les urines de patients atteints de SEP et la mise en oeuvre d'un procédé de détection utilisant un anticorps reconnaissant la protéine.

### Exemple 12 : Production d'anticorps monoclonaux.

La production d'anticorps monoclonaux par ascite impose une compatibilité du système H-2 entre l'hybridome et la souris productrice. 20 souris femelles Balb/c, âgées de 6 semaines, subissent une injection de 0.5ml de Pristane (2-6-10-14 acide tétraméthylpentadécane) dans leur cavité péritonéale, pour la production d'ascite (Porter et al., 1972). Une semaine à 10 jours plus tard, 5.10⁶ à 10.10⁶ hybridomes dilués dans 0.5ml de tampon stérile NaCl 0,145M, Na₂HPO₄ 10 mM, KCl 2.7 mM, KH₂PO₄ 1.5 mM à pH 7.4. sont injectés par voie intrapéritonéale. L'ascite apparaît une à deux semaines plus tard. Les liquides d'ascites présents dans la cavité péritonéale sont alors recueillis avec une seringue après incision du péritoine. Le liquide recueilli est centrifugé à 3000g pendant 15 minutes à température ambiante, filtré sur gaze pour éliminer le gras, puis tamponné en ajoutant 1/20^{ème} de son volume de tris-HCl 1M à pH 8.0. Cette méthode permet d'obtenir des quantités d'anticorps 10 fois supérieures à celles obtenues par culture d'hybridomes.
Les immunoglobulines présentes dans le liquide d'ascite sont relarguées par les sels (sulfate d'ammonium ou sulfate de sodium). Le liquide d'ascite est précipité par le sulfate d'ammonium 40%. Après 20 minutes au froid la solution est centrifugée 15 minutes 8000g à 4°C. Le précipité est lavé et resuspendu à froid dans une solution de sulfate d'ammonium 40% puis de nouveau centrifugé. Le nouveau précipité enrichi en IgG est remis en solution dans du tampon PBS et dialysé la nuit contre le tampon Tris-HCl 25 mM, NaCl 150 mM pH 7,4. Parallèlement une colonne d'agarose-Protéine A (ou protéine G) (commercialisée sous forme lyophilisée, Pierce) est lavée extensivement avec le tampon Tris-HCl 25 mM, NaCl 150mM pH7.4. La solution enrichie en IgG est déposé sur la colonne puis la colonne est lavée. Les IgG retenues par la colonne sont éluées à pH acide (glycine 200 mM pH 2.8). Les fractions éluées sont neutralisées avec un volume de Tris-Base 1M pH 10.5. Le contenu en immunoglobulines de chaque fraction recueillie est quantifiée par lecture d'absorbance à 280 nm (e 1%, 1cm = 14.0 Prahl et Porter 1968). Les fractions riches sont poolées. Le degré de purification des IgGs poolées est analysé par électrophorèse en gel d'acrylamide en présence de SDS. Les IgGs purifiées sont dialysées une nuit contre le tampon Tris-HCl 25 mM, NaCl 150mM pH7.4, filtrées stérilement, aliquotées et conservées à -20°C. leur concentration finale est déterminée par lecture de l'absorbance à 280 nm ou par dosage micro-BCA. Les peptides immunogènes référencés SEQ ID N° 58, SEQ ID N° 54, SEQ ID N° 55, SEQ ID N° 56, SEQ ID N° 57, SEQ ID N° 58, SEQ ID N° 59 et SEQ ID N° 65 ont été utilisés pour la production d'anticorps monoclonaux, selon le protocole décrit ci dessus. Mais, il est à la portée de l'homme du métier de définir d'autres protocoles pour la production d'anticorps monoclonaux, par exemple à partir des techniques décrites par Köhler et Milstein et par Galfre G. *et al*. précédemment cités ou des techniques dérivées de celles ci.

### Production de protéines recombinantes et d'anticorps polyclonaux et monoclonaux.

### Protéines recombinantes :

Les protéines recombinantes GM2AP (SEQ ID NO :73) et Saposine B (SEQ ID NO :74) utilisées pour réaliser la gamme étalon de cette étude ont été produites en système procaryote et purifiées à partir des clones de ces deux protéines obtenus dans notre laboratoire en utilisant les méthodes et protocoles bien connus de l'homme de l'art.

### Anticorps anti-GM2AP ou anti-Saposine B :

Les anticorps anti-GM2AP ou anti-Saposine B utilisés pour réaliser l'étude ont été soit produits dans notre laboratoire ou donnés généreusement.
Des anticorps polyclonaux anti-Saposine B et anti-GM2AP (Li et al, Glycoconjugate, 1984) ont été utilisés pour l'étude (cf les exemples ci-dessous) : ils sont dénommés SAP84 et GM2AP84.

Des anticorps polyclonaux anti-GM2AP ou anti-Saposine B ont été produits et purifiés dans le laboratoire en utilisant les protocoles et méthodes bien connus de l'homme de l'art : 50 µg de protéine GM2AP ou Saposine B procaryote achetée ont été injectés à des lapins aux jours J0, J28 et J56 ; deux injections de rappel ont été réalisés une fois par mois pendant deux mois consécutifs. Les deux anticorps polyclonaux anti-GM2AP et deux anticorps polyclonaux anti-Saposine B ont ainsi été obtenus et leur spécificité vis-à-vis de la protéine recombinante a été vérifiée par Western blot et par Elisa.

Des anticorps polyclonaux anti-peptides GM2AP ou Saposine B ont été produits et purifiés dans le laboratoire en utilisant les protocoles et méthodes bien connus de l'homme de l'art : 75 µg de peptides GM2AP ou Saposine B définis, produits et couplés à KLH dans notre laboratoire ont été injectés aux jours J0, J28 et J56 ; plusieurs boosts ont été réalisés une fois par mois pendant 5 mois consécutifs avec injection de 75 µg à chaque fois. Quatre anticorps polyclonaux anti-peptides GM2AP, quatre anticorps polyclonaux anti-peptides Saposine B et quatre anticorps polyclonaux de lapins anti-peptides MRP14 ont ainsi été obtenus et leur spécificité vis-à-vis de la protéine recombinante a été vérifiée par Western blot et par Elisa. La séquence des peptides GM2AP, Saposine B et MRP14 choisis sont décrites_dans les figures de 1 à 3.

Il a été obtenu :
- un anticorps anti-mélange de deux peptides de 13 et 15 acides aminés de GM2AP : 189-190 ; un anticorps anti-peptide de 18 acides aminés de GM2AP : 191-192 (cf. Figure 1),
- un anticorps anti-mélange de deux peptides de 13 et 19 acides aminés de MRP14 :193 ; un anticorps anti-peptide de 17 acides aminés de MRP14 : 195-196 (cf. Figure 2),
- un anticorps anti-mélange de trois peptides de 12, 15 et 15 acides aminés de Saposine B: 74-75 ; un autre anticorps anti-mélange de 3 peptides de 12, 15 et 15 acides aminés de Saposine B : 72-73 (cf. Figure 3).

Des anticorps monoclonaux anti-fraction native ont été produits et purifiés dans le laboratoire en utilisant les protocoles et méthodes bien connus de l'homme de l'art. La « fraction native » correspond à la fraction d'élution cytotoxique obtenue à partir du pool des 80 litres d'urine de patients SEP et après purification. C'est la dernière fraction d'élution qui contient les trois protéines GM2AP, Saposine B, MRP14. 30 µg de cette fraction de purification ont été injectés à trois souris aux jours J0, J14, J28 et le prélèvement a été effectué à J38. Après « screening » et fusion cellulaire, protocoles connus de l'homme de l'art pour l'établissement d'hybridomes et d'anticorps monoclonaux, les hybridomes ont été ré-injectés à la souris et le liquide d'ascite a été récupéré 10 jours après. Les anticorps ont été purifiés sur colonne sépharose-Protéine A et la spécificité vis-à-vis de la fraction utilisée pour l'immunisation a été vérifiée par Western blot et par Elisa. Ainsi quatre anticorps monoclonaux ont été obtenus : 19C1A7, 3D3F9, 18C8C5 et 7D12A8.

### Exemple 13 : Dosage des protéines MRP14 dans les urines par technique ELISA.

Les protéines MRP14, MRP8 et l'hétérocomplexe MRP8/14 ont été dosés dans des urines humaines en utilisant (i) soit une technique de dosage Elisa selon le procédé connu de l'homme de l'art et en utilisant les anticorps anti-MRP décrits dans les exemples précédents ; (ii) soit le kit 'MRP Enzyme Immunoassay' commercialisé par BMA Biomedicals AG, Augst, Switzerland, en utilisant les anticorps du kit, le protocole étant réalisé suivant la notice du kit..

### Détection de MRP14 et MRP8/14 dans des urines.

Les dosages a été réalisés à partir de 17 urines d'individus issus de la population active (TS), de 27 urines de patients atteints de sclérose en plaques (SEP et de 7 urines de patients atteints d'autres maladies neurologiques (AMN).
- La figure 4 illustre les taux de MRP8 dosés dans ces urines : alors que la concentration en MRP8 est quasiment nulle dans les urines AMN, il n'y a pas vraiment de différence de distribution entre les urines TS et SEP. Notons cependant que les différences observées sont quasiment négligeables car les concentrations dosées sont extrêmement faibles.
- La figure 5 illustre les taux de MRP14 dosés dans les mêmes urines : alors qu'il n'y a pas vraiment de différences de distribution des concentrations entre les urines TS et AMN, les concentrations sont plus élevées dans certaines urines SEP.
- La figure 6 illustre les taux d'hétérodimère MRP8/14 dosés dans les mêmes urines :alors qu'il n'y a pas vraiment de différence entre les concentrations des urines TS et AMN, on observe des plus fortes concentrations dans certaines urines SEP, correspondant peut-être à une sous population de patients SEP caractérisée par une activité de la maladie. MRRP8/14 dosé dans les urines est un marqueur de l'activité de la maladie SEP caractérisée par un pic d'inflammation).
- La figure 7 récapitulative confirme qu'il n'y a pas de différence significative de concentration en MRP8 et en MRP14 entre les urines TS, AMN et SEP, alors qu'une faible différence de concentration en MRP8/14 est observée entre ces urines, cette concentration étant plus élevée en moyenne dans les urines SEP et étant un marqueur de l'activité de la maladie (pic d'inflammation).

### Exemple 14 : Protocoles ELISA utilisés pour le dosage des protéines GM2AP et Saposine B.

Les protéines GM2AP ou Saposine B ont été dosées dans des urines humaines en utilisant des anticorps polyclonaux anti-GM2AP ou anti-Saposine B ? en suivant le protocole Elisa décrit par Gardas et al. (Glycoconjugate Journal 1, 37-42, 1984). Les principales étapes sont brièvement décrites ci-dessous :

A chaque étape, les puits d'une microplaque de 96 puits sont remplis avec 200 µl de la solution désignée. Les puits sont d'abord « coatés » avec une solution de GM2AP (protéine recombinante procaryote) diluée à 50 ng/ml dans un tampon carbonate-bicarbonate, pH 9,6. Après incubation une nuit à 4°C, la solution est éliminée et les puits sont lavés quatre fois avec du tampon PBS pH 7,4 contenant du Tween-20 0.05% (PBS-Tween). Les microplaques ainsi coatées sont stockées à 4°C pendant environ 2 semaines.

Les échantillons d'urine à trois dilutions différentes (20x, 40x et 80x ou d'autres dilutions appropriées) sont incubés avec une dilution appropriée de l'anticorps polyclonal de lapin anti-GM2AP ou anti-Saposine B pendant une nuit à 4°C. Une série de dilutions standard d'une protéine recombinante allant de 2,0 à 62,5 ng/ml est utilisée pour réaliser la gamme étalon et sont traitée de la même façon. Toutes les dilutions sont faites en tampon PBS-Tween contenant 1 mg/ml d'ovalbumine. Ainsi, 0,2 ml de chaque solution incubée est ajoutée dans des puits « coatés » en duplicat et les plaques sont laissées pendant 2 heures à température ambiante. Les puits sont alors lavés quatre fois en PBS-Tween et remplis encore avec une solution d'anticorps de chèvre anti-IgG de lapin couplés à la peroxidase et diluée environ 1200 fois. Après une incubation de 2 heures à température ambiante, les puits sont lavés quatre fois en PBS-Tween et remplis e nouveau avec le réactif de coloration. Le réactif de coloration consiste en 100 mg d'acide 2,2'-azino-di-(3-éthylbenzothiazoline) sulfonique et 10µl de 30% de peroxide d'hydrogène pendant une heure à température ambiante et le degré de coloration de chaque micropuits est estimé par lecture d'absorbance à 405 nm.

Une courbe standard est construite en mettant en abscisse la concentration de GM2AP de la gamme étalon ou de Saposine B avec une échelle logarithmique et en ordonné le pourcentage d'absorbance avec une échelle linéaire. Le pourcentage d'absorbance de l'échantillon est le rapport d'absorbance entre l'échantillon d'urine et le contrôle qui contient seulement l'antisérum, sans l'antigène soluble.

Une solution de protéine recombinante GM2AP produite en système procaryote, et de concentration 3 mg/ml est diluée en tampon carbonate 50 mM, pH 9,6 et 50µl sont ajoutés dans chaque puits d'une microplaque à 96 puits, soit 50µl par puits d'une solution à 0,5 µg/ml. Les plaques ainsi préparées sont incubées une nuit à température ambiante. L'anticorps polyclonal anti-GM2AP produit dans le laboratoire (lapin 79) a été purifié et dilué en tampon PBS-Tween 0,05% en présence de sérum de cheval 10%. Cette solution est diluée au 1/8000^{ème}. La solution est utilisée pour réaliser une gamme étalon avec 8 points de gamme couvrant les concentrations de 0 à 500 ng/ml. Une préincubation est réalisée pendant une nuit à température ambiante ente 100 µl d'anticorps et 100 µl d'échantillon d'urine à doser ou de solution protéine recombinante GM2AP ou Saposine B servant pour la gamme étalon. Après lavage de la microplaque en PBS-Tween, 50µl du mélange d'incubation sont ajoutés par puits, puis incubés pendant deux heures à température ambiante. La microplaque est de nouveau lavée en PBS-Tween, puis 50 µl d'anticorps anti-IgG de lapin couplé à la peroxidase et dilués au 1/5000 sont ajoutés dans chaque micropuits de la plaque et incubés pendant deux heures à température ambiante. Après de nouveaux lavages de la microplaque, 100µl d'OPD sont ajoutés dans chaque puits et incubés pendant 20 minutes à température ambiante. La coloration de chaque puits, proportionnelle à la concentration de GM2AP ou de Saposine B reconnue par l'anticorps spécifique utilisé, est estimée par lecture d'absorbance.

Une solution de protéine recombinante GM2AP ou Saposine B produite en système procaryote, et de concentration 3 mg/ml est diluée en tampon carbonate 50 mM, pH 9,6 et 50µl sont ajoutés dans chaque puits d'une microplaque à 96 puits, soit 50µl par puits d'une solution à 1,5 µg /ml. Les plaques ainsi préparées sont incubées une nuit à température ambiante. Les anticorps polyclonaux anti-peptides GM2AP produits dans le laboratoire (lapin 190 et lapin 191) purifiés sont utilisés seuls ou en mélange dilués au 1/1000 pour chacun en tampon PBS-Tween 0,05% en présence de sérum de cheval 10% . La gamme étalon est réalisée en utilisant de la protéine recombinante procaryote GM2AP ou Saposine B diluée de façon à couvrir la gamme de concentration 0 à 1500 ng/ml avec 8 points. 100 µl d'anticorps (un anticorps ou les deux ensemble) sont pré-incubés en présence de 100µl d'échantillon d'urine à tester ou de solution GM2AP ou Saposine B recombinante, pendant une nuit à température ambiante. Après lavage de la microplaque en PBS-Tween, 50µl du mélange d'incubation est ajouté par puits puis incubés pendant deux heures à température ambiante. La microplaque est de nouveau lavée en PBS-Tween, puis 50 µl d'anticorps anti-IgG de lapin couplé à la peroxidase, dilués au 1/5000, sont ajoutés dans chaque micropuits de la plaque et incubés pendant deux heures à température ambiante. Après lavage de la microplaque, 100µl d'OPD sont ajoutés dans chaque puits et incubés pendant 20 minutes à température ambiante. La coloration de chaque puits, proportionnelle à la concentration de GM2AP ou Saposine B reconnue par l'anticorps spécifique utilisé, est estimée par lecture d'absorbance.

### Exemple 15 : Dosage des protéines GM2AP dans les urines.

La protéine GM2AP a été dosée dans les urines de 22 patients atteints de sclérose en plaques (SEP), 5 patients atteints d'autres maladies neurologiques (OND) et 9 individus choisis parmi la population active et recueillies pendant une visite médicale (Healthy), en suivant le protocole Elisa décrit ci-dessous, utilisant des anticorps polyclonaux anti-GM2AP. Les patients SEP sélectionnés pour cette étude sont des patients tout azimut, c'est-à-dire avec différents stades et profils de la maladies, et différents traitements, etc...

Les résultats du dosage sont rapportés dans la figure 8. Alors que seulement 0/5 urines OND et 2/9 urines dites 'Healthy' présentent une concentration en GM2AP supérieure à 200 ng/ml, 10/22 (soit 45%) présentent une concentration supérieure à 200 ng/ml.

Ces résultats indiquent que si la protéine GM2AP est présente en très faible concentration (<400 ng/ml) dans les urines d'individus de la population active, elle est présente en plus forte concentration dans les urines de patients SEP. Cependant 12 urines SEP présentent également des taux faibles de GM2AP. Parmi ces 12 patients, 10 sont en traitement. Les fortes concentrations urinaires de GM2AP semblent être un marqueur de la pathologie SEP, et plus précisément un marqueur d'un stade ou d'une forme de la maladie, de l'activité de la maladie, et est certainement influencé par tout traitement en cours. Notons que deux individus de la population active ont des concentrations élevées de GM2AP (ces deux cas ont été inclus volontairement dans l'étude, car ils présentaient tous deux une activité gliotoxique dans leur urines contrairement aux autres individus de cette même catégories). Il est impossible de savoir s'il s'agit d'individus sains, ou atteints d'une pathologie, ou des individus atteints d'une sclérose en plaques car les échantillons des individus dits « Healthy » ont été prélevés de manière anonyme, sans connaissance du dossier clinique.

Des concentrations urinaires plus élevées de GM2AP sont détectées dans les urines de patients SEP ; une concentration élevée de GM2AP peut être alors un marqueur de la pathologie SEP, et plus précisément d'une forme de la maladie, d'un stade de la maladie, d'une période d'activité, et peut être influencée par tout traitement en cours. Ces concentrations urinaires élevées en GM2AP peut également avoir une valeur prédictive d'un début d'aggravation de la maladie, ou d'une SEP benigne en début d'évolution, etc ....

Les valeurs absolues des concentrations GM2AP détectées dans les urines sont dépendantes de l'affinité et de la spécificité de l'anticorps utilisé, mais d'une façon générale, la tendance entre les trois groupes d'individus est conservée quelque soit l'anticorps utilisé.

### Exemple 16 : Dosage des protéines Saposine B dans les urines.

La protéine Saposine B a été détectée dans les même échantillons d'urines que ceux utilisés pour l'étude de la détection de GM2AP. Les dosages ont été réalisés en parallèle avec ceux du GM2AP, dans une même étude, en suivant le protocole Elisa décrit ci-dessous, utilisant des anticorps polyclonaux anti-Saposine B.

Les résultats du dosage Saposine B sont reportés dans la Figure 9. 0/5 urines OND et 2/9 urines Healthy présentent une concentration en Saposine B supérieure à 2 µg/ml , alors que 6/22 (soit 27%) présentent une concentration supérieure à 2 µg /ml.

Ces résultats indiquent que la protéine Saposine B est présente dans chaque urine (population dite saine ou population dite malade) à des concentrations non négligeables, c'est-à-dire < 2µg /ml. Ces résultats de dosage sont compatibles avec ceux décrits dans la bibliographie. Cependant même si la Saposine B est présente dans chaque urine, elle semble être présente en plus forte concentration dans certaines urines SEP. Cette augmentation de concentration de saposine B dans les urines Sep est peut-être masquée par la concentration basale de cette protéine à l'état ordinaire. Ainsi les fortes concentrations urinaires de Saposine B semblent être un marqueur de la pathologie SEP, et plus précisément un marqueur d'un stade ou d'une forme de la maladie, de l'activité de la maladie, et est certainement influencée par tout traitement en cours. La Saposine B dosée seule semble être cependant un marqueur un peu moins discriminant d'une forme ou d'une activité de la maladie que le GM2AP. Notons encore uen fois que deux individus de la population active ont des concentrations élevées de Saposine B et que ce sont les deux même individus qui présentaient aussi une forte concentration en GM2AP dans leurs urines.

En conclusion, des concentrations urinaires plus élevées de Saposine B sont détectées dans les urines de patients SEP ; une concentration élevée de Saposine B peut être alors un marqueur de la pathologie SEP, et plus précisément d'une forme de la maladie, d'un stade de la maladie, d'une période d'activité, et peut être influencée par tout traitement en cours. Ces concentrations urinaires élevées en GM2AP peut également avoir une valeur prédictive d'un début d'aggravation de la maladie, ou d'une SEP benigne en début d'évolution, etc .... Mais d'une façon générale, les fortes concentrations de Saposine B seules semblent être des marqueurs moins discriminants que les fortes concentrations de GM2AP seules.

Les valeurs absolues des concentrations Saposine B détectées dans les urines sont dépendantes de l'affinité et de la spécificité de l'anticorps utilisé, mais d'une façon générale, la tendance entre les trois groupes d'individus est conservée quelque soit l'anticorps utilisé.

### Exemple 17 : Co-dosage des protéines GM2AP et Saposine B dans les urines.

La Figure 10 reporte les concentrations de GM2AP dosée dans les échantillons d'urine décrites dans la Figure 5 par rapport à la concentration de Saposine B dosée dans ces mêmes échantillons et décrite dans la Figure 6. Dans ce graphe sont reportés les échantillons SEP (losanges foncés) et les échantillons OND et 'Healthy' (losanges blancs).

Sur ce graphe, il apparaît clairement que :
- plus la concentration en GM2AP est élevée dans les urines, plus la concentration en Saposine B est élevée. (Nous avons montré que ce n'est pas un cas général avec d'autres protéines et que cela ne traduit pas une perturbation rénale, avec le dosage de la créatinine en parallèle pour chacun des échantillons testés.) ;
- les concentrations élevées de GM2AP et Saposine B sont caractéristiques des échantillons SEP (à l'exception des deux urines de la population active, mentionnées ci-dessus).Ces concentrations élevées conjointes de GM2AP et Saposine B sont des marqueurs de la pathologie SEP, plus précisément d'une fenêtre de la maladie (quadran à droite et en haut du graphe).

En conclusion, cette analyse confirme que des concentrations urinaires élevées de GM2AP (>400 ng /ml) et de Saposine B (>2 µg /ml) sont co-détectées dans les urines de patients SEP et peuvent représenter des marqueurs de la pathologie SEP, plus précisément d'une forme de la maladie, d'un stade de la maladie, d'une période d'activité, et peuvent être influencée par tout traitement en cours. Il est avantageux de doser les deux protéines en parallèle dans chaque échantillons, et de considérer les deux concentrations.

Dosage de GM2AP et Saposine B dans l'urine de deux patients en cinétique.

### Patient SEP n°1 - Forme Rémittente Progressive.

Des urines de ce patient ont été prélevées au cours de l'évolution de sa maladie. Le patient a été hospitalisé à J0 pour une poussée. Il a subit à J1 un flash de corticoïdes puis a été suivi dans le temps d'un point de vue clinique (le flash a apporté une amélioration clinique). La figure 11 montre le profil de dosage du GM2AP et de la Saposine B dans ces urines au cours de l'évolution, et la figure 12 montre le profil du produit des deux concentrations en GM2AP et Saposine B, traduisant une co-détection de concentrations élevées. Les concentrations en GM2AP et Saposine B élevées au moment de la poussée et de l'hospitalisation, diminuent progressivement dans le temps après le flash de corticoïdes jusqu'à 90 jours.

### Patient SEP n°2 - Forme Progressive.

Des urines de ce patient ont été prélevées au cours de l'évolution de sa maladie. Le patient a été hospitalisé à J0 pour une poussée. Il a subit à J1 un flash d'Endoxan puis a été suivi dans le temps d'un point de vue clinique (le flash a apporté une amélioration clinique et à J60, des signes d'aggravation de la maladie ont été observés). La figure 13 montre le profil de dosage du GM2AP et de la Saposine B dans ces urines au cours de l'évolution, et la figure 14 montre le profil du produit des deux concentrations en GM2AP et Saposine B, traduisant une co-détection de concentrations élevées. Les concentrations en GM2AP et Saposine B élevées au moment de la poussée et de l'hospitalisation, diminuent progressivement dans le temps après le flash d'Endoxan (ou encore appelé cyclophosphamide) jusqu'à 23 jours et semblent augmenter pour devenir élevées à J60, montrant ainsi une parfaite corrélation avec l'évolution des signes cliniques.

Ces résultats confirment que :
- des concentrations fortes de GM2AP et Sapsoine B dans les urines sont des marqueurs de la pathologie SEP, et en particulier la co-détection des fortes concentrations des deux protéines conjointement (traduit par le produit des deux concentrations) ;
- les fortes concentrations de GM2AP et Saposine B dans les urines sont des marqueurs de l'activité de la maladie (ici pendant la poussée) ou sont des marqueurs influencés par les traitements immuno-suppresseurs comme les corticoïdes ou l'Endoxan qui abaissent les concentrations.

Cet exemple illustre le fait que ces marqueurs peuvent être utilisés entre autres :
- pour réaliser un suivi thérapeutique d'un patient et évaluer le bénéfice thérapeutique d'un traitement pour un patient donné ; ou
- de prédire une aggravation de la maladie, prédire un pic d'activité, etc...
- de décider une (re)prise thérapeutique anticipée sur les signes cliniques

### Exemple 18 : Corrélation ente la détection des protéines MRP 14, GM2AP et Saposine B dans les urines et la gliotoxicité mesurée dans ces urines.

Afin de vérifier une corrélation entre la présence de ces protéines seules ou en combinaison dans les urines et la gliotoxicité des urines, ont été dosées en parallèle les concentrations en protéine d'intérêt et la gliotoxicité d'un échantillonage d'urines de patients atteints de sclérose en plaques (SEP), de patients atteints d'autres maladies neurologiques (OND) et d'individus issus de la population active dit « Healthy ». Parmi les patients SEP, on note des patients avec différentes formes et stades de la maladie, sous traitement ou non, à différentes activités de la maladie.

Les protéines MRP, GM2AP et Saposine B ont été dosées dans des urines humaines en suivant les protocoles Elisa décrits ci-dessus. Les dosages analysés dans cet exemple sont ceux décrits dans les exemples précédents. Chaque échantillon d'urine analysé en Elisa a été analysé par le test MTT pour mesurer la gliotoxicité de chaque échantillon. La gliotoxicité est exprimée en pourcentage de cellules mortes (estimé par colorimétrie en utilisant les sels de tetrazolium) d'une lignée cellulaire astrocytaire murine (CLTT1.1) après 48 heures d'incubation en présence d'urine centrifugée.

La figure 15 représente la concentration en GM2AP en fonction de la gliotoxicité des urines déterminée par test MTT.

22 urines SEP (losanges gris), 5 urines AMN (losanges noirs) et 9 urines dites « Healthy » (losanges noirs) ont été reportés sur le graphe. Ce sont les mêmes urines qui ont été étudiées dans les exemples 15 et 16. On observe que toutes les urines témoins (OND et Healthy) ont des taux en GM2AP faibles (<400 ng/ml) et une gliotoxicité faibles (<15%), à l'exception d'une urine témoin Healthy (déjà commentée dans l'exemple 15) pour laquelle on observe une forte concentration en GM2AP et une gliotoxicité.

Les urines SEP sont réparties en trois sous-populations :
- urines à faible concentration en GM2AP (<400 ng /ml) et faible gliotoxicité (<15%),
- urines à faible concentration en GM2AP (<400 ng /ml) et gliotoxicité (>15%), soit essentiellement 3 urines,
- urines à forte concentration en GM2AP (>400 ng /ml) et forte gliotoxicité (>15%).

Ces trois sous populations traduisent peut-être des sous populations SEP, c'est-à-dire différentes formes ou stades de la maladie, différentes activités de la maladie, différents bénéfices thérapeutiques, ....

Cependant on peut noter que toutes les urines présentant une forte concentration en GM2AP présentent également une forte gliotoxicité.

En conclusion : on observe une corrélation entre concentration urinaire élevée en GM2AP et gliotoxicité (toutes les urines avec une forte concentration en GM2AP sont gliotoxiques (10/10), et toutes les urines avec une faible concentration en GM2AP ne sont pas gliotoxiques (<15%), à l'exception de 3 urines/12 SEP). Ceci traduit l'implication de la protéine GM2AP dans le mécanisme de gliotoxicité, seule ou en combinaison, sous sa forme naturelle ou modifiée, mais reconnaissable par un anticorps anti-GM2AP. De plus la co-détection d'une forte concentration en GM2AP dans les urines et d'une forte gliotoxicité corrèle avec une sous population de patients atteints de SEP.

La figure 16 représente la concentration en Saposine B en fonction de la gliotoxicité des urines déterminée par test MTT.

22 urines SEP (losanges gris), 5 urines AMN (losanges noirs) et 9 urines dites «Healthy » (losanges gris clair) ont été reportés sur le graphe. Ce sont les mêmes urines qui ont été étudiées dans les exemples .15 et 16. On observe que plus les urines sont riches en Saposine B, plus elles sont gliotoxiques. Il y a une corrélation assez nette entre concentration de Saposine B et gliotoxicité des urines.

En conclusion : on observe une corrélation entre concentration urinaire élevée en Saposine B et gliotoxicité. Ceci traduit l'implication de la protéine Saposine B dans le mécanisme de gliotoxicité, seule ou en combinaison, sous sa forme naturelle ou modifiée mais reconnaissable par l'anticorps anti-saposine B utilisé pour le dosage.

La figure 17 représente le produit des concentrations en GM2AP et Saposine B en fonction de la gliotoxicité des urines déterminée par test MTT.

Les 22 urines SEP (losanges gris), 5 urines AMN (losanges noirs) et 9 urines dites «Healthy » (losanges gris clair) des exemples 15 et 16 ont été reportés dans la figure 17. La gliotoxicité de ces urines est analysée en fonction du produit des concentrations en GM2AP et Saposine B, c'est-à-dire en fonction de la co-détection des deux protéines dans les urines. On observe très nettement une corrélation entre le produit des deux concentrations GM2AP et Saposine B et la gliotoxicité, bien plus importante qu'en ne considérant qu'une seule protéine. On observe que 5/5 des urines OND ont un produit de concentration GM2AP et Saposine B faible et une gliotoxicité faible ; 8/9 urines « Healthy » ont un produit de concentration GM2AP et SaposineB faible et/ou une gliotoxicité faible. Par contre, on distingue essentiellement trois sous-populations d'urines SEP :
- urines à faible concentration en GM2AP.Saposine B et faible gliotoxicité (<15%),
- urines à forte concentration en GM2AP. Saposine B et forte gliotoxicité (>15%).

Ces deux sous populations traduisent peut-être des sous populations SEP, c'est-à-dire différentes formes ou stades de la maladie, différentes activités de la maladie, différents bénéfices thérapeutiques, .... Cependant il est très important de noter que toutes les urines présentant une forte concentration en GM2AP et Saposine B, c'est-à-dire ayant conjointement une forte concentration en GM2AP et Saposine B, présentent également une forte gliotoxicité. Les deux sous populations de patients SEP sont d'autant plus marquées et nettes que l'on considère conjointement les trois marqueurs : gliotoxicité, concentration élevée en GM2AP et concentration élevée en Saposine B. Ceci est confirmé à la figure 18.

En conclusion : on observe une corrélation entre concentration urinaire élevée de GM2AP et Saposine B et Gliotoxicité. Toutes les urines avec une forte concentration en GM2AP et Saposine B sont gliotoxiques, et toutes les urines avec une faible concentration en GM2AP et Saposine B ne sont pas gliotoxiques (<15%), à l'exception de 2 urines/22 SEP. Ceci traduit l'implication des deux protéines GM2AP et Saposine conjointement ou en combinaison dans le mécanisme de gliotoxicité, sous leur forme naturelle ou modifiée mais reconnaissable par les anticorps anti-GM2AP et anti-saposine B utilisés pour le dosage. De plus la co-détection d'une forte concentration urinaire en GM2AP et Saposine B et d'une forte gliotoxicité corrèle avec une sous population de patients atteints de SEP (stade, forme, activité, traitement de la maladie ?), par rapport à une autre sous population. Ces trois marqueurs considérés conjointement permettent de discriminer entre deux sous populations de patients SEP.

Evolution de la gliotoxicité et des concentrations en GM2AP et Saposine B en fonction de l'évolution de la maladie de deux patients après et pendant traitement.

La corrélation entre gliotoxicité, forte concentration en GM2AP ET Saposine dans les urines et pathologie SEP a également été confirmée en mesurant ces trois paramètres dans l'urine de deux patients au cours de l'évolution de leur maladie.

Patient n°1 : SEP forme rémittente-progressive, hospitalisé à J0 pour une poussée et ayant reçu un flash de corticoïde à J1. Après le flash, il a montré une amélioration clinique jusqu'à J90 - (cf. figures 11,12),

Patient n°2 : SEP forme progressive, hospitalisé à J0 pour une poussée et ayant reçu un flash d'Endoxan (encore appelé cyclophosphamide) à J1. A J60, il présente de nouveaux des signes cliniques d'aggravation de sa maladie - (cf. figures 13,14).

Pour les deux patients, il a été montré :
- une corrélation entre la gliotoxicité urinaire et l'évolution clinique de la maladie (lorsque les signes cliniques sont sévères, la gliotoxicité est élevée ; lorsque les signes cliniques diminuent suite au traitement, la gliotoxicité diminue et devient stationnaire ; lorsque les signes d'aggravation apparaissent après le traitement, la gliotoxicité semble augmenter de nouveau),
- une corrélation entre le taux de gliotoxicité dans les urines de patients et les concentrations de GM2AP et Saposine B, et
- une corrélation entre les concentrations élevées de GM2AP et Saposine B et l'évolution clinique de la maladie.

En conclusion : le dosage des protéines GM2AP ET Saposine B dans les urines est un bon marqueur discriminatif d'une sous population de la SEP (stade, forme, activité, traitement de la maladie). Les protéines GM2AP et/ou Saposine B sont impliquées dans le mécanisme de gliotoxicité, seules ou en combinaison, sous leur forme naturelle ou sous une forme reconnaissable par les anticorps polyclonaux utilisés pour leur dosage. Comme les protéines GM2AP et Saposine sont co-détectées en forte concentration dans les urines gliotoxiques, il est possible que ces deux protéines agissent en combinaison pour induire la gliotoxicité.

Exemple 19 : Analyse immunohistochimique de l'expression des protéines GM2A, SAPB, MRP14 et MRP8 dans un système de culture producteur de gliotoxine in vitro (cultures de monocytes), ainsi que dans le tissu cérébral normal et pathologique de SEP et de témoins.

Protocole : Des cultures de monocytes d'un patient atteint de SEP et d'un témoin sain ont été réalisées en parallèle, selon le protocole présent décrit brièvement. A partir de sang périphérique de ces deux volontaires prélevé sur ACD, les PBMC (Peripheral Blood Mononuclear Cells) sont isolés sur Ficoll en utilisant la technique connue de l'homme de l'art. Les cellules récupérés (au niveau de l'anneau) sont lavées deux fois en milieu RPMI. Les cellules sont alors énumérées sur Kovas-slide et sont ensemencées en flacon primaire de 25 cm² ou sur lame Labteck (8 cupules) (en permanox) en milieu RPMI supplémenté avec 15% de sérum AB humain à J0. Les cellules sont cultivées sur des lames alvéolées de type « Labtek » afin de disposer d'un support direct pour l'analyse des monocytes qui adhèrent au support et se différencient ultérieurement en macrophages. Pour les lames, 2.10⁶ cellules sont ainsi ensemencées à raison de 0,25 10⁶ cellules/puits. Pour les flacons, 4.10⁶ cellules sont ensemencées à raison de 0,25 10⁶ cellules/puits. A J1, les cellules en suspension sont récupérées et les puits des Labteck ou les flacons sont lavés deux fois en RPMI (au préalable chauffé à 37°C) avant de rajouter du milieu RPMI supplémenté avec 5% de sérum AB humain. A J1, J3, J6, J9, J12 ou 14, J15 le milieu de culture est changé ; les surnageants sont prélevés et les cellules fixées sur lames en utilisant les techniques connues de l'homme de l'art. A chaque changement de milieu, au moins deux lames ont été fixées en paraformaldéhyde et conservées pour l'analyse immunohistochimique.

Composition du milieu : RPMI (500 ml) avec 15ml de glutamate 200 mM, 5 ml de pyruvate de sodium 100 µM, 5 ml d'acides aminés non essentiels (100x), des antibiotiques penicilline et streptomycine 100 000 U /µl et des anticorps anti-interferon humains à 100 U/µl.

Résultats : Quatre cultures de monocytes *in vitro* ont été ainsi étudiées en cinétique : deux cultures de monocytes issus de sang d'individus contrôles et deux cultures de monocytes issus de patients SEP. A différents temps de la culture (J0, J1, J3, J6, J9, J12, ....), les surnageants correspondants ont été également récupérés. Une fois la cinétique complétée, les lames correspondant aux différents jours de cultures ont été incubées en présence d'anticorps polyclonaux anti-GM2A, SAP-B, MRP-8 et MRP14. La gliotoxicité de chaque surnageant ainsi récupéré a été estimé par test MTT. La concentration en protéine GM2AP, MRP14 et Saposine B a également été déterminée dans chaque surnageant par protocole Elisa comme décrit dans les exemples 13 et 14.

Les résultats d'immunofluorescence sur cellules fixées sont résumés ci-dessous ; on peut noter :
- une absence d'expression de MRP8 à tous les stades des 2 cultures
- une expression nette de MRP-14 dans la période entre J9 et J15, retrouvée dans les deux cultures, quoique plus forte dans la culture SEP. Cette expression semble corréler une étape de différenciation macrophagique.
- une très faible expression (faible intensité et faible nombre de cellules) est observée en début de culture dans la culture témoin et correspond vraisemblablement à la présence physiologique de GM2A dans les lysosomes macrophagiques.
- Dans la culture SEP, une expression beaucoup plus nette de GM2A (plus forte intensité et nombre de cellules plus important) est observée, avec un marquage cytoplasmique relativement homogène entre J3 et J6, disparaît à J9 et est à nouveau notée à J14-J15 avec un marquage intense et localisé à la périphérie cytoplasmique, dessinant le contour interne de la membrane plasmique. Ces observations ne sont pas retrouvées dans l'ensemble des lames témoins.

L'analyse avec le anticorps anti-SAP-B n'a pas permis d'obtenir un marquage immuno-histochimique interprétable.

Dans les cultures de monocytes SEP déjà effectuées, 3/3 ont présenté un pic de gliotoxicité à J9 et 2/3 un pic plus faible à J6. Aucun pic n'étant détecté dans les cultures de monocytes de 2/2 témoins non-SEP analysés en parallèle. De même, le dosage des protéines MRP14, GM2AP et Saposine B dans le surnageant des cultures cellulaires au cours de la cinétique a montré que les protéines SapB et GM2AP sont détectées par Elisa dans les surnageants des monocytes SEP et non dans ceux des monocytes témoins, aux jours J6 et surtout J9 de la culture ; les protéines ne sont pas détectées au-delà de cette cinétique. Notons que les anticorps utilisés pour le dosage peuvent reconnaître les formes physiologiques des protéines, mais également des formes complexées et/ou modifiées.

On constate donc que la période J6-J9 pendant laquelle on observe une gliotoxicité la plus importante dans le surnageant, est couverte par la période J3-J15 pendant laquelle on observe une production moins différenciée du témoin négatif de GM2A dans les cellules avec des fluctuations quantitatives et qualitatives de son expression cellulaire (quantité d'expression et localisation cellulaire).

### Exemple 20: Technique d'immunohistologie sur coupes de cerveaux en paraffine.

Les coupes histologique préparées en paraffine sont déparaffinées en xylène et alcool avant de subir un prétraitement qui a pour but de démasquer les antigènes ; ce prétraitement peut correspondre à (i) deux fois 5 minutes sous micro-onde (750W) en présence d'un tampon citrate de sodium, acide citrique, (ii) un traitement à l'acide par incubation 15 minutes dans une solution d'acide périodique 1 % ou par incubation 5 minutes dans une solution d'acide formique 99%. Les peroxydases endogènes sont ensuite bloquées par incubation des lames 30 minutes en eau oxygénée 1% puis lavage extensif en eau pendant 15 minutes. Le bruit de fond est bloqué en incubant les lames 30 minutes en présence de PBS Triton 0.03%, 10% sérum Donkey (pour les anticorps polyclonaux) ou 10% sérum Goat (pour les anticorps monoclonaux). Un marquage avec l'anticorps primaire est réalisé en appliquant 100 à 200 µl de solution d'anticorps primaire par lame (0.5 à 5 µg /ml selon le titre) dans du PBS Triton 0.03% puis en incubant 2 heures à température ambiante. Les lames sont ensuite rincées 3 fois en PBS-Triton pendant 10 minutes. Un marquage anticorps secondaire est réalisé en utilisant des anticorps biotinylés capables de se fixer spécifiquement aux anticorps primaires, par exemples des anti-IgG de lapin ou anti-IgG de souris dilués dans du PBS-Triton 0.03%. les lames sont lavées et incubées dans une solution pendant 2 heures (2 µl complexe streptavidine-biotine-peroxides, 1600 µl PBS-Triton 0.03%). Les lames sont de nouveau lavées avant d'être révélées à l'abri de la lumière dans le tampon A puis rincées à l'eau avant observation microscopique. Tampon A pour 5 lames : 25 ml Tris0.05M pH 7.6, 2.5 ml Imidazole 1M, 15 ml eau stérile, 2 ml DAB 5 mg/ml, 5 ml Nickel d'ammonium 10%, 30 µl H₂O₂ 1%.

Les mêmes anticorps ont été utilisés pour une étude immunohistochimique, selon la technique décrite brièvement ci-dessous, sur lames paraffinées obtenues par coupe au microtome de cerveaux prélevés post-mortem de SEP et de témoins décédés de pathologies non-neurologiques.

Les résultats de l'analyse sont résumés ci-dessous :

Il n'y a pas de marquage des cerveaux « non-SEP » et SEP dans la substance grise et la substance blanche « normale (non lésée) avec les différents anticorps anti- MRP8, MRP14, GM2A. Une réactivité non spécifique n'a pas permis d'interpréter les résultats avec l'anticorps anti-saposine B dans cette application immunohistochimique.

Par contre on note, dans les zones de plaques des cerveaux SEP :
- une réactivité anti-MRP 14 dans les cellules macrophagiques et microgliales, ayant une distribution relativement homogène sur toute l'étendue des zones de démyélinisation (plaques),
- une plus faible (moins fréquente) réactivité anti-MRP8 liée essentiellement aux infiltrats lymphoïdes périvasculaires
- une nette réactivité anti-GM2A dans les macrophages et microgliocytes des zones de plaques, avec une densité particulière dans les zones constituant le « mur glial » en limite périphérique de plaque. Un marquage de quelques astrocytes a aussi été retrouvé dans les zones de démyélinisation.

Ces différentes observations montrent qu'il existe une hyperexpression particulière des protéines MRP-14 et GM2A dans les cultures de monocytes de SEP produisant une activité gliotoxique dans leur surnageant, ainsi que dans les zones définissant des plaques de démyélinisation dans les cerveaux de SEP. Elles témoignent donc de la réalité de la coïncidence entre leur co-expression anormale, la production d'activité gliotoxique et les lésions de démyélinisation.

De plus, leur production anorrmale dans le contexte de la SEP, dans les cellules macrophagiques sanguines ainsi que dans celles du cerveau, indique qu'il est fondé de réaliser leur dosage dans les fluides biologiques pour corréler leur quantité avec l'activité lésionnelle et inflammatoire de la SEP.

### Exemple 21 : Mesure de l'activité des cellules T par prolifération des cellules T (Sredni et al., 1981).

Les cellules T sont lavées deux fois en milieu de culture pour éliminer toute trace d'IL2 présente dans le milieu initial de culture. Des lymphocytes B (EBV-LCL) ou des monocytes/macrophages pris comme cellules présentatrices de l'antigène, sont irradiées à 10000 rads, lavées deux fois avec du milieu de culture (RPMI). 2.10⁴ cellules T (2.10⁵ cellules /ml) et 2.10⁴ cellules B autologues irradiées (2.10⁵ cellules /ml) sont incubées ensemble en présence d'une gamme de concentration croissante de l'antigène sous un volume final de 200 µl dans des micropuits. Après 48 heures de culture à 37°C, 1 µCi de 3H- thymidine dans 50 µl de milieu RPMI est ajouté dans chaque puits. Les cellules T, seules à se diviser, incorporent la thymidine tritiée dans l'ADN. Après 18 heures de culture, les cellules de chaque micropuits sont récoltées sur des pastilles de laine de verre par aspiration. Après lyse osmotique des cellules, la radioactivité incorporée dans l'ADN est absorbée sur les pastilles (cell Harvester 530, Inotech). Chaque pastille séchée est placée dans un tube plastique qui contient 2 ml de scintillant ; la radioactivité b adsorbée sur chacune des pastilles est quantifiée dans un compteur bêta à scintillation liquide (LKB Rackbeta 1217). Les résultats sont exprimés comme moyenne arithmétique de cpm/culture ('coups par minute').

### Exemple 22 : Protocole de détection de l'association entre les peptides et les molécules d'histocompatibilité (approche APC transformées avec un peptide se fixant au CMH I).

### 1) Matériel :

Les sources de molécules d'histocompatibilité sont actuellement de deux types principaux : les cellules mutantes et les molécules d'histocompatibilité purifiées.

La cellule mutante utilisée est la cellule humaine T2 qui et un variant de la lignée T1 produite par fusion du lymphome T CEM et du lymphome B 721.174 (Salter and Cresswell Embo J 1986, 5: 943-949). Cette cellule qui est dépourvue de transporteurs de peptides contient des chaînes lourdes de molécules de classe I libres de peptides qui vont pouvoir accepter de peptides exogènes.

Des molécules d'histocompatibilité de classe I purifiées par chromatographie d'affinité à partir de lignées de cellules B humaines transformées par l'EBV peuvent être également utilisées. Dans ce cas les peptides endogènes doivent être éliminés par un traitement avec de l'urée1.5 M et de la soude 12.5 mM (pH 11.7) pendant 1 heure à 4°C,suivi de leur élimination par une colonne de désalage (PDLO, Pharmacia). Les molécules d'histocompatibilité sont immédiatement remises en présence des peptides à tester dans un tampon PBS avec 0.05% Tween 20, 2 mM EDTA, 0.1% NP40 et6mM CHAPS, en présence de 2µg/ml B2m pour faciliter la réassociation (Gnjatic et al., Eur J Immunol 1995 25 : 1638-1642).

Les peptides testés ont en général 8 à 10 résidus, parfois 11 ou 12. Ils ont été synthétisés par Néosystems (Strasbourg), ou par Chiron mimotopes (Victoria, Australie). Ils sont utilisés à des concentrations variant de 100 µM à0.1 nM.

### 2) Protocole de l'assemblage (Connan et al., Eur J Immunol 1994, 24 : 777 ; Couillin et al. Eur J Immunol 1995, 25 : 728-732).

Des aliquotes de 8.105 cellules dans un volume de 64 µl, répartis dans des tubes microfuge Eppendorf, sont mis en présence d'un tampon de lyse contenant 10 mM PBS, pH 7.5 1% NP40, des inhibiteurs de protéases (1 mM PMSF, 100 µM iodoacétamide, 2 µg /ml aprotinine, 10 µM leupeptine, 10 µM pepstatine et 10 µg/ml inhibiteur de trypsine). La lyse se fait en présence des peptides à tester pendant 30 minutes ou 1 heure à 37°C. Après élimination du matériel non solubilisé par une centrifugation à 15 000 tours /minute à 4°C, le surnageant et additionné de 140 µl de PBS contenant 0.05% de Tween 20, 3 mM d'azide de sodium, 1 mM PMSF et 10 mg /ml d'albumine bovine. Chaque échantillon est incubé pendant 20 heures à 4°C dans 2 puits d'une plaque à microtitration de type Nunc,Maxisorb, préalablement recouverts d'un anticorps monoclonal (10 µg /ml en PBS) qui reconnaît les molécules d'histocompatibilité ayant une(des) conformation(s) conforme(s) pour la présentation de peptides et semblable(s) à celle(s) présente(s) à la surface des cellules. La plaque recouverte d'anticorps est préalablement saturée par de l'albumine bovine à 10 mg /ml dans du PBS-Tween avant la mise de l'échantillon. Le second anticorps qui permet la détection de l'assemblage des molécules d'histocompatibilité est dirigé contre la B2m. Il est couplé soit à la biotine (NHS-LC biotin, Pierce) soit à la phosphatase alcaline (P-552, Sigma) et est incubé à 2 µg /ml pendant une heure à 37°C. Dans le cas de l'emploi de la biotine, une incubation de 45 minutes à 20-25°C avec de la streptavidine couplée à la phosphatase alcaline (E-2636, Sigma) est réalisée. L'activité de la phosphatase alcaline est mesurée en utilisant comme substrat le 4-méthyl-umbelliféryl-phosphate (M-8883, Sigma) à 100 µM dans de la diéthanolamine 50 mM, pH 9.5 avec du MgCl2 1 mM. La lecture est faite à 340/460 nm à l'aide d'un cytofluorimètre.

### 3) Stabilité des complexes HLA/peptides :

La stabilité des complexes précités a été étudiée car elle conditionne la bonne présentation de l'antigène et l'induction de la réponse T. A cet effet, on a utilisé soit du HLA purifié, soit le lysat de la cellule T2. Avec le HLA purifié, on a éliminé les peptides endogènes (comme décrit en 2)) puis on l'a mis en présence du peptide à tester en tube Eppendorf à 37°C, pendant des temps variables de quelques minutes à plusieurs jours. La phase suivante d'incubation sur plaque de 96 puits (comme décrit en 2) avec l'anticorps anti-HLA se fait pendant une heure à 37°C. La révélation est effectuée de manière classique. Avec le lysat de la cellule T2, toutes les incubations sont également faites à 37°C, après ajout de tous les inhibiteurs de protéases.

LISTE DE SEQUENCES

<110> BIOMERIEUX

<120> Utilisation d'un polypeptide pour détecter, prévenir ou
      traiter un état pathologique associé à une maladie
      dégénérative, neurologique ou auto-immune

<130> SEP22

<140>
<141>

<150> FR9909372
<151> 1999-07-15

<160> 75

<170> PatentIn Ver. 2.1

<210> 1
<211> 4393
<212> PRT
<213> Homo sapiens

<400> 1
Met Gly Trp Arg Ala Pro Gly Ala Leu Leu Leu Ala Leu Leu Leu His
  1               5                  10                  15

Gly Arg Leu Leu Ala Val Thr His Gly Leu Arg Ala Tyr Asp Gly Leu
             20                  25                  30

Ser Leu Pro Glu Asp Ile Glu Thr Val Thr Ala Ser Gln Met Arg Trp
         35                  40                  45

Thr His Ser Tyr Leu Ser Asp Asp Glu Asp Met Leu Ala Asp Ser Ile
     50                  55                  60

Ser Gly Asp Asp Leu Gly Ser Gly Asp Leu Gly Ser Gly Asp Phe Gln
 65                  70                  75                  80

Met Val Tyr Phe Arg Ala Leu Val Asn Phe Thr Arg Ser Ile Glu Tyr
                 85                  90                  95

Ser Pro Gln Leu Glu Asp Ala Gly Ser Arg Glu Phe Arg Glu Val Ser
            100                 105                 110

Glu Ala Val Val Asp Thr Leu Glu Ser Glu Tyr Leu Lys Ile Pro Gly
        115                 120                 125

Asp Gln Val Val Ser Val Val Phe Ile Lys Glu Leu Asp Gly Trp Val
    130                 135                 140

Phe Val Glu Leu Asp Val Gly Ser Glu Gly Asn Ala Asp Gly Ala Gln
145                 150                 155                 160

Ile Gln Glu Met Leu Leu Arg Val Ile Ser Ser Gly Ser Val Ala Ser
                165                 170                 175

Tyr Val Thr Ser Pro Gln Gly Phe Gln Phe Arg Arg Leu Gly Thr Val
            180                 185                 190

Pro Gln Phe Pro Arg Ala Cys Thr Glu Ala Glu Phe Ala Cys His Ser
        195                 200                 205

Tyr Asn Glu Cys Val Ala Leu Glu Tyr Arg Cys Asp Arg Arg Pro Asp
    210                 215                 220

Cys Arg Asp Met Ser Asp Glu Leu Asn Cys Glu Glu Pro Val Leu Gly
225                 230                 235                 240

Ile Ser Pro Thr Phe Ser Leu Leu Val Glu Thr Thr Ser Leu Pro Pro
                245                 250                 255

Arg Pro Glu Thr Thr Ile Met Arg Gln Pro Pro Val Thr His Ala Pro
            260                 265                 270

Gln Pro Leu Leu Pro Gly Ser Val Arg Pro Leu Pro Cys Gly Pro Gln
        275                 280                 285

Glu Ala Ala Cys Arg Asn Gly His Cys Ile Pro Arg Asp Tyr Leu Cys
    290                 295                 300

Asp Gly Gln Glu Asp Cys Glu Asp Gly Ser Asp Glu Leu Asp Cys Gly
305                 310                 315                 320

Pro Pro Pro Pro Cys Glu Pro Asn Glu Phe Pro Cys Gly Asn Gly His
                325                 330                 335

Cys Ala Leu Lys Leu Trp Arg Cys Asp Gly Asp Phe Asp Cys Glu Asp
            340                 345                 350

Arg Thr Asp Glu Ala Asn Cys Pro Thr Lys Arg Pro Glu Glu Val Cys
        355                 360                 365

Gly Pro Thr Gln Phe Arg Cys Val Ser Thr Asn Met Cys Ile Pro Ala
    370                 375                 380

Ser Phe His Cys Asp Glu Glu Ser Asp Cys Pro Asp Arg Ser Asp Glu
385                 390                 395                 400

Phe Gly Cys Met Pro Pro Gln Val Val Thr Pro Pro Arg Glu Ser Ile
                405                 410                 415

Gln Ala Ser Arg Gly Gln Thr Val Thr Phe Thr Cys Val Ala Ile Gly
            420                 425                 430

Val Pro Ala Pro Phe Leu Ile Asn Trp Arg Leu Asn Trp Gly His Ile
        435                 440                 445

Pro Ser Gln Pro Arg Val Thr Val Thr Ser Glu Gly Gly Arg Gly Thr
    450                 455                 460

Leu Ile Ile Arg Asp Val Lys Glu Ser Asp Gln Gly Ala Tyr Thr Cys
465                 470                 475                 480

Glu Ala Met Asn Ala Arg Gly Met Val Phe Gly Ile Pro Asp Gly Val
                485                 490                 495

Leu Glu Leu Val Pro Gln Arg Ala Gly Pro Cys Pro Asp Gly His Phe
            500                 505                 510

Tyr Leu Glu His Ser Ala Ala Cys Leu Pro Cys Phe Cys Phe Gly Ile
        515                 520                 525

Thr Ser Val Cys Gln Ser Thr Arg Arg Phe Arg Asp Gln Ile Arg Leu
    530                 535                 540

Arg Phe Asp Gln Pro Asp Asp Phe Lys Gly Val Asn Val Thr Met Pro
545                 550                 555                 560

Ala Gln Pro Gly Thr Pro Pro Leu Ser Ser Thr Gln Leu Gln Ile Asp
                565                 570                 575

Pro Ser Leu His Glu Phe Gln Leu Val Asp Leu Ser Arg Arg Phe Leu
            580                 585                 590

Val His Asp Ser Phe Trp Ala Leu Pro Glu Gln Phe Leu Gly Asn Lys
        595                 600                 605

Val Asp Ser Tyr Gly Gly Ser Leu Arg Tyr Asn Val Arg Tyr Glu Leu
    610                 615                 620

Ala Arg Gly Met Leu Glu Pro Val Gln Arg Pro Asp Val Val Leu Val
625                 630                 635                 640

Gly Ala Gly Tyr Arg Leu Leu Ser Arg Gly His Thr Pro Thr Gln Pro
                645                 650                 655

Gly Ala Leu Asn Gln Arg Gln Val Gln Phe Ser Glu Glu His Trp Val
            660                 665                 670

His Glu Ser Gly Arg Pro Val Gln Arg Ala Glu Leu Leu Gln Val Leu
        675                 680                 685

Gln Ser Leu Glu Ala Val Leu Ile Gln Thr Val Tyr Asn Thr Lys Met
    690                 695                 700

Ala Ser Val Gly Leu Ser Asp Ile Ala Met Asp Thr Thr Val Thr His
705                 710                 715                 720

Ala Thr Ser His Gly Arg Ala His Ser Val Glu Glu Cys Arg Cys Pro
                725                 730                 735

Ile Gly Tyr Ser Gly Leu Ser Cys Glu Ser Cys Asp Ala His Phe Thr
            740                 745                 750

Arg Val Pro Gly Gly Pro Tyr Leu Gly Thr Cys Ser Gly Cys Ser Cys
        755                 760                 765

Asn Gly His Ala Ser Ser Cys Asp Pro Val Tyr Gly His Cys Leu Asn
    770                 775                 780

Cys Gln His Asn Thr Glu Gly Pro Gln Cys Lys Lys Cys Lys Ala Gly

785                 790                 795                 800

Phe Phe Gly Asp Ala Met Lys Ala Thr Ala Thr Ser Cys Arg Pro Cys
                805                 810                 815

Pro Cys Pro Tyr Ile Asp Ala Ser Arg Arg Phe Ser Asp Thr Cys Phe
            820                 825                 830

Leu Asp Thr Asp Gly Gln Ala Thr Cys Asp Ala Cys Ala Pro Gly Tyr
        835                 840                 845

Thr Gly Arg Arg Cys Glu Ser Cys Ala Pro Gly Tyr Glu Gly Asn Pro
    850                 855                 860

Ile Gln Pro Gly Gly Lys Cys Arg Pro Val Asn Gln Glu Ile Val Arg
865                 870                 875                 880

Cys Asp Glu Arg Gly Ser Met Gly Thr Ser Gly Glu Ala Cys Arg Cys
                885                 890                 895

Lys Asn Asn Val Val Gly Arg Leu Cys Asn Glu Cys Ala Asp Arg Ser
            900                 905                 910

Phe His Leu Ser Thr Arg Asn Pro Asp Gly Cys Leu Lys Cys Phe Cys
        915                 920                 925

Met Gly Val Ser Arg His Cys Thr Ser Ser Ser Trp Ser Arg Ala Gln
    930                 935                 940

Leu His Gly Ala Ser Glu Glu Pro Gly His Phe Ser Leu Thr Asn Ala
945                 950                 955                 960

Ala Ser Thr His Thr Thr Asn Glu Gly Ile Phe Ser Pro Thr Pro Gly
                965                 970                 975

Glu Leu Gly Phe Ser Ser Phe His Arg Leu Leu Ser Gly Pro Tyr Phe
            980                 985                 990

Trp Ser Leu Pro Ser Arg Phe Leu Gly Asp Lys Val Thr Ser Tyr Gly
        995                 1000                1005

Gly Glu Leu Arg Phe Thr Val Thr Gln Arg Ser Gln Pro Gly Ser Thr
    1010                1015                1020

Pro Leu His Gly Gln Pro Leu Val Val Leu Gln Gly Asn Asn Ile Ile
1025                1030                1035                1040

Leu Glu His His Val Ala Gln Glu Pro Ser Pro Gly Gln Pro Ser Thr
                1045                1050                1055

Phe Ile Val Pro Phe Arg Glu Gln Ala Trp Gln Arg Pro Asp Gly Gln
            1060                1065                1070

Pro Ala Thr Arg Glu His Leu Leu Met Ala Leu Ala Gly Ile Asp Thr
        1075                1080                1085

Leu Leu Ile Arg Ala Ser Tyr Ala Gln Gln Pro Ala Glu Ser Arg Val
    1090                1095                1100

Ser Gly Ile Ser Met Asp Val Ala Val Pro Glu Glu Thr Gly Gln Asp
1105                1110                1115                1120

Pro Ala Leu Glu Val Glu Gln Cys Ser Cys Pro Pro Gly Tyr Arg Gly
                1125                1130                1135

Pro Ser Cys Gln Asp Cys Asp Thr Gly Tyr Thr Arg Thr Pro Ser Gly
            1140                1145                1150

Leu Tyr Leu Gly Thr Cys Glu Arg Cys Ser Cys His Gly His Ser Glu
        1155                1160                1165

Ala Cys Glu Pro Glu Thr Gly Ala Cys Gln Gly Cys Gln His His Thr
    1170                1175                1180

Glu Gly Pro Arg Cys Glu Gln Cys Gln Pro Gly Tyr Tyr Gly Asp Ala
1185                1190                1195                1200

Gln Arg Gly Thr Pro Gln Asp Cys Gln Leu Cys Pro Cys Tyr Gly Asp
                1205                1210                1215

Pro Ala Ala Gly Gln Ala Ala His Thr Cys Phe Leu Asp Thr Asp Gly
            1220                1225                1230

His Pro Thr Cys Asp Ala Cys Ser Pro Gly His Ser Gly Arg His Cys
        1235                1240                1245

Glu Arg Cys Ala Pro Gly Tyr Tyr Gly Asn Pro Ser Gln Gly Gln Pro
    1250                1255                1260

Cys Gln Arg Asp Ser Gln Val Pro Gly Pro Ile Gly Cys Asn Cys Asp
1265                1270                1275                1280

Pro Gln Gly Ser Val Ser Ser Gln Cys Asp Ala Ala Gly Gln Cys Gln
                1285                1290                1295

Cys Lys Ala Gln Val Glu Gly Leu Thr Cys Ser His Cys Arg Pro His
            1300                1305                1310

His Phe His Leu Ser Ala Ser Asn Pro Asp Gly Cys Leu Pro Cys Phe
        1315                1320                1325

Cys Met Gly Ile Thr Gln Gln Cys Ala Ser Ser Ala Tyr Thr Arg His
    1330                1335                1340

Leu Ile Ser Thr His Phe Ala Pro Gly Asp Phe Gln Gly Phe Ala Leu
1345                1350                1355                1360

Val Asn Pro Gln Arg Asn Ser Arg Leu Thr Gly Glu Phe Thr Val Glu
                1365                1370                1375

Pro Val Pro Glu Gly Ala Gln Leu Ser Phe Gly Asn Phe Ala Gln Leu
            1380                1385                1390

Gly His Glu Ser Phe Tyr Trp Gln Leu Pro Glu Thr Tyr Gln Gly Asp
        1395                1400                1405

Lys Val Ala Ala Tyr Gly Gly Lys Leu Arg Tyr Thr Leu Ser Tyr Thr
    1410                1415                1420

Ala Gly Pro Gln Gly Ser Pro Leu Ser Asp Pro Asp Val Gln Ile Thr
1425                1430                1435                1440

Gly Asn Asn Ile Met Leu Val Ala Ser Gln Pro Ala Leu Gln Gly Pro
                1445                1450                1455

Glu Arg Arg Ser Tyr Glu Ile Met Phe Arg Glu Glu Phe Trp Arg Arg
            1460                1465                1470

Pro Asp Gly Gln Pro Ala Thr Arg Glu His Leu Leu Met Ala Leu Ala
        1475                1480                1485

Asp Leu Asp Glu Leu Leu Ile Arg Ala Thr Phe Ser Ser Val Pro Leu
    1490                1495                1500

Val Ala Ser Ile Ser Ala Val Ser Leu Glu Val Ala Gln Pro Gly Pro
1505                1510                1515                1520

Ser Asn Arg Pro Arg Ala Leu Glu Val Glu Glu Cys Arg Cys Pro Pro
                1525                1530                1535

Gly Tyr Ile Gly Leu Ser Cys Gln Asp Cys Ala Pro Gly Tyr Thr Arg
            1540                1545                1550

Thr Gly Ser Gly Leu Tyr Leu Gly His Cys Glu Leu Cys Glu Cys Asn
        1555                1560                1565

Gly His Ser Asp Leu Cys His Pro Glu Thr Gly Ala Cys Ser Gln Cys
    1570                1575                1580

Gln His Asn Ala Ala Gly Glu Phe Cys Glu Leu Cys Ala Pro Gly Tyr
1585                1590                1595                1600

Tyr Gly Asp Ala Thr Ala Gly Thr Pro Glu Asp Cys Gln Pro Cys Ala
                1605                1610                1615

Cys Pro Leu Thr Asn Pro Glu Asn Met Phe Ser Arg Thr Cys Glu Ser
            1620                1625                1630

Leu Gly Ala Gly Gly Tyr Arg Cys Thr Ala Cys Glu Pro Gly Tyr Thr
        1635                1640                1645

Gly Gln Tyr Cys Glu Gln Cys Gly Pro Gly Tyr Val Gly Asn Pro Ser
    1650                1655                1660

Val Gln Gly Gly Gln Cys Leu Pro Glu Thr Asn Gln Ala Pro Leu Val
1665                1670                1675                1680

Val Glu Val His Pro Ala Arg Ser Ile Val Pro Gln Gly Gly Ser His
                1685                1690                1695

Ser Leu Arg Cys Gln Val Ser Gly Arg Gly Pro His Tyr Phe Tyr Trp
            1700                1705                1710

Ser Arg Glu Asp Gly Arg Pro Val Pro Ser Gly Thr Gln Gln Arg His

1715                 1720                 1725

Gln Gly Ser Glu Leu His Phe Pro Ser Val Gln Pro Ser Asp Ala Gly
    1730                 1735                 1740

Val Tyr Ile Cys Thr Cys Arg Asn Leu His Arg Ser Asn Thr Ser Arg
1745                 1750                 1755                 1760

Ala Glu Leu Leu Val Thr Glu Ala Pro Ser Lys Pro Ile Thr Val Thr
                 1765                 1770                 1775

Val Glu Glu Gln Arg Ser Gln Ser Val Arg Pro Gly Ala Asp Val Thr
             1780                 1785                 1790

Phe Ile Cys Thr Ala Lys Ser Lys Ser Pro Ala Tyr Thr Leu Val Trp
         1795                 1800                 1805

Thr Arg Leu His Asn Gly Lys Leu Pro Thr Arg Ala Met Asp Phe Asn
    1810                 1815                 1820

Gly Ile Leu Thr Ile Arg Asn Val Gln Leu Ser Asp Ala Gly Thr Tyr
1825                 1830                 1835                 1840

Val Cys Thr Gly Ser Asn Met Phe Ala Met Asp Gln Gly Thr Ala Thr
                 1845                 1850                 1855

Leu His Val Gln Ala Ser Gly Thr Leu Ser Ala Pro Val Val Ser Ile
             1860                 1865                 1870

His Pro Pro Gln Leu Thr Val Gln Pro Gly Gln Leu Ala Glu Phe Arg
         1875                 1880                 1885

Cys Ser Ala Thr Gly Ser Pro Thr Pro Thr Leu Glu Trp Thr Gly Gly
    1890                 1895                 1900

Pro Gly Gly Gln Leu Pro Ala Lys Ala Gln Ile His Gly Gly Ile Leu
1905                 1910                 1915                 1920

Arg Leu Pro Ala Val Glu Pro Thr Asp Gln Ala Gln Tyr Leu Cys Arg
                 1925                 1930                 1935

Ala His Ser Ser Ala Gly Gln Gln Val Ala Arg Ala Val Leu His Val
             1940                 1945                 1950

His Gly Gly Gly Gly Pro Arg Val Gln Val Ser Pro Glu Arg Thr Gln
         1955                 1960                 1965

Val His Ala Gly Arg Thr Val Arg Leu Tyr Cys Arg Ala Ala Gly Val
    1970                 1975                 1980

Pro Ser Ala Thr Ile Thr Trp Arg Lys Glu Gly Gly Ser Leu Pro Pro
1985                 1990                 1995                 2000

Gln Ala Arg Ser Glu Arg Thr Asp Ile Ala Thr Leu Leu Ile Pro Ala
                 2005                 2010                 2015

Ile Thr Thr Ala Asp Ala Gly Phe Tyr Leu Cys Val Ala Thr Ser Pro
             2020                 2025                 2030

Ala Gly Thr Ala Gln Ala Arg Ile Gln Val Val Val Leu Ser Ala Ser
        2035                2040                2045

Asp Ala Ser Gln Pro Pro Val Lys Ile Glu Ser Ser Ser Pro Ser Val
    2050                2055                2060

Thr Glu Gly Gln Thr Leu Asp Leu Asn Cys Val Val Ala Gly Ser Ala
2065                2070                2075                2080

His Ala Gln Val Thr Trp Tyr Arg Arg Gly Gly Ser Leu Pro His His
                2085                2090                2095

Thr Gln Val His Gly Ser Arg Leu Arg Leu Pro Gln Val Ser Pro Ala
            2100                2105                2110

Asp Ser Gly Glu Tyr Val Cys Arg Val Glu Asn Gly Ser Gly Pro Lys
        2115                2120                2125

Glu Ala Ser Ile Thr Val Ser Val Leu His Gly Thr His Ser Gly Pro
    2130                2135                2140

Ser Tyr Thr Pro Val Pro Gly Ser Thr Arg Pro Ile Arg Ile Glu Pro
2145                2150                2155                2160

Ser Ser Ser His Val Ala Glu Gly Gln Thr Leu Asp Leu Asn Cys Val
                2165                2170                2175

Val Pro Gly Gln Ala His Ala Gln Val Thr Trp His Lys Arg Gly Gly
            2180                2185                2190

Ser Leu Pro Ala Arg His Gln Thr His Gly Ser Leu Leu Arg Leu His
        2195                2200                2205

Gln Val Thr Pro Ala Asp Ser Gly Glu Tyr Val Cys His Val Val Gly
    2210                2215                2220

Thr Ser Gly Pro Leu Glu Ala Ser Val Leu Val Thr Ile Glu Ala Ser
2225                2230                2235                2240

Val Ile Pro Gly Pro Ile Pro Pro Val Arg Ile Glu Ser Ser Ser Ser
                2245                2250                2255

Thr Val Ala Glu Gly Gln Thr Leu Asp Leu Ser Cys Val Val Ala Gly
            2260                2265                2270

Gln Ala His Ala Gln Val Thr Trp Tyr Lys Arg Gly Gly Ser Leu Pro
        2275                2280                2285

Ala Arg His Gln Val Arg Gly Ser Arg Leu Tyr Ile Phe Gln Ala Ser
    2290                2295                2300

Pro Ala Asp Ala Gly Gln Tyr Val Cys Arg Ala Ser Asn Gly Met Glu
2305                2310                2315                2320

Ala Ser Ile Thr Val Thr Val Thr Gly Thr Gln Gly Ala Asn Leu Ala
                2325                2330                2335

Tyr Pro Ala Gly Ser Thr Gln Pro Ile Arg Ile Glu Pro Ser Ser Ser
            2340                2345                2350

Gln Val Ala Glu Gly Gln Thr Leu Asp Leu Asn Cys Val Val Pro Gly
        2355                2360                2365

Gln Ser His Ala Gln Val Thr Trp His Lys Arg Gly Gly Ser Leu Pro
    2370                2375                2380

Val Arg His Gln Thr His Gly Ser Leu Leu Arg Leu Tyr Gln Ala Ser
2385                2390                2395                2400

Pro Ala Asp Ser Gly Glu Tyr Val Cys Arg Val Leu Gly Ser Ser Val
                2405                2410                2415

Pro Leu Glu Ala Ser Val Leu Val Thr Ile Glu Pro Ala Gly Ser Val
            2420                2425                2430

Pro Ala Leu Gly Val Thr Pro Thr Val Arg Ile Glu Ser Ser Ser Ser
        2435                2440                2445

Gln Val Ala Glu Gly Gln Thr Leu Asp Leu Asn Cys Leu Val Ala Gly
    2450                2455                2460

Gln Ala His Ala Gln Val Thr Trp His Lys Arg Gly Gly Ser Leu Pro
2465                2470                2475                2480

Ala Arg His Gln Val His Gly Ser Arg Leu Arg Leu Leu Gln Val Thr
                2485                2490                2495

Pro Ala Asp Ser Gly Glu Tyr Val Cys Arg Val Val Gly Ser Ser Gly
            2500                2505                2510

Thr Gln Glu Ala Ser Val Leu Val Thr Ile Gln Gln Arg Leu Ser Gly
        2515                2520                2525

Ser His Ser Gln Gly Val Ala Tyr Pro Val Arg Ile Glu Ser Ser Ser
    2530                2535                2540

Ala Ser Leu Ala Asn Gly His Thr Leu Asp Leu Asn Cys Leu Val Ala
2545                2550                2555                2560

Ser Gln Ala Pro His Thr Ile Thr Trp Tyr Lys Arg Gly Gly Ser Leu
                2565                2570                2575

Pro Ser Arg His Gln Ile Val Gly Ser Arg Leu Arg Ile Pro Gln Val
            2580                2585                2590

Thr Pro Ala Asp Ser Gly Glu Tyr Val Cys His Val Ser Asn Gly Ala
        2595                2600                2605

Gly Ser Arg Glu Thr Ser Leu Ile Val Thr Ile Gln Gly Ser Gly Ser
    2610                2615                2620

Ser His Val Pro Arg Val Ser Pro Pro Ile Arg Ile Glu Ser Ser Ser
2625                2630                2635                2640

Pro Thr Val Val Glu Gly Gln Thr Leu Asp Leu Asn Cys Val Val Ala

2645                2650                2655

Arg Gln Pro Gln Ala Ile Ile Thr Trp Tyr Lys Arg Gly Gly Ser Leu
        2660                2665                2670

Pro Ser Arg His Gln Thr His Gly Ser His Leu Arg Leu His Gln Met
    2675                2680                2685

Ser Val Ala Asp Ser Gly Glu Tyr Val Cys Arg Ala Asn Asn Asn Ile
    2690                2695                2700

Asp Ala Leu Glu Ala Ser Ile Val Ile Ser Val Ser Pro Ser Ala Gly
2705                2710                2715                2720

Ser Pro Ser Ala Pro Gly Ser Ser Met Pro Ile Arg Ile Glu Ser Ser
                2725                2730                2735

Ser Ser His Val Ala Glu Gly Glu Thr Leu Asp Leu Asn Cys Val Val
            2740                2745                2750

Pro Gly Gln Ala His Ala Gln Val Thr Trp His Lys Arg Gly Gly Ser
        2755                2760                2765

Leu Pro Ser Tyr His Gln Thr Arg Gly Ser Arg Leu Arg Leu His His
    2770                2775                2780

Val Ser Pro Ala Asp Ser Gly Glu Tyr Val Cys Arg Val Met Gly Ser
2785                2790                2795                2800

Ser Gly Pro Leu Glu Ala Ser Val Leu Val Thr Ile Glu Ala Ser Gly
                2805                2810                2815

Ser Ser Ala Val His Val Pro Ala Pro Gly Gly Ala Pro Pro Ile Arg
            2820                2825                2830

Ile Glu Pro Ser Ser Ser Arg Val Ala Glu Gly Gln Thr Leu Asp Leu
        2835                2840                2845

Lys Cys Val Val Pro Gly Gln Ala His Ala Gln Val Thr Trp His Lys
    2850                2855                2860

Arg Gly Gly Asn Leu Pro Ala Arg His Gln Val His Gly Pro Leu Leu
2865                2870                2875                2880

Arg Leu Asn Gln Val Ser Pro Ala Asp Ser Gly Glu Tyr Ser Cys Gln
                2885                2890                2895

Val Thr Gly Ser Ser Gly Thr Leu Glu Ala Ser Val Leu Val Thr Ile
            2900                2905                2910

Glu Pro Ser Ser Pro Gly Pro Ile Pro Ala Pro Gly Leu Ala Gln Pro
        2915                2920                2925

Ile Tyr Ile Glu Ala Ser Ser Ser His Val Thr Glu Gly Gln Thr Leu
    2930                2935                2940

Asp Leu Asn Cys Val Val Pro Gly Gln Ala His Ala Gln Val Thr Trp
2945                2950                2955                2960

Tyr Lys Arg Gly Gly Ser Leu Pro Ala Arg His Gln Thr His Gly Ser
                2965                2970                2975

Gln Leu Arg Leu His His Val Ser Pro Ala Asp Ser Gly Glu Tyr Val
            2980                2985                2990

Cys Arg Ala Ala Gly Gly Pro Gly Pro Glu Gln Glu Ala Ser Phe Thr
        2995                3000                3005

Val Thr Val Pro Pro Ser Glu Gly Ser Ser Tyr Arg Leu Arg Ser Pro
    3010                3015                3020

Val Ile Ser Ile Asp Pro Pro Ser Ser Thr Val Gln Gln Gly Gln Asp
3025                3030                3035                3040

Ala Ser Phe Lys Cys Leu Ile His Asp Gly Ala Ala Pro Ile Ser Leu
                3045                3050                3055

Glu Trp Lys Thr Arg Asn Gln Glu Leu Glu Asp Asn Val His Ile Ser
            3060                3065                3070

Pro Asn Gly Ser Ile Ile Thr Ile Val Gly Thr Arg Pro Ser Asn His
        3075                3080                3085

Gly Thr Tyr Arg Cys Val Ala Ser Asn Ala Tyr Gly Val Ala Gln Ser
    3090                3095                3100

Val Val Asn Leu Ser Val His Gly Pro Pro Thr Val Ser Val Leu Pro
3105                3110                3115                3120

Glu Gly Pro Val Trp Val Lys Val Gly Lys Ala Val Thr Leu Glu Cys
                3125                3130                3135

Val Ser Ala Gly Glu Pro Arg Ser Ser Ala Arg Trp Thr Arg Ile Ser
            3140                3145                3150

Ser Thr Pro Ala Lys Leu Glu Gln Arg Thr Tyr Gly Leu Met Asp Ser
        3155                3160                3165

His Thr Val Leu Gln Ile Ser Ser Ala Lys Pro Ser Asp Ala Gly Thr
    3170                3175                3180

Tyr Val Cys Leu Ala Gln Asn Ala Leu Gly Thr Ala Gln Lys Gln Val
3185                3190                3195                3200

Glu Val Ile Val Asp Thr Gly Ala Met Ala Pro Gly Ala Pro Gln Val
                3205                3210                3215

Gln Ala Glu Glu Ala Glu Leu Thr Val Glu Ala Gly His Thr Ala Thr
            3220                3225                3230

Leu Arg Cys Ser Ala Thr Gly Ser Pro Ala Arg Thr Ile His Trp Ser
        3235                3240                3245

Lys Leu Arg Ser Pro Leu Pro Trp Gln His Arg Leu Glu Gly Asp Thr
    3250                3255                3260

Leu Ile Ile Pro Arg Val Ala Gln Gln Asp Ser Gly Gln Tyr Ile Cys
3265                3270                3275                3280

Asn Ala Thr Ser Pro Ala Gly His Ala Glu Ala Thr Ile Ile Leu His
                3285                3290                3295

Val Glu Ser Pro Pro Tyr Ala Thr Thr Val Pro Glu His Ala Ser Val
            3300                3305                3310

Gln Ala Gly Glu Thr Val Gln Leu Gln Cys Leu Ala His Gly Thr Pro
        3315                3320                3325

Pro Leu Thr Phe Gln Trp Ser Arg Val Gly Ser Ser Leu Pro Gly Arg
    3330                3335                3340

Ala Thr Ala Arg Asn Glu Leu Leu His Phe Glu Arg Ala Ala Pro Glu
3345                3350                3355                3360

Asp Ser Gly Arg Tyr Arg Cys Arg Val Thr Asn Lys Val Gly Ser Ala
                3365                3370                3375

Glu Ala Phe Ala Gln Leu Leu Val Gln Gly Pro Pro Gly Ser Leu Pro
            3380                3385                3390

Ala Thr Ser Ile Pro Ala Gly Ser Thr Pro Thr Val Gln Val Thr Pro
        3395                3400                3405

Gln Leu Glu Thr Lys Ser Ile Gly Ala Ser Val Glu Phe His Cys Ala
    3410                3415                3420

Val Pro Ser Asp Arg Gly Thr Gln Leu Arg Trp Phe Lys Glu Gly Gly
3425                3430                3435                3440

Gln Leu Pro Pro Gly His Ser Val Gln Asp Gly Val Leu Arg Ile Gln
                3445                3450                3455

Asn Leu Asp Gln Ser Cys Gln Gly Thr Tyr Ile Cys Gln Ala His Gly
            3460                3465                3470

Pro Trp Gly Lys Ala Gln Ala Ser Ala Gln Leu Val Ile Gln Ala Leu
        3475                3480                3485

Pro Ser Val Leu Ile Asn Ile Arg Thr Ser Val Gln Thr Val Val Val
    3490                3495                3500

Gly His Ala Val Glu Phe Glu Cys Leu Ala Leu Gly Asp Pro Lys Pro
3505                3510                3515                3520

Gln Val Thr Trp Ser Lys Val Gly Gly His Leu Arg Pro Gly Ile Val
                3525                3530                3535

Gln Ser Gly Gly Val Val Arg Ile Ala His Val Glu Leu Ala Asp Ala
            3540                3545                3550

Gly Gln Tyr Arg Cys Thr Ala Thr Asn Ala Ala Gly Thr Thr Gln Ser
        3555                3560                3565

His Val Leu Leu Leu Val Gln Ala Leu Pro Gln Ile Ser Met Pro Gln

3570                    3575                    3580

Glu Val Arg Val Pro Ala Gly Ser Ala Ala Val Phe Pro Cys Ile Ala
3585                    3590                    3595                    3600

Ser Gly Tyr Pro Thr Pro Asp Ile Ser Trp Ser Lys Leu Asp Gly Ser
                3605                    3610                    3615

Leu Pro Pro Asp Ser Arg Leu Glu Asn Asn Met Leu Met Leu Pro Ser
            3620                    3625                    3630

Val Gln Pro Gln Asp Ala Gly Thr Tyr Val Cys Thr Ala Thr Asn Arg
        3635                    3640                    3645

Gln Gly Lys Val Lys Ala Phe Ala His Leu Gln Val Pro Glu Arg Val
    3650                    3655                    3660

Val Pro Tyr Phe Thr Gln Thr Pro Tyr Ser Phe Leu Pro Leu Pro Thr
3665                    3670                    3675                    3680

Ile Lys Asp Ala Tyr Arg Lys Phe Glu Ile Lys Ile Thr Phe Arg Pro
                3685                    3690                    3695

Asp Ser Ala Asp Gly Met Leu Leu Tyr Asn Gly Gln Lys Arg Val Pro
            3700                    3705                    3710

Gly Ser Pro Thr Asn Leu Ala Asn Arg Gln Pro Asp Phe Ile Ser Phe
        3715                    3720                    3725

Gly Leu Val Gly Gly Arg Pro Glu Phe Arg Phe Asp Ala Gly Ser Gly
    3730                    3735                    3740

Met Ala Thr Ile Arg His Pro Thr Pro Leu Ala Leu Gly His Phe His
3745                    3750                    3755                    3760

Thr Val Thr Leu Leu Arg Ser Leu Thr Gln Gly Ser Leu Ile Val Gly
                3765                    3770                    3775

Asp Leu Ala Pro Val Asn Gly Thr Ser Gln Gly Lys Phe Gln Gly Leu
            3780                    3785                    3790

Asp Leu Asn Glu Glu Leu Tyr Leu Gly Gly Tyr Pro Asp Tyr Gly Ala
        3795                    3800                    3805

Ile Pro Lys Ala Gly Leu Ser Ser Gly Phe Ile Gly Cys Val Arg Glu
    3810                    3815                    3820

Leu Arg Ile Gln Gly Glu Glu Ile Val Phe His Asp Leu Asn Leu Thr
3825                    3830                    3835                    3840

Ala His Gly Ile Ser His Cys Pro Thr Cys Arg Asp Arg Pro Cys Gln
                3845                    3850                    3855

Asn Gly Gly Gln Cys His Asp Ser Glu Ser Ser Ser Tyr Val Cys Val
            3860                    3865                    3870

Cys Pro Ala Gly Phe Thr Gly Ser Arg Cys Glu His Ser Gln Ala Leu
        3875                    3880                    3885

His Cys His Pro Glu Ala Cys Gly Pro Asp Ala Thr Cys Val Asn Arg
    3890                3895                3900

Pro Asp Gly Arg Gly Tyr Thr Cys Arg Cys His Leu Gly Arg Ser Gly
3905                3910                3915                3920

Leu Arg Cys Glu Glu Gly Val Thr Val Thr Thr Pro Ser Leu Ser Gly
                3925                3930                3935

Ala Gly Ser Tyr Leu Ala Leu Pro Ala Leu Thr Asn Thr His His Glu
            3940                3945                3950

Leu Arg Leu Asp Val Glu Phe Lys Pro Leu Ala Pro Asp Gly Val Leu
        3955                3960                3965

Leu Phe Ser Gly Gly Lys Ser Gly Pro Val Glu Asp Phe Val Ser Leu
    3970                3975                3980

Ala Met Val Gly Gly His Leu Glu Phe Arg Tyr Glu Leu Gly Ser Gly
3985                3990                3995                4000

Leu Ala Val Leu Arg Thr Ala Glu Pro Leu Ala Leu Gly Arg Trp His
                4005                4010                4015

Arg Val Ser Ala Glu Arg Leu Asn Lys Asp Gly Ser Leu Arg Val Asn
            4020                4025                4030

Gly Gly Arg Pro Val Leu Arg Ser Ser Pro Gly Lys Ser Gln Gly Leu
        4035                4040                4045

Asn Leu His Thr Leu Leu Tyr Leu Gly Gly Val Glu Pro Ser Val Pro
    4050                4055                4060

Leu Ser Pro Ala Thr Asn Met Ser Ala His Phe Arg Gly Cys Val Gly
4065                4070                4075                4080

Glu Val Ser Val Asn Gly Lys Arg Leu Asp Leu Thr Tyr Ser Phe Leu
                4085                4090                4095

Gly Ser Gln Gly Ile Gly Gln Cys Tyr Asp Ser Ser Pro Cys Glu Arg
            4100                4105                4110

Gln Pro Cys Gln His Gly Ala Thr Cys Met Pro Ala Gly Glu Tyr Glu
        4115                4120                4125

Phe Gln Cys Leu Cys Arg Asp Gly Ile Lys Gly Asp Leu Cys Glu His
    4130                4135                4140

Glu Glu Asn Pro Cys Gln Leu Arg Glu Pro Cys Leu His Gly Gly Thr
4145                4150                4155                4160

Cys Gln Gly Thr Arg Cys Leu Cys Leu Pro Gly Phe Ser Gly Pro Arg
                4165                4170                4175

Cys Gln Gln Gly Ser Gly His Gly Ile Ala Glu Ser Asp Trp His Leu
            4180                4185                4190

Glu Gly Ser Gly Gly Asn Asp Ala Pro Gly Gln Tyr Gly Ala Tyr Phe
        4195                4200                4205

His Asp Asp Gly Phe Leu Ala Phe Pro Gly His Val Phe Ser Arg Ser
    4210                4215                4220

Leu Pro Glu Val Pro Glu Thr Ile Glu Leu Glu Val Arg Thr Ser Thr
4225                4230                4235                4240

Ala Ser Gly Leu Leu Leu Trp Gln Gly Val Glu Val Gly Glu Ala Gly
                4245                4250                4255

Gln Gly Lys Asp Phe Ile Ser Leu Gly Leu Gln Asp Gly His Leu Val
            4260                4265                4270

Phe Arg Tyr Gln Leu Gly Ser Gly Glu Ala Arg Leu Val Ser Glu Asp
        4275                4280                4285

Pro Ile Asn Asp Gly Glu Trp His Arg Val Thr Ala Leu Arg Glu Gly
    4290                4295                4300

Arg Arg Gly Ser Ile Gln Val Asp Gly Glu Glu Leu Val Ser Gly Arg
4305                4310                4315                4320

Ser Pro Gly Pro Asn Val Ala Val Asn Ala Lys Gly Ser Ile Tyr Ile
                4325                4330                4335

Gly Gly Ala Pro Asp Val Ala Thr Leu Thr Gly Gly Arg Phe Ser Ser
            4340                4345                4350

Gly Ile Thr Gly Cys Val Lys Asn Leu Val Leu His Ser Ala Arg Pro
        4355                4360                4365

Gly Ala Pro Pro Pro Gln Pro Leu Asp Leu Gln His Arg Ala Gln Ala
    4370                4375                4380

Gly Ala Asn Thr Arg Pro Cys Pro Ser
4385                4390

<210> 2
<211> 195
<212> PRT
<213> Homo sapiens

<400> 2
Asp Ala Pro Gly Gln Tyr Gly Ala Tyr Phe His Asp Asp Gly Phe Leu
1               5                   10                  15

Ala Phe Pro Gly His Val Phe Ser Arg Ser Leu Pro Glu Val Pro Glu
            20                  25                  30

Thr Ile Glu Leu Glu Val Arg Thr Ser Thr Ala Ser Gly Leu Leu Leu
        35                  40                  45

Trp Gln Gly Val Glu Val Gly Glu Ala Gly Gln Gly Lys Asp Phe Ile
    50                  55                  60

Ser Leu Gly Leu Gln Asp Gly His Leu Val Phe Arg Tyr Gln Leu Gly
65                  70                  75                  80

Ser Gly Glu Ala Arg Leu Val Ser Glu Asp Pro Ile Asn Asp Gly Glu
                85                  90                  95

Trp His Arg Val Thr Ala Leu Arg Glu Gly Arg Arg Gly Ser Ile Gln
            100                 105                 110

Val Asp Gly Glu Glu Leu Val Ser Gly Arg Ser Pro Gly Pro Asn Val
        115                 120                 125

Ala Val Asn Ala Lys Gly Ser Val Tyr Ile Gly Gly Ala Pro Asp Val
    130                 135                 140

Ala Thr Leu Thr Gly Gly Arg Phe Ser Ser Gly Ile Thr Gly Cys Val
145                 150                 155                 160

Lys Asn Leu Val Leu His Ser Ala Arg Pro Gly Ala Pro Pro Pro Gln
                165                 170                 175

Pro Leu Asp Leu Gln His Arg Ala Gln Ala Gly Ala Asn Thr Arg Pro
            180                 185                 190

Cys Pro Ser
        195

<210> 3
<211> 508
<212> PRT
<213> Homo sapiens

<400> 3
Arg Thr Cys Arg Cys Lys Asn Asn Val Val Gly Arg Leu Cys Asn Glu
1               5                   10                  15

Cys Ala Asp Arg Ser Phe His Leu Ser Thr Arg Asn Pro Asp Gly Cys
            20                  25                  30

Leu Lys Cys Phe Cys Met Gly Val Ser Arg His Cys Thr Ser Ser Ser
        35                  40                  45

Trp Ser Arg Ala Gln Leu His Gly Ala Ser Glu Glu Pro Gly His Phe
    50                  55                  60

Ser Leu Thr Asn Ala Ala Ser Thr His Thr Thr Asn Glu Gly Ile Phe
65                  70                  75                  80

Ser Pro Thr Pro Gly Glu Leu Gly Phe Ser Ser Phe His Arg Leu Leu
                85                  90                  95

Ser Gly Pro Tyr Phe Trp Ser Leu Pro Ser Arg Phe Leu Gly Asp Lys
            100                 105                 110

Val Thr Ser Tyr Gly Gly Glu Leu Arg Phe Thr Val Thr Gln Arg Ser

        115                 120                 125

Gln Pro Gly Ser Thr Pro Leu His Gly Gln Pro Leu Val Val Leu Gln
    130                 135                 140

Gly Asn Asn Ile Ile Leu Glu His His Val Ala Gln Glu Pro Ser Pro
145                 150                 155                 160

Gly Gln Pro Ser Thr Phe Ile Val Pro Phe Arg Glu Gln Ala Trp Gln
                165                 170                 175

Arg Pro Asp Gly Gln Pro Ala Thr Arg Glu His Leu Leu Met Ala Leu
            180                 185                 190

Ala Gly Ile Asp Thr Leu Leu Ile Arg Ala Ser Tyr Ala Gln Gln Pro
        195                 200                 205

Ala Glu Ser Arg Leu Ser Gly Ile Ser Met Asp Val Ala Val Pro Glu
    210                 215                 220

Glu Thr Gly Gln Asp Pro Ala Leu Glu Val Glu Gln Cys Ser Cys Pro
225                 230                 235                 240

Pro Gly Tyr Leu Gly Pro Ser Cys Gln Asp Cys Asp Thr Gly Tyr Thr
                245                 250                 255

Arg Thr Pro Ser Gly Leu Tyr Leu Gly Thr Cys Glu Arg Cys Ser Cys
            260                 265                 270

His Gly His Ser Glu Ala Cys Glu Pro Glu Thr Gly Ala Cys Gln Gly
        275                 280                 285

Cys Gln His His Thr Glu Gly Pro Arg Cys Glu Gln Cys Gln Pro Gly
    290                 295                 300

Tyr Tyr Gly Asp Ala Gln Arg Gly Thr Pro Gln Asp Cys Gln Leu Cys
305                 310                 315                 320

Pro Cys Tyr Gly Asp Pro Ala Ala Gly Gln Ala Ala Leu Thr Cys Phe
                325                 330                 335

Leu Asp Thr Asp Gly His Pro Thr Cys Asp Ala Cys Ser Pro Gly His
            340                 345                 350

Ser Gly Arg His Cys Glu Arg Cys Ala Pro Gly Tyr Tyr Gly Asn Pro
        355                 360                 365

Ser Gln Gly Gln Pro Cys Gln Arg Asp Ser Gln Val Pro Gly Pro Ile
    370                 375                 380

Gly Cys Asn Cys Asp Pro Gln Gly Ser Val Ser Ser Gln Cys Asp Ala
385                 390                 395                 400

Ala Gly Gln Cys Gln Cys Lys Ala Gln Val Glu Gly Leu Thr Cys Ser
                405                 410                 415

His Cys Arg Pro His His Phe His Leu Ser Ala Ser Asn Pro Asp Gly
            420                 425                 430

Cys Leu Pro Cys Phe Cys Met Gly Ile Thr Gln Gln Cys Ala Ser Ser
        435                 440                 445

Ala Tyr Thr Arg His Leu Ile Ser Thr His Phe Ala Pro Gly Asp Phe
    450                 455                 460

Gln Gly Phe Ala Leu Val Asn Pro Gln Arg Asn Ser Arg Leu Thr Gly
465                 470                 475                 480

Glu Phe Thr Val Glu Pro Val Pro Glu Gly Ala Gln Leu Ser Phe Gly
                485                 490                 495

Asn Phe Ala Gln Leu Gly His Glu Ser Phe Tyr Trp
            500                 505

<210> 4
<211> 199
<212> PRT
<213> Homo sapiens

<400> 4
Met Lys Trp Val Trp Ala Leu Leu Leu Leu Ala Ala Trp Ala Ala Ala
 1               5                  10                  15

Glu Arg Asp Cys Arg Val Ser Ser Phe Arg Val Lys Glu Asn Phe Asp
            20                  25                  30

Lys Ala Arg Phe Ser Gly Thr Trp Tyr Ala Met Ala Lys Lys Asp Pro
        35                  40                  45

Glu Gly Leu Phe Leu Gln Asp Asn Ile Val Ala Glu Phe Ser Val Asp
    50                  55                  60

Glu Thr Gly Gln Met Ser Ala Thr Ala Lys Gly Arg Val Arg Leu Leu
65                  70                  75                  80

Asn Asn Trp Asp Val Cys Ala Asp Met Val Gly Thr Phe Thr Asp Thr
                85                  90                  95

Glu Asp Pro Ala Lys Phe Lys Met Lys Tyr Trp Gly Val Ala Ser Phe
            100                 105                 110

Leu Gln Lys Gly Asn Asp Asp His Trp Ile Val Asp Thr Asp Tyr Asp
        115                 120                 125

Thr Tyr Ala Val Gln Tyr Ser Cys Arg Leu Leu Asn Leu Asp Gly Thr
    130                 135                 140

Cys Ala Asp Ser Tyr Ser Phe Val Phe Ser Arg Asp Pro Asn Gly Leu
145                 150                 155                 160

Pro Pro Glu Ala Gln Lys Ile Val Arg Gln Arg Gln Glu Glu Leu Cys
                165                 170                 175

Leu Ala Arg Gln Tyr Arg Leu Ile Val His Asn Gly Tyr Cys Asp Gly

180                 185                 190

Arg Ser Glu Arg Asn Leu Leu
        195

<210> 5
<211> 199
<212> PRT
<213> Homo sapiens

<400> 5
Met Lys Trp Val Trp Ala Leu Leu Leu Leu Ala Ala Trp Ala Ala Ala
1               5                   10                  15

Glu Arg Asp Cys Arg Val Ser Ser Phe Arg Val Lys Glu Asn Phe Asp
            20                  25                  30

Lys Ala Arg Phe Ser Gly Thr Trp Tyr Ala Met Ala Lys Lys Asp Pro
        35                  40                  45

Glu Gly Leu Phe Leu Gln Asp Asn Ile Val Ala Glu Phe Ser Val Asp
    50                  55                  60

Glu Thr Gly Gln Met Ser Ala Thr Ala Lys Gly Arg Val Arg Leu Leu
65                  70                  75                  80

Asn Asn Trp Asp Val Cys Ala Asp Met Val Gly Thr Phe Thr Asp Thr
                85                  90                  95

Glu Asp Pro Ala Lys Phe Lys Met Lys Tyr Trp Gly Val Ala Ser Phe
            100                 105                 110

Leu Gln Lys Gly Asn Asp Asp His Trp Ile Val Asp Thr Asp Tyr Asp
        115                 120                 125

Thr Tyr Ala Val Gln Tyr Ser Cys Arg Leu Leu Asn Leu Asp Gly Thr
    130                 135                 140

Cys Ala Asp Ser Tyr Ser Phe Val Phe Ser Arg Asp Pro Asn Gly Leu
145                 150                 155                 160

Pro Pro Glu Ala Gln Lys Ile Val Arg Gln Arg Gln Glu Glu Leu Cys
                165                 170                 175

Leu Ala Arg Gln Tyr Arg Leu Ile Val His Asn Gly Tyr Cys Asp Gly
            180                 185                 190

Arg Ser Glu Arg Asn Leu Leu
        195

<210> 6
<211> 199
<212> PRT
<213> Homo sapiens

<400> 6
Met Lys Trp Val Trp Ala Leu Leu Leu Leu Ala Ala Trp Ala Ala Ala
1               5                   10                  15

Glu Arg Asp Cys Arg Val Ser Ser Phe Arg Val Lys Glu Asn Phe Asp
            20                  25                  30

Lys Ala Arg Phe Ser Gly Thr Trp Tyr Ala Met Ala Lys Lys Asp Pro
        35                  40                  45

Glu Gly Leu Phe Leu Gln Asp Asn Ile Val Ala Glu Phe Ser Val Asp
    50                  55                  60

Glu Thr Gly Gln Met Ser Ala Thr Ala Lys Gly Arg Val Arg Leu Leu
65                  70                  75                  80

Asn Asn Trp Asp Val Cys Ala Asp Met Val Gly Thr Phe Thr Asp Thr
                85                  90                  95

Glu Asp Pro Ala Lys Phe Lys Met Lys Tyr Trp Gly Val Ala Ser Phe
            100                 105                 110

Leu Gln Lys Gly Asn Asp Asp His Trp Ile Val Asp Thr Asp Tyr Asp
        115                 120                 125

Thr Tyr Ala Val Gln Tyr Ser Cys Arg Leu Leu Asn Leu Asp Gly Thr
    130                 135                 140

Cys Ala Asp Ser Tyr Ser Phe Val Phe Ser Arg Asp Pro Asn Gly Leu
145                 150                 155                 160

Pro Pro Glu Ala Gln Lys Ile Val Arg Gln Arg Gln Glu Glu Leu Cys
                165                 170                 175

Leu Ala Arg Gln Tyr Arg Leu Ile Val His Asn Gly Tyr Cys Asp Gly
            180                 185                 190

Arg Ser Glu Arg Asn Leu Leu
        195

<210> 7
<211> 182
<212> PRT
<213> Homo sapiens

<400> 7
Glu Arg Asp Cys Arg Val Ser Ser Phe Arg Val Lys Glu Asn Phe Asp
1               5                   10                  15

Lys Ala Arg Phe Ser Gly Thr Trp Tyr Ala Met Ala Lys Lys Asp Pro
            20                  25                  30

Glu Gly Leu Phe Leu Gln Asp Asn Ile Val Ala Glu Phe Ser Val Asp
        35                  40                  45

Glu Thr Gly Gln Met Ser Ala Thr Ala Lys Gly Arg Val Arg Leu Leu
    50                  55                  60

Asn Asn Trp Asp Val Cys Ala Asp Met Val Gly Thr Phe Thr Asp Thr
65                  70                  75                  80

Glu Asp Pro Ala Lys Phe Lys Met Lys Tyr Trp Gly Val Ala Ser Phe
                85                  90                  95

Leu Gln Lys Gly Asn Asp Asp His Trp Ile Val Asp Thr Asp Tyr Asp
            100                 105                 110

Thr Tyr Ala Val Gln Tyr Ser Cys Arg Leu Leu Asn Leu Asp Gly Thr
        115                 120                 125

Cys Ala Asp Ser Tyr Ser Phe Val Phe Ser Arg Asp Pro Asn Gly Leu
    130                 135                 140

Pro Pro Glu Ala Gln Lys Ile Val Arg Gln Arg Gln Glu Glu Leu Cys
145                 150                 155                 160

Leu Ala Arg Gln Tyr Arg Leu Ile Val His Asn Gly Tyr Cys Asp Gly
                165                 170                 175

Arg Ser Glu Arg Asn Leu
            180

<210> 8
<211> 193
<212> PRT
<213> Homo sapiens

<400> 8
Met Gln Ser Leu Met Gln Ala Pro Leu Leu Ile Ala Leu Gly Leu Leu
1               5                   10                  15

Leu Ala Thr Pro Ala Gln Ala His Leu Lys Lys Pro Ser Gln Leu Ser
            20                  25                  30

Ser Phe Ser Trp Asp Asn Cys Asp Glu Gly Lys Asp Pro Ala Val Ile
        35                  40                  45

Arg Ser Leu Thr Leu Glu Pro Asp Pro Ile Val Val Pro Gly Asn Val
    50                  55                  60

Thr Leu Ser Val Val Gly Ser Thr Ser Val Pro Leu Ser Ser Pro Leu
65                  70                  75                  80

Lys Val Asp Leu Val Leu Glu Lys Glu Val Ala Gly Leu Trp Ile Lys
                85                  90                  95

Ile Pro Cys Thr Asp Tyr Ile Gly Ser Cys Thr Phe Glu His Phe Cys
            100                 105                 110

Asp Val Leu Asp Met Leu Ile Pro Thr Gly Glu Pro Cys Pro Glu Pro
        115                 120                 125

Leu Arg Thr Tyr Gly Leu Pro Cys His Cys Pro Phe Lys Glu Gly Thr
    130                 135                 140

Tyr Ser Leu Pro Lys Ser Glu Phe Val Val Pro Asp Leu Glu Leu Pro
145                 150                 155                 160

Ser Trp Leu Thr Thr Gly Asn Tyr Arg Ile Glu Ser Val Leu Ser Ser
                165                 170                 175

Ser Gly Lys Arg Leu Gly Cys Ile Lys Ile Ala Ala Ser Leu Lys Gly
            180                 185                 190

Ile

<210> 9
<211> 193
<212> PRT
<213> Homo sapiens

<400> 9
Met Gln Ser Leu Met Gln Ala Pro Leu Leu Ile Ala Leu Gly Leu Leu
  1               5                  10                  15

Leu Ala Thr Pro Ala Gln Ala His Leu Lys Lys Pro Ser Gln Leu Ser
            20                  25                  30

Ser Phe Ser Trp Asp Asn Cys Phe Glu Gly Lys Asp Pro Ala Val Ile
        35                  40                  45

Arg Ser Leu Thr Leu Glu Pro Asp Pro Ile Val Val Pro Gly Asn Val
    50                  55                  60

Thr Leu Ser Val Val Gly Ser Thr Ser Val Pro Leu Ser Ser Pro Leu
65                  70                  75                  80

Lys Val Asp Leu Val Leu Glu Lys Glu Val Ala Gly Leu Trp Ile Lys
                85                  90                  95

Ile Pro Cys Thr Asp Tyr Ile Gly Ser Cys Thr Phe Glu His Phe Cys
            100                 105                 110

Asp Val Leu Asp Met Leu Ile Pro Thr Gly Glu Pro Cys Pro Glu Pro
        115                 120                 125

Leu Arg Thr Tyr Gly Leu Pro Cys His Cys Pro Phe Lys Glu Gly Thr
    130                 135                 140

Tyr Ser Leu Pro Lys Ser Glu Phe Ala Val Pro Asp Leu Glu Leu Pro
145                 150                 155                 160

Ser Trp Leu Thr Thr Gly Asn Tyr Arg Ile Glu Ser Val Leu Ser Ser
                165                 170                 175

Ser Gly Lys Arg Leu Gly Cys Ile Lys Ile Ala Ala Ser Leu Lys Gly

            180                 185                 190

Ile

<210> 10
<211> 178
<212> PRT
<213> Homo sapiens

<400> 10
Leu Leu Ala Thr Pro Ala Gln Ala His Leu Lys Lys Pro Ser Gln Leu
1               5                   10                  15

Ser Ser Phe Ser Trp Asp Asn Cys Asp Glu Gly Lys Asp Pro Ala Val
            20                  25                  30

Ile Arg Ser Leu Thr Leu Glu Pro Asp Pro Ile Val Val Pro Gly Asn
        35                  40                  45

Val Thr Leu Ser Val Val Gly Ser Thr Ser Val Pro Leu Ser Ser Pro
    50                  55                  60

Leu Lys Val Asp Leu Val Leu Glu Lys Glu Val Ala Gly Leu Trp Ile
65                  70                  75                  80

Lys Ile Pro Cys Thr Asp Tyr Ile Gly Ser Cys Thr Phe Glu His Phe
                85                  90                  95

Cys Asp Val Leu Asp Met Leu Ile Pro Thr Gly Glu Pro Cys Pro Glu
            100                 105                 110

Pro Leu Arg Thr Tyr Gly Leu Pro Cys His Cys Pro Phe Lys Glu Gly
        115                 120                 125

Thr Tyr Ser Leu Pro Lys Ser Glu Phe Val Val Pro Asp Leu Glu Leu
    130                 135                 140

Pro Ser Trp Leu Thr Thr Gly Asn Tyr Arg Ile Glu Ser Val Leu Ser
145                 150                 155                 160

Ser Ser Gly Lys Arg Leu Gly Cys Ile Lys Ile Ala Ala Ser Leu Lys
                165                 170                 175

Gly Ile

<210> 11
<211> 200
<212> PRT
<213> Homo sapiens

<400> 11
Arg Ala Gly Pro Pro Phe Pro Met Gln Ser Leu Met Gln Ala Pro Leu

1                   5                   10                  15

Leu Ile Ala Leu Gly Leu Leu Leu Ala Ala Pro Ala Gln Ala His Leu
                20                  25                  30

Lys Lys Pro Ser Gln Leu Ser Ser Phe Ser Trp Asp Asn Cys Asp Glu
            35                  40                  45

Gly Lys Asp Pro Ala Val Ile Arg Ser Leu Thr Leu Glu Pro Asp Pro
    50                  55                  60

Ile Ile Val Pro Gly Asn Val Thr Leu Ser Val Met Gly Ser Thr Ser
65                  70                  75                  80

Val Pro Leu Ser Ser Pro Leu Lys Val Asp Leu Val Leu Glu Lys Glu
                85                  90                  95

Val Ala Gly Leu Trp Ile Lys Ile Pro Cys Thr Asp Tyr Ile Gly Ser
            100                 105                 110

Cys Thr Phe Glu His Phe Cys Asp Val Leu Asp Met Leu Ile Pro Thr
        115                 120                 125

Gly Glu Pro Cys Pro Glu Pro Leu Arg Thr Tyr Gly Leu Pro Cys His
    130                 135                 140

Cys Pro Phe Lys Glu Gly Thr Tyr Ser Leu Pro Lys Ser Glu Phe Val
145                 150                 155                 160

Val Pro Asp Leu Glu Leu Pro Ser Trp Leu Thr Thr Gly Asn Tyr Arg
                165                 170                 175

Ile Glu Ser Val Leu Ser Ser Ser Gly Lys Arg Leu Gly Cys Ile Lys
            180                 185                 190

Ile Ala Ala Ser Leu Lys Gly Ile
        195                 200

<210> 12
<211> 189
<212> PRT
<213> Homo sapiens

<400> 12
Met Gln Ala Pro Leu Leu Ile Ala Leu Gly Leu Leu Leu Ala Thr Pro
1               5                   10                  15

Ala Gln Ala His Leu Lys Lys Pro Ser Gln Leu Ser Ser Phe Ser Trp
            20                  25                  30

Asp Asn Cys Asp Glu Gly Lys Asp Pro Ala Val Ile Arg Ser Leu Thr
        35                  40                  45

Leu Glu Pro Asp Pro Ile Val Val Pro Gly Asn Val Thr Leu Ser Val
    50                  55                  60

Val Gly Ser Thr Ser Val Pro Leu Ser Ser Pro Leu Lys Val Asp Leu
65                  70                  75                  80

Val Leu Glu Lys Glu Val Ala Gly Leu Trp Ile Lys Ile Pro Cys Thr
                85                  90                  95

Asp Tyr Ile Gly Ser Cys Thr Phe Glu His Phe Cys Asp Val Leu Asp
            100                 105                 110

Met Leu Ile Pro Thr Gly Glu Pro Cys Pro Glu Pro Leu Arg Thr Tyr
        115                 120                 125

Gly Leu Pro Cys His Cys Pro Phe Lys Glu Gly Thr Tyr Ser Leu Pro
    130                 135                 140

Lys Ser Glu Phe Val Val Pro Asp Leu Glu Leu Pro Ser Trp Leu Thr
145                 150                 155                 160

Thr Gly Asn Tyr Arg Ile Glu Ser Val Leu Ser Ser Ser Gly Lys Arg
                165                 170                 175

Leu Gly Cys Ile Lys Ile Ala Ala Ser Leu Lys Gly Ile
            180                 185

<210> 13
<211> 193
<212> PRT
<213> Homo sapiens

<400> 13
Met Gln Ser Leu Met Gln Ala Pro Leu Leu Ile Ala Leu Gly Leu Leu
1               5                   10                  15

Leu Ala Thr Pro Ala Gln Ala His Leu Lys Lys Pro Ser Gln Leu Ser
            20                  25                  30

Ser Phe Ser Trp Asp Asn Cys Asp Glu Gly Lys Asp Pro Ala Val Ile
        35                  40                  45

Arg Ser Leu Thr Leu Glu Pro Asp Pro Ile Val Val Pro Gly Asn Val
    50                  55                  60

Thr Leu Ser Val Val Gly Ser Thr Ser Val Pro Leu Ser Ser Pro Leu
65                  70                  75                  80

Lys Val Asp Leu Val Leu Glu Lys Glu Val Ala Gly Leu Trp Ile Lys
                85                  90                  95

Ile Pro Cys Thr Asp Tyr Ile Gly Ser Cys Thr Phe Glu His Phe Cys
            100                 105                 110

Asp Val Leu Asp Met Leu Ile Pro Thr Gly Glu Pro Cys Pro Glu Pro
        115                 120                 125

Leu Arg Thr Tyr Gly Leu Pro Cys His Cys Pro Phe Lys Glu Gly Thr
    130                 135                 140

Tyr Ser Leu Pro Lys Ser Glu Phe Val Val Pro Asp Leu Glu Leu Pro
145                 150                 155                 160

Ser Trp Leu Thr Thr Gly Asn Tyr Arg Ile Glu Ser Val Leu Ser Ser
                165                 170                 175

Ser Gly Lys Arg Leu Gly Cys Ile Lys Ile Ala Ala Ser Leu Lys Gly
            180                 185                 190

Ile

<210> 14
<211> 193
<212> PRT
<213> Homo sapiens

<400> 14
Met Gln Ser Leu Met Gln Ala Pro Leu Leu Ile Ala Leu Gly Leu Leu
1               5                   10                  15

Leu Ala Thr Pro Ala Gln Ala His Leu Lys Lys Pro Ser Gln Leu Ser
            20                  25                  30

Ser Phe Ser Trp Asp Asn Cys Asp Glu Gly Lys Asp Pro Ala Val Ile
        35                  40                  45

Arg Ser Leu Thr Leu Glu Pro Asp Pro Ile Val Val Pro Gly Asn Val
    50                  55                  60

Thr Leu Ser Val Val Gly Ser Thr Ser Val Pro Leu Ser Ser Pro Leu
65                  70                  75                  80

Lys Val Asp Leu Val Leu Glu Lys Glu Val Ala Gly Leu Trp Ile Lys
                85                  90                  95

Ile Pro Cys Thr Asp Tyr Ile Gly Ser Cys Thr Phe Glu His Phe Cys
            100                 105                 110

Asp Val Leu Asp Met Leu Ile Pro Thr Gly Glu Pro Cys Pro Glu Pro
        115                 120                 125

Leu Arg Thr Tyr Gly Leu Pro Cys His Cys Pro Phe Lys Glu Gly Thr
    130                 135                 140

Tyr Ser Leu Pro Lys Ser Glu Phe Val Val Pro Asp Leu Glu Leu Pro
145                 150                 155                 160

Ser Trp Leu Thr Thr Gly Asn Tyr Arg Ile Glu Ser Val Leu Ser Ser
                165                 170                 175

Ser Gly Lys Arg Leu Gly Cys Ile Lys Ile Ala Ala Ser Leu Lys Gly
            180                 185                 190

Ile

<210> 15
<211> 193
<212> PRT
<213> Homo sapiens

<400> 15
Met Gln Ser Leu Met Gln Ala Pro Leu Leu Ile Ala Leu Gly Leu Leu
1               5                   10                  15

Leu Ala Thr Pro Ala Gln Ala His Leu Lys Lys Pro Ser Gln Leu Ser
            20                  25                  30

Ser Phe Ser Trp Asp Asn Cys Asp Glu Gly Lys Asp Pro Ala Val Ile
        35                  40                  45

Arg Ser Leu Thr Leu Glu Pro Asp Pro Ile Val Val Pro Gly Asn Val
    50                  55                  60

Thr Leu Ser Val Val Gly Ser Thr Ser Val Pro Leu Ser Ser Pro Leu
65                  70                  75                  80

Lys Val Asp Leu Val Leu Glu Lys Glu Val Ala Gly Leu Trp Ile Lys
                85                  90                  95

Ile Pro Cys Thr Asp Tyr Ile Gly Ser Cys Thr Phe Glu His Phe Cys
            100                 105                 110

Asp Val Leu Asp Met Leu Ile Pro Thr Gly Glu Pro Cys Pro Glu Pro
        115                 120                 125

Leu Arg Thr Tyr Gly Leu Pro Cys His Cys Pro Phe Lys Glu Gly Thr
    130                 135                 140

Tyr Ser Leu Pro Lys Ser Glu Phe Val Val Pro Asp Leu Glu Leu Pro
145                 150                 155                 160

Ser Trp Leu Thr Thr Gly Asn Tyr Arg Ile Glu Ser Val Leu Ser Ser
                165                 170                 175

Ser Gly Lys Arg Leu Gly Cys Ile Lys Ile Ala Ala Ser Leu Lys Gly
            180                 185                 190

Ile

<210> 16
<211> 193
<212> PRT
<213> Homo sapiens

<400> 16
Met Gln Ser Leu Met Gln Ala Pro Leu Leu Ile Ala Leu Gly Leu Leu

1                5                   10                  15

Leu Ala Thr Pro Ala Gln Ala His Leu Lys Lys Pro Ser Gln Leu Ser
            20                  25                  30

Ser Phe Ser Trp Asp Asn Cys Asp Glu Gly Lys Asp Pro Ala Val Ile
        35                  40                  45

Arg Ser Leu Thr Leu Glu Pro Asp Pro Ile Val Val Pro Gly Asn Val
    50                  55                  60

Thr Leu Ser Val Val Gly Ser Thr Ser Val Pro Leu Ser Ser Pro Leu
65                  70                  75                  80

Lys Val Asp Leu Val Leu Glu Lys Glu Val Ala Gly Leu Trp Ile Lys
                85                  90                  95

Ile Pro Cys Thr Asp Tyr Ile Gly Ser Cys Thr Phe Glu His Phe Cys
            100                 105                 110

Asp Val Leu Asp Met Leu Ile Pro Thr Gly Glu Pro Cys Pro Glu Pro
        115                 120                 125

Leu Arg Thr Tyr Gly Leu Pro Cys His Cys Pro Phe Lys Glu Gly Thr
    130                 135                 140

Tyr Ser Leu Pro Lys Ser Glu Phe Val Val Pro Asp Leu Glu Leu Pro
145                 150                 155                 160

Ser Trp Leu Thr Thr Gly Asn Tyr Arg Ile Glu Ser Val Leu Ser Ser
                165                 170                 175

Ser Gly Lys Arg Leu Gly Cys Ile Lys Ile Ala Ala Ser Leu Lys Gly
            180                 185                 190

Ile

<210> 17
<211> 114
<212> PRT
<213> Homo sapiens

<400> 17
Met Thr Cys Lys Met Ser Gln Leu Glu Arg Asn Ile Glu Thr Ile Ile
1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Asn Ala Asp Lys Gln Leu Ser Phe Glu Glu Phe Ile
65                  70                  75                  80

Met Leu Met Ala Arg Leu Thr Trp Ala Ser His Glu Lys Met His Glu
                85                  90                  95

Gly Asp Glu Gly Pro Gly His His His Lys Pro Gly Leu Gly Glu Gly
            100                 105                 110

Thr Pro

<210> 18
<211> 93
<212> PRT
<213> Homo sapiens

<400> 18
Met Leu Thr Glu Leu Glu Lys Ala Leu Asn Ser Ile Ile Asp Val Tyr
1               5                   10                  15

His Lys Tyr Ser Leu Ile Lys Gly Asn Phe His Ala Val Tyr Arg Asp
            20                  25                  30

Asp Leu Lys Lys Leu Leu Glu Thr Glu Cys Pro Gln Tyr Ile Arg Lys
        35                  40                  45

Lys Gly Ala Asp Val Trp Phe Lys Glu Leu Asp Ile Asn Thr Asp Gly
    50                  55                  60

Ala Val Asn Phe Gln Glu Phe Leu Ile Leu Val Ile Lys Met Gly Val
65                  70                  75                  80

Ala Ala His Lys Lys Ser His Glu Glu Ser His Lys Glu
                85                  90

<210> 19
<211> 92
<212> PRT
<213> Homo sapiens

<400> 19
Met Thr Lys Leu Glu Glu His Leu Glu Gly Ile Val Asn Ile Phe His
1               5                   10                  15

Gln Tyr Ser Val Arg Lys Gly His Phe Asp Thr Leu Ser Lys Gly Glu
            20                  25                  30

Leu Lys Gln Leu Leu Thr Lys Glu Leu Ala Asn Thr Ile Lys Asn Ile
        35                  40                  45

Lys Asp Lys Ala Val Ile Asp Glu Ile Phe Gln Gly Leu Asp Ala Asn
    50                  55                  60

Gln Asp Glu Gln Val Asp Phe Gln Glu Phe Ile Ser Leu Val Ala Ile
65                  70                  75                  80

Ala Leu Lys Ala Ala His Tyr His Thr His Lys Glu
                85                  90

<210> 20
<211> 92
<212> PRT
<213> Homo sapiens

<400> 20
Met Thr Lys Leu Glu Glu His Leu Glu Gly Ile Val Asn Ile Phe His
1               5                   10                  15

Gln Tyr Ser Val Arg Lys Gly His Phe Asp Thr Leu Ser Lys Gly Glu
            20                  25                  30

Leu Lys Gln Leu Leu Thr Lys Glu Leu Ala Asn Thr Ile Lys Asn Ile
        35                  40                  45

Lys Asp Lys Ala Val Ile Asp Glu Ile Phe Gln Gly Leu Asp Ala Asn
    50                  55                  60

Gln Asp Glu Gln Val Asp Phe Gln Glu Phe Ile Ser Leu Val Ala Ile
65                  70                  75                  80

Ala Leu Lys Ala Ala His Tyr His Thr His Lys Glu
                85                  90

<210> 21
<211> 91
<212> PRT
<213> Homo sapiens

<400> 21
Thr Lys Leu Glu Glu His Leu Glu Gly Ile Val Asn Ile Phe His Gln
1               5                   10                  15

Tyr Ser Val Arg Lys Gly His Phe Asp Thr Leu Ser Lys Gly Glu Leu
            20                  25                  30

Lys Gln Leu Leu Thr Lys Glu Leu Ala Asn Thr Ile Lys Asn Ile Lys
        35                  40                  45

Asp Lys Ala Val Ile Asp Glu Ile Phe Gln Gly Leu Asp Ala Asn Gln
    50                  55                  60

Asp Glu Gln Val Asp Phe Gln Glu Phe Ile Ser Leu Val Ala Ile Ala
65                  70                  75                  80

Leu Lys Ala Ala His Tyr His Thr His Lys Glu
                85                  90

<210> 22
<211> 93
<212> PRT
<213> Homo sapiens

<400> 22
Met Leu Thr Glu Leu Glu Lys Ala Leu Asn Ser Ile Ile Asp Val Tyr
1               5                   10                  15

His Lys Tyr Ser Leu Ile Lys Gly Asn Phe His Ala Val Tyr Arg Asp
            20                  25                  30

Asp Leu Lys Lys Leu Leu Glu Thr Glu Cys Pro Gln Tyr Ile Arg Lys
        35                  40                  45

Lys Gly Ala Asp Val Trp Phe Lys Glu Leu Asp Ile Asn Thr Asp Gly
    50                  55                  60

Ala Val Asn Phe Gln Glu Phe Leu Ile Leu Val Ile Lys Met Gly Val
65                  70                  75                  80

Ala Ala His Lys Lys Ser His Glu Glu Ser His Lys Glu
                85                  90

<210> 23
<211> 92
<212> PRT
<213> Homo sapiens

<400> 23
Met Thr Lys Leu Glu Glu His Leu Glu Gly Ile Val Asn Ile Phe His
1               5                   10                  15

Gln Tyr Ser Val Arg Lys Gly His Phe Asp Thr Leu Ser Lys Gly Glu
            20                  25                  30

Leu Lys Gln Leu Leu Thr Lys Glu Leu Ala Asn Thr Ile Lys Asn Ile
        35                  40                  45

Lys Asp Lys Ala Val Ile Asp Glu Ile Phe Gln Gly Leu Asp Ala Asn
    50                  55                  60

Gln Asp Glu Gln Val Asp Phe Gln Glu Phe Ile Ser Leu Val Ala Ile
65                  70                  75                  80

Ala Leu Lys Ala Ala His Tyr His Thr His Lys Glu
                85                  90

<210> 24
<211> 85
<212> PRT
<213> Homo sapiens

<400> 24
Asp Asn Gly Asp Val Cys Gln Asp Cys Ile Gln Met Val Thr Asp Ile
1               5                   10                  15

Gln Thr Ala Val Arg Thr Asn Ser Thr Phe Val Gln Ala Leu Val Glu
            20                  25                  30

His Val Lys Glu Glu Cys Asp Arg Leu Gly Pro Gly Met Ala Asp Ile
        35                  40                  45

Cys Lys Asn Tyr Ile Ser Gln Tyr Ser Glu Ile Ala Ile Gln Met Met
    50                  55                  60

Met His Met Gln Asp Gln Gln Pro Lys Glu Ile Cys Ala Leu Val Gly
65                  70                  75                  80

Phe Cys Asp Glu Val
                85

<210> 25
<211> 381
<212> PRT
<213> Homo sapiens

<400> 25
Met Ala Glu Ser His Leu Leu Gln Trp Leu Leu Leu Leu Leu Pro Thr
1               5                   10                  15

Leu Cys Gly Pro Gly Thr Ala Ala Trp Thr Thr Ser Ser Leu Ala Cys
            20                  25                  30

Ala Gln Gly Pro Glu Phe Trp Cys Gln Ser Leu Glu Gln Ala Leu Gln
        35                  40                  45

Cys Arg Ala Leu Gly His Cys Leu Gln Glu Val Trp Gly His Val Gly
    50                  55                  60

Ala Asp Asp Leu Cys Gln Glu Cys Glu Asp Ile Val His Ile Leu Asn
65                  70                  75                  80

Lys Met Ala Lys Glu Ala Ile Phe Gln Asp Thr Met Arg Lys Phe Leu
                85                  90                  95

Glu Gln Glu Cys Asn Val Leu Pro Leu Lys Leu Leu Met Pro Gln Cys
            100                 105                 110

Asn Gln Val Leu Asp Asp Tyr Phe Pro Leu Val Ile Asp Tyr Phe Gln
        115                 120                 125

Asn Gln Ile Asp Ser Asn Gly Ile Cys Met His Leu Gly Leu Cys Lys
    130                 135                 140

Ser Arg Gln Pro Glu Pro Glu Gln Glu Pro Gly Met Ser Asp Pro Leu
145                 150                 155                 160

Pro Lys Pro Leu Arg Asp Pro Leu Pro Asp Pro Leu Leu Asp Lys Leu
                165                 170                 175

Val Leu Pro Val Leu Pro Gly Ala Leu Gln Ala Arg Pro Gly Pro His
            180                 185                 190

Thr Gln Asp Leu Ser Glu Gln Gln Phe Pro Ile Pro Leu Pro Tyr Cys
        195                 200                 205

Trp Leu Cys Arg Ala Leu Ile Lys Arg Ile Gln Ala Met Ile Pro Lys
    210                 215                 220

Gly Ala Leu Arg Val Ala Val Ala Gln Val Cys Arg Val Val Pro Leu
225                 230                 235                 240

Val Ala Gly Gly Ile Cys Gln Cys Leu Ala Glu Arg Tyr Ser Val Ile
                245                 250                 255

Leu Leu Asp Thr Leu Leu Gly Arg Met Leu Pro Gln Leu Val Cys Arg
            260                 265                 270

Leu Val Leu Arg Cys Ser Met Asp Asp Ser Ala Gly Pro Arg Ser Pro
        275                 280                 285

Thr Gly Glu Trp Leu Pro Arg Asp Ser Glu Cys His Leu Cys Met Ser
    290                 295                 300

Val Thr Thr Gln Ala Gly Asn Ser Ser Glu Gln Ala Ile Pro Gln Ala
305                 310                 315                 320

Met Leu Gln Ala Cys Val Gly Ser Trp Leu Asp Arg Glu Lys Cys Lys
                325                 330                 335

Gln Phe Val Glu Gln His Thr Pro Gln Leu Leu Thr Leu Val Pro Arg
            340                 345                 350

Gly Trp Asp Ala His Thr Thr Cys Gln Ala Leu Gly Val Cys Gly Thr
        355                 360                 365

Met Ser Ser Pro Leu Gln Cys Ile His Ser Pro Asp Leu
    370                 375                 380

<210> 26
<211> 379
<212> PRT
<213> Homo sapiens

<400> 26
Met Ala Glu Ser His Leu Leu Gln Trp Leu Leu Leu Leu Leu Pro Thr
1               5                   10                  15

Leu Cys Gly Pro Gly Thr Ala Ala Trp Thr Thr Ser Ser Leu Ala Cys
            20                  25                  30

Ala Gln Gly Pro Glu Phe Trp Cys Gln Ser Leu Glu Gln Ala Leu Gln
        35                  40                  45

Cys Arg Ala Leu Gly His Cys Leu Gln Glu Val Trp Gly His Val Gly
    50                  55                  60

Ala Asp Asp Leu Cys Gln Glu Cys Glu Asp Ile Val His Ile Leu Asn
65                  70                  75                  80

Lys Met Ala Lys Glu Ala Ile Phe Gln Asp Thr Met Arg Lys Phe Leu
                85                  90                  95

Glu Gln Glu Cys Asn Val Leu Pro Leu Lys Leu Leu Met Pro Gln Cys
            100                 105                 110

Asn Gln Val Leu Asp Asp Tyr Phe Pro Leu Val Ile Asp Tyr Phe Gln
        115                 120                 125

Asn Gln Thr Asp Ser Asn Gly Ile Cys Met His Leu Gly Cys Lys Ser
    130                 135                 140

Arg Gln Pro Glu Pro Glu Gln Glu Pro Gly Met Ser Asp Pro Leu Pro
145                 150                 155                 160

Lys Pro Leu Arg Asp Pro Leu Pro Asp Pro Leu Leu Asp Lys Leu Val
                165                 170                 175

Leu Pro Val Leu Pro Gly Ala Leu Gln Ala Arg Pro Gly Pro His Thr
            180                 185                 190

Gln Asp Leu Ser Glu Gln Gln Phe Pro Ile Pro Leu Pro Tyr Cys Trp
        195                 200                 205

Cys Arg Ala Leu Ile Lys Arg Ile Gln Ala Met Ile Pro Lys Gly Ala
    210                 215                 220

Leu Arg Val Ala Val Ala Gln Val Cys Arg Val Val Pro Leu Val Ala
225                 230                 235                 240

Gly Gly Ile Cys Gln Cys Leu Ala Glu Arg Tyr Ser Val Ile Leu Leu
                245                 250                 255

Asp Thr Leu Leu Gly Arg Met Leu Pro Gln Leu Val Cys Arg Leu Val
            260                 265                 270

Leu Arg Cys Ser Met Asp Asp Ser Ala Gly Pro Arg Ser Pro Thr Gly
        275                 280                 285

Glu Trp Leu Pro Arg Asp Ser Glu Cys His Leu Cys Met Ser Val Thr
    290                 295                 300

Thr Gln Ala Gly Asn Ser Ser Glu Gln Ala Ile Pro Gln Ala Met Leu
305                 310                 315                 320

Gln Ala Cys Val Gly Ser Trp Leu Asp Arg Glu Lys Cys Lys Gln Phe
                325                 330                 335

Val Glu Gln His Thr Pro Gln Leu Leu Thr Leu Val Pro Arg Gly Trp
            340                 345                 350

Asp Ala His Thr Thr Cys Gln Ala Leu Gly Val Cys Gly Thr Met Ser
        355                 360                 365

Ser Pro Leu Gln Cys Ile His Ser Pro Asp Leu
    370                 375

<210> 27
<211> 527
<212> PRT
<213> Homo sapiens

<400> 27
Met Tyr Ala Leu Phe Leu Leu Ala Ser Leu Leu Gly Ala Ala Leu Ala
1               5                   10                  15

Gly Pro Val Leu Gly Leu Lys Glu Cys Thr Arg Gly Ser Ala Val Trp
            20                  25                  30

Cys Gln Asn Val Lys Thr Ala Ser Asp Cys Gly Ala Val Lys His Cys
        35                  40                  45

Leu Gln Thr Val Trp Asn Lys Pro Thr Val Lys Ser Leu Pro Cys Asp
    50                  55                  60

Ile Cys Lys Asp Val Val Thr Ala Ala Gly Asp Met Leu Lys Asp Asn
65                  70                  75                  80

Ala Thr Glu Glu Glu Ile Leu Val Tyr Leu Glu Lys Thr Cys Asp Trp
                85                  90                  95

Leu Pro Lys Pro Asn Met Ser Ala Ser Cys Lys Glu Ile Val Asp Ser
            100                 105                 110

Tyr Leu Pro Val Ile Leu Asp Ile Ile Lys Gly Glu Met Ser Arg Pro
        115                 120                 125

Gly Glu Val Cys Ser Ala Leu Asn Leu Cys Glu Ser Leu Gln Lys His
    130                 135                 140

Leu Ala Glu Leu Asn His Gln Lys Gln Leu Glu Ser Asn Lys Ile Pro
145                 150                 155                 160

Glu Leu Asp Met Thr Glu Val Val Ala Pro Phe Met Ala Asn Ile Pro
                165                 170                 175

Leu Leu Leu Tyr Pro Gln Asp Gly Pro Arg Ser Lys Pro Gln Pro Lys
            180                 185                 190

Asp Asn Gly Asp Val Cys Gln Asp Cys Ile Gln Met Val Thr Asp Ile
        195                 200                 205

Gln Thr Ala Val Arg Thr Asn Ser Thr Phe Val Gln Ala Leu Val Glu
    210                 215                 220

His Val Lys Glu Glu Cys Asp Arg Leu Gly Pro Gly Met Ala Asp Ile
225                 230                 235                 240

Cys Lys Asn Tyr Ile Ser Gln Tyr Ser Glu Ile Ala Ile Gln Met Met
                245                 250                 255

Met His Met Gln Asp Gln Gln Pro Lys Glu Ile Cys Ala Leu Val Gly
            260                 265                 270

Phe Cys Asp Glu Val Lys Glu Met Pro Met Gln Thr Leu Val Pro Ala
        275                 280                 285

Lys Val Ala Ser Lys Asn Val Ile Pro Ala Leu Glu Leu Val Glu Pro
    290                 295                 300

Ile Lys Lys His Glu Val Pro Ala Lys Ser Asp Val Tyr Cys Glu Val
305                 310                 315                 320

Cys Glu Phe Leu Val Lys Glu Val Thr Lys Leu Ile Asp Asn Asn Lys
                325                 330                 335

Thr Glu Lys Glu Ile Leu Asp Ala Phe Asp Lys Met Cys Ser Lys Leu
            340                 345                 350

Pro Lys Ser Leu Ser Glu Glu Cys Gln Glu Val Val Asp Thr Tyr Gly
        355                 360                 365

Ser Ser Ile Leu Ser Ile Leu Leu Glu Glu Val Ser Pro Glu Leu Val
    370                 375                 380

Cys Ser Met Leu His Leu Cys Ser Gly Thr Arg Leu Pro Ala Leu Thr
385                 390                 395                 400

Val His Val Thr Gln Pro Lys Asp Gly Gly Phe Cys Glu Val Cys Lys
                405                 410                 415

Lys Leu Val Gly Tyr Leu Asp Arg Asn Leu Glu Lys Asn Ser Thr Lys
            420                 425                 430

Gln Glu Ile Leu Ala Ala Leu Glu Lys Gly Cys Ser Phe Leu Pro Asp
        435                 440                 445

Pro Tyr Gln Lys Gln Cys Asp Gln Phe Val Ala Glu Tyr Glu Pro Val
    450                 455                 460

Leu Ile Glu Ile Leu Val Glu Val Met Asp Pro Ser Phe Val Cys Leu
465                 470                 475                 480

Lys Ile Gly Ala Cys Pro Ser Ala His Lys Pro Leu Leu Gly Thr Glu
                485                 490                 495

Lys Cys Ile Trp Gly Pro Ser Tyr Trp Cys Gln Asn Thr Glu Thr Ala
            500                 505                 510

Ala Gln Cys Asn Ala Val Glu His Cys Lys Arg His Val Trp Asn
        515                 520                 525

<210> 28

<211> 523
<212> PRT
<213> Homo sapiens

<400> 28
Met Tyr Ala Leu Phe Leu Leu Ala Ser Leu Leu Gly Ala Ala Leu Ala
1               5                   10                  15

Gly Pro Val Leu Gly Leu Lys Glu Cys Thr Arg Gly Ser Ala Val Trp
            20                  25                  30

Cys Gln Asn Val Lys Thr Ala Ser Asp Cys Gly Ala Val Lys His Cys
        35                  40                  45

Leu Gln Thr Val Trp Asn Lys Pro Thr Val Lys Ser Leu Pro Cys Asp
    50                  55                  60

Ile Cys Lys Asp Val Val Thr Ala Ala Gly Asp Met Leu Lys Asp Asn
65                  70                  75                  80

Ala Thr Glu Glu Glu Ile Leu Val Tyr Leu Glu Lys Thr Cys Asp Trp
                85                  90                  95

Leu Pro Lys Pro Asn Met Ser Ala Ser Cys Lys Glu Ile Val Asp Ser
            100                 105                 110

Tyr Leu Pro Val Ile Leu Asp Ile Ile Lys Gly Glu Met Ser Arg Pro
        115                 120                 125

Gly Glu Val Cys Ser Ala Leu Leu Cys Glu Ser Leu Gln Lys His Leu
    130                 135                 140

Ala Glu Leu Asn His Gln Lys Gln Leu Glu Ser Asn Lys Ile Pro Glu
145                 150                 155                 160

Leu Asp Met Thr Glu Val Val Ala Pro Phe Met Ala Asn Ile Pro Leu
                165                 170                 175

Leu Leu Tyr Pro Gln Asp Gly Pro Arg Ser Lys Pro Gln Pro Lys Asp
            180                 185                 190

Asn Gly Asp Val Cys Gln Asp Cys Ile Gln Met Val Thr Asp Ile Gln
        195                 200                 205

Thr Ala Val Arg Thr Asn Ser Thr Phe Val Gln Ala Leu Val Glu His
    210                 215                 220

Val Lys Glu Glu Cys Asp Arg Leu Gly Pro Gly Met Ala Asp Ile Cys
225                 230                 235                 240

Lys Asn Tyr Ile Ser Gln Tyr Ser Glu Ile Ala Ile Gln Met Met Met
                245                 250                 255

His Met Gln Pro Lys Glu Ile Cys Ala Leu Val Gly Phe Cys Asp Glu
            260                 265                 270

Val Lys Glu Met Pro Met Gln Thr Leu Val Pro Ala Lys Val Ala Ser
        275                 280                 285

Lys Asn Val Ile Pro Ala Leu Glu Leu Val Glu Pro Ile Lys Lys His
    290                 295                 300

Glu Val Pro Ala Lys Ser Asp Val Tyr Cys Glu Val Cys Glu Phe Leu
305                 310                 315                 320

Val Lys Glu Val Thr Lys Leu Ile Asp Asn Asn Lys Thr Glu Lys Glu
                325                 330                 335

Ile Leu Asp Ala Phe Asp Lys Met Cys Ser Lys Leu Pro Lys Ser Leu
            340                 345                 350

Ser Glu Glu Cys Gln Glu Val Val Asp Thr Tyr Gly Ser Ser Ile Leu
        355                 360                 365

Ser Ile Leu Leu Glu Glu Val Ser Pro Glu Leu Val Cys Ser Met Leu
    370                 375                 380

His Leu Cys Ser Gly Thr Arg Leu Pro Ala Leu Thr Val His Val Thr
385                 390                 395                 400

Gln Pro Lys Asp Gly Gly Phe Cys Glu Val Cys Lys Lys Leu Val Gly
                405                 410                 415

Tyr Leu Asp Arg Asn Leu Glu Lys Asn Ser Thr Lys Gln Glu Ile Leu
            420                 425                 430

Ala Ala Leu Glu Lys Gly Cys Ser Phe Leu Pro Asp Pro Tyr Gln Lys
        435                 440                 445

Gln Cys Asp Gln Phe Val Ala Glu Tyr Glu Pro Val Leu Ile Glu Ile
    450                 455                 460

Leu Val Glu Val Met Asp Pro Ser Phe Val Cys Leu Lys Ile Gly Ala
465                 470                 475                 480

Cys Pro Ser Ala His Lys Pro Leu Leu Gly Thr Glu Lys Cys Ile Trp
                485                 490                 495

Gly Pro Ser Tyr Trp Cys Gln Asn Thr Glu Thr Ala Ala Gln Cys Asn
            500                 505                 510

Ala Val Glu His Cys Lys Arg His Val Trp Asn
        515                 520

<210> 29
<211> 380
<212> PRT
<213> Homo sapiens

<400> 29
Met Ala Glu Ser His Leu Leu Gln Trp Leu Leu Leu Leu Leu Pro Thr
1               5                   10                  15

Leu Cys Gly Pro Gly Thr Ala Ala Trp Thr Thr Ser Ser Leu Ala Cys

            20                  25                  30

Ala Gln Gly Pro Glu Phe Trp Cys Gln Ser Leu Glu Gln Ala Leu Gln
        35                  40                  45

Cys Arg Ala Leu Gly His Cys Leu Gln Glu Val Trp Gly His Val Gly
    50                  55                  60

Ala Asp Asp Leu Cys Gln Glu Cys Glu Asp Ile Val His Ile Leu Asn
65                  70                  75                  80

Lys Met Ala Lys Glu Ala Ile Phe Gln Asp Thr Met Arg Lys Phe Leu
                85                  90                  95

Glu Gln Glu Cys Asn Val Leu Pro Leu Lys Leu Leu Met Pro Gln Cys
            100                 105                 110

Asn Gln Val Leu Asp Asp Tyr Phe Pro Leu Val Ile Asp Tyr Phe Gln
        115                 120                 125

Asn Gln Thr Asp Ser Asn Gly Ile Cys Met His Gly Leu Cys Lys Ser
    130                 135                 140

Arg Gln Pro Glu Pro Glu Gln Glu Pro Gly Met Ser Asp Pro Leu Pro
145                 150                 155                 160

Lys Pro Leu Arg Asp Pro Leu Pro Asp Pro Leu Leu Asp Lys Leu Val
                165                 170                 175

Leu Pro Val Leu Pro Gly Ala Leu Gln Ala Arg Pro Gly Pro His Thr
            180                 185                 190

Gln Asp Leu Ser Glu Gln Gln Phe Pro Ile Pro Leu Pro Tyr Cys Trp
        195                 200                 205

Leu Cys Arg Ala Leu Ile Lys Arg Ile Gln Ala Met Ile Pro Lys Gly
    210                 215                 220

Ala Leu Ala Val Ala Val Ala Gln Val Cys Arg Val Val Pro Leu Val
225                 230                 235                 240

Ala Gly Gly Ile Cys Gln Cys Leu Ala Glu Arg Tyr Ser Val Ile Leu
                245                 250                 255

Leu Asp Thr Leu Leu Gly Arg Met Leu Pro Gln Leu Val Cys Arg Leu
            260                 265                 270

Val Leu Arg Cys Ser Met Asp Asp Ser Ala Gly Pro Arg Ser Pro Thr
        275                 280                 285

Gly Glu Trp Leu Pro Arg Asp Ser Glu Cys His Leu Cys Met Ser Val
    290                 295                 300

Thr Thr Gln Ala Gly Asn Ser Ser Glu Gln Ala Ile Pro Gln Ala Met
305                 310                 315                 320

Leu Gln Ala Cys Val Gly Ser Trp Leu Asp Arg Glu Lys Cys Lys Gln
                325                 330                 335

Phe Val Glu Gln His Thr Pro Gln Leu Leu Thr Leu Val Pro Arg Gly
        340                 345                 350

Trp Asp Ala His Thr Thr Cys Gln Ala Leu Gly Val Cys Gly Thr Met
    355                 360                 365

Ser Ser Pro Leu Gln Cys Ile His Ser Pro Asp Leu
    370                 375                 380

<210> 30
<211> 4124
<212> ADN
<213> Homo sapiens

<400> 30
   atgagagaat gggttctgct catgtccgtg ctgctctgtg gcctggctgg ccccacacac 60
   ctgttccagc caagcctggt gctggacatg gccaaggtcc tcttggataa ctactgcttc 120
   ccggagaacc tgctgggcat gcaggaagcc atccagcagg ccatcaagag ccatgagatt 180
   ctgagcatct cagacccgca gacgctggcc agtgtgctga cagccggggt gcagagctcc 240
   ctgaacgatc ctcgcctggt catctcctat gagcccagca cccccgagcc tcccccacaa 300
   gtcccagcac tcaccagcct ctcagaagag gaactgcttg cctggctgca aaggggcctc 360
   cgccatgagg ttctggaggg taatgtgggc tacctgcggg tggacagcgt cccgggccag 420
   gaggtgctga gcatgatggg ggagttcctg gtggcccacg tgtgggggaa tctcatgggc 480
   acctccgcct tagtgctgga tctccggcac tgcacaggag gccaggtctc tggcattccc 540
   tacatcatct cctacctgca cccagggaac accatcctgc acgtggacac tatctacaac 600
   cgcccctcca acaccaccac ggagatctgg accttgcccc aggtcctggg agaaaggtac 660
   ggtgccgaca aggatgtggt ggtcctcacc agcagccaga ccaggggcgt ggccgaggac 720
   atcgcgcaca tccttaagca gatgcgcagg gccatcgtgg tgggcgagcg gactggggga 780
   ggggccctgg acctccggaa gctgaggata ggcgagtctg acttcttctt cacggtgccc 840
   gtgtccaggt ccctggggcc ccttggtgga ggcagccaga cgtgggaggg cagcggggtg 900
   ctgccctgtg tggggactcc ggccgagcag gccctggaga aagccctggc catcctcact 960
   ctgcgcagcg cccttccagg ggtagtccac tgcctccagg aggtcctgaa ggactactac 1020
   acgctggtgg accgtgtgcc caccctgctg cagcacttgg ccagcatgga cttctccacg 1080
   gtggtctccg aggaagatct ggtcaccaag ctcaatgccg gcctgcaggc tgcgtctgag 1140
   gatcccaggc tcctggtgcg agccatcggg cccacagaaa ctccttcttg gcccgcgccc 1200
   gacgctgcag ccgaagactc accaggggtg gccccagagt tgcctgagga cgaggctatc 1260
   cggcaagcac tggtggactc tgtgttccag gtgtcggtgc tgccaggcaa tgtgggctac 1320
   ctgcgcttcg atagttttgc tgacgcctcc gtcctgggtg tgttggcccc atatgtcctg 1380
   cgccaggtgt gggagccgct acaggacacg gagcacctca tcatggacct gcgccacaac 1440
   cctggagggc catcctctgc tgtgcccctg ctcctgtcct acttccaggg ccctgaggcc 1500
   ggccccgtgc acctcttcac cacctatgat cgccgcacca acatcacgca ggagcacttc 1560
   agccacatgg agctcccggg cccacgctac agcacccaac gtggggtgta tctgctcacc 1620
   agccaccgca ccgccacggc cgcggaggag ttcgccttcc ttatgcagtc gctgggctgg 1680
   gccacactgg taggtgagat caccgcgggc aacctgctgc acacccgcac ggtgccgctg 1740
   ctggacacac ccgaaggcag cctcgcgctc accgtgccgg tcctcacctt catcgacaat 1800
   cacggcgagg cctggctggg tggtggagtg gtgcccgatg ccatcgtgct ggccgaggag 1860
   gccctggaca aagcccagga agtgctggag ttccaccaaa gcctgggggc cttggtggag 1920
   ggcacagggc acctgctgga ggcccactat gctcggccag aggtcgtggg gcagaccagt 1980
   gccctcctgc gggccaagct ggcccagggc gcctaccgca cagctgtgga cttggagtct 2040
   ctggcctctc agctcacagc agacctccag gaggtgtctg gggaccaccg cttgctagtg 2100
   ttccacagcc ctggcgagct ggtggtagag gaagcacccc caccaccccc tgctgtcccc 2160
   tctccagagg agctcaccta ccttattgag gccctgttca agacagaggt gctgcccggc 2220
   cagctgggct acctgcgttt tgacgccatg gctgaactgg agacagtgaa ggccgtgggg 2280
   ccacagctgg tgcggctggt atggcaacag ctggtggaca cggctgcgct ggtgatcgac 2340
   ctgcgctaca accctggcag ctactccacg gccatcccgc tgctctgctc ctacttcttt 2400
   gaggcagagc cccgccagca cctgtattct gtctttgaca gggccacctc aaaagtcacg 2460
   gaggtgtgga ccttgcccca ggtcgccggc cagcgctacg gctcacacaa ggacctctac 2520
   atcctgatga gccacaccag tggctctgcg gccgaggcct ttgcacacac catgcaggac 2580
   ctgcagcggg ccacggtcat tggggagccc acggccggag gcgcactctc tgtgggcatc 2640
   taccaggtgg gcagcagccc cttatatgca tccatgccca cccagatggc catgagtgcc 2700
   accacaggca aggcctggga cctggctggt gtggagcccg acatcactgt gcccatgagc 2760
   gaagcccttt ccatagccca ggacatagtg gctctgcgtg ccaaggtgcc cacggtgctg 2820
   cagacggccg ggaagctggt ggctgataac tatgcctctg ccgagctggg ggccaagatg 2880
   gccaccaaac tgagcggtct gcagagccgc tactccaggg tgacctcaga agtggcccta 2940
   gccgagatcc tgggggctga cctgcagatg ctctccggag acccacacct gaaggcagcc 3000
   catatccctg agaatgccaa ggaccgcatt cctggaattg tgcccatgca gatcccttcc 3060
   cctgaagtat ttgaagagct gatcaagttt tccttccaca ctaacgtgct tgaggacaac 3120
   attggctact tgaggtttga catgtttggg gacggtgagc tgctcaccca ggtctccagg 3180
   ctgctggtgg agcacatctg gaagaagatc atgcacacgg atgccatgat catcgacatg 3240
   aggttcaaca tcggtggccc cacatcctcc attcccatct tgtgctccta cttctttgat 3300
   gaaggccctc cagttctgct ggacaagatc tacagccggc ctgatgactc tgtcagtgaa 3360
   ctctggacac acgcccaggt tgtaggtgaa cgctatggct ccaagaagag catggtcatt 3420
   ctgaccagca gtgtgacggc cggcaccgcg gaggagttca cctatatcat gaagaggctg 3480
   ggccgggccc tggtcattgg ggaggtgacc agtgggggct gccagccacc acagacctac 3540
   cacgtggatg acaccaacct ctacctcact atccccacgg cccgttctgt gggggcctcg 3600
   gatggcagct cctgggaagg ggtgggggtg acaccccatg tggttgtccc tgcagaagag 3660
   gctctcgcca gggccaagga gatgctccag cacaaccagc tgagggtgaa gcggagccca 3720
   ggcctgcagg accacctgta gggaagggcc ccataggcag agccccaggg cagacagaac 3780
   ctctgggaca cacaccaagg gcactcctgc aggtggcccg gcctgaggtt cccaggagca 3840
   gcaaaggggc ctgctgagct ctggttaggt tacagctgga ggtgtgtata tatacacaca 3900
   cacacatgta tatacacata tatatgtgta tgtatatata tgtatatata tatggctttc 3960
   caataaccac ctaaatttta acaaaggttc cttctaagtg gtagaacttg ggtggtattt 4020
   tttaccttcc ttcttcatac tttgctcttt ttcttaaata ctcattaatg tgcatatatc 4080
   attattttca gatgcagcta tcattattcc aaaatacaaa ataa 4124

<210> 31
<211> 579
<212> ADN
<213> Homo sapiens

<400> 31
atgcarwsny tnatgcargc nccnytnytn athgcnytng gnytnytnyt ngcnacnccn 60
gcncargcnc ayytnaaraa rccnwsncar ytnwsnwsnt tywsntggga yaaytgygay 120
garggnaarg ayccngcngt nathmgnwsn ytnacnytng arccngaycc nathgtngtn 180
ccnggnaayg tnacnytnws ngtngtnggn wsnacnwsng tnccnytnws nwsnccnytn 240
aargtngayy tngtnytnga raargargtn gcnggnytnt ggathaarat hccntgyacn 300
gaytayathg gnwsntgyac nttygarcay ttytgygayg tnytngayat gytnathccn 360
acnggngarc cntgyccnga rccnytnmgn acntayggny tnccntgyca ytgyccntty 420
aargarggna cntaywsnyt nccnaarwsn garttygtng tnccngayyt ngarytnccn 480
wsntggytna cnacnggnaa ytaymgnath garwsngtny tnwsnwsnws nggnaarmgn 540
ytnggntgya thaarathgc ngcnwsnytn aarggnath 579

<210> 32
<211> 633
<212> ADN
<213> Homo sapiens

<400> 32
tttctttgcg taaccaatac tggaaggcat ttaaaggacc tctgccgcct cagaccttgc 60
agttaactcc gccctgaccc acccttcccg atgcagtccc tgatgcaggc tcccctcctg 120
atcgccctgg gcttgcttct cgcgacccct gcgcaagccc acctgaaaaa ggtgagtgca 180

ccctctttta agagtctgtt tgcagcctcc tggcccagct acgggtgtgc gggtctggct 240
gagatatggg ggtggccact ccgttctcta gaattggttc tctgcactag agccttccaa 300
agtaactaat tatgggattc tggtctgtac aatgagggtg gcctctaaag acttgttctg 360
ctccaggccc tttttggaga gattaatctc acgtctgcac tctcctgccc tccctccaag 420
cgccggagtg aaaatgcaga cagccttaaa actaaggcat tgcccccaag agattcagtc 480
ctgttaaccc tgcaccttac tcctgacccc cactccttat gtcccccatg ataaggcctg 540
ctgcctcatc tcttcccctg ctcgaatgcc ctgaggtctt cctgagagtt gggagggttt 600
gagagctttc caaggccaag aggattcact aag 633

<210> 33
<211> 1047
<212> ADN
<213> Homo sapiens

<400> 33
caggagcttg ccctcttgct gggattccaa cgctggctgg agaggagtgg gcagcaggga 60
ggtgggaagt cagagaaggt gcccaccaaa ggcctattag gtcagtctcc tgtttggaag 120
ttccaggtct atcatatcct gccttatagt ttacaataca cttttgggag attatgtctt 180
ttgagtcttt tagtttagtc ctgcctataa aatgagtagg ataagtgtta tcccaggttc 240
ataggtatgg agtctcatag atgaggctca gggacggggg tgcctcaccc aaggtcacac 300
tgccaggagc tcatttttcc tgtgatctgt gatagtttct tttgtcaacc tttttcttct 360
tctccttcct tgctgcctga ttgtccccag ccatcccagc tcagtagctt ttcctgggat 420
aactgtgatg aagggaagga ccctgcggtg atcagaagcc tgactctgga gcctgacccc 480
atcgtcgttc ctggaaatgt gaccctcagt gtcgtgggca gcaccagtgt cccccctgagt 540
tctcctctga aggtgagcct gggggtgggt ggagaagggg aggtgcgagg gtctggccag 600
caggggtact ggggcatgta tgcttgggga actgtgaaga atttcagaat cctggattcc 660
cagagaatag tacaggacat gtagattcag acactctttc acaggttcat ggaatctcag 720
gatcataaga ttgaaaggaa tctctgatgt cagcgccagc aacttcctgg tgagggcagg 780
agtgacggat accttgcacc tggcagaagc gtcctggcct tctctgggcc tggtggccaa 840
ctgctcatta ttatctgaca gctctggttg gccaatttgg ttttgctgtt aattataaaa 900
ttgatatacc aattagccag taatatatag tcactttaga aaacacaagt ggtcaaaaaa 960
taaataaaat aggccaagtg tggtaacttc atgcctgtaa ttcccacacc cttaggaggc 1020
tgaaggtggg tgggatcctt tttgagg 1047

<210> 34
<211> 1706
<212> ADN
<213> Homo sapiens

<400> 34
acagtagatg ccagtgcatt tcaatgcaag tgttagagcc aatcaatggg tagtgactac 60
ctaaagaatt ttaagactat ggattgagca tgatggctca cggcctgtaa tcccagcctt 120
tggaaggtga aggtgaaagg attgcttgag gccaggagtt ccagaccagc ttgggcaaca 180
aagtgagccc catctctaca aaaaatacaa aattagctgg gtgtggtggc atgtgcctgt 240
ctgtgtttcc cacctacatg ggaggctgag gcaggaggat cgtctgagcc caggagtttg 300
aggctgcagt gagtgcagtg agccatgata caaaaaaaaa aaataaagaa ttctaagtct 360
atgtatagtt cagtgtaggg ggaaaattca catttgatta ttaatgtctg ccatgggcac 420
aataatacac tatactcaca catgggccac aatgttgcca ttcctagaac agactatctc 480
taagatctca tccagttaaa aattctatga ttaaaatata ttgctgcttt tttgaagaca 540
gaagagctgg tatgtttgcc ctggaattta cacttataac ctttttcaaa cctttgtttt 600
attttttttt accaggtgga tttagttttg gagaaggagg tggctggcct ctggatcaag 660
atcccatgca cagactacat tggcagctgt acctttgaac acttctgtga tgtgcttgac 720
atgttaattc ctactgggga gccctgccca gagcccctgc gtacctatgg gcttccttgc 780
cactgtccct tcaaagaagt aagtacttag ggaggagaga gcgttacccc tgtggctaaa 840
gagatggggt ttggagagaa gggtctttgc attctccttc tgcagatctg catgtctctg 900
gatttgtaag ccagtgtgac ctatcaggaa tcacttatct tccgggagcc tcagttatcc 960

atctacgaaa tgggagactt gaacttagat gtgatcttca gggcccttta tccatataat 1020
ccatgctcta cagtgctatg gccgtctctc atcttgtgcg gctgttttga gaatgggaag 1080
aggggtggta gttcatggct gcaatcctag cagtggctct aggagaaaga ccccatcagt 1140
aggctcccac tgactggcgg tccactggct ttcccgcagg gaacctactc actgcccaag 1200
agcgaattcg ttgtgcctga cctggagctg cccagttggc tcaccaccgg gaactaccgc 1260
atagagagcg tcctgagcag cagtgggaag cgtctgggct gcatcaagat cgctgcctct 1320
ctaaagggca tatagcatgg catctgccac agcagaatgg agcggtgtga ggaaggtccc 1380
ttttcctctg ttttgtgttt gccaaggcca aactcccact ctctgccccc ctttaatccc 1440
ctttctacag tgagtccact accctcactg aaaatcattt tgtaccactt acatttaggg 1500
ctggggcaag cagccctgac ctaagggaga atgagttgga cagttcttga tagcccaggg 1560
catctgctgg gctgaccacg ttactcatcc ccgttaacat tctctctaaa gagcctcgtt 1620
catttccaaa gcagttaagg aatgggaaca gagtgtttta ggacctgaag aatctttatg 1680
actctctctc tttctctctt tttttt 1706

<210> 35
<211> 633
<212> ADN
<213> Homo sapiens

<400> 35
tttctttgcg taaccaatac tggaaggcat ttaaaggacc tctgccgcct cagaccttgc 60
agttaactcc gccctgaccc acccttcccg atgcagtccc tgatgcaggc tcccctcctg 120
atcgccctgg gcttgcttct cgcgacccct gcgcaagccc acctgaaaaa ggtgagtgca 180
ccctctttta agagtctgtt tgcagcctcc tggcccagct acgggtgtgc gggtctggct 240
gagatatggg ggtggccact ccgttctcta gaattggttc tctgcactag agccttccaa 300
agtaactaat tatgggattc tggtctgtac aatgagggtg gcctctaaag acttgttctg 360
ctccaggccc tttttggaga gattaatctc acgtctgcac tctcctgccc tccctccaag 420
cgccggagtg aaaatgcaga cagccttaaa actaaggcat tgcccccaag agattcagtc 480
ctgttaaccc tgcaccttac tcctgacccc cactccttat gtcccccatg ataaggcctg 540
ctgcctcatc tcttcccctg ctcgaatgcc ctgaggtctt cctgagagtt gggagggttt 600
gagagctttc caaggccaag aggattcact aag 633

<210> 36
<211> 1047
<212> ADN
<213> Homo sapiens

<400> 36
caggagcttg ccctcttgct gggattccaa cgctggctgg agaggagtgg gcagcaggga 60
ggtgggaagt cagagaaggt gcccaccaaa ggcctattag gtcagtctcc tgtttggaag 120
ttccaggtct atcatatcct gccttatagt ttacaataca cttttgggag attatgtctt 180
ttgagtcttt tagtttagtc ctgcctataa aatgagtagg ataagtgtta tcccaggttc 240
ataggtatgg agtctcatag atgaggctca gggacggggg tgcctcaccc aaggtcacac 300
tgccaggagc tcatttttcc tgtgatctgt gatagtttct tttgtcaacc tttttcttct 360
tctccttcct tgctgcctga ttgtccccag ccatcccagc tcagtagctt ttcctgggat 420
aactgtgatg aagggaagga ccctgcggtg atcagaagcc tgactctgga gcctgacccc 480
atcgtcgttc ctggaaatgt gaccctcagt gtcgtgggca gcaccagtgt ccccctgagt 540
tctcctctga aggtgagcct gggggtgggt ggagaagggg aggtgcgagg gtctggccag 600
caggggtact ggggcatgta tgcttgggga actgtgaaga atttcagaat cctggattcc 660
cagagaatag tacaggacat gtagattcag acactctttc acaggttcat ggaatctcag 720
gatcataaga ttgaaaggaa tctctgatgt cagcgccagc aacttcctgg tgagggcagg 780
agtgacggat accttgcacc tggcagaagc gtcctggcct tctctgggcc tggtggccaa 840
ctgctcatta ttatctgaca gctctggttg gccaatttgg ttttgctgtt aattataaaa 900
ttgatatacc aattagccag taatatatag tcactttaga aaacacaagt ggtcaaaaaa 960
taaataaaat aggccaagtg tggtaacttc atgcctgtaa ttcccacacc cttaggaggc 1020
tgaaggtggg tgggatcctt tttgagg 1047

<210> 37
<211> 1706
<212> ADN
<213> Homo sapiens

<400> 37
acagtagatg ccagtgcatt tcaatgcaag tgttagagcc aatcaatggg tagtgactac 60
ctaaagaatt ttaagactat ggattgagca tgatggctca cggcctgtaa tcccagcctt 120
tggaaggtga aggtgaaagg attgcttgag gccaggagtt ccagaccagc ttgggcaaca 180
aagtgagccc catctctaca aaaaatacaa aattagctgg gtgtggtggc atgtgcctgt 240
ctgtgtttcc cacctacatg ggaggctgag gcaggaggat cgtctgagcc caggagtttg 300
aggctgcagt gagtgcagtg agccatgata caaaaaaaaa aaataaagaa ttctaagtct 360
atgtatagtt cagtgtaggg ggaaaattca catttgatta ttaatgtctg ccatgggcac 420
aataatacac tatactcaca catgggccac aatgttgcca ttcctagaac agactatctc 480
taagatctca tccagttaaa aattctatga ttaaaatata ttgctgcttt tttgaagaca 540
gaagagctgg tatgtttgcc ctggaattta cacttataac ctttttcaaa cctttgtttt 600
attttttttt accaggtgga tttagttttg gagaaggagg tggctggcct ctggatcaag 660
atcccatgca cagactacat tggcagctgt acctttgaac acttctgtga tgtgcttgac 720
atgttaattc ctactgggga gccctgccca gagcccctgc gtacctatgg gcttccttgc 780
cactgtccct tcaaagaagt aagtacttag ggaggagaga gcgttacccc tgtggctaaa 840
gagatggggt ttggagagaa gggtctttgc attctccttc tgcagatctg catgtctctg 900
gatttgtaag ccagtgtgac ctatcaggaa tcacttatct tccgggagcc tcagttatcc 960
atctacgaaa tgggagactt gaacttagat gtgatcttca gggcccttta tccatataat 1020
ccatgctcta cagtgctatg gccgtctctc atcttgtgcg gctgttttga gaatggaaag 1080
aggggtggta gttcatggct gcaatcctag cagtggctct aggagaaaga ccccatcagt 1140
aggctcccac tgactggcgg tccactggct ttcccgcagg gaacctactc actgcccaag 1200
agcgaattcg ttgtgcctga cctggagctg cccagttggc tcaccaccgg gaactaccgc 1260
atagagagcg tcctgagcag cagtgggaag cgtctgggct gcatcaagat cgctgcctct 1320
ctaaagggca tatagcatgg catctgccac agcagaatgg agcggtgtga ggaaggtccc 1380
ttttcctctg ttttgtgttt gccaaggcca aactcccact ctctgccccc ctttaatccc 1440
ctttctacag tgagtccact accctcactg aaaatcattt tgtaccactt acattttagg 1500
ctggggcaag cagccctgac ctaagggaga atgagttgga cagttcttga tagcccaggg 1560
catctgctgg gctgaccacg ttactcatcc ccgttaacat tctctctaaa gagcctcgtt 1620
catttccaaa gcagttaagg aatgggaaca gagtgtttta ggacctgaag aatctttatg 1680
actctctctc tttctctctt tttttt 1706

<210> 38
<211> 1043
<212> ADN
<213> Homo sapiens

<400> 38
tttctttgcg taaccaatac tggaaggcat ttaaaggacc tctgccgcct cagaccttgc 60
agttaactcc gccctgaccc acccttcccg atgcagtccc tgatgcaggc tcccctcctg 120
atcgccctgg gcttgcttct cgcgacccct gcgcaagccc acctgaaaaa gccatcccag 180
ctcagtagct tttcctggga taactgtgat gaagggaagg accctgcggt gatcagaagc 240
ctgactctgg agcctgaccc catcgtcgtt cctggaaatg tgaccctcag tgtcgtgggc 300
agcaccagtg tccccctgag ttctcctctg aaggtggatt tagttttgga gaaggaggtg 360
gctggcctct ggatcaagat cccatgcaca gactacattg gcagctgtac ctttgaacac 420
ttctgtgatg tgcttgacat gttaattcct actggggagc cctgcccaga gcccctgcgt 480
acctatgggc ttccttgcca ctgtcccttc aaagaaggaa cctactcact gcccaagagc 540
gaattcgttg tgcctgacct ggagctgccc agttggctca ccaccgggaa ctaccgcata 600
gagagcgtcc tgagcagcag tgggaagcgt ctgggctgca tcaagatcgc tgcctctcta 660
aagggcatat agcatggcat ctgccacagc agaatggagc ggtgtgagga aggtcccttt 720
tcctctgttt tgtgtttgcc aaggccaaac tcccactctc tgcccccctt taatcccctt 780

tctacagtga gtccactacc ctcactgaaa atcattttgt accacttaca ttttaggctg 840
gggcaagcag ccctgaccta agggagaatg agttggacag ttcttgatag cccagggcat 900
ctgctgggct gaccacgtta ctcatccccg ttaacattct ctctaaagag cctcgttcat 960
ttccaaagca gttaaggaat gggaacagag tgttttagga cctgaagaat ctttatgact 1020
ctctctcttt ctctcttttt ttt 1043

<210> 39
<211> 1047
<212> ADN
<213> Homo sapiens

<400> 39
caggagcttg ccctcttgct gggattccaa cgctggctgg agaggagtgg gcagcaggga 60
ggtgggaagt cagagaaggt gcccaccaaa ggcctattag gtcagtctcc tgtttggaag 120
ttccaggtct atcatatcct gccttatagt ttacaataca cttttgggag attatgtctt 180
ttgagtcttt tagtttagtc ctgcctataa aatgagtagg ataagtgtta tcccaggttc 240
ataggtatgg agtctcatag atgaggctca gggacggggg tgcctcaccc aaggtcacac 300
tgccaggagc tcatttttcc tgtgatctgt gatagtttct tttgtcaacc tttttcttct 360
tctccttcct tgctgcctga ttgtccccag ccatcccagc tcagtagctt ttcctgggat 420
aactgtgatg aagggaagga ccctgcggtg atcagaagcc tgactctgga gcctgacccc 480
atcgtcgttc ctggaaatgt gaccctcagt gtcgtgggca gcaccagtgt ccccctgagt 540
tctcctctga aggtgagcct gggggtgggt ggagaagggg aggtgcgagg gtctggccag 600
caggggtact ggggcatgta tgcttgggga actgtgaaga atttcagaat cctggattcc 660
cagagaatag tacaggacat gtagattcag acactctttc acaggttcat ggaatctcag 720
gatcataaga ttgaaaggaa tctctgatgt cagcgccagc aacttcctgg tgagggcagg 780
agtgacggat accttgcacc tggcagaagc gtcctggcct tctctgggcc tggtggccaa 840
ctgctcatta ttatctgaca gctctggttg gccaatttgg ttttgctgtt aattataaaa 900
ttgatatacc aattagccag taatatatag tcactttaga aaacacaagt ggtcaaaaaa 960
taaataaaat aggccaagtg tggtaacttc atgcctgtaa ttcccacacc cttaggaggc 1020
tgaaggtggg tgggatcctt tttgagg 1047

<210> 40
<211> 1705
<212> ADN
<213> Homo sapiens

<400> 40
acagtagatg ccagtgattt caatgcaagt gttagagcca atcaatgggt agtgactacc 60
taaagaattt taagactatg gattgagcat gatggctcac ggcctgtaat cccagccttt 120
ggaaggtgaa ggtgaaagga ttgcttgagg ccaggagttc cagaccagct tgggcaacaa 180
agtgagcccc atctctacaa aaaatacaaa attagctggg tgtggtggca tgtgcctgtc 240
tgtgtttccc acctacatgg gaggctgagg caggaggatc gtctgagccc aggagtttga 300
ggctgcagtg agtgcagtga gccatgatac aaaaaaaaaa aataaagaat tctaagtcta 360
tgtatagttc agtgtagggg gaaaattcac atttgattat taatgtctgc catgggcaca 420
ataatacact atactcacac atgggccaca atgttgccat tcctagaaca gactatctct 480
aagatctcat ccagttaaaa attctatgat taaaatatat tgctgctttt ttgaagacag 540
aagagctggt atgtttgccc tggaatttac acttataacc tttttcaaac ctttgtttta 600
ttttttttta ccaggtggat ttagttttgg agaaggaggt ggctggcctc tggatcaaga 660
tcccatgcac agactacatt ggcagctgta cctttgaaca cttctgtgat gtgcttgaca 720
tgttaattcc tactggggag ccctgcccag agcccctgcg tacctatggg cttccttgcc 780
actgtccctt caaagaagta agtacttagg gaggagagag cgttacccct gtggctaaag 840
agatggggtt tggagagaag ggtctttgca ttctccttct gcagatctgc atgtctctgg 900
atttgtaagc cagtgtgacc tatcaggaat cacttatctt ccgggagcct cagttatcca 960
tctacgaaat gggagacttg aacttagatg tgatcttcag ggccctttat ccatataatc 1020
catgctctac agtgctatgg ccgtctctca tcttgtgcgg ctgttttgag aatggaagaa 1080
ggggtggtag ttcatggctg caatcctagc agtggctcta ggagaaagac cccatcagta 1140

ggctcccact gactggcggt ccactggctt tcccgcaggg aacctactca ctgcccaaga 1200
gcgaattcgt tgtgcctgac ctggagctgc ccagttggct caccaccggg aactaccgca 1260
tagagagcgt cctgagcagc agtgggaagc gtctgggctg catcaagatc gctgcctctc 1320
taaagggcat atagcatggc atctgccaca gcagaatgga gcggtgtgag gaaggtccct 1380
tttcctctgt tttgtgtttg ccaaggccaa actcccactc tctgcccccc tttaatcccc 1440
tttctacagt gagtccacta ccctcactga aaatcatttt gtaccactta cattttaggc 1500
tggggcaagc agccctgacc taagggagaa tgagttggac agttcttgat agcccagggc 1560
atctgctggg ctgaccacgt tactcatccc cgttaacatt ctctctaaag agcctcgttc 1620
atttccaaag cagttaagga atgggaacag agtgttttag gacctgaaga atctttatga 1680
ctctctctct ttctctcttt ttttt 1705

<210> 41
<211> 1043
<212> ADN
<213> Homo sapiens

<400> 41
tttctttgcg taaccaatac tggaaggcat ttaaaggacc tctgccgcct cagaccttgc 60
agttaactcc gccctgaccc acccttcccg atgcagtccc tgatgcaggc tcccctcctg 120
atcgccctgg gcttgcttct cgcgacccct gcgcaagccc acctgaaaaa gccatcccag 180
ctcagtagct ttcctgggga taactgtgat gaagggaagg accctgcggt gatcagaagc 240
ctgactctgg agcctgaccc catcgtcgtt cctggaaatg tgaccctcag tgtcgtgggc 300
agcaccagtg tcccccctgag ttctcctctg aaggtggatt tagttttgga gaaggaggtg 360
gctggcctct ggatcaagat cccatgcaca gactacattg gcagctgtac ctttgaacac 420
ttctgtgatg tgcttgacat gttaattcct actggggagc cctgcccaga gcccctgcgt 480
acctatgggc ttccttgcca ctgtcccttc aaagaaggaa cctactcact gcccaagagc 540
gaattcgttg tgcctgacct ggagctgccc agttggctca ccaccgggaa ctaccgcata 600
gagagcgtcc tgagcagcag tgggaagcgt ctgggctgca tcaagatcgc tgcctctcta 660
aagggcatat agcatggcat ctgccacagc agaatggagc ggtgtgagga aggtcccttt 720
tcctctgttt tgtgtttgcc aaggccaaac tcccactctc tgcccccctt taatcccctt 780
tctacagtga gtccactacc ctcactgaaa atcattttgt accacttaca ttttaggctg 840
gggcaagcag ccctgaccta agggagaatg agttggacag ttcttgatag cccagggcat 900
ctgctgggct gaccacgtta ctcatccccg ttaacattct ctctaaagag cctcgttcat 960
ttccaaagca gttaaggaat gggaacagag tgttttagga cctgaagaat ctttatgact 1020
ctctctcttt ctctcttttt ttt 1043

<210> 42
<211> 342
<212> ADN
<213> Homo sapiens

<400> 42
atgacntgya aratgwsnca rytngarmgn aayathgara cnathathaa yacnttycay 60
cartaywsng tnaarytngg ncayccngay acnytnaayc arggngartt yaargarytn 120
gtnmgnaarg ayytncaraa yttyytnaar aargaraaya araaygaraa rgtnathgar 180
cayathatgg argayytnga yacnaaygcn gayaarcary tnwsnttyga rgarttyath 240
atgytnatgg cnmgnytnac ntgggcnwsn caygaraara tgcaygargg ngaygarggn 300
ccnggncayc aycayaarcc nggnytnggn garggnacnc cn 342

<210> 43
<211> 4195
<212> ADN
<213> Homo sapiens

<400> 43

ttccaccttt tggctcttgt aaataatgct gctatgaaca tgaatgtaca aacatctgtt 60
tgaatccctg cattcaattc ttttgcatat atacccagga gcagaatgat ggatcatatg 120
gtaattctgt gtttatttat ttgaggaaca aacttgccgt tttccataac agctgcacta 180
ttttacattc ccactaacag tgcattaggc ttccaattct ctatgccctc accaacactt 240
gttttctggg ttttaaaaga agtagtagtc atccttgtag gtgtcaggtg gtatctcatt 300
gtcgttttgc ttcatgtttt cctaaagatt agtaattttc atatgcttat tgaccatttg 360
tatatcttct tcggagaagt gtctatttga gtctttcccc aattttgatt ggtttgtttg 420
tttttttgttg ttgagttgta gggattcttt tatattctgg atattaatcc cttatcagat 480
atttgtttta caaatatttt ctttgtaaca acagaaacac accacagtct tcaaggttgg 540
aagccagtta atctgagtag cattttgtta gtggtgggga gaggatttgt tcctcctgaa 600
atcctgggga attggccacc tcctcttctc ctcttaggca tgaagcgcgt ctggcttctc 660
caaagaactc ttcccctcca ctacctcaga gttagcttcc tctcttcagc cagtgatcct 720
ggggtcccag acacaataat taaccaagag agggtgaaag gctccctgct gtgtttatgc 780
aatggctcag gcccttgtga agtgccgagg gaccccaagc agcctccatc tcccagggca 840
tggtccatcc ccagctttca cagaacagga aagctgtgga ggagtgtggg cagcagggta 900
ggaatggata tagcccttgg caacaacaca tttccccaca aagcacccac ccaaaagaac 960
aacaacgata gttttagttt ttagtaatga gaacaatagt tctcatgact aaaagccatc 1020
agccaggaca ctgttctcaa cccttttgcg gtctttggac cctttgaaac tctgacagaa 1080
gccatggagg aatgttctca ctgagtgcat gcactcaaaa tgatgcattc aacttcaatt 1140
cagtttcagg gatgtatggc ctgaccacca atgcagggga ttagcaatcg caatagtgga 1200
gagggcatgg gagtgggaat ctggctggat caagcaagtg gatgccagca gcccagaaaa 1260
agagcccccc tacctgcttt ttccttcctg ggcactattg cccagcaaat gccttcctct 1320
ttccgcttct cctacctccc cacccaaaat tttcattctg cacagtgatt gccacattca 1380
ctggttgaga aacagagact gtagcaactc tggcagggag aagctgtctc tgatggcctg 1440
aagctgtggg cagctggcca agcctaaccg ctataaaaag gagctgcctc tcagccctgc 1500
atgtctcttg tcagctgtct ttcagaagac ctggtaagtg ggactgtctg ggttggcccc 1560
gcactttggg cttctcttgg ggagggtcag ggaagtggag cagccttcct gagagaggag 1620
agagaaagct cagggaggtc tggagcaaag atactcctgg aggtggggag tgaggcaggg 1680
ataaggaagg agagtatcct ccagcacctt ccagtgggta agggcacatt gtctcctagg 1740
ctggactttt cttgagcaga gggtggggtg gtaaggaaag tctacgggcc cccgtgtgtg 1800
tgcacatgtc tctgtgtgaa tggacccttc cccttcccac acgtgtatcc ctatcatccc 1860
acccttccca ccagaggcca tagccatctg ctggtttggt tatttgagag tgcaggccag 1920
gacaaggcca tcgcttgggg catgaatcct ctgcgtactg ccctggccag atgcaaattc 1980
cctgccatgg gattccccag aaggttctgt ttttcaggtg gggcaagttc cgtgggcatc 2040
atgttgaccg agctggagaa agccttgaac tctatcatcg acgtctacca caagtactcc 2100
ctgataaagg ggaatttcca tgccgtctac agggatgacc tgaagaaatt gctagagacc 2160
gagtgtcctc agtatatcag ggtgaggagg ggctgggtgt ggcgggggct ctctgcctgg 2220
tcctggggct gccctgggcc agcggtcctc cctgccaccc ttcatagatg ctatgcctcg 2280
gctctctctg agatctttaa actctggctt cttcctcctc aatcttgaca gaaaaagggt 2340
gcagacgtct ggttcaaaga gttggatatc aacactgatg gtgcagttaa cttccaggag 2400
ttcctcattc tggtgataaa gatgggcgtg gcagcccaca aaaaaagcca tgaagaaagc 2460
cacaaagagt agctgagtta ctgggcccag aggctgggcc cctggacatg tacctgcaga 2520
ataataaagt catcaatacc tcatgcctct ctcttatgct tttgtggaat gaggttcctc 2580
ggtgtggagg gagggttgga aaacccaaag gaagaaaaag aaatctatgt tatcccaccc 2640
tacctctcac aagcctttcc tgctttaccc ctcacctggc ctctgcccca cattccttca 2700
gcccctcatt tcgagcattg gatttgaggc ttaaggattc aaaaagtcgt catgaatata 2760
gctgatgatt ttatagtggt tctgaaatgg gtcggggatt tgggaacagg gtggtagtat 2820
aagaacaact gatactgttc tctaagctaa atcttagctt ccagctacct gtcttagatg 2880
tggctcttgg gaaccttaga gtgatagcta catagaagtg tgtgggtgtg tgtgtgtgtg 2940
tctgtgtgtg tgtgtgtgag agagagacag acagaaagag agcaagagag ggaagggggg 3000
agaggctgat tgtgtgtgtg gtgtgatgta ggtggacaat gttcagagtc ctccattaac 3060
aggataatcc tcacacctgt ccacatacct gtagtttgtc cttggggatt ttgaaaattt 3120
ttcctccctc tccactccca aactcccaac tcaattaaat gataaaggaa taggcaaata 3180
ggaaaataaa ttagtaaaac ttaagtcaaa gaataggtta ttcatacgct gcctatggga 3240
ttctatgctt tgtgatcaga aaattatcta aaaaatactt cccaagggct ggtacaaggg 3300
aggccagaag acgagtggtt cttctctgag gtggacatta aaaaaagaag aaaatgaagg 3360
ggaacctttt gacaagaatg tcaccccaaa ctggatttcc atgctgtggt gtggggaatt 3420
ttctgttgtc ctcacttagg tgctggggca gtggtgttag tgatgggtaa aaaggtagga 3480

agctgtcaca gaatcactaa accagggttc ttaacttgtc tgtctataca tctctgaaat 3540
tgggttgaag ttgtgtgcat cattttgagt gacgcactga gaacattcct ccacggcttc 3600
catcgagagt ctcgaaaagg cccaacacct caaaaaggtt aagaacactt gtcctgctta 3660
ctggttttta gtaacaaatg gcagagtatt tctctctgtc tctctctctt tttttttttt 3720
tttttttgag acacagggtc ttgtctgtca cgtggactag agtacaatgg gcatgatcat 3780
gggctcactg tagcctcgaa cacctgggct caagtaatcc tcccacctca gcctctttag 3840
tagctgggac tacagcatga gccactgccc ttggctaatt tttaaattat ttttttgtag 3900
agatggaaac ttgctatgtt gcccaggcta gtctcaaact cctggactca agcgatcctc 3960
ctaccttggc ctcccaaagt gctgagatta cagtgtgatc cacaccacac ctggccaaag 4020
attggagtat ttttattgct attgttgtgc tgggtgggtg ggtgggtgta tgctttgtgg 4080
ggacgtgtgt tgttgccaag ggctaaatca gttcctaccc tgctgcccac agtcctccac 4140
agctttcctg ctctgtgaag ctaaggatac accccgatga taagctgtca acata 4195

<210> 44
<211> 477
<212> ADN
<213> Homo sapiens

<400> 44
tttttttttt tttttttgg ataaagactt atttattatt tatcttatca tttcccagaa 60
caaaggccat tgagtaagcc attcccttta aacttggttg ggcagctgtc acatggctga 120
cctcttaatt acttcccaca gcctttgcca tgactgtggc catgcccacg tgggttgttc 180
tcatgcagct tctcatgaca ggcaaagatc aactttgcca tcagcatcat acactcctca 240
aagctcagct gattgtcctg gtttgtgtcc aggtcctcca tgatgtcatt tatgagggct 300
tcatttctct tctctttctt cataaaaggt tgccaaactg tgcttcccac catttggtct 360
gaattccttc ttgctcaggg tgtaggggng ggtcttcctt cttaaagtat tgatgaaagg 420
gggccagatg gggggttat gctgcgctcc atctgaaaag tggctttggt gggccat 477

<210> 45
<211> 406
<212> ADN
<213> Homo sapiens

<400> 45
   tttttttttt tttttttttt ttttggagga agagacttta tttggcccca gcccctagcc 60
   ccacagccaa gacagtttga cataacaggc cccggggccc tggttgggta gaggcagggt 120
   ggcctggcct cctgattagt ggctgtggcc gtggccacca tgactgtggc cgtggccggg 180
   gccactgtga tcttggccac tgtggtctta gggggtgccc tccccgaggc ctggcttatg 240
   gtggtggcca gggccctcgt caccctcgtg cattttttcg tgggaggccc aggttagcct 300
   cgccatcagc atgatgaact cctggagctc agctgcttgt ctgcatttgg gtccaggtcc 360
   tccatgatgt gttctatgac cttttcattc ttattctcct tcttga 406

<210> 46
<211> 425
<212> ADN
<213> Homo sapiens

<400> 46
ggaggaagag actttatttg gccccagccc ctatccccac agccaagaca gtttgacata 60
acaggccccg gggccctggt tgggtaaagg cagggtggcc tggcctcctg attagtggct 120
gtggccgtgg ccaccatgac tgtggccgtg gccgtggcca ctgtgatctt ggccactgtg 180
gtcttagggg gtgccctccc cgaggcctgg cttatggtgg tggccagggc cctcgtcacc 240
ctcgtgcatc ttctcgtggg aggcccaggt tagcctcgcc atcagcatga tgaactcctc 300
gaagctcagc tgcttgtctg catttgtgtc caggtcctcc atgatgtgtt ctatgacctt 360
ttcattctta ttctccttct tgagaaaatt ttgcagatct tttcgcacca gctcttngaa 420

ttccc 425

<210> 47
<211> 565
<212> ADN
<213> Homo sapiens

<400> 47
   aattcgctcg gctttgacag agtgcaagac gatgacttgc aaaatgtcgc agctggaacg 60
   caacatagag accatcatca acaccttcca ccaatactct gtgaagctgg ggcacccaga 120
   caccctgaac cagggggaat tcaaagagct ggtgcgaaaa gatctgcaaa attttctcaa 180
   gaaggagaat aagaatgaaa aggtcataga acacatcatg gaggacctgg acacaaatgc 240
   agacaagcag ctgagcttcg aggagttcat catgctgatg gcgaggctaa cctgggcctc 300
   ccacgagaag atgcacgagg gtgacgaggg ccctggccac caccataagc caggcctcgg 360
   ggagggcacc ccctaagacc acagtggcca agatcacagt ggccacggcc atggccacag 420
   tcatggtggc cacggccaca ggccactaat caggaggcca ggccaccctg cctctaccca 480
   accagggccc cggggcctgt tatgtcaaac tgtcttggct gtggggctag gggctggggc 540
   caaataaagt ctcttcctcc aagct 565

<210> 48
<211> 430
<212> ADN
<213> Homo sapiens

<400> 48
gacttggagg aagagacttt atttggcccc agcccctagc cccacagcca agacagtttg 60
acataacagg ccccggggcc ctggttgggt agaggcaggg tggcctggcc tcctgattag 120
tggctgtggc cgtggccacc atgactgtgg ccgtggccgt ggccactgtg atcttggcca 180
ctgtggtctt agggggtgcc ctccccgagg cctggcttat ggtggtggcc agggccctcg 240
tcaccctcgt gcatcttctc gtgggaggcc caggttagcc tcgccatcag catgatgaac 300
tcctcgaagc tcagctgctt gtctgcattt gtgtccaggt cctccatgat gtgttctatg 360
accttttcat tcttattctc cttcttgaga aaattttgca gatcttttcg caccagctct 420
ttgaattccc 430

<210> 49
<211> 305
<212> ADN
<213> Homo sapiens

<400> 49
tgacttggag gaaaaaactt tatttggccc cagcccctag ccccacagcc aaaacagttt 60
gacataacag gccccggggc cctggttggg tagaggcagg ggggcctggc ctcctgatta 120
gtggctgtgg ccggggccac catgactgtg gccggggccg gggccactgt gatcttgcca 180
ctggggtctt agggggtgcc ctccccgagg cctggtttat ggtggtggcc agggcccttg 240
tcacccttgt gcatttttc gtgggaggcc caggttagcc tcgccatcag catgatgaac 300
tcctc 305

<210> 50
<211> 452
<212> ADN
<213> Homo sapiens

<400> 50
ggaggaagag actttatttg gccccagccc ctagccccac agccaagaca gtttgacata 60

acaggccccg gggccctggt tgggtagagg cagggtggcc tggcctcctg attagtggct 120
gtggccgtgg ccaccatgac tgtggccgtg gccgtggcca ctgtgatctt ggccactgtg 180
gtcttagggg gtgccctccc cgaggcctgg cttatggtgg tggccagggc cctcgtcacc 240
ctcgtgcatt ttctcgtggg aggcccaggt tagcctcgcc atcagcatga tgaactcctc 300
gaagctcagc tgcttgtctg catttgtgtc caggtcctcc atgatgtgtt ctatgacctt 360
ttcattctta ttctccttct tgagaaaatt ttgcagatct tttcgcacca gctctttgaa 420
ttccccctgg ttcagggtgt ctgggtgccc ca 452

<210> 51
<211> 4439
<212> ADN
<213> Homo sapiens

<400> 51
atcactgtgg agtaggggaa gggcactcct ggggtggcaa ggtgggaggt gggccctgtg 60
ttcccacagt gggcagggag gtagtgaaag ggaagctggc cggacaggaa gggccattcc 120
aagagggctt tgtgcgcagg gctaagccaa gctttctcca taggcaatgg ggagcaactg 180
gaggttcgta gcaggagaag gacacatcaa gcccaccagg aggctaagta aaaacagttg 240
tctcccaagt tataagttcc tggaaccctt gctgggagca ggatttagaa aaatgatgct 300
gagagatgct agaaacatat tcgccctgag gctctctcac tcagactgca agaggaaggt 360
atcatcagaa ttgcccttaa ccaggaacca gaatagctgg gtccccttcc tgccaagtca 420
gcaaccagct atgtgacctt gctcaggtcc atctccgggt gtcagtttct tcatctacaa 480
tgcaagaggg ttgcccacct ctgagaaccc ttctaacccc aaatctcacc ctatgaatct 540
aagaacacaa cccctcgcca tcctaagtat cacagagcca ggcaagcatg ggtgagagct 600
cagaccatcc ttgttggact aaaaggaagg ggcagactgc catgggggc agccgagagg 660
gtcaggcccc cataggtcct cagcctgctt caacctcaaa ggggatgggg ggctgagtgg 720
tgccagagga gcagcaggct cgctcgggga gagtagggcc ttaggataga agggaaatga 780
actaaacaac cagcttcctg caaaccagtt tcaggccagg gctgggaatt tcacaaaaaa 840
gcagaaggcg ctctgtgaac atttcctgcc ccgccccagc cccttcctg gcagcattag 900
cacactgctc acctgtgaag caatcttccg gagacagggc caaagggcaa gtgccccagt 960
caggagctgc ctataaatgc cgagcctgca cagctctggc aaacactctg tgtggctcct 1020
cggctttggt aagtgagctg ccagcttccc caggcagaag cctgcctgcc gattccttct 1080
ttccttccct gacccaactt ccttccaaat cctcctccta gaagccctcc ttggttggcc 1140
ctgcctactt taaagcttct ttcacatttt cttaggtcat gttcccctgg ggcctcctgc 1200
cctcaaatgc tttgcttttt ggcactctgt agatattcta aaaaatcatt ttgtacatgt 1260
gtgtgacagg ccatctccca gttaagttgc agcctgtgct ttctttttat tttgcacttc 1320
ccccactatt tctgtgagtg cttagtagga agtgtcaaag aagcttgaca gcattttctt 1380
ctaagtgtcc caactcttgg ttttccatta cacagacaga gtgcaagacg atgacttgca 1440
aaatgtcgca gctggaacgc aacatagaga ccatcatcaa caccttccac caatactctg 1500
tgaagctggg gcacccagac accctgaacc aggggaatt caaagagctg gtgcgaaaag 1560
atctgcaaaa ttttctcaag gtagggctgg actctggcag gtctgaccca gcctcaccgc 1620
agtttgggtt gacaagggag gatgggagta tgggctacag caatcaaggg gaagatttga 1680
gctcctggag cccagcccca agacgcagcg agtgtcctgt tatacagggc aggtgctcac 1740
agttacacag gacgacaggg tcaagaaatt gctcaattga acacctgcta tttgtcgggc 1800
cctgttctgg gcagagggat gtagtggtaa atgggagccc actattccat gaggagacac 1860
acagtaaagt tgttggccaa taaagagcac agataaagcc aaatgccaat aagtgcctgg 1920
aagaaaatga gatagagtgc gctgtgggca atggggctgg gtggggtgga ggtgaccagt 1980
tagggtacat gagaagggcc tctttgagga ggtaacattt gagctgagcc ccgaatgttg 2040
gggagggaag cccctgagga tgacacttgg cacaaagctg aggagaccct aagcctcagg 2100
gcgaacttgg ggtggaagac ttgggggctt ttctaatcct aagggtctgc ggtggaaaat 2160
gaatgcataa agagcacatg gagagcacct gcacagcact cagggaactg ggaggttttt 2220
cccccgctcc aaaaatgatt aggcagttct aagaaaaagg ctgagcactt ccaacagcct 2280
ttttgttttc ttttcaaatt tggggaaagt cgggaaacag aggcctgcat taagaagggt 2340
ggaacacatg ggtctcagtc tcagttccag tcccggagcc agacatcctg gggtaggtcc 2400
ccagccctcc cagtgcccct ccctccgcct tggtaaggtg gagaattgca gccttcagag 2460
ttaggggccc tgacagctct ccataggtgg aggcctcagg caggcaggat gctgggtggg 2520
gtaggcaaga aagggcccag cagagaggcc gcatcggaaa actatcctcc atgtgacccc 2580

ctatgcccgc ttcacccccc acctgacatc ccccaccaga agcaaagcga tgctgtggga 2640
aaggaagcag agcctcatgg atgggctgca caggagagtg ctcgcattgg ctgggtaccc 2700
cacaggttct gggaggggac ttagcgaggt gactcagtgc ctcggcctcc caaagtgctg 2760
ggattacaag catgagccac cctgtccgac catctcccct tttatacttt atcacaccct 2820
tgaggtcagc ggagcacata ctctgctctc tgaccctcca tctcccctgc ccacacctag 2880
gtttttctag tgtttccccg ttgtattggt tgaaataagt ttcactaatt ggtaacctcc 2940
agagggaagg gaagggaggg caggggaagg agtgaagtgc agaggggtag cagagtggaa 3000
ctggcctcta agtcagatct gaatttgcat gccctcaata gtcaagcctg tgaaaactaa 3060
tgaccctctc taggactggt ttcaagtctt cctccaggaa gataccattc ctagctgtta 3120
aagttgttat aaggaccaaa tgaggtgaca tttccaggct tactcatgcc atgaccaggg 3180
caagaccctg gaactcagct tcctcttcta taaatagaga atcagcaccc aagtcacagg 3240
gtcatggagg gaataaactg gagagcgttt ggtatgtgct cagtgtctgc tccattgtgc 3300
gcactcagcc tatggtcatt tttaattttt aaatccagcc ccagggtcga ggcttccttg 3360
tacatttgcc agctggtcat ttactgtgct cccagtcccc acctctggcc acacccagct 3420
ctcacagcct tctctcccca cccgcagaag gagaataaga atgaaaaggt catagaacac 3480
atcatggagg acctggacac aaatgcagac aagcagctga gcttcgagga gttcatcatg 3540
ctgatggcga ggctaacctg ggcctcccac gagaagatgc acgagggtga cgagggccct 3600
ggccaccacc ataagccagg cctcggggag ggcacccct aagaccacag tggccaagat 3660
cacagtggcc acggccacgg ccacagtcat ggtggccacg gccacaggcc actaatcagg 3720
aggccaggcc accctgcctc tacccaacca gggccccggg gctgttatgt caaactgtct 3780
tggctgtggg gctaggggct ggggcaaata agtctcttcc tccaagtcag tgctctgtgt 3840
gcttcttcca cctcttctcc aaccctgcct tcccagggct ctggcattta gacagccctg 3900
tccttatctg tgactcagcc ccctcattca gtattaacaa aatgagaagc agcaaaacat 3960
gggtctgtgc tgggcccctt ggctcacctc cctgaccatg tcctcacctc tgacttcagg 4020
ccccactgtt cagatcccag gctccctgcc ccatctcaga caccctgtcc agcctgtcca 4080
gcctgacaaa tggcccttgt cactgtacac tgtagaaagc aaaaaggcat atctctaccc 4140
cttgatatgc ctgctacctc accaaccagc cccaagcctg tcttcaccca tcactgtcta 4200
cacagccctc tctctctcct aacagaattc tattcctctg aaagtcttca gaaactggac 4260
ctagatagtg ccatgtctgg ggaggaatat ggcaccaggc agtggaaaca aggacagatc 4320
ggtgtgttat ctcacatttg atcagagagc atgatctctc ttaacagacc tgccacccta 4380
atcaacggga gtgctcacac aagtgggagt ctgagagctt agccctatgc ccaccctgg 4439

<210> 52
<211> 565
<212> ADN
<213> Homo sapiens

<400> 52
   aattcgctcg gctttgacag agtgcaagac gatgacttgc aaaatgtcgc agctggaacg 60
   caacatagag accatcatca acaccttcca ccaatactct gtgaagctgg ggcacccaga 120
   caccctgaac cagggggaat tcaaagagct ggtgcgaaaa gatctgcaaa attttctcaa 180
   gaaggagaat aagaatgaaa aggtcataga acacatcatg gaggacctgg acacaaatgc 240
   agacaagcag ctgagcttcg aggagttcat catgctgatg gcgaggctaa cctgggcctc 300
   ccacgagaag atgcacgagg gtgacgaggg ccctggccac caccataagc caggcctcgg 360
   ggagggcacc ccctaagacc acagtggcca agatcacagt ggccacggcc atggccacag 420
   tcatggtggc cacggccaca ggccactaat caggaggcca ggccaccctg cctctaccca 480
   accagggccc cggggcctgt tatgtcaaac tgtcttggct gtggggctag gggctggggc 540
   caaataaagt ctcttcctcc aagct 565

<210> 53
<211> 255
<212> ADN
<213> Homo sapiens

<400> 53
gayaayggng aygtntgyca rgaytgyath caratggtna cngayathca racngcngtn 60

mgnacnaayw snacnttygt ncargcnytn gtngarcayg tnaargarga rtgygaymgn 120
ytnggnccng gnatggcnga yathtgyaar aaytayathw sncartayws ngarathgcn 180
athcaratga tgatgcayat gcargaycar carccnaarg arathtgygc nytngtnggn 240
ttytgygayg argtn 255

<210> 54
<211> 2724
<212> ADN
<213> Homo sapiens

<400> 54
cgcgctatgt acgccctctt cctcctggcc agcctcctgg gcgcggctct agccggcccg 60
gtccttggac tgaaagaatg caccaggggc tcggcagtgt ggtgccagaa tgtgaagacg 120
gcgtccgact gcggggcagt gaagcactgc ctgcagaccg tttggaacaa gccaacagtg 180
aaatcccttc cctgcgacat atgcaaagac gttgtcaccg cagctggtga tatgctgaag 240
gacaatgcca ctgaggagga gatccttgtt tacttggaga agacctgtga ctggcttccg 300
aaaccgaaca tgtctgcttc atgcaaggag atagtggact cctacctccc tgtcatcctg 360
gacatcatta aaggagaaat gagccgtcct ggggaggtgt gctctgctct caacctctgc 420
gagtctctcc agaagcacct agcagagctg aatcaccaga agcagctgga gtccaataag 480
atcccagagc tggacatgac tgaggtggtg gcccccttca tggccaacat ccctctcctc 540
ctctaccctc aggacggccc ccgcagcaag ccccagccaa aggataatgg ggacgtttgc 600
caggactgca ttcagatggt gactgacatc cagactgctg tacggaccaa ctccaccttt 660
gtccaggcct tggtggaaca tgtcaaggag gagtgtgacc gcctgggccc tggcatggcc 720
gacatatgca agaactatat cagccagtat tctgaaattg ctatccagat gatgatgcac 780
atgcaaccca aggagatctg tgcgctggtt gggttctgtg atgaggtgaa agagatgccc 840
atgcagactc tggtccccgc caaagtggcc tccaagaatg tcatccctgc cctggaactg 900
gtggagccca ttaagaagca cgaggtccca gcaaagtctg atgtttactg tgaggtgtgt 960
gaattcctgg tgaaggaggt gaccaagctg attgacaaca acaagactga gaaagaaata 1020
ctcgacgctt ttgacaaaat gtgctcgaag ctgccgaagt ccctgtcgga agagtgccag 1080
gaggtggtgg acacgtacgg cagctccatc ctgtccatcc tgctggagga ggtcagccct 1140
gagctggtgt gcagcatgct gcacctctgc tctggcacgc ggctgcctgc actgaccgtt 1200
cacgtgactc agccaaagga cggtggcttc tgcgaagtgt gcaagaagct ggtgggttat 1260
ttggatcgca acctggagaa aaacagcacc aagcaggaga tcctggctgc tcttgagaaa 1320
ggctgcagct tcctgccaga cccttaccag aagcagtgtg atcagtttgt ggcagagtac 1380
gagcccgtgc tgatcgagat cctggtggag gtgatggatc cttccttcgt gtgcttgaaa 1440
attggagcct gcccctcggc ccataagccc ttgttgggaa ctgagaagtg tatatggggc 1500
ccaagctact ggtgccagaa cacagagaca gcagcccagt gcaatgctgt cgagcattgc 1560
aaacgccatg tgtggaacta ggaggaggaa tattccatct tggcagaaac cacagcattg 1620
gtttttttct acttgtgtgt ctgggggaat gaacgcacag atctgtttga ctttgttata 1680
aaaatagggc tcccccacct cccccatttc tgtgtccttt attgtagcat tgctgtctgc 1740
aagggagccc ctagcccctg gcagacatag ctgcttcagt gccccttttc tctctgctag 1800
atggatgttg atgcactgga ggtcttttag cctgcccttg catggcgcct gctggaggag 1860
gagagagctc tgctggcatg agccacagtt tcttgactgg aggccatcaa ccctcttggt 1920
tgaggccttg ttctgagccc tgacatgtgc ttgggcactg gtgggcctgg gcttctgagg 1980
tggcctcctg ccctgatcag ggaccctccc cgctttcctg ggcctctcag ttgaaccaaa 2040
gcagcaaaac aaaggcagtt ttatatgaaa gattagaagc ctggaataat caggcttttt 2100
aaatgatgta attcccactg taatagcata gggattttgg aagcagctgc tggtggcttg 2160
ggacatcagt ggggccaagg gttctctgtc cctggttcaa ctgtgatttg gctttcccgt 2220
gtctttcctg gtgatgcctt gtttggggtt ctgtgggttt gggtgggaag agggcccatc 2280
tgcctgaatg taacctgcta gctctccgaa gccctgcggg cctggcttgt gtgagcgtgt 2340
ggacagtggt ggccgcgctg tgcctgctcg tgttgcctac atgtccctgg ctgttgaggc 2400
gctgcttcag cctgcacccc tccctttgtc tcatagatgc tccttttgac cttttcaaat 2460
aaatatggat ggcaagctcc taggcctctg cttcctggta gagggcggca tgccgaaggg 2520
tctgctgggt gtggattgga tgctggggtg tgggggttgg aagctgtctg tggcccactt 2580
gggcacccac gcttctgtcc acttctggtt gccaggagac agcaagcaaa gccagcagga 2640
catgaagttg ctattaaatt gacttcgtga tttttgtttt gcactaaagt ttctgtgatt 2700
taacaataaa attctgttag ccag 2724

<210> 55
<211> 2171
<212> ADN
<213> Homo sapiens

<400> 55
   cgcgctatgt acgccctctt cctcctggcc agcctcctgg gcgcggctct agccggcccg 60
   gtccttggac tgaaagaatg caccaggggc tcggcagtgt ggtgccagaa tgtgaagacg 120
   gcgtccgact gcggggcagt gaagcactgc ctgcagaccg tttggaacaa gccaacagtg 180
   aaatcccttc cctgcgacat atgcaaagac gttgtcaccg cagctggtga tatgctgaag 240
   gacaatgcca ctgaggagga gatccttgtt tacttggaga agacctgtga ctggcttccg 300
   aaaccgaaca tgtctgcttc atgcaaggag atagtggact cctacctccc tgtcatcctg 360
   gacatcatta aaggagaaat gagccgtcct ggggaggtgt gctctgctct caacctctgc 420
   gagtctctcc agaagcacct agcagagctg aatcaccaga agcagctgga gtccaataag 480
   atcccagagc tggacatgac tgaggtggtg gccccattca tggccaacat ccctctcctc 540
   ctctaccctc aggacggccc ccgcagcaag ccccagccaa aggataatgg ggacgtttgc 600
   caggactgca ttcagatggt gactgacatc cagactgctg tacggaccaa ctccaccttt 660
   gtccaggcct tggtggaaca tgtcaaggag gagtgtgacc gcctgggccc tggcatggcc 720
   gacatatgca agaactatat cagccagtat tctgaaattg ctatccagat gatgatgcac 780
   atgcaaccca aggagatctg tgcgctggtt gggttctgtg atgaggtgaa agagatgccc 840
   atgcagactc tggtccccgc caaagtggcc tccaagaatg tcatccctgc cctggaactg 900
   gtggagccca ttaagaagca cgaggtccca gcaaagtctg atgtttactg tgaggtgtgt 960
   gaattcctgg tgaaggaggt gaccaagctg attgacaaca acaagactga gaaagaaata 1020
   ctcgacgctt ttgacaaaat gtgctcgaag ctgccgaagt ccctgtcgga agagtgccag 1080
   gaggtggtgg acacgtacgg cagctccatc ctgtccatcc tgctggagga ggtcagccct 1140
   gagctggtgt gcagcatgct gcacctctgc tctggcacgc ggctgcctgc actgaccgtt 1200
   cacgtgactc agccaaagga cggtggcttc tgcgaagtgt gcaagaagct ggtgggttat 1260
   ttggatcgca acctggagaa aaacagcacc aagcaggaga tcctggctgc tcttgagaaa 1320
   ggctgcagct tcctgccaga cccttaccag aagcagtgtg atcagtttgt ggcagagtac 1380
   gagcccgtgc tgatcgagat cctggtggag gtgatggatc cttccttcgt gtgcttgaaa 1440
   attggagcct gcccctcggc ccataagccc ttgttgggaa ctgagaagtg tatatggggc 1500
   ccaagctact ggtgccagaa cacagagaca gcagcccagt gcaatgctgt cgagcattgc 1560
   aaacgccatg tgtggaacta ggaggaggaa tattccatct tggcagaaac cacagcattg 1620
   gtttttttct acttgtgtgt ctgggggaat gaacgcacag atctgtttga ctttgttata 1680
   aaaatagggc tcccccacct cccccatttc tgtgtccttt attgtagcat tgctgtctgc 1740
   aagggagccc ctagcccctg gcagacatag ctgcttcagt gccccttttc tctctgctag 1800
   atggatgttg atgcactgga ggtcttttag cctgcccttg catggcgcct gctggaggag 1860
   gagagagctc tgctggcatg agccacagtt tcttgactgg aggccatcaa ccctcttggt 1920
   tgaggccttg ttctgagccc tgacatgtgc ttgggcactg gtgggcctgg gcttctgagg 1980
   tggcctcctg ccctgatcag ggaccctccc cgctttcctg ggcctctcag ttgaaccaaa 2040
   gcagcaaaac aaaggcagtt ttatatgaaa gattagaagc ctggaataat caggcttttt 2100
   aaatgatgta attcccactg taatagcata gggattttgg aagcagctgc tggtggcttg 2160
   ggacatcagt g 2171

<210> 56
<211> 35465
<212> ADN
<213> Homo sapiens

<400> 56
gatcttggct cactgcaacc tccgcctcca aggttcaagc gatcctccca cctcagcctc 60
ccaagtagct gggattacaa gcgtgtgcta tcacacctgg ctaattttta tatttttggt 120
agagatgggg tttcaccttg ttggttaggc tggtcttgaa ctcctgacct caggtgatct 180
gcctgcctca gcctcccaaa gtgctgggat tacaggtgtg agccaccgcg cccagcctga 240
ccctttcttt ctctactggc aaaactcctg ctccttttta aagccaagct catgtcacct 300

cctctgtgaa gtcctcgctg actccccaag cggtcagtgt ctctctcgta tgggctcccc 360
ggcccctgca ctgctctcca tcacaccctg accactctgg gcagtggccc ccctccccac 420
ccactgacta tgggctcctt gaaggcaggg cctgggtctg ccccatctct gtgtccccag 480
caatgctggg catgagtcag cctcagaaga catctgctga atggctgcaa accagaggaa 540
atatctccag cctcaggctg ggacccctcc cctctctcct cccacctctg acttcatacc 600
actcaccctc cagagtcttc aatgcccact attacttcac acagttggcc tgtgacaggc 660
aatcaggtca tcgtccacgg ctaccaggtg tttcatgtct actgtgactt ccaggaccac 720
aagccctttt gcgcccacca tgtcttcacc taagagatct tcaaagccca gtatgtctct 780
ggcacccagt ggatcctcca tgcccactgc ggatcccaag cctcctgcct ccttgaagtc 840
caccaaatca gcaacaccca acagatcctt agtgcccacc aaaccagcga catcccgtaa 900
ctcagtcatg agcccaagca gttccaagtc caccaaatcg accagtacaa aaagagcccc 960
ttctaaccgg cccagcagca ggtcccgagt ccgcagcaaa gcaagaacac ccagcagggt 1020
gagcaccgac accaggacca gcaaagccag caaggccagc gacgtgagat gccaccagcg 1080
gaggggcaca cacagccggg gtaggacacc tggcagaagg ggaagccgca gctccaagag 1140
gtcacccagc agggccagca ctcctggcag gataagaact catggtgcca gaccaggcat 1200
ggccagcagg gtgagaactc ccacttcaca gcaaaaaggg agccggggaa agagttacgg 1260
ccggcctaga accagcaaca gggaaaggag tgacagccag cctagaaatc tgagcaagaa 1320
gagttaccgc ccaccaggag gctcaggtat agggaggagt tccgagctgg ctgtaactcc 1380
cagtacagcc aagtgtcaaa ccccgactgg aattccctcc aaggagaaga gtgacaaccc 1440
atctccatcc tcatcaagga aggtgaagag ctacggtcag atgatcatcc ccagtaggga 1500
aaagagttac agccccactg aaatgtccag cagggtcaag agttataacc aggccagcac 1560
ccgcagcagg ccgcaaagtc acagccaatc tagaagcccc agaaggtcaa gaagtggcag 1620
tcagaagagg acgcacagca gagtgagaag tcacagttgg aagagaaacc atagcagggc 1680
aagaagtcgc acccggaagg gaattctgag ccagatggga agacacagcc agtctagaag 1740
ccacagcaag gggaaaagtc aaaaccaatc tagaaccccc agaagaggaa gaagtcacaa 1800
ctggtctaga aaccccagca aggaaagaag tcatagccat tccagaagct ccagcaaaga 1860
gagagatcac aggggatcta gcagccccag gaaggagagt ggtcgcagtc aatcaggaag 1920
ccccaacaag cagagagatc acagccgatc tagaagtccc aacaaggcga gagatcgcag 1980
ccgatctaga agtccctaca aggcgagaga tcgcagccga tctagaagtc ccaacaaggc 2040
gagagattgc agccgatcta gaagtcccta caaggcgaga gatcgcagcc gatctagaag 2100
tcccaacaag gcaagagatc atagccgatc tagaagtccc aacaaggcga gagatcgcag 2160
ccgatctaga agccccagca aggaaagaga tcacagccaa cttggaagcc ccagcaaaga 2220
gagagatcac agacgatcta gaagccccag caaggagaga cagtgcagac aatctagaag 2280
ctccagcaaa gagagagatc acagacgatc tagaagcccc agcaaggaga gacagcgcag 2340
acaatctaga agccccaaca aggagagaga tcgcagccaa tctagaagcc ccagcgagga 2400
gagagagcac agacaatcca gaagccccag caaagagaga gatcgcagac gatggagaag 2460
ccccagcaag gagagagagc gcagacaatc tagaagctcc agcgaggaga gagatcacag 2520
ccgatctaga agccccaata agcagagtgg ttacagtcga cctagagcct ccagcaagga 2580
gaaagctcat agccgatcta gaacccccag caaagaagga aatcatagcc aatctagaac 2640
ctctagcaag gagagcgacc ccagtcaatc tacagtcccc agaagtcccg actggaagag 2700
atcccctact aggacaagca gtctcagtca gaatagaacc cctagcaaga caagcagcca 2760
ctccccatca acatttccca gtgggggcca aaccctaagc caggatgaca gtcaagccga 2820
cgccaccacc tctaaggcca ccttacctgg ggaaaggtct tcatcatctt cttccaagct 2880
ggcgtagccc ccagtctcag ctggctcacg ggtctctgtc atgaccgggg gaggggacag 2940
gagacaggag cagagcagca gctgagcagc gtccctcccc ggccagctct ccacagccac 3000
acctccggcc acaagttctc taatacagga tgttggcagg tagagaggga tgctggatag 3060
ggggaaagga aagacctgtg atgattcaat aaatttttac atagcaccca tccccaccaa 3120
gcccaactgt gtgctcactg ctggcatggg gcacagagga ccccagctct gtccctgact 3180
gtctacaggg tcttgactgc aagccctgcc cctctctagg tctttttttt ttttgagaca 3240
gagtctctct ctgttgccca ggctggagtg cagtggtgtg atctcagctc actgcaacct 3300
ccacctccca ggctcaagca attctcctac ctcagcttcc cgagtagctg gaactacaag 3360
tgtgcgtcct cacgcccggc taattttgta tttttagtag agatggggct tcaccatgtt 3420
ggccaggctg ggctcgaact cctgacctca ggtgatccac atgcctcaac ctcgcaaagt 3480
gctgggatta taggcatgag ccaccgcacc cgtccccctc tctaggtctt aatttccgca 3540
tgtgggcaac aaggctgcct tctggttctt attcagtggg gtagggagag gtgacactcc 3600
aaatattcaa cagtggggac tggtgtgggc accaatcaga actgagagtg gagcgggacg 3660
gataccaggc cttaaccctt tagttgctgg accatgggga ggtctggggt tggggaagtg 3720
ttatggggaa aaaaaaccct caaactgtgt ttttcctcta ctctcacact atcacaacaa 3780

tcatcaacac agaattctgt gaccaaatgt gtggggcttt ttccccacac actacacagc 3840
agacaacagc taggtgtccc ctccgattcc attccaacgc tgtccccaca cccagctaat 3900
ttttgtattt ttggaagaga cagggtttca ccatgttgcc cagagctcaa gcaatctgcc 3960
cacttcagcc ctccaaagtg ctgggattac aggcgtgagc caccacaccc gactttttta 4020
aaaaaataaa aataaggccg ggcgcagtga cccatgcctg taatcccagc actttgggag 4080
gccgaggtgg gcagatcacc tgagctcagg agtttgacac cagcctaggc aacatggcaa 4140
acttgtctct aaaaaaaaaa aaaaaattac aaaagttagc cggtgtggtg gcatgtgctt 4200
atagtcccag ctacctgaga ggctgaggca ggaggataaa ttgagcctgg aaggtcaagg 4260
ctgcagtgag ccgtgacctt gccactgcac tcaagcctgg atgacccatc ttacaaaaaa 4320
aaaatttttg ctggagctgc tcacagaact caaggaaatg cttacttaga tttactggtt 4380
tattatagag gatattgcaa agaacaaaga tgaagagatg tgtagggcaa ggtataaggg 4440
aaggggcagg gagcttcacg ccctccctgg ggtgctaccc tacaggaacc ctcaggtggt 4500
tagctatgcg gaagctctcc aaacccagtc ctcttgggtt tttacggagg ctttaagaca 4560
gcagcattgg gcatggactt ctctgaaaag tgtcttaaga ccaacaatca agaaggtggg 4620
gaagattaga gtcttgccct ggggcaggaa atggagggca ggaggaggtc agagagattc 4680
tgtttcttca gacctgcccc aggcctaagg tacacaacat tataacaaga gactgtaaca 4740
aaggctgtag gagttaccag ccaggaactg tggatgaaaa ccaatatatt tatatatata 4800
ataccacaag gggggtccaa agtggcagtt agggacaggg agtacttgtg tagcagtgac 4860
acaccaaccc atctggaagt attttaatat ttaaacaatt ggtatggcta tactagtttg 4920
tgattatcag ccttagttct gtatcaattg gcaagatagt gtctaggttt gccacactct 4980
agctgtgtag caccaagcaa agaacttaac ttctctagcc tgtttccttc tctggaagaa 5040
aggggcttcc aggccttaac tcacgtactc cccataacta gactgggaat tatctccttt 5100
gtacagatga ggaaacagac acagaggtga taagtgagta gcccaaggtc accatctggt 5160
aagtggatga actaggattg gaagccagac ctttcataaa atgatttctc agctcaaaag 5220
gtttttctga agattcagta ggctcactga tagaaattgc tggtgtgtgg ctggtattcc 5280
atcaagagtg gccattacta ctcccacccc tgcccctcta taaactccag atgttccaga 5340
cctctcatct ctccctgtgc acacaaggcc ttttcacatc tgtgggtctt agtacaccca 5400
ctgttgctgt caagaatgtc ctcctcctcc tttttttttt tttttttgag atggagtctc 5460
actttgttgc ccaggctgga gtacagtagc gcgatctcag ctcactgcaa cctctaccct 5520
gcatcagcct ccctagtagc tgggattaca ggcagccacc accaccatgc ccggctaatt 5580
ttttggtatt tttagtagag acagggtttc attatgtcag ccaggctggt ctcaaactcc 5640
tgacctcagg tgatccattt accttggcct cccagagtgc tgggattaca ggcaagagcc 5700
accacgccca gccctccttc cccctttttg gcctggagaa ctccttttca cccttcaaag 5760
cccaccacaa acataagaac ctctatactt cttgcccgct gaaatactgc ctctgccagg 5820
aagccttctg tgacttctct ctctccctct tcaccaacgg accgcccccg ccccccacca 5880
accccaccac acacacacac cactactgtc ttccactgta ctccctgaca gtagagaacc 5940
aagcagggcc agttgatgca gcctcagcta tatctcttac atgccaaggc ccatgcactg 6000
gggatacaat ggtggaaaat acatggtccc ttcaaagtct ggatgtcaag tttaatgctg 6060
gggactaaag agaaaagctt cagattgaaa cctggaggtg gctggggcaa aggaccattg 6120
gcatcattgg cagggcaact tcctaaagaa agcacctaaa tcttggcttt taaagacaga 6180
tttcataatt ggcagaggag aattctaatg ataccctatt gcctacaggg ccccatctaa 6240
tttgggaatt ctactttata ccaagataag attgccagat ttagcaaata aaaacagaag 6300
acatccaatt aatttttttg tttgtttttg ggtttttgtt gcggagatgg tgtctcacta 6360
tgttgcgaag gctgctgtca aattcctggc tcaaacaatc ctcctgcctt ggcctcccac 6420
ttcccaaagt gctgggatta caggcatgag ctaccacacc tggcccttat ttatttattt 6480
atttaatttt ctttttttggg acggagtgtc actctgtcgc ccaggttgga gcgcagtagc 6540
gcgatctcgg ctcactgcaa cctctgcctc ctgggttcaa gcgattatcc tgccccagcc 6600
tcccaagtag ctgggactac aggcgcgtgc caccatgccc ggcttttttt tttttttttt 6660
tttttttttt gagacggagt cttgctctgt cgcccaggct ggagtgcagt ggcacgatct 6720
cggctcactg caagctccgc ctcctgggtt cacgccattc tcctgcctca gccttccgag 6780
tagctgggac tacaggcgcc tgccaccacg cccgactatt ttttgtattt ttagtagaga 6840
tggggtttca ccgtgttagc caggatgatc tcgatctcct gacctcgtga tccacccgcc 6900
tcggcctccc aaagtgctgg gattacaggc gtgagccacc gcgcccagcc tacttattta 6960
tattttttaa gagacagggt ctcgctcagt tgcccaggct ggagtgcagt agggtgatct 7020
gtaggaaagg ggcttccagg ccttaactca tgtactcccc cataaccagg ttgggaggtt 7080
agctcactgt aacctcaaac tcctgtgctc aaggtaccct actagcccct aggagagcag 7140
ctgggactac aggtatgcgc caccatgcca ggcttaattt ttactttttt tttttttttt 7200
tttttttgta gagacggggg tctcactata ttgcccaggc tggtcttgaa ctcctggtct 7260

caagcgatcc tcctgcctta gcctcccaaa gtattggtat cactgcaact agcccaaaga 7320
attaatatag ctatgttcca tgtgatattt gggacatact tttctaaaag gttgtatctt 7380
ttggatataa ttgtttatct gaaattcaaa tttaactaga cattgtatat tttatacggc 7440
aaccacacac ctgggacaat caagacattc cctgaagtta ccaggagaca atgcccatca 7500
gcctacactt ttccaagccc acgtcacaca aggccccttc cagagtattc cagacgtcag 7560
gtagggccat cccttggttc acaagtccca ctcctaccac gcctatggca gccaaactga 7620
aaggcaaaca cagtgctgga gaccccacaa tgccctgggc ctatagcagt caattcccaa 7680
gatgccccgc gtgaacacaa taggcacccg ttccaatgct cgagcaaaga gaccaggcca 7740
aaaccttcca ctacggacaa ataacggcca gttcccacaa ttcgttgtgg cagttcttcc 7800
caggatgcct taggcctata gcgaccacct tcccagactc cccgtgtgga agcgctccaa 7860
gcctccagga cggtcagcgg caggtgtggg ataaaaggaa ccggtctcga caaggatctg 7920
ggacactctt tcccaggatg caccaggcct acgactagcg gaccgactcc cacagcgctt 7980
caaggcggag cgctcggttc tcccaggatg ccccagggcg gcacaaacgc gtagggggag 8040
aaaaagaagc cctcgggtca ccacggcccc agaccgccgg ctccccggtg acgggagtcg 8100
tcgctcccat catgcagcgg ggccgtagcg cccgcttccc ggcatgcctc gcgcacccct 8160
gcccgggaca ctcaccggcg ccggcggccc ccgctccggc tctgcggcgg cggctgcacg 8220
cccagcctct gcgcctgcgt cgcaagtagg gtaggacagc gcgcaggggg cgtgaagagc 8280
ctagggcgct tgcgcggcga gacggactag tcctgtagcg ctgtgggaag aggggctatg 8340
cgcgtcgggc cgtcgacgag acccgcgcgg ggggcgccgt gctttgcccc tcgctgcctg 8400
ggtttacttg gtacagcccg cggcccaaag gaacaagaag ctgaagggtt cgcgcgtgcg 8460
tgtgcggggc aggaacgcgc cttacaaaac tgggatgcgc tgggggtgga gggcgctagt 8520
tcggactgga tcctgggccc gaggcctgct tatttgcata atcctagcgc gggacaatga 8580
aaggcctccc gcactggaag gagtgatttg catattcccc ggaggggcct tactccagag 8640
cgcagtgatt agcatatggc gggggcaacc tgagcaaagc gcatgcgcgc aggactgca 8700
gactgacgcg aagtgggtag ccttgtcttc gtaggggatc agtttgcatc ctgagagagg 8760
gcacgagggc caggacccct cccaaccagg ataaaggttt attgatctcc taggtgtcag 8820
gccccatgct ggcggattct gtggtttctg cagtgaacca tactcctgta ctcacggcac 8880
cccagtcgaa ggagatacgc acctaattag acaactacta cccagaaggt cagacctgga 8940
gtgaggaaca caggggggctg tgggagccta agaggcgctt gccccggcct ctggttctag 9000
aaagacttcc aggaggtggt gatccttaag ccaagtacga ataggagcca actagaatgg 9060
gaatgggtct ggcagaatga actgcaagcg ccaaggccca gaggccaaaa aaaaaaaaaa 9120
aaaaatagaa gcgcatgttt tgattgagga agcaagagca gcttagtatg cctagaacct 9180
aactggagac gggaaatggt tctatagacg atgttagagt tcaactatgg ctacattcca 9240
gtcttcctgt aagtgacttt gtcacattct ggcttaaaac tcccccaaag ggatcccatt 9300
aggaaaaaaa aaaaatccaa aaatctttat catggcctca gggctataca cctggtctgg 9360
ccgtgcttat ctttctgacc ccacctactt cctcctccct ccatttctgt ccagctccac 9420
cttaccccaa actctttacc agctcgggcc tctgctcttg ccgttccctc cgcctgaaaa 9480
tgcttttccc tctgaccttt gaatacctac tcttgtgctc accattcata tcttggtaca 9540
gatgtcaatc tgagaggctt ttcctgatct ctccataata gcacttacac atttgactgg 9600
agttatggat aaatcgggat tggccatgag ttggtggtgg ttgtaactgg catgaagagt 9660
acatggggct gggcgcggtg gctcacgccc gtaatcccag cactttggga ggccgaggct 9720
ggtgtatcac ctgaggtcag gagcttgaga ccagcctggg caacatggtg aaaccctgcc 9780
tctattaaaa ctacaaaaat tagccagggg ttatgggggg tgcctgtaat ccttgctact 9840
tgggaggctg aggcacgaag atcacttgaa ccctggaggc agaggttgca ttgagtcgag 9900
attgagccac tgcactccag cctgggccac ccagcgagac tctgggtctc gcctgtaatc 9960
ccagcacttt gggaggccga ggcgggcgga tcacgtcaga agatcgagac catcctggcc 10020
atcctagacc atttctacta aaaatacaaa aaaaaaaaaa aaaaaattag ccgggcgtgg 10080
tggcaggcgc ctgtagtccc agctactcgg gaggctgagg caggagaatg gcgtgaacac 10140
gggaggcgga gcttgcagtg atccgagatg gcgctactgc actccagcct gggcgacaga 10200
gcgagacttg gtctcaaaaa aaagagtaca tgggacgtta ttgtcctgtc tactcctgtg 10260
ggtttgaagt tttccataat gacaatggca taccacatca ccatactctg catttatatt 10320
aatagttctt atcacaatct gaactttctt tgcttccttg ttttgagtgt tttcctcatg 10380
aaagcttcat gagggtaaga atggagtcgc ccttttttcac tttgggttct caatgcttag 10440
agcaggatca gatttcagat tagtgtagcg ctgtctttaa cacttaacat ttgcctgttt 10500
tattcaccat ggactctaga actttgagca gcacctggca catcgtaaga ggttattttt 10560
taaagttaga ataatacatc taaaatgtac atgaatgaat gagaggcctg ggatgccaga 10620
ctaaagagct ttgacttggt ctaaaggtga tggggagcta ggcaaaggtt ttgagagttt 10680
aactttaatt caaagttccc ttggagacta atgtctgggg tagggggaag ccagggtaag 10740

ggtccgggcc atggaatggg gtagctcagt cgctatcaaa aagacaagac tgtgactatt 10800
tggctgaaga aatggccaaa cccaggtttc tggggaggtc gaggtaccct cagtgaggtc 10860
aggaccttct cctggcctat actgtccacc agcaaccatc acactcctcc ctcccctctc 10920
ccttagttcc cctcccaatg gtacagccct tgacagcagg acagacacac agccacccca 10980
aacacttgtt ctctcctcag tttaatggtg gttagtgaga ttgccaaacc ccctccccat 11040
tcccctcccc accccgtaca aaatgtgtgt gtggtttttt gtttttgtt ttttgttttt 11100
taacaagaaa aagggggcaa aagccaggaa tggggagagg ggggtgcaat ctgatatttt 11160
catacagact tttgattttt taatatatta tatataaaac catgaagacc acgaatcctc 11220
cccaaactcc tttcccctc cccgggggc ctggaggaga gatggggaag gcccccccag 11280
gagtgggtgg acagagagac aaatatggat gggacagacg ttgggggaga aggtagagag 11340
aaggggagcc caggaacctg gggaaggggg attggagaaa agggttgggg ctgtctccct 11400
cactgccccc atcaaagtta tgacacaaag acacagaatc cctatttcca cgccctcccc 11460
ccacccatcc ccccaccgtg caaacatggc tttgcaaaga agtgcccaga gctctgtgga 11520
actcttacaa tggctggcat ggggtctagg acccccaaag aaatctgtgt tccccttccc 11580
tgcccccccc acccttccca gaaactgacc ccctccccac aagacctggt tttgtagcct 11640
aggggccctg gccttccccc agttatcttc ccccaaccca atccctactg ccctcactgg 11700
acttgggggg tctggacctt tggcccctgc cccctggggg acccagacct ctgggccctc 11760
acttctggcc cttacagaga tccaggcatc caacaccccc atccctgccc aagcgtctga 11820
ggtgttagtg gtggggggag aagcccacca tcccagactc tggtaaatgt ctttgctggt 11880
tccttgcagc tggcagtggg ggggacccca gcccaggccc aggcctaggc ctgggtgggg 11940
gatagggtca gatgaagaat tcctctttcc tcttgtgtcc gtcgctgcca ttgaggaagg 12000
cttctcttgc ttctccctgt tcatccaagc cactggcttc gtgggtcaga taggaacctg 12060
aggggtgac agacccccgg ggcagggggg acatatttgt ggatccagga gttggacaga 12120
agtataaggg aagagggaga cagacaagac acatgccagg cgaaggaaga gggagaaacg 12180
gaacacacag ggagaggcag agaaagaggt aaacagtggc agagaaagag gtaaaagcag 12240
aattaggaag actccaaaag ctcaccgaaa gtgccaccct tatcctttct cttggaggta 12300
tttccttgcc ctgctcccag cgaattcagc aattaggaaa ataaattgtt ttattcaaat 12360
ccatgctctt tttttcccct aatttttgt atttttagta gaaaaggggc tgcgccatgg 12420
tgcccaggct ggtctcgacc tcctagcttc tcaagtgctt tatccgcctt ggcctcccaa 12480
cgtgctggga ttacaggcgt gagccaccgc gcccaaccgc aaatctatgc ttttaattca 12540
gcttctaaat tctacccctt ttcgagtatt gtgccgaaag ccccgccccc tttgtcatct 12600
ccgcccccgg tgcggcggga tttggaatcc agagcctagg ctccgccctc tcgttaccct 12660
ggctctaggc cccgcctctt tccgagccct acaaccaacc aaccgtagag tccaggcccc 12720
gtcccactca cccttctgcc gtaccgagca ccagaccatg cccactagca cacatatgat 12780
cagaaacacc agcagcgcca ggatgccgcc cacaatggca tagggaaccg acgtctgagc 12840
ctctaccacc gcaccagggt ctgccagagg gacacggcac aggaccaggt catcagagga 12900
cgatcccagt ctggccccat cgctgccaag cttttaagcc attctgcaca cgtctaaccg 12960
tgccctttta tgtgccacac ccctcaaaaa ttactgccac cttgtagtct cttctctttc 13020
cagatgcttg ttggtttgta cactgcccga cccctcccct gagtcatgtt acattttcct 13080
tttctttttc ttgttttctt ttgcagagac gggggtctca ctatgtggcc caggctgatc 13140
ttaaactcct gggctcaagc gatcctccgg cctaggcctc ccaaagtact gggattagag 13200
gcgtgagcga ccgcacccag ccatcccttt tcttttgact caagtttctt cctccactaa 13260
gaaacagagt ccaagaaaca ggtccaagtc ccttcccacc ttgtctaaaa cgctccaagt 13320
atttaaagtg ctgggcccaa ctaccaaaat ttctgcccca ccgtcataga gctaaacaca 13380
gaacagctgt gtgctagagc ccattccaac caccttacat atttagttca cataatcttc 13440
acaacagcct tgttatatag gtgctattgt ttatttccac tttactgatg ggtaaactga 13500
ggcgcagaca ggttcggtta cctgcaatag aatgcagcca acccgaattt gagccccgcg 13560
ggccagtctg gtcccaaaac aaaaagaact ctgttggctg ccgaacccct gagttatgtg 13620
gcctctttgc tcaagccccg cccccgccac ctggcgcccc gcccccgccc tcagtcggcc 13680
gcagcctgct ctcaccgtag accacaagta cgtagagcgc cctcgcatgg ccgtgcttat 13740
tggacgcctc gcaagtgtag gtgccgttat ccgcggatac cagacccggc agcgtgagcg 13800
tctctcccac ggcctccgcc ctctccggca aagactcatt cccgcggttc cagcggatct 13860
ggtttggcct gggtggggat aaagtatagt gagagttagg aaccgaggtg ccagcaccca 13920
attctgactt gtcaagaatc tagacatgca actctcatcc cgcagggacc tccaaataag 13980
aggcttcctg ctatctcttt cctttctgga aaaccaacag tcctgggcct acttccaccc 14040
atcaccaagg tctcaggaat tctagcccag gctgaacatg gtggcttatg cctgcaatcc 14100
cagcacttta ggaggctgag acgggaggac tgcttaaggc cagcagttcc agaccagcct 14160
gggcaacaca gggagacccc gtcactacaa ttaaaaaata ataataataa taataataat 14220

tctagccctc ccacgccatt ccatcctcag caaccaggag tctgaggctg cacagcttca 14280
gtattgggga gtctgagcct ccagattcct cctccctcag gatccaggag tccaggtccc 14340
agatccctat tcgtccaggt ccccagctct ctcctcctca ggacccagga atccaggtcc 14400
tagctccctg tttgtccagg tcctcagctc tctcctcctt aggacccagg agtccaagtc 14460
cctggtccct gttcttccag gtccccagct ttctcctcct gaggacgcag gaggccccca 14520
gagctcacct ggggttcccc gtgacagcac acgtcaacac cagcgtgtct ccctccctca 14580
ccacagcttg ggaggcatga atccgggccg tgggggagtc tgttaggcaa aagtaagagg 14640
agagagtagt ttccaagcca tcacgcagga caagggggac cctcgcgggt gcgggtggct 14700
ggcgttggga tcccttgggt cctggcccgc cggtcactta cactgcacat ccagcacgta 14760
ctgcgtctgc ttgctgtgtc cggagggcag cgcctggttc tgcgcctcac agatgatgat 14820
accaccgtcg tccttacggt ccacacgaaa ccgtactgtg cttgccacgc tccagacctt 14880
gccattttcc tggctgctgc tcactcctgc cacaccccgg tcagacactg tcaggccaca 14940
attccggctc catccaccca cccacccgag ccaacgccaa agcaggctat ttgccaagct 15000
ccacccctta cccacaggcc ccgcctcttg tcctccaagc tacgcccctc ccctaaccaa 15060
gcccacgtgc ctcctcccaa agctcttccc tctttcacgc tcatgctttc tcgtctatca 15120
atccatttaa ttgctatata tataaaaaca taaatttata tatatactta gagacagggt 15180
ctcacaatgt tgggcaggtt gaactcctga cctcaagcaa tcctcccatc tcagcctccc 15240
aaagtgctag gactacaggc gtgagccacc gcgctcgaca tcaaccacta catattgaat 15300
gtccagtgtc tgtgaaaacc tgtggctcct ctccacatat aaacaacctc tcctaagtcc 15360
cacctcctcc ccatcccttg tcagcactcg gcccagggta cctttcagct ccttgcggtc 15420
ccggtaccag cgcagggtgg cagccggacg ggaccgcgga acgaggcagc tgagctccac 15480
ctcgccgccc tctaccgcct gctcccggac ctccaccaca ggattctctg gggccactgc 15540
cgcagggaga agggaagtaa ggggttaaag aaggcacgaa cgtgggctca aagcgatcga 15600
gctgcctgtt cccagcgacc atagggaacc agggtcccag gtggcagggg tcaaagggga 15660
gaggtcagga gccagatgcc catccaggat gttaaaaata gccatggtct gaaagtctca 15720
ggagaagaga gaagcagaga agaaaggagg agaggatgcg tctgacaagg gggagggcgt 15780
tacctagtac cgtgagcgtg gcaatctggt ggtgggtgtc ttctgtgtag agctggcaga 15840
aatagccccc ctcgtcctcc aggcgggcat ctgagagccg gatccgcacc cggcgtgggg 15900
agaactcctc aagctggaaa cgctcatcct tcaaggctag agagagtgag ggggaaggtg 15960
tgaatttcgg gagtcctggc ctcacaagtc ccacccttcc gacaggagct tagagtccag 16020
ccctctgcct cttttctcca gccatatcta tgagtctgag gtgtccaact atttactccc 16080
ttgaggaccc agcattattc aagtcctcct gcctgcagga ccagcagtcc gggaccccag 16140
ccctttcttc tccgagaccc aggagaccaa actctcaggt gtgtcctctt tcaggacatg 16200
ggagcctggg ccccagccct ctcttccttt aagactcctg agtctggtcc ccagcactca 16260
ccacgggtgc cattgaagaa gagggtctgc cgggctgggt tctggatgac aactatggac 16320
ccatcatact ggtgcagacg gcaggtgatc tcagccaccc caccctcagc cactgtcacg 16380
ttctctgtct gtacttcctg tcctgcccct ggacgattag acaaagagac aggatagaag 16440
acttactgag agctgcaatt caattttttc tttctccctc ttccccatcc aaacctccaa 16500
tccctctctt tcccctcatt cattccattg cactgaacat ttcctgcagg ctagagtcca 16560
ggacagggag gaaatctgct ccctactcta aaagagctgc agtcaagatt tagtagaata 16620
tgctctaatg agggcagcac agggcacact aggagcccag agcaagggag gactattata 16680
gaattgccta gagagatggg tagccagaga gggctctgca agaaagctcc attggatctg 16740
gatcttaaag agtaagcagg aggctgagcg cggtggctca tgcctgtaat cccagcactt 16800
tgagaggccg aggtgggcgg atcgcaaggt caagagatag agaccatcct ggccaacatg 16860
gtgaaaccct gtcactacta aaaatacaaa aaaaaaaaaa aaattagctg ggtgtggtgg 16920
tgcgcacctg tagtcccagc tactcgggag gctgaggcag gggaatcgct tgaacccggg 16980
agttggaagt tgcagtgagc cgagatggag ccactgcact ccaggctggg cgacagagcg 17040
agactctgtc tcaaaaaaaa aaagaaagaa aaaaaagagt aagcaggagt tcacaaggtg 17100
tgggagactg ctgtgtgttc accaagcctc atctttcaca cctgggcaca tgttgtagcc 17160
cgtttgcaaa gatagccgta atattctcct gtccctggac atgccctttg caagttgatt 17220
ttgccattcc tcccattgag aaggcacttt gtcccctact agtctgggta agccttgaga 17280
gttgctttga ccaatagaat ttgctagaag tgatattgag cctaggcctg aagaggcctt 17340
gtagcttcca ctcctgccct aagactgttg catgaagata cccagactag tgtctttgca 17400
gatgaacaat catggtgaaa gagaagccca gccggcagcc agcaccaatc gccagctgtg 17460
tgagtgtggc catcctggat catccagccc cagctgcccc accagctgac agcagccaca 17520
caagtgaccc cagttgagac caataaaaga tctgcccatc tgatacagcc caaactgctg 17580
aaccccagaa tcatgaacaa ataaggtggt ggttgtttta agctcctaag ttgtgggtga 17640
tctgttctac tgctaaagtt aactgataca atacataatt aggctatact tcccagcatc 17700

ctttatagtt aggtggggcc atgtgaccaa ttctggccaa tgggatgtag gtggaagaga 17760
aacacctctt gcagcctgac ccatctccct cataatcctt cacactggct gaacagagag 17820
gactccaagg agcctagagg agggcagaat cacaagccag aaggaacctg ggtctctaac 17880
tgactgtccc ccatgacccg cctgtatagg actgtgatat gagcaagaaa tatacctttt 17940
tgttaagcca ttgagatttc aggggtgtct gttacagcct ttaacctacc ctgattaatc 18000
catcagaaaa acaaggtggg gaatctagaa ccatcagaga aaagcattta ggaaagctga 18060
aagccaagac taatcatcag cattaatatc atcatctgtt gtcttcaaaa taacaataac 18120
ccccatagct accaattatt aggtacttgc agtgttagtc cctgtgctaa gggcattacc 18180
catataactt acctttaatc ctcacaatcc ctgtgtaagg tagacatgat tattatcatt 18240
attattatta ttttgggaca gagtattgct ctgttgccca ggctggagtg cagtggtgtg 18300
atctcagctc attgaaacct ccacctccca agttcaagcg attcttcagc ctcagcctcc 18360
caagtagctg gaattacagg catgcaccac catgccgggc taatttttat ttttagtaga 18420
gacagagttt agccatattg gcctggctgg tctcgaactc ctggcctcaa gtgatccgcc 18480
tgcctcagcc tcccaaagtc cagggattac aggtgcgacc caccgcgcct ggccaattat 18540
tattattatt tttaatttga gacaaggtca ggctggagtg cagtggcacg atctcagctc 18600
actgcaatgt ctgcctccca ggctcgagtg atcccacctc agcctcccca gtagctggaa 18660
ctacaggtgc acaacatcac acctggctaa cttttgtatt tttttagaga cggagtttca 18720
ccgtgttgcc caggctggtc ttgaacttgc gagctcaagt gaactgcctg cttcggcctc 18780
ccaaagtgct gggattacag gcatgagcca ctgtgcccgg cctgcgctat tattatcccc 18840
attttgcccg gcctgcgcta ctattatccc cattttcccc catttccatt tttcttttct 18900
tttttttttt tttttttttt tgagacattg tcttgctctg tcgcccaggc tagagtgcag 18960
tggtacgatc tcggctcact gcaacctcca cttcccgggt tcaagcaatt ctcctgcctc 19020
agcctcccaa gtagctggga ttataggcac ctgccactgc acttggctaa tctttgtgtt 19080
tttagtaaag acggggtctc accatcttgg ccaggctggt ctggaactcc tgacctcgtg 19140
atccacccgc ctcggcctcc caaagtgctg ggattacagg cttgagctat cgtgtcctgc 19200
tcccattccc attttatagg tgagaaaatt ggcccacaga gatgaaatga cttgcccaag 19260
ttcacagcca agagtggcag tgccaaaatc ttcgtccaaa tctctgattc tgtatcctga 19320
atctgtatat ccactcctgg ctgtctggat taagtgtcca tcattggcag ggggttgtga 19380
gagccgcttg tgatgggcct cgaatgccaa cctaggagat ttgctttcat cctaagggcc 19440
agtgaaggtt ttgaagcagg aatatgccat gattagatct ggctatttgt ctttaagtgc 19500
tggataacta tccatgtctt ttacattcag gtgctgggtt gcattcattc aggagtattt 19560
cctgagcatc acgtaggttt tcaggggctg agtagtcaga gatgagttag atgaggtccc 19620
tgccctttaa gatttatggg aaggtaggaa ccaatcacgg taatcaaaag tgttatgtgg 19680
ctgggcacgg tggctcacac ctgtaatccc agcactttgg gaggccgagg tgggcggatc 19740
acaaggtcag gagttcgaga ccagcctgac caacatggtg aaaccccgtc tgtactaaaa 19800
atacaaaaat tagccaggtg tggtggtggg tgcttgtaat tccagctact caggaggctg 19860
aggcataaga atcgcttgaa cctgggaggc agaggttgca gtgagccaag atcgcgccac 19920
tgcagtccag cctgggtgac agagcaagac tccgtttcaa aaaagaaaaa aaaaaaagaa 19980
ataaataaaa gaaagtgtta tgttttctgt aagagggtag gtaacctaat ttggaagttg 20040
aggggtagaa aagattattt ctgggggatg gagacagaga cttctggctt cctattctga 20100
catccatttt tccctttctc ctcagtaaaa gaaaagaaca ctggttgtat tttatggttg 20160
cactatgtcc agcagaaaaa ggcattcctc agtctccttg cagcaaggta aagccatctg 20220
ataaaatttt gtccagttgg atataagcca aaatgttgcg tgacaatttt gggaggactt 20280
cctgaaacag gtggacaaac cctttttcta ctgagtcacc tttgtgccac ctggaactaa 20340
cagtgtgacg cgtggaattt aggcagccat attgaaccat gaggacaaga gcagtgggga 20400
tggcggaacc aagagctgga aggtgcctga gtctctggtg aagatgtgga gctgctgtaa 20460
cagccctcaa ctcctagttc tggacttctt ttatgtttta gtgtaacgct ttgggtattt 20520
ttattttttt aatttatttt agagatgagg tctcactatg ttgcctaggc tggactcaaa 20580
ctcttatgct caagcagtcc tcctgcctca gcttcatgag tagctgaaac tatagcactt 20640
tgggtatttc agccactgtt tgaggttttt ctagcacctc ctggaatatc aagcttaaca 20700
tgtccaatcc ttgccccaga tattttcctc cccaaatttt ctcaatctca ataaatgtca 20760
ccaccatcca cctggttgct caggtcaaaa acctagaaat cattcaagtt ctctcccttt 20820
ccctcatccc caatatccat tccatcagca acatctgtcc attctacctc caagacatat 20880
cccagatctc atcacctttg tctgcctctc ctaccctcac tctcatccag catcatccct 20940
cacctggact ctgcaaaagc ctactcgtgg gtctgtctgc atccctgtct gcctcctcca 21000
gggccattct ccacccagtg gccggatcga tttttcaaag aggtaaatca gatcaattca 21060
cctttctgct taaaaccctc cgagggctgc ccgtaacatg tagaataaaa tagagacccc 21120
ttcccgggga cttcaaggtg ctatatggcc tggccccttg ctgaccttac ttcactctgg 21180

gctcgctagc cttgctgtcc ctcaaacatg ctgagctcgc tcccaccaca gggccttttc 21240
ccttttcttc cttctgcctg gaatgttctt ctccccacct cccaagcccc atcttcccag 21300
ggctgactcc tgttcccatt tgggtctcaa atcatatcag taccttctca gagaggcctt 21360
ccctcactgc tcatcccttc acctttagaa cactttcttt tcttttaaga gacaaagtca 21420
gcccagtgcg gtggctcacg cctgtaatac cagcactttt gagaggccaa ggcgggcaga 21480
tcacctcagg tcaggagttc aagaccagcc tggccaacgt ggcgaaaccc cgtctctact 21540
aaaaaaatac aaaaattagc taggcagtgg tagcccgggc tactcaggag gctgaggcag 21600
aattgcttga acccaggagg cagaggttgc agtgagccga gattgagcca ctgcacccca 21660
acctgggtga cagagagaga ctctgtctca aaaaaaaaaa aaaaaaaaag agacagggta 21720
ttgctctgtc acccaggctg gagtgcagtg gtgcaatcat ggctcactgc agcctcgaac 21780
tcctgggctc aagccatcct cccacctcag cctcctaagt agctgagatt ataggctcct 21840
cccaccacac ctggctaatt tttgtgcttt ttgtggagac acagattctc catgttgccc 21900
aggctggtct ccaactcctg gggtcaaagg atcctcctgc ctcggcttcc caaagtgctg 21960
ggattacagg cgtgagccac tgcgcctggc ccagaacact tgctatttcc tcaccattgc 22020
tttatttctt ctatgaagat ttcactggaa ttatcagatt aatttgctta tttgtttact 22080
gtctgtttgt cacccatgac tggaatgtat actctaggaa ggcagggata taatccaatg 22140
ggtttactgc tgcaccccta gtacccagaa gagtgcttgg cacctgataa gtgtctgggg 22200
aacttgctac atgaattaca tgtgtcagat gggatatctg ttcgtctttc ttctctcttt 22260
tttctttctc tctttctctc tctctttctt tctctttctt tctttttctt tttttgaga 22320
taaggtctcg ctctgtcacc caggctagag tgcagtggtg caatcatggc tcactgcaac 22380
cttgaacatg tgggctcaag cgatcctccc acctcaggct accaaatagc taagactaca 22440
gaggtgcgta gctatgccca gctaattaaa aaaaaaaaaa tttttttttt tttttagaga 22500
tgggggtctc aatatcttgc ccaggttggt cttgaactcc taggctcaag caatccccct 22560
gccttggcct cccaaagtgc tgggattata ggcatgagcc attgcagctg gcccagacag 22620
aatctcattt cagcccgaca actttgtgac atcattattt tcatcttaaa cacctaggtt 22680
gatcccagct caaccacttg ccatctgtgt gacctgtggg caagtgacct tacctttcgg 22740
agcctcagtt gccccatcta taaaatggga atgatgccag tgcctgcctc ataaggatga 22800
gccccgctcc tgaagctcag ggagccctct ctgcaaggct gttttagtgc aacctccgga 22860
aacatgccca tgcatgtgaa aactggcatg cacattctgg tgcttttaaa aacatctcga 22920
agcctatcca cagatcctgg acctcaagac tggttcagtg ctagcccccc atttacaga 22980
tgtggagaat gaggcttagc gggtcccagg caagtcagtg gcaaaactca ccatctcctg 23040
ggagccatca ggttcctctg gatctgcccc caccaaattt atcccctgct ctctgcttga 23100
gggtgcacat ggggtgaggg tgggggtctt ttgttttact ccctccccct cctgaggagt 23160
cagtaaccaa cagtgtctgt gcctggaata ttaatgtctc agcagctttt gtttgggggg 23220
ttgggggtgg tgggggcggg actttctggt cagagagggg ctgagctttg gggactgagg 23280
cactggccct ttaaactgtg ttgacagcca ggagtcgtca tggggatggt gcttggaaaa 23340
ggggacaggg agggtttggg aaagagtggc ggagcaggta atgcgtaaga cccaggaatc 23400
cagcccccaa ctacctcctc tcccaggacc caggagtcta ggctcccagc ccctcctcca 23460
tcaggttcca ggagtctgga accccggctt ctttccgcct tagacccagg aattcagccc 23520
ccaaccacct cctctctcag gttcccgaaa tccagacccc tagccccctt ctcgatcagg 23580
acccaggagt ctgggctgtc agcagcccct tccttcaaac ctaggagtca gagcccccag 23640
ccctctccta gcttagacac aggagtctgg gcctccagcc ccctcctcct tcaggaccca 23700
ggagccaggg gtccagagta cacagctggt ggatgtttcc acggagacta agcagggtgg 23760
ggggagcgct tcctgggtcc tgagtcagcg aatacccaag ggagtctcaa ggtcatagtt 23820
ccgggaaggt caccaccacc ccctctgtat ccgctcccca gggggctcct ggcatcctgc 23880
ctccttcccc cttcctccct tagggaggtg gtacatccct gcgtcctgac tgaacccccc 23940
tcagcccccc atcaatggcg gagtccgaac atcctcgcac aaagcgtcaa ttcttcccca 24000
gctcagcctt gtgaaggcgc ctgtattcgc aggacctagg cgtcagggtc tcagcccctc 24060
ctccctcaga aacctgcagt ggaatccccc gcctccagcc ccttcctccc tcaggaccca 24120
ggagtctgta tcctcatccc ttcctccctc aagacctagg agtgtggact cccagccccc 24180
ttttccttcc ggacacagga gttccagccc tcggccctct cctctcttaa acccaggggt 24240
ctaagacccc agcctcctcc tccctcaaac tcaggagtct aagatcccag gcccctcctc 24300
cctcagactc aggagtctaa gatcccaggc ccctcctccc tcagactcag gagtctaaga 24360
ccccaggccc ctcctccctc agactcagga gtctaagatc ccaggcccct cctccctcag 24420
acccaggagt ctaagacccc agcccctcct ccctcagact caggagtcta agaccccagc 24480
ccctcctccc tcagactcag gagtctaaga ccccagcccc ctcctccctg gacccaggag 24540
cctaagacct cagccccctc ctccttgaga cccaggagtc taagacccta gctccctcct 24600
cctttagacc cattagtcca ggcccccaga ccctcctcca tcagacccag gagtccaggc 24660

ccccagcccc tcctccatca gatccagccc ctcctctcct gaaaactttt gactctaact 24720
ccccagtcct caacccctag aagcacagtc ctgcctttcc tcaatcctct gtcccctccc 24780
atctggggac ctaggcatca ggtgggggcg taggggtgag tcagcaacct cacacacaaa 24840
gtccccgctg tggcccccac attcctggga tattcgggac tccctggatt ccaggcctca 24900
ggcccagcca gggagtgggg agtcccccag aggtcctccc tgggtgtggg gtacgagagg 24960
aattcctgct ccgggaaggg tgcaggcctg cactgagctc cctctgtccg aacctccacg 25020
cccagtgccc tctattcacc ccctcttccc agaagagccc aggctcagca cctgcccctt 25080
gccccactgg gtgcccacgg aggagcctgc gtgcctgctc cctatgggcc tggggtctgc 25140
acaggcggaa atcagtgggt gcttccgttc tgatgccaca ggccattgga tgctggcggg 25200
tctgactgtc tccaggccac cccccacccc tcccagagag agaaagctgc ctttgtgttc 25260
tccaagatgg ggacaggcca ggctcgcacg acattaaccc agccttaggc cccagccctg 25320
ctgtgtctaa ggtcttggaa tccactgcag aacctgaccc ccacccccag gctctgggga 25380
cacaggcgcc tggctcatgg gtgggtgggt gggggggtca gtgatagaaa cctccaaaac 25440
ctgttccttg ggtgactca caatggaggg agggtccccc tattctcaag agtggctggt 25500
cagaatttta gcaggaaaaa gtgagtcacc ctgggaagga aacattattt agggaccaac 25560
aactgccccc tccacaagac ccctcaactc ctaatagcct ctctattctt tctttgtatt 25620
ggatatctgt ttcctctcct cctttctgtt ctacccagtt tctggctgcg ggtcccattt 25680
ctgcctgggt gcatccctgg gcaggcaacc catccctccc tcttgctttc tctcctctgc 25740
ccaccctgga tccttctttg ggcataaatc tcatcttctt ctgctatgct cagaagatga 25800
atgaaccagg agagagagaa catgttttta aaatggcgca aatgcacccc atctcccccg 25860
attcctgctg gctgggcaag gtgagagagg aagaagtgac taagagagaa atgtgggaac 25920
aacagatacc ccctaaaatg tggtagccaa ggccactgag aaatatccaa tggaaaggag 25980
agcaggaagg gccctccaag accacatgct acagcctcct accccatgct ttacagaacg 26040
ggaaagtaag gcccagagag ggacaaggac tgatgcaaaa ttatactaaa gggtcctggg 26100
taaggcttgg acccaagttc cttagctccc agctgagagc tcttcccatg acaccaagct 26160
cagtttctac tggtaaaagc cacatactat ttactttaga gaaagtttac agagagggtt 26220
agggtgccag gaagcagtga cttggaaatc aaacgaggga cagggctgta gacctaactc 26280
ccagaagcac cagagaaagg cttttgcacg gggcgggtgg tcaccttaag ctatattctg 26340
atcctgagaa ttcaaagtct gatgattcta agctgtcagg attctaaatg tcatagatgt 26400
caagatccag gaactccaag acatcaagat ttcacgattt ttaagacgtc aagatgctag 26460
catgctaaca ccatcacggt tctagaactt taaaggtgtc aagattctaa agccttctgg 26520
attctagaat cctgtagatg tcagcattct aaagtaccat caggttcttt atttactgga 26580
ttcattagtt ccaggattct atgagcctgg tgtttagcct aaaaaataaa gataaattaa 26640
aattgatgga aatgtcactg aggtaccaaa gttctcatct gggaaattgt ggcatgtctg 26700
ttgtaaagaa aggaggtaat gatgcaagtt ctaaagcagt cacagaagac tagagaagaa 26760
agaaagacag tgagaggaca gctttgcccc tcatcctggc cgaggtgagg atggctctgc 26820
ctcaaaccct ggagtgggga acatgtaacc gcactcaact tgccagaaac cccttcacgg 26880
tctgagctgg cgttcccttt catgtcactg agttcaacat cctcacttta cagaaagaga 26940
aacagaagcc tggagagagg aaggtgttta ccattggctg cgatggcaaa tggcaagagc 27000
caagatttaa gcccaggccg ccagccccat gccacctggt tataactcct ctcaccaatc 27060
tctgccgaac acccagccct cctgcttctg cctagccacc ttccaatcct ctgttccttc 27120
caaaagtggc cttatccacc agggaggggt gacccgtggc aggttcaaga cttacacagt 27180
gtgagagtgt gtgtgggtga catttcctga ccttgtcccc attctcaggg tcacccaacc 27240
tcgggggtct ccagcttctc acagtgtgtg atgagggtat gtggatggct ccctggatgt 27300
cctggacagg ggcttctctg tgagtcaagc ctgggtgtgt gaatgggtga gcagggtttg 27360
gagaggcatt cgctgaatcc acgtgtgtgc ctacacgcca aggtccccca ttctcacttc 27420
cccacacaca tgcacacaga tgttcccctc cagggctctt tagaatgccc tgcctgactg 27480
aattcctctt caggggcaca gagggataga gagagggagg aaggtaggat gggaatggga 27540
gatcccggga tggaggctgt aagcgtagag agaggaggca cagcagaaag acagggatgg 27600
agatagtggg acagagaagg gggaaagaga caggtgacag aaagggttag agaaacgagt 27660
gacagaaaga caggggacag agacaagggg atggggcaga taggggacag agaaaaaggg 27720
acagaaaaac aagggtgaca gcgagacaga gacagggacc aagaataggg gcagagaggg 27780
agggcagaaa tccgggggaa agagaataga caggatgatg gaggggacag agtgacccag 27840
gaaaagggga cagagaccag gggacagagg taggggacaa agacagaata gatgaggaac 27900
accgaggcaa gaagagaggg agacagacag aaggagggac aggacttcga gactgaggga 27960
tagaggacaa gggtaggggg acgaggagcc agacgggggg gttcagagac gggcggacag 28020
agggacgcag agactggaca gaaggacagc gggaccggcc tggggagggc ggacttgtgt 28080
gtgtaggggg gtctcgggcc ctttgtcccc gccgggatcc agcctgcgcg ggtggggggg 28140

ctgcggcacg gcggccgggc cccgcgcccc ctcccccgct cgtcgctccc ggctcccggc 28200
ccgcgctgcg ctttgtcccg gggagggggc ccggcccggc cccgcgcgca ttgttcggcc 28260
tctgcggccc cgaggctgcc gggctgtcac cacagcgcgc cccccgcccc agcccggccg 28320
gccgaccccg gcccccgacc ctacctggcc ccgccgcggc cgcccacagc agcagcagcg 28380
gccactggaa gcgccgggcc cggcccatgg tgccgccgcc gccgccgccg ccgctcgctc 28440
ccggcccggc acctgcaccg cccgcgccgc ccgccccgcc ccccgcgccc cgccccctgc 28500
ccgcccgggg gcggggcgcc gaggccgggg cggggccggg gaggggaggg ggagacggag 28560
gagaggcccg gagacaatcg gggggacggc acggtggggg aacggtgcgg ggtgcgaaag 28620
ctggagagga gaggggtgag gagggcggga aggggtgcgc gggagggcga cagcggcgtg 28680
ggagcaggtg ggggatctcg gtgagcgcgg gaaatggagg gtgttgggtg agggtgctgc 28740
gtgcgggccc aggtgctgcg cgcgagggtg cggagttgct ggcatgcagg gtgcttgcgc 28800
tgcgcggagg ggagggtggc agggtgttgc tggaggctgt gcgagggtgg gggcgcgggc 28860
gtcgtggggt gcggtgtgtg cgaaggagga gcgtggccag cgtgacgggg gagcgtaagg 28920
gagggagtgc gacgtgggaa aggtgagtgt gagaggcgtg ctgcgggcag gtgggtgtct 28980
ggagtctagc gagaggctgt gagctgagcc accgggacag gggaggctgc agctggaggt 29040
ccggagggtc cggaggtcga ggcaggtcaa ggatctccca gggcagggcg aggctggggc 29100
tcaggagtgg ggtggggtca gttccctccc tccctctctc ctgtcctgac ctgaaaaccc 29160
cgtgtttccg cgtcattctc cgggaggggc cccctgaaag tgaactaact ggaaggaagc 29220
ctgaatcctg ggtcccagga gggagaggct cctgtgaaca ccttccaagc cctggcgtcc 29280
cctctcctcc ctgctgtctc cctgccccag cctctctccc tctctctgca tgtatttgcc 29340
tctgcccttc ctctctcccc atctttgagg gtgactcacc cctccagact taggtccctt 29400
ctccctcctg ggagtgggtt tccctgagcc cacttctgtg acaccctgta gacctgatgc 29460
gggatcatta cctatgggac ccagaaagag tgagaaacca tggaaagaag gcctcgacct 29520
ctctcatgcc catttgtcag gcaaactgag gtccagaagt gccaattatg aacatctttc 29580
cttcccccct ccccctcccc cgcccagacg gagtctcgct ctgttgccca ggctggagtg 29640
cagtggcacg atctcgactc actgcaacct ctgcctccca ggttccagtg attctcctgc 29700
ctcagcctcc cgagtagctg agattacagg cgcccgccac catgcctagc taatttttat 29760
atttttagta gagacggagt tttgccatgc tggccaggct ggtcttgaac tccttacctc 29820
aggtgatcca tctgtctggc ctcccaaagt gctggattac aggcgtgagc caccatgcct 29880
ggctgaaaat ccttactttt tattccgact aaaaaatttt acatccagtc ccacaaggga 29940
cttcagcttc acacaccctt tctgtcctca gtacccagct cccagtatcc tttctgacct 30000
caaaaccata gctaccatca acccttgtgt cccaggacca tggctcccag tgtcttctct 30060
gtcctcaggg tccaagctcc catcaactcc tgtgtcctca ggaccacggc tcccagcatc 30120
ctctctgtcc ttcaggtcca agctcccatc aacccctgtg aagcaggacc atggctccca 30180
gcatcctctc tgtcctcagg gtccaagctc ctatcaactc ctgtgtcccc aggacgatgg 30240
ctccagcaat cctctctgtc ctgagagccc aagcttctaa ctgcccctgt gtccccagat 30300
ccatagccct gagcaacttc cttctttttc agtcctcagc ttcccagctt ctgtagactt 30360
gggaagagat agtctctaat cctctttcca gggctcacat tctgtgactt ttgctagatg 30420
ggagaggaat gtttgatctg cctttggaat actggtccaa ggggtaacta gtagttgcct 30480
tttcccgcag gagccaatag gcccgctcac tctgtgctct gacagatgtc tcctgctcca 30540
gctgaagggg aaccttggga gatgttggtt tggttctcac ctgtcatcct taagtcccac 30600
cattccatgt gaagacatca caagagtagt ggtcctgacg ggcgcgttgg ctcacacctg 30660
taatcccagc actttgggag gccaaggtgg gccgatcact tgaggtcagg agtttgagac 30720
cagcctgacc aaccggccaa catggtgaaa caccatcttt accaaaaaaa aaaaaaaaaa 30780
ttagcaaggc gtggtggcac gtgcctgtaa tcccagctgg tcggaaggct gaggcatgag 30840
aatcccctga acttgggagg cagaggttgc agtgagctaa gatcatgcca ctgcactcca 30900
gcctgggtga cagaatgaga ctcagtctaa ataataataa taataataat aataataata 30960
ataataataa taaatagaat agtggtcctg tccccatcct acttcagggt accctgtcca 31020
ttagggattt agtgcaagtg acagcaagtg caacccaact ggtttgagag aaagagaact 31080
ggttcacaca taacaaaaag tccttctatg gctggctttg gcgaggtctg tcaatctctg 31140
tcctaaggat gcatggctcc cctcctgtag caagatggct ggcagatacc cctggggcca 31200
gattcatatt tggggtgatt aagattctgc aagagagaga caacctttat ttcacacagc 31260
ttttcaattg ttgcctgtcc ctggtgagac tcggagacct agctcttgcc tggtttctaa 31320
actttcaata acaccgtttt tgcttaagtc agcacaaaca gattttattt cttgcaagca 31380
aagattcctg aacaacaact tcagagccgt taacaatgag gtcctgatca caagctatgg 31440
tataggacgt gagaaatttg tccctagcct caatatctgc tggagggcat catggaataa 31500
gtatttctat cctctgatcc ccactgtagg gcatcatggg atatataatc ctaaccttca 31560
atctctgcca tagagtttca taggcaatgc agtcctagcc tcaatatgtt gtagggaatt 31620

atgggaaagg tgaaattatc ctcaattata atacagagca tctcagaaaa tgtcgtttta 31680
gcctcatctc tgctgtaggg catcatggga gatatacttc tggcccaatt tttgttgtaa 31740
gttgccatag aagatgcagt ctttccttcc ttcccttttt tcttttcttt ctttctttct 31800
tttttttttt ttttattatg tagagacagg gtctctcgct atgttgccca ggctggtcct 31860
gaactcctgg gctcaagcag ttctcctgcc ttggcctccc aaagtgctgg gattacaggc 31920
aagagccatt gcacccagtc ccttctctcc tttctttctt catcacctgc catattccag 31980
gcactaggaa taaatcatca agtaaataaa cggccttacc ctccctggca attataatgg 32040
ggaaagttag ctaaaaacaa acaaaaatta ctgttccatt taaccatcgc tgaataacaa 32100
aataccccag aacgtagtgg tgtgaaacaa caacctttta atttatgat tctgtgagtc 32160
aggaattgga gcaggattgg tgtgtatctg cttcatgatg aactggagcc aaaaatgaac 32220
tagctggaac agctggagat ggaggggagg ggcatcaagg gccatatatc taaggctggt 32280
ggttggtgtt gtgggttttg aatagtgtcc tccaagtaaa atatatgttg aagttctagc 32340
ccctggtatc tgtacatgtg accttatttg gaaataaaat ctttgcaaat gtaattcact 32400
tttttgtttg tttgtttgtt tgctcgagac tgagtctcgc tctgtcaccc aggctggagt 32460
gcagtggcat gatctcggct cactgtaacc ttcacctcct gggttcaagc gattctcctg 32520
cctcagcctc ccaagtagct gggattatag gcacgtgtca ccatgcccag ctaatttttg 32580
tattttcagt agggacgggg tttcaccatg ttggccaggc tggtctcgaa ctcctgacct 32640
caaatgatct gccacctcag cctcccaaag tgctgggatt ataggcatgg ggcactgcat 32700
cctgcccaga tgtgattaac ttctaacccc tggtatcttt gcatgtgact ttatttggaa 32760
ataaggtggg tttttttctt gttttttttt ttttttttga gacagtttca ctttgtcgct 32820
caggctggag ttcagttgca taatctcagc tcactgaaac ctctgcctcc gaggctcaag 32880
cgatcctccc gcctcagtct cccgagtcac tgggactacg ggcaagcgcc accacacccg 32940
gctaattgtt gcagtttttg tagagatggg gttttgccat gttgcccagg cggtctccaa 33000
ttgccaccct caagcaattc atccgcctcg gcctcccaga gtgctggaat tataggtgtg 33060
agccatggcg cccggccaga aagtctttgc agatttagtt gaattaatga ctaaatgttt 33120
ccatgctgag ttagagtggg ctctaaatcc aatgattgat atggggttat aaggagagat 33180
atttggagac atagccacag tcccagggaa ggtggacatt ggaagacaga ggtagggatt 33240
agagtgatgc agctacaagc caaggaatgg caaagattgc tggcagtccc tcagaagcaa 33300
aggagaggca aggaagggtt cttcccctga gacttttttt tttttttttg agacggagtc 33360
tcactgctgt cagcctcagc tggagtgcaa tggcgcgatc tcggctcact gcaacctctg 33420
cctcccaggt tccagcaatt ctcctgcctc agcctcccga gtaactgaga ttacaggcac 33480
ccgccaccat gcctggctag tttttgcatt tttagtagag atgggatttc accctgttgg 33540
ccaggctggt ctcgaactcc tgacctcagg tgatccaccc gcctcggcct cccaaagtgc 33600
tgggattaca ggtgtcagcc ccggagactt taaaagcatg gctcttcccc tgacgcttta 33660
aaagcgtggc tcttcccgtg agacttcaac accttggttt tggacattta gcattcagaa 33720
ctgtgagaga acaagtttct agtgtgtgtg tgtgtgtgtg tgtgtgtgtg tgtgtgtgtg 33780
tgtgtgtgta tgtgttttag acagaggctc attctgttgc ccaggctgga gtgcagtggt 33840
tcaatctcgg ctcactgcaa actccgcttc tcagattcaa gtgattctta tgcctcagcc 33900
tcccaagtag ctggaattac agaggagcgc catcacagcc ggctattttt tttttttttt 33960
tttgtacttt tagtagagac agggtttcac tgtgttggcc aggctggtct caaattcctg 34020
gcctcaagtg atatgcctgc cttggcctcc caaagtgctg ggattacagg tgtaagccac 34080
cacacctggc ctaagtttct gtgtgtgtgt gtgtgtgttt tgttttgttt tttttttttt 34140
tttgagtgga gtctcgctct gttgcccagg ctggagtgca gtggcatgat ctcgactcac 34200
tgcaagctcc gcctcccggg ttcacgccat tctcctgcct cagcctcccg agtagctggg 34260
actacaggca cccaccacca cgcccagtta atttttgtat tttttaatag tgacagggtt 34320
tcatcatgtt agccaggatg gtctcgatct cctgacctcg tgatccgccc gcctcagcct 34380
cccgaattgc tgggattaca ggcatgagcc accaaacccg gccaagtttc tgtggtttta 34440
agccaccttg cttgtaagat ttgtgtgtgt gtgtttttaa tttttattt ttaagtatta 34500
tgaatacata atagtggtgt atatttacag gacatatgta atatggtttt gggttttagt 34560
gttttttttt tggagacaga gtctggctct gttgcccagg ctggagtaca gtggtgggat 34620
catggctcac tgcagccttg acctcccggg ctcaagggat cctcctgcct cagcctccca 34680
tgtaactagg accacaggca tgccccacca catccagcca attttttttt atttttagtg 34740
gagatgaggt ctcactgtgt tgcccaggct gatcttgaac tcctgagctc aagagatctt 34800
cctttctcac cctcccaaag tgctaggact acaggcatga gccactgtgc ctgtccttcc 34860
atgatgtttt gatataggca cacaatgtgt tagtttataa agtttgtaat aatttatcac 34920
aggcagccct aggaaactaa tatagccaag tttcctgttt cttctctata tcacatctgc 34980
tggggctaca tgtccaaggt ggcttcttca cccacttgtc tggtgcctgg gctgagatgg 35040
ctgaaacatc tggggctcta tctccacatg gcatttatac atgagtagct tgggcttcct 35100

cacagcatgg tggtctcagg gcagtagtac ttttacatgg caaccagctt ccccagagtg 35160
agcgttctaa gattcagaaa gtgaaaaatg aaagtttctt aaaacttggt tccagaacat 35220
agcacagcaa aacttccacc acattctact ggtcaaagca gtcacagagt cactcatatt 35280
caagaggcag aagtacagac ctcacttctt taagccacta cagtgacagg tggtgatatg 35340
tcattagaga aagccctaaa caagaacctt gtccctcacc tgcccccaaa taccatggaa 35400
gatgtctttt tttttttttt tttttttttg gggatagtct cactgtgtca tgcagtggtg 35460
tgatc 35465

<210> 57
<211> 14327
<212> ADN
<213> Homo sapiens

<400> 57
ggccggcgag cgggcggctg cgggcggcgc ggagcgggcg gcgcggagcg agcgagcgag 60
agagcggcgc gggccgggcc atggggtggc gggcgccggg cgcgctgctg ctggcgctgc 120
tgctgcacgg gcggctgctg gcggtgaccc atgggctgag ggcatacgat ggcttgtctc 180
tgcctgagga catagagacc gtcacagcaa gccaaatgcg ctggacacat tcgtaccttt 240
ctgatgatga gtacatgctg gctgacagca tctcaggaga cgacctgggc agtggggacc 300
tgggcagcgg ggacttccag atggtttatt tccgagccct ggtgaatttc actcgctcca 360
tcgagtacag ccctcagctg gaggatgcag gctccagaga gtttcgagag gtgtccgagg 420
ctgtggtaga cacgctggag tcggagtact tgaaaattcc cggagaccag gttgtcagtg 480
tggtgttcat caaggagctg gatggctggg tttttgtgga gctcgatgtg ggctcggaag 540
ggaatgcgga tggtgctcag attcaggaga tgctgctcag ggtcatctcc agcggctctg 600
tggcctccta cgtcacctct ccccagggat tccagttccg acgcctgggc acagtgcccc 660
agttcccaag agcctgcacg gaggccgagt ttgcctgcca cagctacaat gagtgtgtgg 720
ccctggagta tcgctgtgac cggcggcccg actgcaggga catgtctgat gagctcaatt 780
gtgaggagcc agtcctgggt atcagcccca cattctctct ccttgtggag acgacatctt 840
taccgccccg gccagagaca accatcatgc gacagccacc agtcacccac gctcctcagc 900
ccctgcttcc cggttccgtc aggcccctgc cctgtgggcc ccaggaggcc gcatgccgca 960
atgggcactg catccccaga gactacctct gcgacggaca ggaggactgc gaggacggca 1020
gcgatgagct agactgtggc cccccgccac cctgtgagcc caacgagttc ccctgcggga 1080
atggacattg tgccctcaag ctgtggcgct gcgatggtga ctttgactgt gaggaccgaa 1140
ctgatgaagc caactgcccc accaagcgtc ctgaggaagt gtgcgggccc acacagttcc 1200
gatgcgtctc taccaacatg tgcatcccag ccagcttcca ctgtgacgag gagagcgact 1260
gtcctgaccg gagcgacgag tttggctgca tgcccccca ggtggtgaca cctccccggg 1320
agtccatcca ggcttcccgg ggccagacag tgaccttcac ctgcgtggcc attggcgtcc 1380
ccacccccat catcaattgg aggctcaact ggggccacat cccctctcat cccagggtga 1440
cagtgaccag cgagggtggc cgtggcacac tgatcatccg tgatgtgaag gagtcagacc 1500
agggtgccta cacctgtgag gccatgaacg cccggggcat ggtgtttggc attcctgacg 1560
gtgtccttga gctcgtccca caacgaggcc cctgccctga cggccacttc tacctggagc 1620
acagcgccgc ctgcctgccc tgcttctgct ttggcatcac cagcgtgtgc cagagcaccc 1680
gccgcttccg ggaccagatc aggctgcgct ttgaccaacc cgatgacttc aagggtgtga 1740
atgtgacaat gcctgcgcag cccggcacgc cacccctctc ctccacgcag ctgcagatcg 1800
acccatccct gcacgagttc cagctagtag acctgtcccg ccgcttcctc gtccacgact 1860
ccttctgggc tctgcctgaa cagttcctgg gcaacaaggt ggactcctat ggcggctccc 1920
tgcgttacaa cgtgcgctac gagttggccc gtggcatgct ggagccagtg cagcggccgg 1980
acgtggtcct cgtgggtgcc gggtaccgcc tcctctcccg aggccacaca cccacccaac 2040
ctggtgctct gaaccagcgc caggtccagt tctctgagga gcactgggtc catgagtctg 2100
gccggccggt gcagcgcgcg gagctgctgc aggtgctgca gagcctggag gccgtgctca 2160
tccagaccgt gtacaacacc aagatggcta gcgtgggact tagcgacatc gccatggata 2220
ccaccgtcac ccatgccacc agccatggcc gtgcccacag tgtggaggag tgcagatgcc 2280
ccattggcta ttctggcttg tcctgcgaga gctgtgatgc ccacttcact cgggtgcctg 2340
gtgggcccta cctgggcacc tgctctggtt gcagttgcaa tggccatgcc agctcctgtg 2400
accctgtgta tggccactgc ctgaattgcc agcacaacac ggagggggca cagtgcaaca 2460
agtgcaaggc tggcttcttt ggggacgcca tgaaggccac ggccacttcc tgccggccct 2520
gcccttgccc atacatcgat gcctcccgca gattctcaga cacttgcttc ctggacacgg 2580

atggccaagc cacatgtgac gcctgtgccc caggctacac tggccgccgc tgtgagagct 2640
gtgcccccgg atacgagggc aaccccatcc agcccggcgg gaagtgcagg cccgtcaacc 2700
aggagattgt gcgctgtgac gagcgtggca gcatggggac ctccggggag gcctgccgct 2760
gtaagaacaa tgtggtgggg cgcttgtgca atgaatgtgc tgacggctct ttccacctga 2820
gtacccgaaa ccccgatggc tgcctcaagt gcttctgcat gggtgtcagt cgccactgca 2880
ccagctcttc atggagccgt gcccagttgc atggggcctc tgaggagcct ggtcacttca 2940
gcctgaccaa cgccgcaagc acccacacca ccaacgaggg catcttctcc cccacgcccg 3000
gggaactggg attctcctcc ttccacagac tcttatctgg accctacttc tggagcctcc 3060
cttcacgctt cctgggggac aaggtgacct cctatggagg agagctgcgc ttcacagtga 3120
cccagaggtc ccagccgggc tccacacccc tgcacgggca gccgttggtg gtgctgcaag 3180
gtaacaacat catcctagag caccatgtgg cccaggagcc cagccccggc cagcccagca 3240
ccttcattgt gcctttccgg gagcaagcat ggcagcggcc cgatgggcag ccagccacac 3300
gggagcacct gctgatggca ctggcaggca tcgacaccct cctgatccga gcatcctacg 3360
cccagcagcc cgctgagagc agggtctctg gcatcagcat ggacgtggct gtgcccgagg 3420
aaaccggcca ggaccccgcg ctggaagtgg aacagtgctc ctgcccaccc gggtaccgtg 3480
ggccgtcctg ccaggactgt gacacaggct acacacgcac gcccagtggc ctctacctgg 3540
gtacctgtga acgctgcagc tgccatggcc actcagaggc ctgcgagcca gaaacaggtg 3600
cctgccaggg ctgccagcat cacacggagg gccctcggtg tgagcagtgc cagccaggat 3660
actacgggga cgcccagcgg gggacaccac aggactgcca gctgtgcccc tgctacggag 3720
accctgctgc cggccaggct gcccacactt gttttctgga cacagacggc caccccacct 3780
gtgatgcgtg ctccccaggc cacagtgggc gtcactgtga gaggtgcgcc cctggctact 3840
atggcaaccc cagccagggc cagccatgcc agagagacag ccaggtgcca gggcccatag 3900
gctgcaactg tgacccccaa ggcagcgtca gcagccagtg tgatgctgct ggtcagtgcc 3960
agtgcaaggc ccaggtagaa ggcctcactt gcagccactg ccggccccac cacttccacc 4020
tgagtgccag caacccagac ggctgcctgc cctgcttctg tatgggcatc acccagcagt 4080
gcgccagctc tgcctacaca cgccacctga tctccaccca ctttgcccct ggggacttcc 4140
aaggctttgc cctggtgaac ccacagcgaa acagccgcct gacaggagaa ttcactgtgg 4200
aacccgtgcc cgagggtgcc cagctctctt ttggcaactt tgcccaactc ggccatgagt 4260
ccttctactg gcagctgccg gagacatacc agggagacaa ggtggcggcc tacggtggga 4320
agttgcgata caccctctcc tacacagcag gcccacaggg cagcccactc tcggaccccg 4380
atgtgcagat cacgggcaac aacatcatgc tagtggcctc ccagccagcg ctgcagggcc 4440
cagagaggag gagctacgag atcatgttcc gagaggaatt ctggcgccgg cccgatgggc 4500
agccggccac acgcgagcac ctcctgatgg cactggccga cctggatgag ctcctgatcc 4560
gggccacgtt ctcctccgtg ccgctggtgg ccagcatcag cgcagtcagc ctggaggtcg 4620
cccagccggg gccctcaaac agaccccgcg ccctcgaggt ggaggagtgc cgctgcccgc 4680
caggctacat cggtctgtcc tgccaggact gtgcccccgg ctacacgcgc accgggagtg 4740
ggctctacct cggccactgc gagctatgtg aatgcaatgg ccactcagac ctgtgccacc 4800
cagagactgg ggcctgctcg caatgccagc acaacgccgc aggggagttc tgcgagcttt 4860
gtgcccctgg ctactacgga gatgccacag ccgggacgcc tgaggactgc cagccctgtg 4920
cctgcccact gaccaaccca gagaacatgt ttcccgcacc ctgtgagagc ctgggagccg 4980
gcgggtaccg ctgcacggcc tgcgaacccg gctacactgg ccagtactgt gagcagtgtg 5040
gcccaggtta cgtgggtaac cccagtgtgc aagggggcca gtgcctgcca gagacaaacc 5100
aagccccact ggtggtcgag gtccatcctg ctcgaagcat agtgccccaa ggtggctccc 5160
actccctgcg gtgtcaggtc agtgggagcc caccccacta cttctattgg tcccgtgagg 5220
atgggcggcc tgtgcccagc ggcacccagc agcgacatca aggctccgag ctccacttcc 5280
ccagcgtcca gccctcggat gctggggtct acatttgcac ctgccgtaat ctccaccaat 5340
ccaataccag ccgggcagag ctgctggtca ctgaggctcc aagcaagccc atcacagtga 5400
ctgtggagga gcagcggagc cagagcgtgc gccccggagc tgacgtcacc ttcatctgca 5460
cagccaaaag caagtcccca gcctataccc tggtgtggac ccgcctgcac aacgggaaac 5520
tgcccacccg agccatggat ttcaatggca tcctgaccat tcgcaacgtc cagctgagtg 5580
atgcaggcac ctacgtgtgc accggctcca acatgtttgc catggaccag ggcacagcca 5640
ctctacatgt gcaggcctcg ggcaccttgt ccgcccccgt ggtctccatc catccgccac 5700
agctcacagt gcagcccggg caactggcgg agttccgctg cagcgccaca gggagcccca 5760
cgcccaccct cgagtggaca gggggccccg gcggccagct ccctgcgaag gcacaaatcc 5820
acggcggcat cctgcgcctg ccagctgtcg agcccacgga tcaggcccag tacttgtgcc 5880
gagcccacag cagcgctggg cagcaggtgg ccagggctgt gctccacgtg catgggggcg 5940
gtgggcccag agtccaagtg agcccagaga ggacccaggt ccacgcaggc cggaccgtca 6000
ggctgtactg cagggctgca ggcgtgccta gcgccaccat cacctggagg aaggaagggg 6060

gcagcctccc accacaggcc cggtcagagc gcacagacat cgcgacactg ctcatcccag 6120
ccatcacgac tgctgacgcc ggcttctacc tctgcgtggc caccagccct gcaggcactg 6180
cccaggcccg gatgcaagtg gttgtccttt cagcctcaga tgccagccca ccgggggtca 6240
agattgagtc ctcatcgcct tctgtgacag aagggcaaac actcgacctc aactgtgtgg 6300
tggcagggtc agcccatgcc caggtcacct ggtacaggcg agggggtagc ctgcctcccc 6360
acacccaggt gcacggctcc cgtctgcggc tcccccaggt ctcaccagct gattctggag 6420
aatatgtgtg ccgtgtggag aatggatcgg gccccaagga ggcctccatt actgtgtctg 6480
tgctccacgg cacccattct ggccccagct acaccccagt gcccggcagc acccggccca 6540
tccgcatcga gccctcctcc tcacacgtgg cggaagggca gaccctggat ctgaactgcg 6600
tggtgcccgg gcaggcccac gcccaggtca cgtggcacaa gcgtgggggc agcctccctg 6660
cccggcacca gacccacggc tcgctgctgc ggctgcacca ggtgaccccg gccgactcag 6720
gcgagtatgt gtgccatgtg gtgggcacct ccggcccccct agaggcctca gtcctggtca 6780
ccatcgaagc ctctgtcatc cctggaccca tcccacctgt caggatcgag tcttcatcct 6840
ccacagtggc cgagggccag accctggatc tgagctgcgt ggtggcaggg caggcccacg 6900
cccaggtcac atggtacaag cgtgggggca gcctccctgc ccggcaccag gttcgtggct 6960
cccgcctgta catcttccag gcctcacctg ccgatgcggg acagtacgtc tgccgggcca 7020
gcaacggcat ggaggcctcc atcacggtca cagtaactgg gacccagggg gccaacttag 7080
cctaccctgc cggcagcacc cagcccatcc gcatcgagcc ctcctcctcg caagtggcgg 7140
aagggcagac cctggatctg aactgcgtgg tgcccgggca gtcccatgcc caggtcacgt 7200
ggcacaagcg tgggggcagc ctccctgtcc ggcaccagac ccacggctcc ctgctgagac 7260
tctaccaagc gtcccccgcc gactcgggcg agtacgtgtg ccgagtgttg ggcagctccg 7320
tgcctctaga ggcctctgtc ctggtcacca ttgagcctgc gggctcagtg cctgcacttg 7380
gggtcacccc cacggtccgg atcgagtcat cgtcttcgca agtggccgag gggcagaccc 7440
tggacctgaa ctgcctcgtt gctggtcagg cccatgccca ggtcacgtgg cacaagcgcg 7500
ggggcagcct cccggcccgg caccaggtgc atggctcgag gctacgcctg ctccaggtga 7560
ccccagctga ttcaggggag tacgtgtgcc gtgtggtcgg cagctcaggt acccaggaag 7620
cctcagtcct tgtcaccatc cagcagcgcc ttagtggctc ccactcccag ggtgtggcgt 7680
accccgtccg catcgagtcc tcctcagcct ccctggccaa tggacacacc ctggacctca 7740
actgcctggt tgccagccag gctccccaca ccatcacctg gtataagcgt ggaggcagct 7800
tacccagccg gcaccagatc gtgggctccc ggctgcggat ccctcaggtg actccggcag 7860
actcgggcga gtacgtgtgt cacgtcagta acggtgcagg ctcccgggag acctcgctca 7920
tcgtcaccat ccagggcagc ggttcctccc acgtgcccag cgtctcccca ccgatcagga 7980
tcgagtcgtc ttcccccacg gtggtggaag ggcagacctt ggatctgaac tgcgtggtcg 8040
ccaggcagcc ccaggctatc atcacatggt acaagcgtgg gggcagcctt ccctcccgac 8100
accagaccca tggctcccac ctgcggttgc accaaatgtc tgtggctgac tcgggcgagt 8160
atgtgtgccg ggccaacaac aacatcgatg ccctggaggc ctccatcgtc atctccgtct 8220
cccctagcgc cggcagcccc tccgcccctg gcagctccat gcccatcaga attgagtcat 8280
cctcctcaca cgtggccgaa ggggagaccc tggatctgaa ctgcgtggtc cccgggcagg 8340
cccatgccca ggtcacttgg cacaagcgtg gggcagcct ccccagtcac catcagaccc 8400
gcggctcacg gctgcggctg caccatgtgt ccccggccga ctcgggtgaa tacgtgtgcc 8460
gggtgatggg cagctctggc cccctggagg cctcagtcct ggtcaccatc gaagcctctg 8520
gctcaagtgc tgtccacgtc cccgccccag gtggagcccc acccatccgc atcgagccct 8580
cctcctcccg agtggcagaa gggcagaccc tggatctgaa gtgcgtggtg cccgggcagg 8640
cccacgccca ggtcacatgg cacaagcgtg gaggaaacct ccctgcccgg caccaggtcc 8700
acggcccact gctgaggctg aaccaggtgt ccccggctga ctctggcgag tactcgtgcc 8760
aagtgaccgg aagctcaggc accctggagg catctgtcct ggtcacaatt gagccctcca 8820
gcccaggacc cattcctgct ccaggactgg cccagcccat ctacatcgag gcctcctctt 8880
cacacgtgac tgaagggcag actctggatc tgaactgtgt ggtgcccggg caggcccatg 8940
cccaggtcac gtggtacaag cgcgggggca gcctccccgc ccggcaccag acccatggct 9000
cccagctgcg gctccacctc gtctcccctg ccgactcagg cgagtatgtg tgtcgtgcag 9060
ccagcggccc aggccctgag caagaagcct ccttcacagt caccgtcccg cccagtgagg 9120
ggtcttccta ccgccttagg agcccggtca tctccatcga cccgcccagc agcaccgtgc 9180
agcagggcca ggatgccagc ttcaagtgcc tcatccatga cggggcagcc cccatcagcc 9240
tcgagtggaa gacccggaac caggagctgg aggacaacgt ccacatcagt cccaatggct 9300
ccatcatcac catcgtgggc acccggccca gcaaccacgg tacctaccgc tgcgtggcct 9360
ccaatgccta cggtgtggcc cagagtgtgg tgaacctcag tgtgcacggg ccccctacag 9420
tgtccgtgct ccccgagggc cccgtgtggg tgaaagtggg aaaggctgtc accctggagt 9480
gtgtcagtgc cggggagccc cgctcctctg ctcgttggac ccggatcagc agcacccctg 9540

ccaagttgga gcagcggaca tatgggctca tggacagcca cgcggtgctg cagatttcat 9600
cagctaaacc atcagatgcg ggcacttatg tgtgccttgc tcagaatgca ctaggcacag 9660
cacagaagca ggtggaggtg atcgtggaca cgggcgccat ggccccaggg gcccctcagg 9720
tccaagctga agaagctgag ctgactgtgg aggctggaca cacggccacc ttgcgctgct 9780
cagccacagg cagccccgcg cccaccatcc actggtccaa gctgcgttcc ccactgccct 9840
ggcagcaccg gctggaaggt gacacactca tcataccccg ggtagcccag caggactcgg 9900
gccagtacat ctgcaatgcc actagccctg ctgggcacgc tgaggccacc atcatcctgc 9960
acgtggagag cccaccatat gccaccacgg tcccagagca cgcttcggtg caggcagggg 10020
agacggtgca gctccagtgc ctggctcacg ggacaccccc actcaccttc cagtggagcc 10080
gcgtgggcag cagccttcct gggagggcga ccgccaggaa cgagctgctg cactttgagc 10140
gtgcagcccc tgaggactca ggccgctacc gctgccgggt caccaacaag gtgggctcag 10200
ccgaggcctt tgcccagctg ctcgtccaag gccctcccgg ctctctccct gccacctcca 10260
tcccagcagg gtccacgccc accgtgcagg tcacgcctca gctagagacc aagagcattg 10320
gggccagcgt tgagttccac tgtgctgtgc ccagcgacca gggtacccag ctccgttggt 10380
tcaaggaagg gggtcagctg cctccgggtc acagcgtgca ggatggggtg ctccgaatcc 10440
agaacttgga ccagagctgc caagggacgt atatatgcca ggcccatgga ccttggggga 10500
aggcccaggc cagtgcccag ctggttatcc aagccctgcc ctcggtgctc atcaacatcc 10560
ggacctctgt gcagaccgtg gtggttggcc acgccgtgga gttcgaatgc ctggcactgg 10620
gtgaccccaa gcctcaggtg acatggagca aagttggagg gcacctgcgg ccaggcattg 10680
tgcagagcgg aggtgtcgtc aggatcgccc acgtagagct ggctgatgcg ggacagtatc 10740
gctgcactgc caccaacgca gctggcacca cacaatccca cgtcctgctg cttgtgcaag 10800
ccttgcccca gatctcaatg ccccaagaag tccgtgtgcc tgctggttct gcagctgtct 10860
tcccctgcat agcctcaggc taccccactc ctgacatcag ctggagcaag ctggatggca 10920
gcctgccacc tgacagccgc ctggagaaca acatgctgat gctgccctca gtccgacccc 10980
aggacgcagg tacctacgtc tgcaccgcca ctaaccgcca gggcaaggtc aaagcctttg 11040
cccacctgca ggtgccagag cgggtggtgc cctacttcac gcagaccccc tactccttcc 11100
taccgctgcc caccatcaag gatgcctaca ggaagttcga gatcaagatc accttccggc 11160
ccgactcagc cgatgggatg ctgctgtaca atgggcagaa gcgagtccca gggagcccca 11220
ccaacctggc caaccggcag cccgacttca tctccttcgg cctcgtgggg ggaaggcccg 11280
agttccggtt cgatgcaggc tcaggcatgg ccaccatccg ccatcccaca ccactggccc 11340
tgggccattt ccacaccgtg accctgctgc gcagcctcac ccagggctcc ctgattgtgg 11400
gtgacctggc cccggtcaat gggacctccc agggcaagtt ccagggcctg gatctgaacg 11460
aggaactcta cctgggtggc tatcctgact atggtgccat ccccaaggcg gggctgagca 11520
gcggcttcat aggctgtgtc cgggagctgc gcatccaggg cgaggagatc gtcttccatg 11580
acctcaacct cacggcgcac ggcatctccc actgccccac ctgtcgggac cggccctgcc 11640
agaatggcgg tcagtgccat gactctgaga gcagcagcta cgtgtgcgtc tgcccagctg 11700
gcttcaccgg gagccgctgt gagcactcgc aggccctgca ctgccatcca gaggcctgtg 11760
ggcccgacgc cacctgtgtg aaccggcctg acggtcgagg ctacacctgc cgctgccacc 11820
tgggccgctc ggggttgcgg tgtgaggaag gtgtgacagt gaccaccccc tcgctgtcgg 11880
gtgctggctc ctacctggca ctgcccgccc tcaccaacac acaccacgag ctacgcctgg 11940
acgtggagtt caagccactc gcccctgacg gggtcctgct gttcagcggg gggaagagcg 12000
ggcctgtgga ggacttcgtg tccctggcga tggtgggcgg ccacctggag ttccgctatg 12060
agttggggtc agggctggcc gttctgcgga gcgccgagcc gctggccctg ggccgctggc 12120
accgtgtgtc tgcagagcgt ctcaacaagg acggcagcct gcgggtgaat ggtggacgcc 12180
ctgtgctgcg ctcctcgccc ggcaagagcc agggcctcaa cctgcacacc ctgctctacc 12240
tggggggtgt ggagccttcc gtgccactgt ccccggccac caacatgagc gctcacttcc 12300
gcggctgtgt gggcgaggtg tcagtgaatg gcaaacggct ggacctcacc tacagtttcc 12360
taggcagcca gggcatcggg caatgctatg atagctcccc atgtgagcgc cagccttgcc 12420
aacatggtgc cacgtgcatg cccgctggcg agtatgagtt ccagtgcctg tgtcgagatg 12480
gattcaaagg agacctgtgt gagcacgagg agaacccctg ccagctccgt gaaccctgtc 12540
tgcatggggg cacctgccag ggcacccgct gcctctgcct ccctggcttc tctggcccac 12600
gctgccaaca aggctctgga catggcatag cagagtccga ctggcatctt gaaggcagcg 12660
gggcaatga tgcccctggg cagtacggag cctatttcca cgatgatggc ttcctcgcct 12720
tccctggcca tgtcttctcc aggagcctgc ccgaggtgcc cgagaccatc gagctggagg 12780
ttcggaccag cacagccagt ggcctcctgc tctggcaggg tgtggaggtg ggagaggccg 12840
gccaaggcaa ggacttcatc agcctcgggc ttcaagacgg gcaccttgtc ttcaggtacc 12900
agctgggtag tggggaggcc cgcctggtct ctgaggaccc catcaatgac ggcgagtggc 12960
accgggtgac agcactgcgg gagggccgca gaggttccat ccaagtcgac ggtgaggagc 13020

tggtcagcgg ccggtcccca ggtcccaacg tggcagtcaa cgccaagggc agcgtctaca 13080
tcggcggagc ccctgacgtg gccacgctga ccgggggcag attctcctcg ggcatcacag 13140
gctgtgtcaa gaacctggtg ctgcactcgg cccgacccgg cgccccgccc ccacagcccc 13200
tggacctgca gcaccgcgcc caggccgggg ccaacacacg cccctgcccc tcgtaggcac 13260
ctgcctgccc cacacggact cccgggccac gccccagccc gacaatgtcg agtatattat 13320
tattaatatt attatgaatt tttgtaagaa accgaggcga tgccacgctt tgctgctacc 13380
gccctgggct ggactggagg tgggcatgcc accctcacac acacagctgg gcaaagccac 13440
aaggctggcc agcaaggcag gttggatggg agtgggcacc tcagaaagtc accaggactt 13500
ggggtcagga acagtggctg ggtgggccca gaactgcccc cactgtcccc ctacccaccg 13560
atggagcccc cagatagagc tgggtggcct gtttctgcag cccttgggca gttctcactc 13620
ctaggagagc caacctcggc ttgtgggctg gtgccccaca gctacctgag acgggcatcg 13680
caggagtctc tgccacccac tcaggattgg gaattgtctt tagtgccggc tgtggagcaa 13740
aaggcagctc acccctgggc aggcggtccc catccccacc agctcgtttt tcagcacccc 13800
cacccacctc cacccagccc ctggcacctc ctctggcaga ctccccctcc taccacgtcc 13860
tcctggcctg cattcccacc ccctcctgcc agcacacagc ctggggtccc tccctcaggg 13920
gctgtaaggg aaggcccacc ccaactctta ccaggagctg ctacaggcag agcccagcac 13980
tgatagggcc ccgcccaccg ggccccgccc accccaggcc acatccccac ccatctggaa 14040
gtgaaggccc agggactcct ccaacagaca acggacggac ggatgccgct ggtgctcagg 14100
aagagctagt gccttaggtg gggaaaggca ggactcacga ctgagagaga gaggaggggg 14160
atatgaccac cctgccccat ctgcaggagc ctgaagatcc agctcaagtg ccatcctgcc 14220
agtggccccc agactgtggg gttgggacgc ctggcctctg tgtcctagaa gggaccctcc 14280
tgtggtcttt gtcttgattt ttcttaataa acggtgctat ccccgcc 14327

<210> 58
<211> 15
<212> PRT
<213> Homo sapiens

<400> 58
Ile Pro Thr Gly Glu Pro Cys Pro Glu Pro Leu Arg Thr Tyr Gly
1               5                   10                  15

<210> 59
<211> 13
<212> PRT
<213> Homo sapiens

<400> 59
Ile Glu Ser Val Leu Ser Ser Ser Gly Lys Arg Leu Gly
1               5                   10

<210> 60
<211> 18
<212> PRT
<213> Homo sapiens

<400> 60
Ala Thr Pro Ala Gln Ala His Leu Lys Lys Pro Ser Gln Leu Ser Ser
1               5                   10                  15

Phe Ser

<210> 61
<211> 15
<212> PRT
<213> Homo sapiens

<400> 61
Arg Ile Gln Ala Met Ile Pro Lys Gly Ala Leu Arg Val Ala Val
1               5                   10                  15

<210> 62
<211> 15
<212> PRT
<213> Homo sapiens

<400> 62
Gly Ile Cys Gln Cys Leu Ala Glu Arg Tyr Ser Val Ile Leu Leu
1               5                   10                  15

<210> 63
<211> 17
<212> PRT
<213> Homo sapiens

<400> 63
Glu Lys Met His Glu Gly Asp Glu Gly Pro Gly His His His Lys Pro
1               5                   10                  15

Gly

<210> 64
<211> 13
<212> PRT
<213> Homo sapiens

<400> 64
Asp Leu Gln Asn Phe Leu Lys Lys Glu Asn Lys Asn Glu
1               5                   10

<210> 65
<211> 19
<212> PRT
<213> Homo sapiens

<400> 65
Val Lys Leu Gly His Pro Asp Thr Leu Asn Gln Gly Glu Phe Lys Glu
1               5                   10                  15

Leu Val Arg

<210> 66
<211> 48
<212> ADN
<213> Homo sapiens

<400> 66
ttywsntggg ayaaytgytt ygarggnaar gayccngcng tnathmgn 48

<210> 67
<211> 48
<212> ADN
<213> Homo sapiens

<400> 67
taywsnytnc cnaarwsnga rttygcngtn ccngayytng arytnccn 48

<210> 68
<211> 16
<212> PRT
<213> Homo sapiens

<400> 68
Phe Ser Trp Asp Asn Cys Phe Glu Gly Lys Asp Pro Ala Val Ile Arg
1               5                   10                  15

<210> 69
<211> 585
<212> ADN
<213> Homo sapiens

<400> 69
gaygcnccng gncartaygg ngcntaytty caygaygayg gnttyytngc nttyccnggn 60
caygtnttyw snmgnwsnyt nccngargtn ccngaracna thgarytnga rgtnmgnacn 120
wsnacngcnw snggnytnyt nytntggcar ggngtngarg tnggngargc nggncarggn 180
aargayttya thwsnytngg nytncargay ggncayytng tnttymgnta ycarytnggn 240
wsnggngarg cnmgnytngt nwsngargay ccnathaayg ayggngartg gcaymgngtn 300
acngcnytnm gngarggnmg nmgnggnwsn mgncargtng ayggngarga rytngtnwsn 360
ggnmgnwsnc cnggnccnaa ygtngcngtn aaygcnaarg gnwsngtnta yathggnggn 420
gcnccngayg tngcnacnyt nacnggnggn mgnttywsnw snggnathac nggntgygtn 480
aaraayytng tnytncayws ngcnmgnccn ggngcnccnc cnccncarcc nytngayytn 540
carcaymgng cncargcngg ngcnaayacn mgnccntgyc cnwsn 585

<210> 70
<211> 597
<212> ADN
<213> Homo sapiens

<400> 70

atgaartggg tntgggcnyt nytnytnytn gcngcntggg cngcngcnga rmgngaytgy 60
mgngtnwsnw snttymgngt naargaraay ttygayaarg cnmgnttyws nggnacntgg 120
taygcnatgg cnaaraarga yccngarggn ytnttyytnc argayaayat hgtngcngar 180
ttywsngtng aygaracngg ncaratgwsn gcnacngcna arggnmgngt nmgnytnytn 240
aayaaytggg aygtntgygc ngayatggtn ggnacnttya cngayacnga rgayccngcn 300
aarttyaara tgaartaytg gggngtngcn wsnttyytnc araarggnaa ygaygaycay 360
tggathgtng ayacngayta ygayacntay gcngtncart aywsntgymg nytnytnaay 420
ytngayggna cntgygcnga ywsntaywsn ttygtnttyw snmgngaycc naayggnytn 480
ccnccngarg cncaraarat hgtnmgncar mgncargarg arytntgyyt ngcnmgncar 540
taymgnytna thgtncayaa yggntaytgy gayggnmgnw sngarmgnaa yytnytn 597

<210> 71
<211> 579
<212> ADN
<213> Homo sapiens

<400> 71
atgcarwsny tnatgcargc nccnytnytn athgcnytng gnytnytnyt ngcnacnccn 60
gcncargcnc ayytnaaraa rccnwsncar ytnwsnwsnt tywsntggga yaaytgytty 120
garggnaarg ayccngcngt nathmgnwsn ytnacnytng arccngaycc nathgtngtn 180
ccnggnaayg tnacnytnws ngtngtnggn wsnacnwsng tnccnytnws nwsnccnytn 240
aargtngayy tngtnytnga raargargtn gcnggnytnt ggathaarat hccntgyacn 300
gaytayathg gnwsntgyac nttygarcay ttytgygayg tnytngayat gytnathccn 360
acnggngarc cntgyccnga rccnytnmgn acntayggny tnccntgyca ytgyccntty 420
aargarggna cntaywsnyt nccnaarwsn garttygcng tnccngayyt ngarytnccn 480
wsntggytna cnacnggnaa ytaymgnath garwsngtny tnwsnwsnws nggnaarmgn 540
ytnggntgya thaarathgc ngcnwsnytn aarggnath 579

<210> 72
<211> 16
<212> PRT
<213> Homo sapiens

<400> 72
Tyr Ser Leu Pro Lys Ser Glu Phe Ala Val Pro Asp Leu Glu Leu Pro
1               5                   10                  15

<210> 73
<211>
<212> PRT
<213> Homo sapiens

<400> 73

MQSLMQAPLL IALGLLLATP AQAHLKKPSQ
LSSFSWDNCD EGKDPAVIRS LTLEPDPIVV
PGNVTLSVVG STSVPLSSPL KVDLVLEKEV
AGLWIKIPCT DYIGSCTFEH FCDVLDMLIP
TGEPCPEPLR TYGLPCHCPF KEGTYSLPKS
EFVVPDLELP SWLTTGNYRI ESVLSSSGKR
LGCIKIAASLKGI

<210> 74
<211>
<212> PRT
<213> Homo sapiens

<400> 74

GDVCQDCIQM VTDIQTAVRT NSTFVQALVE
HVKEECDRLG PGMADICKNY ISQYSEIAIQ
MMMHMQDQQP KEICALVGFC DEV

<210> 75
<211>
<212> PRT
<213> Homo sapiens

<400> 75

MTCKMSQLER NIETIINTFH QYSVKLGHPD
TLNQGEFKEL VRKDLQNFLK KENKNEKVIE
HIMEDDLDTN ADKQLSFEEF IMLMARLTWA
SHEKMHEGDE GPGHHHKPGL GEGTP

## Revendications

1. Utilisation d'au moins un polypeptide pour obtenir une composition diagnostique ou pronostique destinée à détecter ou pronostiquer un état pathologique associé à la sclérose en plaques, ledit polypeptide étant choisi parmi :
les polypeptides comprenant, ou consistant en une séquence choisie parmi SEQ ID N° 63, SEQ ID N° 64 et SEQ ID N° 65
les polypeptides comprenant, ou consistant en une protéine choisie parmi les protéines dont la séquence peptidique à l'état natif correspond à SEQ ID N° 17, SEQ ID N° 18, SEQ ID N° 19, SEQ ID N° 20, SEQ ID N° 21, SEQ ID N° 22, SEQ ID N° 23, et les séquences peptidiques qui présentent au moins 70 % d'identité, de préférence au moins 80 % d'identité et avantageusement au moins 98 % d'identité avec l'une quelconque des séquences peptidiques SEQ ID N° 17 à SEQ ID N° 23, et les séquences peptidiques appartenant à la même famille que la calgranuline B.

2. Utilisation selon la revendication 1, d'au moins deux polypeptides en combinaison tels que définis à la revendication 1.

3. Utilisation d'au moins un fragment nucléotidique, pour obtenir une composition diagnostique ou pronostique, destinée à détecter ou pronostiquer un état pathologique associé à la sclérose en plaques, selon laquelle ledit fragment nucléotidique est choisi parmi des fragments qui codent pour au moins un polypeptide tel que défini à la revendication 1.

4. Utilisation d'au moins un fragment nucléotidique pour obtenir une composition diagnostique ou pronostique destinée à détecter ou pronostiquer un état pathologique associé à la sclérose en plaques, selon laquelle ledit fragment est un fragment d'une séquence nucléique choisie parmi l'une quelconque des séquences SEQ ID N° 42, SEQ ID N° 43, SEQ ID N° 44, SEQ ID N° 45, SEQ ID N° 46, SEQ ID N° 47, SEQ ID N° 48, SEQ ID N° 49, SEQ ID N° 50, SEQ ID N° 51, SEQ ID N° 52 et leurs séquences complémentaires.

5. Utilisation d'un ligand spécifique d'un polypeptide ou d'un fragment nucléotidique selon l'une quelconque des revendications précédentes pour obtenir une composition diagnostique ou pronostique, destinée à détecter ou pronostiquer un état pathologique associé à la sclérose en plaques .

6. Procédé pour détecter au moins une protéine associée à la sclérose en plaques, dans un échantillon biologique, **caractérisé en ce que** l'on met en contact l'échantillon biologique avec au moins un ligand spécifique d'au moins un polypeptide tel que défini à la revendication 1, puis on détecte la formation d'un complexe entre ledit polypeptide et ledit ligand.

7. Procédé selon la revendication 6, **caractérisé en ce que** ledit ligand est un anticorps monoclonal, un anticorps polyclonal, un récepteur, un substrat d'activité enzymatique ou une enzyme dont ledit polypeptide est un cofacteur.

8. Procédé pour détecter au moins un ligand associé à la sclérose en plaques, dans un échantillon biologique, **caractérisé en ce que** l'on met en contact l'échantillon biologique avec au moins un polypeptide tel que défini à la revendication 1, puis on détecte la formation d'un complexe entre ledit polypeptide et ledit ligand.

9. Procédé selon la revendication 8, **caractérisé en ce que** le ligand est un anticorps, un récepteur, un substrat d'activité enzymatique ou une enzyme dont ledit polypeptide est un cofacteur.

10. Procédé selon l'une quelconque des revendications 6 à 9, **caractérisé en ce que** la séquence dudit polypeptide comprend ou consiste en une séquence peptidique choisie parmi l'une quelconque de SEQ ID N° 17 à 23.

11. Procédé pour détecter, dans un échantillon d'un fluide biologique, au moins un polypeptide tel que défini dans la revendication 1, selon lequel, éventuellement après purification dudit échantillon, on analyse par spectrométrie de masse le profil de masse obtenu à partir dudit échantillon et on compare à un profil de masse de référence.

12. Utilisation d'au moins un polypeptide tel que défini à la revendication 1, de préférence un polypeptide dont la séquence peptidique comprend SEQ ID NO : 17, pour obtenir une composition diagnostique ou pronostique destinée à détecter ou pronostiquer un état pathologique associé à la sclérose en.

13. Procédé de détection, dans un échantillon, d'une valeur de concentration d'au moins un polypeptide tel que défini dans la revendication 1 , ladite concentration étant associée à un état pathologique, **caractérisé en ce qu'**on dose ledit polypeptide, le dosage permettant d'obtenir une valeur de concentration qui est comparée à une valeur seuil représentative de la sclérose en plaques.

14. Utilisation d'au moins un polypeptide tel que défini à la revendication 1, pour tester l'efficacité d'un agent thérapeutique pour un état associé à la sclérose en plaques.

15. Utilisation d'au moins un fragment nucléotidique, pour tester l'efficacité d'un agent thérapeutique pour un état pathologique associé à la sclérose en plaques, selon laquelle ledit fragment nucléotidique est choisi parmi les fragments qui codent pour un polypeptide tel que défini à la revendication 1.
